(19) Europäisches Patentamt
European Patent Office
Office européen des brevets

(11) EP 3 524 245 A1

(12) **EUROPEAN PATENT APPLICATION**

(43) Date of publication:
**14.08.2019 Bulletin 2019/33**

(51) Int Cl.:
***A61K 31/4458*** *(2006.01)* ***A61P 29/00*** *(2006.01)*
***C07D 213/38*** *(2006.01)* ***C07D 213/53*** *(2006.01)*

(21) Application number: **19162452.7**

(22) Date of filing: **23.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**04.02.2011 US 201161439483 P**
**04.02.2011 US 201161439473 P**
**20.09.2010 US 38444710 P**
**17.09.2010 US 38381110 P**
**23.03.2010 US 72934610**

(62) Document number(s) of the earlier application(s) in accordance with Art. 76 EPC:
**11714880.9 / 2 549 995**

(71) Applicant: **Philip Morris Products S.A.**
**2000 Neuchatel (CH)**

(72) Inventors:
- **WILLIAMS, Jonnie R.**
  **Glen Allen, Virginia 23060 (US)**
- **PUTHIAPARAMPIL, Tom Thomas**
  **560099 Bangalore (IN)**
- **DAVID, Thomas Kanathkunn**
  **560099 Bangalore (IN)**
- **RAJU, Muppala Sarveswara**
  **Upper Saddle River, New Jersey 97458 (US)**

(74) Representative: **Script IP Limited**
**Turnpike House**
**18 Bridge Street**
**Frome Somerset BA11 1BB (GB)**

Remarks:
This application was filed on 13-03-2019 as a divisional application to the application mentioned under INID code 62.

# (54) USE OF ANATABINE TO TREAT INFLAMMATION AND METHODS OF SYNTHESIZING ANATABINE

(57) There is disclosed herein anatabine glutarate and compositions and uses thereof.

EP 3 524 245 A1

## Description

RELATED APPLICATIONS

This application claims priority to U.S. Application No. 61/439,483, filed February 4, 2011, U.S. Application No. 61/439,473, filed February 4, 2011; U.S. Application No. 61/384,447, filed September 20, 2010; U.S. Application No. 61/383,811, filed September 17, 2010; and U.S. Application No. 12/729,346, filed March 23, 2010; the disclosure of each of which is hereby incorporated by reference in its entirety.

BACKGROUND

Inflammation is a protective response to harmful stimuli, such as oxidative stress, irritants, pathogens, and damaged cells. The inflammatory response involves the production and release of inflammatory modulators that heal injured tissue and destroy damaged cells, by directly or indirectly producing and/or signaling the release of agents that produce reactive oxygen species. Thus, an appropriate inflammatory response involves a balance between the destruction of damaged cells and the healing of injured tissue.

An unchecked inflammatory response can lead to oxidative stress and the onset of various inflammatory disease pathologies. In fact, inflammatory processes underlie a wide variety of pathologies, including immune and autoimmune diseases, gastrointestinal diseases, various types of cancer, vascular disorders, heart disease, and neurodegenerative diseases. There is a need in the art for agents that can reduce inappropriate levels of inflammation.

SUMMARY

A pharmaceutical composition comprising a therapeutically effective dose of an isolated form of anatabine or a pharmaceutically acceptable salt thereof can be administered to an individual to reduce a symptom of a disorder comprising an NFκB-mediated inflammatory component or to reduce the risk of developing such a disorder.

In some embodiments the NFκB-mediated inflammatory component is chronic inflammation which occurs, for example, in thyroiditis, cancer, arthritis, Alzheimer's disease, and multiple sclerosis.

In other embodiments, a therapeutically effective dose of an isolated form of anatabine or a pharmaceutically acceptable salt thereof can be administered to an individual for treating alopecia areata or other disorders associated with hair loss.

In some embodiments therapeutically effective doses of anatabine are provided in an extended release formulation. In other embodiments, isolated forms of anatabine or pharmaceutically effective salts thereof can be provided in a bottled water product comprising about 100 ml to about 2,000 ml purified water and from about 0.00001 to about 0.0001 wt% of anatabine or a water-soluble salt of anatabine.

In another aspect, a method is provided to prepare anatabine synthetically. In a first step, benzylhydrylidene-pyridin-3-ylmethyl-amine (Formula I) is prepared by reacting 3-aminomethylpyridine with benzophenoneimine, or benzophenoneimine substituted in one or both aromatic rings.

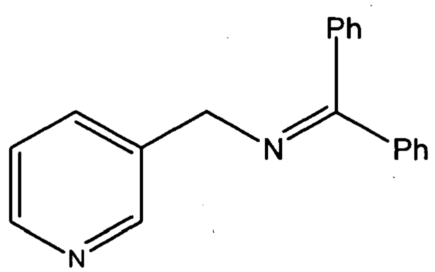

Formula I

The compound of Formula I may then be reacted with a non-nucleophilic base and a dielectrophile to obtain a compound of Formula II.

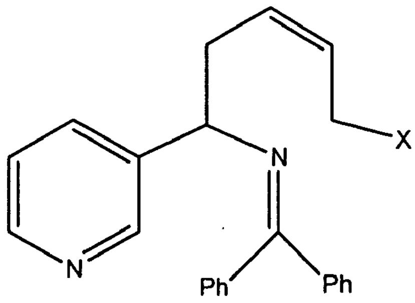

Formula II

The compound of Formula II is then acidified to provide a compound of Formula III.

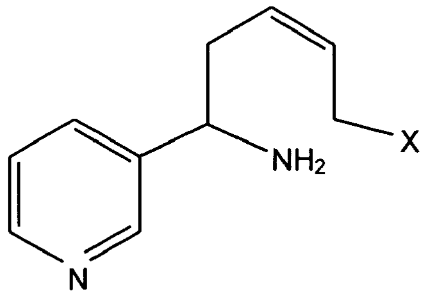

Formula III

The compound of Formula III is basified to yield anatabine.

In another aspect, a novel method is provided to recover anatabine from a reaction product by contacting the reaction product with methyl t-butyl ether (MTBE). Subsequence distillation yields anatabine with a purity greater than 99%.

In yet another aspect, acceptable pharmaceutical or food-grade salts of anatabine are prepared.

In an alternative embodiment, methods are provided for synthesizing other minor alkaloids, such as anabasine, nornicotine, N-methylanabasine, or anabaseine. A compound of Formula I may be prepared as previously described and reacted with a dielectrophile to yield a compound of Formula IIa:

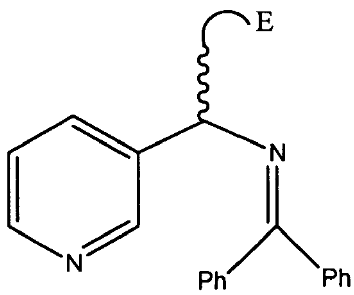

Formula IIa

The dielectrophile may be selected according to the desired alkaloid. The compound of Formula IIa is then acidified to provide a compound of Formula IIIa.

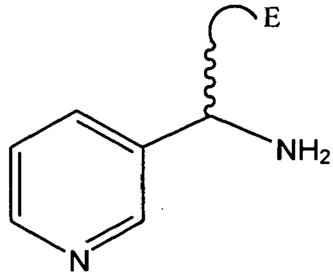

Formula IIIa

Basification of the compound of Formula IIIa results in the desired alkaloid by intramolecular N-alkylation.

BRIEF DESCRIPTION OF THE DRAWINGS

FIG. 1. Graph showing effects of anatabine (AN) on TNFa-induced NFκB activity *in vitro.* See Example 1.

**FIG. 2.** Graph showing effects of a crude extract of smokeless tobacco on TNFα-induced NFKB activity *in vitro.* See Example 1.

**FIG. 3.** Graph showing effects of nicotine and of an alkaloid extract of smokeless tobacco on TNFa-induced NFκB activity *in vitro.* See Example 1.

**FIG. 4.** Graph showing the results of a cytotoxicity assay measuring release of lactate dehydrogenase (LDH) using supernatant from the cells assayed in **FIG. 1.** See Example 2.

**FIG. 5.** Graph showing the results of a cytotoxicity assay using supernatant from the cells assayed in **FIG. 2.** See Example 2.

**FIG. 6.** Graph showing the results of a cytotoxicity assay using supernatant from the cells assayed in **FIG. 3.** See Example 2.

**FIG. 7.** Graph showing concentrations in rat plasma as a function of time of anatabine and nicotine after a single intravenous bolus injection.

**FIG. 8.** Graph showing concentrations of anatabine and nicotine in rat plasma as a function of time (semi-log).

**FIG. 9.** Graph showing $AUC_{0\rightarrow\infty}$ versus dose for both anatabine and nicotine in male and female rats.

**FIG. 10.** Graph showing concentrations of anatabine or nicotine in rat brain extracts following a single intravenous bolus dose.

**FIG. 11.** Graph showing mean concentration of anatabine and nicotine in rat brain extracts 0.5 hours after a single intravenous bolus dose.

**FIG. 12.** Nicotine product ion scan.

**FIG. 13.** Nicotine sample chromatogram.

**FIG. 14.** Anatabine product ion scan.

**FIG. 15.** Anatabine sample chromatogram.

**FIG. 16.** Nicotine-d3 product ion scan.

**FIG. 17.** Nicotine-d3 sample chromatogram.

**FIG. 18.** Anatabine-d4 product ion scan.

**FIG. 19.** Anatabine-d4 sample chromatogram.

**FIG. 20.** Graph showing mean body weights (± Std Dev) for each treatment group and gender.

**FIGS. 21A-21B.** Graphs showing mean (± SEM) concentration of anatabine in plasma for male or female rats. **FIG. 21A**, 0.6 mg/kg body weight (BW); **FIG. 21B**, 6.0 mg/kg BW.

**FIGS. 22A-22B.** Graphs showing mean (± SEM) concentration of anatabine in plasma for male and female rats combined. **FIG. 22A**, 0.6 mg/kg BW; **FIG. 22B**, 6.0 mg/kg BW.

**FIGS. 23A-23B.** Graphs showing mean (± SEM), maximal (Cp, max), and minimal (Cp, min) concentrations of anatabine in plasma for male or female rats. **FIG. 23A**, 0.6 mg/kg BW; **FIG. 23B**, 6.0 mg/kg BW.

**FIG. 24.** Graph showing effect of S-(-)-anatabine on TNFa-induced NFκB activity *in vitro.*

DETAILED DESCRIPTION

This disclosure describes methods of using a composition comprising an isolated form of anatabine or a salt thereof to treat disorders comprising an inflammatory component, including chronic, low-level inflammation. Anatabine is an alkaloid present in tobacco and, in lower concentrations, in a variety of foods, including green tomatoes, green potatoes, ripe red peppers, tomatillos, and sundried tomatoes. Preparation of isolated forms of anatabine is described below. Use of such isolated forms avoids the toxicity associated with tobacco, tobacco extracts, alkaloid extracts, and nicotine.

Without being bound by this explanation, data presented in Examples 1 and 2 below indicate that anatabine reduces transcription mediated by nuclear factor κB (NFκB). NFκB is a transcription factor which operates in cells involved in inflammatory and immune reactions. As documented in Table 1, NFκB-mediated transcription is associated with numerous disorders, including those with an inflammatory component, an aberrant immune response, and/or inappropriate cell proliferation. Isolated forms of anatabine or salts of such isolated forms anatabine are particularly useful for treating disorders comprising an "NFκB-mediated inflammatory component," *i.e.* inflammation characterized by, caused by, resulting from, or affected by NFκB-mediated transcription.

In other embodiments, a therapeutically effective dose of an isolated form of anatabine or a pharmaceutically acceptable salt thereof can be administered to an individual for treating alopecia areata or other disorders associated with hair loss.

"Treat" as used herein refers to reducing a symptom of the inflammation or resulting disorder but does not require complete cure, either of the inflammation or the disorder. "Reduction of a symptom" of a disorder with an NFκB-mediated inflammatory component includes but is not limited to elimination of the symptom as well as reduction in frequency, severity, or duration of the symptom. Reduction of a symptom can be recognized subjectively by the individual or an observer of the individual or can be confirmed by clinical and/or laboratory findings.

An isolated form of anatabine or a pharmaceutically acceptable salt of such an isolated form of anatabine is administered to the individual at a dose sufficient to reduce a symptom of a disorder with an NFκB-mediated-transcription component. "Individual" as used herein includes warm-blooded animals, typically mammals, including humans and other primates. Doses typically range from about 1 μg/kg to about 7 mg/kg body weight (*e.g.*, about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 μg/kg or about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 mg/kg), about 1.5 μg/kg to about 5 μg/kg, about 1 μg/kg to about 10 μg/kg, about 0.01 mg/kg to about 7 mg/kg body weight, about 0.1 mg/kg to about 5 mg/kg; about 0.1 mg/kg to about 2 mg/kg, about 1 mg/kg to about 3 mg/kg, about 0.5 mg/kg to about 2 mg/kg, about 1 mg/kg to about 2 mg/kg, about 3 mg/kg to about 5 mg/kg, about 2 mg/kg to about 4 mg/kg, about 2 mg/kg to about 5 mg/kg, or about 0.5 mg/kg to about 1.5 mg/kg. Certain factors may influence the dose sufficient to reduce a symptom of a disorder (*i.e.*, an effective dose), including the severity of the disease or disorder, previous treatments, the general health, age, and/or weight of the individual, the frequency of treatments, the rate of release from the composition, and other diseases present. This dose may vary according to factors such as the disease state, age, and weight of the subject. For example, higher doses may be administered for treatments involving conditions which are at an advanced stage and/or life-threatening. Dosage regimens also may be

adjusted to provide the optimum therapeutic response.

In some embodiments tablets comprising about 600 μg anatabine citrate are administered from once to 25 times daily (*e.g.*, 1, 2, 3, 4, 5, 6, 7, 8, 9, 10, 11, 12, 13, 14, 15, 16, 17, 18, 19, 20, 21, 22, 23, 24, or 25) times daily.

In some embodiments the dose sufficient to reduce the symptom of the disorder can include a series of treatments. For example, an individual can be treated with a dose of an isolated form of anatabine or a salt thereof several times per day (*e.g.*, 2-12 or 4-10 times per day), once daily, or less frequently such as 1-6 times per week. Treatments may span between about 1 to 10 weeks (*e.g.*, between 2 to 8 weeks, between 3 to 7 weeks, for about 1, 2, 3, 4, 5, 6, 7, 8, 9, or 10 weeks). It will also be appreciated that a dose regimen used for treatment may increase or decrease over the course of a particular treatment.

In some embodiments the individual is an animal, such as a companion animal, a service animal, a farm animal, or a zoo animal. Such animals include, but are not limited to, canines (including dogs, wolves), felines (including domestic cats, tigers, lions), ferrets, rabbits, rodents (*e.g.*, rats, mice), guinea pigs, hamsters, gerbils, horses, cows, pigs, sheep, goats, giraffes, and elephants.

In some embodiments an isolated form of anatabine or a pharmaceutically acceptable salt of an isolated form of anatabine can be administered to reduce the risk of developing a disorder comprising an NFκB-mediated inflammatory component (*i.e.*, prophylactically). One can readily identify individuals with an increased risk or family history of a disorder. Other recognized indices of elevated risk of certain disorders can be determined by standard clinical tests or medical history.

NFκB-mediated transcription is implicated in an enormous variety of maladies. Based on S-(-)-anatabine's surprising efficacy in interfering with or interrupting this pivotal inflammatory-related activity, anatabine can be expected to have a wide range of therapeutic utilities.

In some embodiments the disorder is thyroiditis. In some embodiments the disorder is an immune or autoimmune disorder. In some embodiments the disorder is arthritis, such as rheumatoid arthritis, primary and secondary osteoarthritis (also known as degenerative joint disease). In some embodiments the disorder is a spondyloarthropathy, such as psoriatic arthritis, juvenile chronic arthritis with late pannus onset, and enterogenic spondyloarthropathies such as enterogenic reactive arthritis, urogenital spondyloarthropathy, and undifferentiated spondylarthropathy. In some embodiments the disorder is a myopathy, such as "soft tissue rheumatism" (*e.g.*, tennis elbow, frozen shoulder, carpal tunnel syndrome, plantar fasciitis, and Achilles tendonitis).

In some embodiments the disorder is diabetes, either type I diabetes or type II diabetes. In other embodiments the disorder is a gastrointestinal inflammatory disorder, such as an inflammatory bowel disease. Examples of inflammatory bowel disease include, but are not limited to, Crohn's disease, Barrett's syndrome, ileitis, irritable bowel syndrome, irritable colon syndrome, ulcerative colitis, pseudomembranous colitis, hemorrhagic colitis, hemolytic-uremic syndrome colitis, collagenous colitis, ischemic colitis, radiation colitis, drug and chemically induced colitis, diversion colitis, colitis in conditions such as chronic granulomatous disease, celiac disease, celiac sprue, food allergies, gastritis, infectious gastritis, enterocolitis (*e.g.*, Helicobacter pylori-infected chronic active gastritis), and pouchitis.

In other embodiments the disorder is graft-versus-host-disease (GVHD), systemic lupus erythematosus (SLE), lupus nephritis, Addison's disease, Myasthenia gravis, vasculitis (*e.g.*, Wegener's granulomatosis), autoimmune hepatitis, osteoporosis, and some types of infertility.

In some embodiments the disorder is vascular inflammatory disease, associated vascular pathologies, atherosclerosis, angiopathy, inflammation-induced atherosclerotic or thromboembolic macroangiopathy, coronary artery disease, cerebrovascular disease, peripheral vascular disease, cardiovascular circulatory disease such as as ischemia/reperfusion, peripheral vascular disease, restenosis following angioplasty, inflammatory aortic aneurysm, vasculitis, stroke, spinal cord injury, congestive heart failure, hemorrhagic shock, ischemic heart disease/reperfusion injury, vasospasm following subarachnoid hemorrhage, vasospasm following cerebrovascular accident, pleuritis, pericarditis, inflammation-induced myocarditis, or a cardiovascular complication of diabetes.

In some embodiments the disorder is brain swelling or a neurodegenerative disease such as multiple sclerosis, Alzheimer's disease, or Parkinson's disease. In other embodiments the disorder is inflammation related to a kidney disease, nephritis, glomerulonephritis, dialysis, peritoneal dialysis, pericarditis, chronic prostatitis, vasculitis, gout, or pancreatitis.

In some embodiments the disorder is an anemia. In other embodiments the disorder is an ulcer-related disease, such as peptic ulcer disease, acute pancreatitis, or aphthous ulcer. In other embodiments the disorder is related to an age-related disease, such as atherosclerosis, fibrosis, and osteoporosis, or a disorder associated with pre-maturity, such as retinopathy, chronic lung disease, arthritis, and digestive problems.

In other embodiments the disorder is preeclampsia, inflammation related to chemical or thermal trauma due to burns, acid, and alkali, chemical poisoning (MPTP/concavalin/chemical agent/pesticide poisoning), snake, spider, or other insect bites, adverse effects from drug therapy (including adverse effects from amphotericin B treatment), adverse effects from immunosuppressive therapy (*e.g.*, interleukin-2 treatment), adverse effects from OKT3 treatment, adverse effects from GM-CSF treatment, adverse effects of cyclosporine treatment, and adverse effects of aminoglycoside

treatment, stomatitis and mucositis due to immunosuppression, or exposure to ionizing radiation, such as solar ultraviolet exposure, nuclear power plant or bomb exposure, or radiation therapy exposure, such as for therapy for cancer.

In some embodiments the disorder is a cancer, such as acute lymphoblastic leukemia, acute myeloid leukemia, adrenocortical carcinoma, AIDS-related lymphoma, anal cancer, appendix cancer, grade I (anaplastic) astrocytoma, grade II astrocytoma, grade III astrocytoma, grade IV astrocytoma, atypical teratoid/rhabdoid tumor of the central nervous system, basal cell carcinoma, bladder cancer, breast cancer, breast sarcoma, bronchial cancer, bronchoalveolar carcinoma, Burkitt lymphoma, cervical cancer, chronic lymphocytic leukemia, chronic myelogenous leukemia, colon cancer, colorectal cancer, craniopharyngioma, cutaneous T-cell lymphoma, endometrial cancer, endometrial uterine cancer, ependymoblastoma, ependymoma, esophageal cancer, esthesioneuroblastoma, Ewing's sarcoma, extracranial germ cell tumor, extragonadal germ cell tumor, extrahepatic bile duct cancer, fibrous histiocytoma, gallbladder cancer, gastric cancer, gastrointestinal carcinoid tumor, gastrointestinal stromal tumor, gestational trophoblastic tumor, gestational trophoblastic tumor, glioma, hairy cell leukemia, head and neck cancer, heart cancer, hepatocellular cancer, Hilar cholangiocarcinoma, Hodgkin's lymphoma, hypopharyngeal cancer, intraocular melanoma, islet cell tumor, Kaposi sarcoma, Langerhans cell histiocytosis, large-cell undifferentiated lung carcinoma, laryngeal cancer, lip cancer, lung adenocarcinoma, lymphoma, macroglobulinemia, malignant fibrous histiocytoma, medulloblastoma, medulloepithelioma, melanoma, Merkel cell carcinoma, mesothelioma, endocrine neoplasia, multiple myeloma, mycosis fungoides, myelodysplasia, myelodysplastic/ myeloproliferative neoplasms, myeloproliferative disorders, nasal cavity cancer, nasopharyngeal cancer, neuroblastoma, non-Hodgkin's lymphoma, oral cancer, oropharyngeal cancer, osteosarcoma, ovarian clear cell carcinoma, ovarian epithelial cancer, ovarian germ cell tumor, pancreatic cancer, papillomatosis, paranasal sinus cancer, parathyroid cancer, penile cancer, pharyngeal cancer, pineal parenchymal tumor, pineoblastoma, pituitary tumor, plasma cell neoplasm, plasma cell neoplasm, pleuropulmonary blastoma, primary central nervous system lymphoma, prostate cancer, rectal cancer, renal cell cancer, respiratory tract cancer with chromosome 15 changes, retinoblastoma, rhabdomyosarcoma, salivary gland cancer, Sezary syndrome, small cell lung cancer, small intestine cancer, soft tissue sarcoma, squamous cell carcinoma, squamous non-small cell lung cancer, squamous neck cancer, supratentorial primitive neuroectodermal tumor, supratentorial primitive neuroectodermal tumor, testicular cancer, throat cancer, thymic carcinoma, thymoma, thyroid cancer, cancer of the renal pelvis, urethral cancer, uterine sarcoma, vaginal cancer, vulvar cancer, Waldenström macroglobulinemia, or Wilms tumor.

In other embodiments, disorders associated with inflammation in the stomach and/or esophagus, such as acid reflux, may be treated.

Anatabine may have a positive effect on telomere length. Thus, in some embodiments anatabine can be administered to reduce and/or reverse cellular senescence (*i.e.*, aging). Conditions associated with cellular senescence can be treated. For example, anatabine can be administered to reduce and/or reverse senescence of:

a. cells with replicative capacity in the central nervous system, including astrocytes, endothelial cells, and fibroblasts which play a role in age-related diseases as Alzheimer's disease, Parkinson's disease, Huntington's disease, and stroke;

b. cells with finite replicative capacity in the integument, including fibroblasts, sebaceous gland cells, melanocytes, keratinocytes, Langerhan's cells, and hair follicle cells, which may play a role in age-related diseases of the integument such as dermal atrophy, elastolysis and skin wrinkling, sebaceous gland hyperplasia, senile lentigo, graying of hair and hair loss, chronic skin ulcers, and age-related impairment of wound healing;

c. cells with finite replicative capacity in the articular cartilage, such as chondrocytes and lacunal and synovial fibroblasts, which play a role in degenerative joint disease;

d. cells with finite replicative capacity in the bone, such as osteoblasts and osteoprogenitor cells, which play a role in osteoporosis;

e. cells with finite replicative capacity in the immune system such as B and T lymphocytes, monocytes, neutrophils, eosinophils, basophils, NK cells and their respective progenitors, which may play a role in age-related immune system impairment;

f. cells with a finite replicative capacity in the vascular system including endothelial cells, smooth muscle cells, and adventitial fibroblasts, which may play a role in age-related diseases of the vascular system including atherosclerosis, calcification, thrombosis, and aneurysms;

g. cells with a finite replicative capacity in the eye such as pigmented epithelium and vascular endothelial cells which may play an important role in age-related macular degeneration; and

h. cells with abnormal poliferative capacity, such as cancer cells.

Isolated forms of anatabine and pharmaceutically acceptable salts of such isolated forms of anatabine can be used in conjunction with (*i.e.*, before, after, or at the same time as) other therapies for any disorder with an NFκB-mediated component. In some embodiments, these therapies include other products that inhibit production of NFκB-mediated inflammatory species. These products include, but are not limited to, dexamethasone, glucocorticoids (*e.g.*, prednisone, methyl prednisolone), cyclosporine, tacrolimus, deoxyspergualin, non-steroidal antiinflammatory drugs (NSAIDs) such as aspirin and other salicylates, tepoxalin, synthetic peptide proteosome inhibitors, antioxidants (*e.g.*, N-acetyl-L-cysteine, vitamin C, vitamin E, dithiocarbamate derivatives, curcumin), IL-10, nitric oxide, cAMP, gold-containing compounds, and gliotoxin.

Isolated forms of anatabine and pharmaceutically acceptable salts of such isolated forms of anatabine can also be used to improve erectile dysfunction, either administered alone of in conjunction with other therapies such as tadalafil (*e.g.* CIALIS®), vardenafil (*e.g.*, LEVITRA®, STAXYN®), and sildenafil (*e.g.*, VIAGRA®).

**Anatabine**

Anatabine can be prepared synthetically. Such synthetic preparation techniques produce isolated forms of anatabine, either as a racemic mixture or as isolated enantiomers, S-(-)-anatabine or R-(+)-anatabine. The chemical structure of anatabine (1,2,3,6-tetrahydro-[2,3']bipyridinyl) is illustrated below, in which * designates an asymmetric carbon.

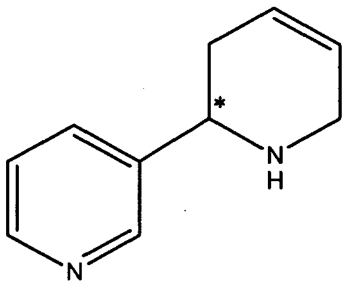

In one aspect of the invention, methods are provided for synthesizing anatabine, especially methods that are useful in larger scale syntheses. In Step 1, 3-aminomethyl pyridine is reacted with benzophenoneimine (or benzophenoneimine substituted in either or both rings with nitrogen, a halogen, or an alkyl group) to form benzylhydrylidene-pyridin-3-ylmethyl-amine, also referred to herein as Formula I:

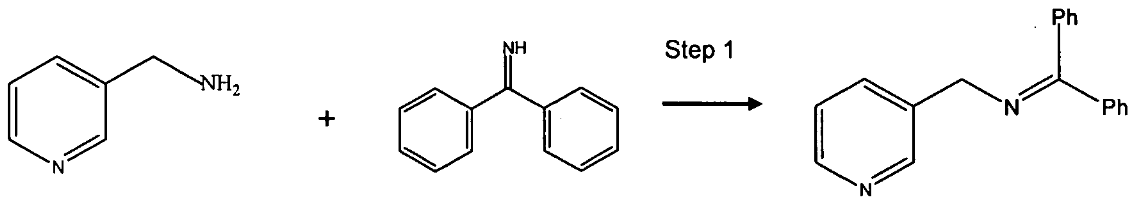


3-aminomethyl pyridine | benzophenoneimine | Formula I

Step 1 may be performed at any suitable temperature range. The reaction is exothermic, normally resulting in a temperature increase from ambient to about 45 °C to 55 °C. If desired, the reaction mixture may be heated or cooled. In general, the rate of reaction increases at higher temperature and decreases at lower temperatures. For example, step 1 may be performed at about 30 °C to about 60 °C. Most often, step 1 is performed at a temperature of about 45 °C to about 55 °C.

The amount of time needed to complete Step 1 may vary depending on such factors such as reaction temperature and pressure. Normally, the reaction is completed in about 4 to about 9 hours, more usually from about 5 to about 7 hours.

Step 1 is particularly advantageous as it can be scaled up to facilitate larger scale production. For example, the amount of starting material (e.g. combined weight of 3-aminomethylpyridine and benzophenoneimine) used for a batch may be about 500 mg, about 1 g, about 5 g, about 10 g, about 20 g, about 25 g, about 30 g, about 40 g, about 50 g, about 100 g, about 200 g, about 500 g, about 1 kg, about 5 kg, or about 10 kg. Often, a medium scale synthesis starts

with 20 g to 30 g of starting material. A large scale synthesis may start with 1 kg to 5 kg or more of starting material.

Using benzophenoneimine provides several key advantages over earlier methods and facilitates larger-scale synthesis as described above. First, the reaction by-product is ammonia rather than water. The ammonia escapes from the reaction mixture. By avoiding the presence of water as byproduct, the reaction temperature-50 °C, for example-is much lower than the ~85 °C used for the water-removing benzene reflux step in the method of Deo et al., "Regioselective alkylation of N-(diphenylmethylidine)-3-(aminomethyl) pyridine: A simple route to minor tobacco alkaloids and related compounds," Tetrahedron Letters, Vol. 37, Issue 8, 19 Feb. 1996, pp. 1137-1140. Using lower temperatures in the preparation of the compound offers improved economic and environmental efficiencies. Also, by omitting benzene from the reaction, the synthesis is safer and more environmentally-friendly. Avoiding the presence of water is also beneficial in terms of improved product stability because anatabine is moisture-sensitive.

Second, because benzophenoneimine is liquid, Step 1 may proceed in a solvent-less reaction medium such that the purity of the isolated intermediate is enhanced and avoids the need for a workup reaction. Purity of Formula I compound typically is about 93-95% whereas the Deo et al. method yields only about 83-85% purity of the intermediate.

The purity of Formula I compound may be as high as about 100%, about 99%, about 98%, about 97%, about 96%, about 95%, about 94%, or about 93%. Often, the Formula I compound is about 93 % to about 95% pure.

In step 2i, the compound of Formula I is reacted with a non-nucleophilic base, such as lithium diisopropylamide (LDA), and a dielectrophile, such as cis-1,4-dichloro-2-butene to form a compound of Formula II. The non-nucleophilic base typically is added in a molar excess, for example, at about 1.2 equivalents (eq), about 1.3 eq, about 1.4 eq, about 1.5 eq, about 1.6 eq, about 1.7 eq, about 1.8 eq, about 1.9 eq, or about 2.0 eq. Often the non-nucleophilic base is added in an amount of about 1.3 eq to about 1.7 eq, more usually from about 1.4 to about 1.6 eq.

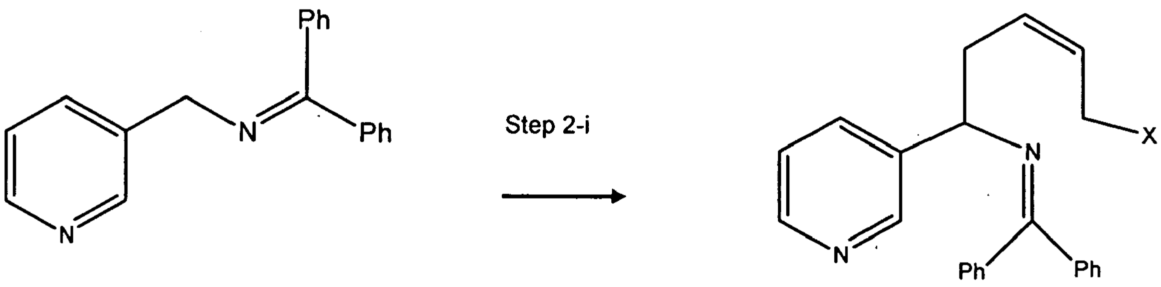


Formula I Formula II

The non-nucleophilic base is typically supplied in a chilled state to provide improved reagent stability. For example, LDA may be supplied at a temperature of about -30 °C, about -20 °C, about -10 °C, about -5 °C, about 0 °C, about 5 °C, or about 10 °C. Most often the non-nucleophilic base is provided at a temperature of about -30 °C to about 0 °C.

The Step 2i reaction may be incubated for a time suitable for the reaction to go to completion or substantial completion. For example, the reaction time for Step 2i often is about 10 minutes, about 20 minutes, about 30 minutes, about 40 minutes, about 50 minutes, or about 1 hour. Often, the reaction is incubated for about 20 to about 40 minutes.

As an alternative to LDA in step 2i, other strong bases such potassium tert butoxide ($K^tOBu$) may be used. Potassium tert butoxide ($K^tOBu$) was found to provide improved yield and purity. Using $K^tOBu$ we obtained an anatabine yield of 25% with 97% purity. Other alternatives to LDA include sodium hydride, sodamide, and other alkyllithium reagents.

Following addition of the non-nucleophilic base, a dielectrophile of Formula IV is added:

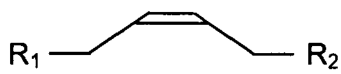

Formula IV

wherein each of $R_1$ and $R_2$ is independently selected from the group consisting of chlorine, (Cl); bromine, (Br), and iodine, (I), tosylate, mesylate, and triflate. For example, $R_1$ and $R_2$ may both be Cl, such that the dielectrophile is cis-1,4-dichloro-2-butene. The dielectrophile may be added neat or in a suitable solvent, such as THF.

For step 2ii, Formula II compound is hydrolyzed with acid. Treatment with acid may be for any suitable time. For example, HCl (10%) treatment may be for about 5 minutes, about 10 minutes, about 20 minutes, about 30 minutes, or for about 1 hour. Often, acid treatment is for about 10 minutes to about 20 minutes. Other suitable acids include dilute sulfuric acid and phosphoric acid. For step 2iii, Formula III compound is basified, such as by treatment with solid $K_2CO_3$,

then aqueous KOH solution (e.g., 40%) to achieve N-alkylation to yield anatabine. Other bases, such as Diaza(1,3)bicyclo[5.4.0]undecane (DBU), di-isopropyl ethylamine, or triethylamine, may also be used.

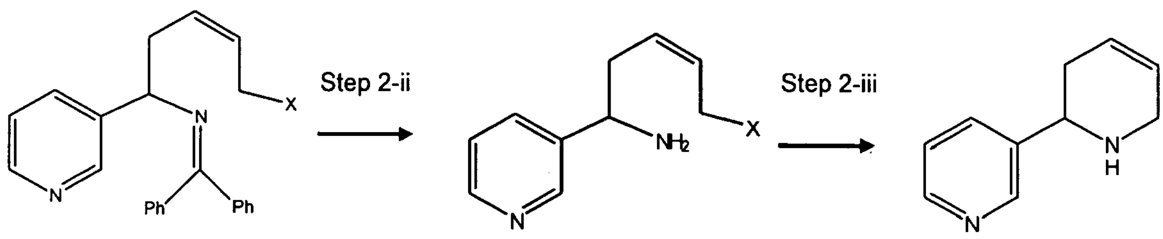


Formula II    Formula III    anatabine

In another aspect, a method of recovering and purifying anatabine comprises extracting anatabine from the reaction product of Step 2iii using MTBE in a distillation process. The anatabine containing solution is basified to saturation with KOH and $K_2CO_3$. Addition of MTBE to the solution induces phase separation, with the anatabine separated into the organic phase, which provides an additional increase in anatabine purity. Subsequent distillation, followed by acid-base work up typically results in an anatabine purity of 99%.

In another aspect, a method of recovering and purifying anatabine comprises extracting anatabine from the reaction product of Step 2iii using MTBE in a distillation process. The anatabine containing solution is basified to saturation with KOH and $K_2CO_3$. Addition of MTBE to the solution induces phase separation, with the anatabine separated into the organic phase, which provides an additional increase in anatabine purity. Subsequent distillation, followed by acid-base work up typically results in an anatabine purity of 99%.

Advantageously, this purification approach can be scaled up to industrial-scale manufacture. Purification by chromatography, which does not scale up from small-scale production, is avoided. Moreover, MTBE is more environmentally friendly than halogenated compounds such as chloroform. MTBE also improves extraction efficiency relative to halogenated compounds such as chloroform.

Anatabine prepared according to the methods disclosed herein displays good stability at a variety of temperatures. See, for example, Tables 54 and 55 in Examples 11 and 12, respectively. Purity of the anatabine may be retained for extended periods of time. For example, the anatabine may exhibit the indicated purity at 12 days, at 18 days, at 21 days, at 1 month, at 3 months, at 6 months, at 9 months, at 12 months at 18 months, at 24 months, and at 36 months, or longer. Further, anatabine remains stable when stored at a variety of temperatures. For example, the anatabine retains the indicated purity when stored at -20 °C to -10 °C, -20 °C to 0 °C, -10 °C to 0 °C, 0 °C to 4 °C, 2 °C to 8 °C, 5 °C to 10 °C, 10 °C to 15 °C, 15 °C to 25 °C, or 25 °C to 28 °C. Stability is determined by any suitable art-recognized method. For example, anatabine stability may be monitored by high performance liquid chromatography (HPLC), liquid chromatography-mass spectroscopy (LC-MS), or nuclear magnetic resonance (NMR).

The synthesis described herein may yield significantly lower concentrations of byproducts, such as benzophenone, which can lower reaction yields and introduce impurities into the product. Concentrations of benzophenone, for example, are typically less than about 3 % as compared to 8-12 % that were found when reproducing the Deo et al. synthesis. Moreover, yield was 25% for the present invention but only 10% for the Deo et al synthesis.

In an alternative embodiment, methods are provided for synthesizing other minor alkaloids, such as anabasine, nornicotine, N-methylanabasine, or anabaseine. A compound of Formula I may be prepared as previously described and reacted with a dielectrophile to yield a compound of Formula IIa:

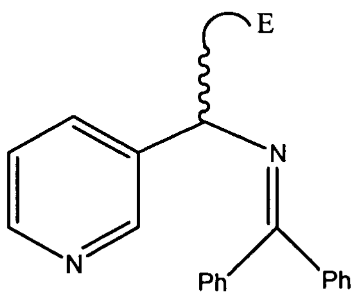

Formula IIa

The dielectrophile may be selected according to the desired alkaloid. For example, as described in Deo et al., anabasine may be synthesized using 1,4-diiodo-butane; nornicotine may be synthesized using 1,3-diiodo-propane or methanesulfonic acid 4-ethoxy-butyl ester, and so on. The compound of Formula IIa is then acidified as previously described to yield a compound of Formula IIIa.

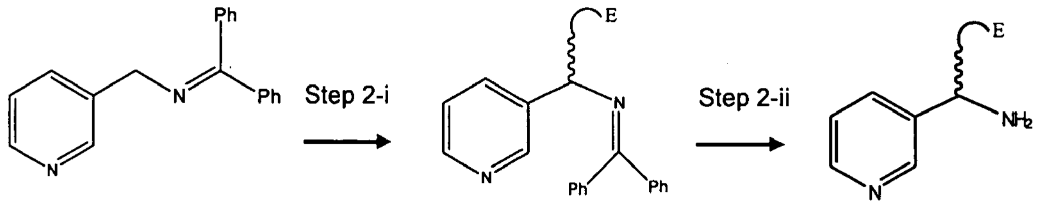


Formula I Formula IIa Formula IIIa

Basification of the compound of Formula IIIa results in the desired alkaloid by intramolecular N-alkylation as previously described. The synthetic alkaloids may be provided in a variety of compositions. For example, the compositions may be in the form of a beverage, a chew, a tablet, a lozenge, a gum, and the like. Additional inactive ingredients may be added to improve taste or stability. Optionally, other components such as sweetening and flavoring agents may be added. Other active ingredients may be provided as well.

In some embodiments, an isolated form of anatabine can be provided as one or more pharmaceutically acceptable (or food grade) salts of anatabine. Anatabine may be adsorbed on a cation exchange resin such as polymethacrilic acid (Amberlite IRP64 or Purolite C115HMR), as described in U.S. Patent 3,901,248, the disclosure of which is hereby incorporated by reference in its entirety. Such cation exchange resins have been used commercially in nicotine replacement therapy, e.g., nicotine polacrilex. Unless otherwise clear from context, the term "anatabine" as used herein refers collectively to anatabine and its pharmaceutically acceptable salts. In general, salts may provide improved chemical purity, stability, solubility, and/or bioavailability relative to anatabine in its native form. Non-limiting examples of possible anatabine salts are described in P. H. Stahl et al., Handbook of Pharmaceutical Salts: Properties, Selection and Use, Weinheim/Zürich: Wiley-VCH/VHCA, 2002, including salts of 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (- L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (- L), salicylic acid, sebacic acid, stearic acid, succinic acid, sulfuric acid, tartaric acid (+ L), thiocyanic acid, toluenesulfonic acid (p), and undecylenic acid.

As an alternative to preparing anatabine synthetically, anatabine can be obtained by extraction from tobacco or other plants, such as members of the Solanaceae family, such as datura, mandrake, belladonna, capsicum, potato, nicotiana, eggplant, and petunia. For example, a tobacco extract may be prepared from cured tobacco stems, lamina, or both. In the extraction process, cured tobacco material is extracted with a solvent, typically water, ethanol, steam, or carbon dioxide. The resulting solution contains the soluble components of the tobacco, including anatabine. Anatabine may be purified from the other components of the tobacco using suitable techniques such as liquid chromatography.

As part of the purification process, tobacco material may be substantially denicotinized to remove a majority of other alkaloids such as nicotine, nornicotine, and anabasine. Denicotinizing is usually carried out prior to extraction of anatabine. Methods that may be used for denicotinizing tobacco materials are described, for example, in U.S. Patent 5,119,835, the disclosure of which is hereby incorporated by reference. In general, tobacco alkaloids may be extracted from tobacco material with carbon dioxide under supercritical conditions. The tobacco alkaloids may then be separated from the carbon dioxide by dissolving an organic acid or a salt thereof, such as potassium monocitrate, in the carbon dioxide.

Flue (bright) varieties of tobacco are often used, *i.e.*, Virginia flue. Other tobacco varieties may be used, such as Burley, dark-fired, and/or other commercial tobacco varieties. Methods for forming aqueous tobacco extracts also are known in the art as described, for example, in U.S. Patent 5,065,775, the disclosure of which is hereby incorporated by reference in its entirety. Additional details on methodology for extracting anatabine from tobacco are disclosed in co-pending and commonly owned U.S. Published Application No. 2010/0154810 A1, the disclosure of which is hereby incorporated by reference in its entirety.

In some aspects, a pharmaceutical composition for use in the disclosed methods comprises an isolated form

of anatabine and a pharmaceutically acceptable vehicle, diluent, or carrier. An "isolated form of anatabine," as used herein, refers to anatabine that either has been prepared synthetically or has been substantially separated from plant materials in which it occurs naturally. The isolated form of anatabine as described herein should have a very high purity (including enantiomeric purity in the case of an isolated enantiomer). In the case of synthetic anatabine, for example, purity refers to the ratio of the weight of anatabine to the weight of the end reaction product. In the case of isolating anatabine from plant material, for example, purity refers to the ratio of the weight of anatabine to the total weight of the anatabine-containing extract. Usually, the level of purity is at least about 95%, more usually at least about 96%, about 97%, about 98%, or higher. For example, the level of purity may be about 98.5%, 99.0%, 99.1%, 99.2%, 99.3%, 99.4%, 99.5%, 99.6%, 99.7%, 99.8%, 99.9%, or higher. In some aspects, the composition contains levels of other alkaloids, such as nicotine, nornicotine, and anabasine, which are individually about 1 $\mu$g/g or less, and which may be about 0.5 $\mu$g/g or less, about 0.3 $\mu$g/g or less, about 0.1 $\mu$g/g or less, or lower.

Anatabine exists in tobacco and other plants as a racemic mixture of R-(+)-anatabine and S-(-)-anatabine, whose structures are illustrated below.

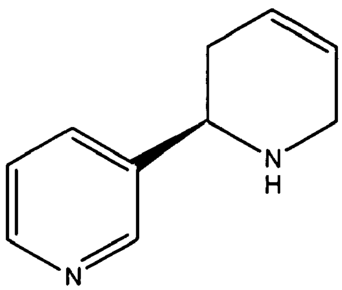

R-(+)-anatabine

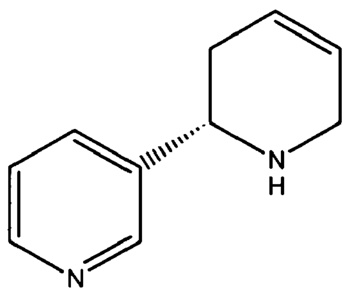

S-(-)-anatabine

Methods for selectively preparing the anatabine enantiomers are described, for example, in "A General Procedure for the Enantioselective Synthesis of the Minor Tobacco Alkaloids Nornicotine, Anabasine, and Anatabine," The AAPS Journal 2005; 7(3) Article 75. Any of the methods or compositions described herein may involve providing anatabine as a racemic mixture of its two enantiomers, as a purified form of S-(-)-anatabine, or as a purified form of R-(+)-anatabine. Unless otherwise clear from context, the term "anatabine" is used herein to refer to any of (1) a racemic mixture of anatabine (R,S) or a salt thereof, (2) a purified form of S-(-)-anatabine or a salt thereof, or (3) a purified form of R-(+)-anatabine or a salt thereof.

Isolated forms of anatabine or pharmaceutically acceptable salts of anatabine can be provided together with other ingredients, for example, in the form of an elixir, a beverage, a chew, a tablet, a lozenge, a gum, and the like. In one embodiment, for example, a beverage may be in the form of a bottled water product containing about 100 ml to about 2,000 ml purified water and from about 0.00001 to about 0.0001 wt% of a water-soluble salt of anatabine. Additional inactive ingredients may be added to improve product characteristics, such as taste, color/clarity, and/or stability. The bottled water product may also contain other beneficial components, such as vitamins, proteinaceous ingredients, or the like. A composition alternatively may be provided in a solid (e.g., powder) form, such as in a packet, which can be combined with water or other liquid (e.g., by an end user) to prepare a beverage.

Pharmaceutical compositions may be formulated together with one or more acceptable pharmaceutical or food grade carriers or excipients. As used herein, the term "acceptable pharmaceutical or food grade carrier or excipient" means a non-toxic, inert solid, semisolid or liquid filler, diluent, encapsulating material or formulation auxiliary of any type. For example, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The pharmaceutical composition may be prepared by any suitable technique and is not limited by any particular method for its production. For example, anatabine can be combined with excipients and a binder, and then granulated. The granulation can be dry-blended any remaining ingredients, and compressed into a solid form such as a tablet.

The pharmaceutical compositions may be administered by any suitable route. For example, the compositions

may be administered orally, parenterally, by inhalation spray, topically, rectally, nasally, buccally, vaginally, via an implanted reservoir, or ingested as a dietary supplement or food. The term parenteral as used herein includes subcutaneous, intracutaneous, intravenous, intramuscular, and intracranial injection or infusion techniques. Most often, the pharmaceutical compositions are readily administered orally and ingested.

The pharmaceutical compositions may contain any conventional non-toxic pharmaceutically-acceptable carriers, adjuvants or vehicles. In some cases, the pH of the formulation may be adjusted with acceptable pharmaceutical or food grade acids, bases or buffers to enhance the stability of the formulated composition or its delivery form.

Liquid dosage forms for oral administration include acceptable pharmaceutical or food grade emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylsulfoxide (DMSO) dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, lozenges, pills, powders, and granules. In such solid dosage forms, the active compound is mixed with at least one inert, acceptable pharmaceutical or food grade excipient or carrier such as sodium citrate or dicalcium phosphate and/or a) fillers or extenders such as starches, lactose, sucrose, glucose, mannitol, and silicic acid, b) binders such as carboxymethylcellulose, alginates, gelatin, polyvinylpyrrolidone, sucrose and acacia, c) humectants such as glycerol, d) disintegrating agents such as agaragar, calcium carbonate, potato or tapioca starch, alginic acid, certain silicates, and sodium carbonate, e) solution retarding agents such as paraffin, f) absorption accelerators such as quaternary ammonium compounds, g) wetting agents such as cetyl alcohol and glycerol monostearate, h) absorbents such as kaolin and bentonite clay, i) lubricants such as talc, calcium stearate, magnesium stearate, solid polyethylene glycols, sodium lauryl sulfate, and mixtures thereof, and j) sweetening, flavoring, perfuming agents, and mixtures thereof. In the case of capsules, lozenges, tablets and pills, the dosage form may also comprise buffering agents.

The solid dosage forms of tablets, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract or, optionally, in a delayed or extended manner. Examples of embedding compositions which can be used include polymeric substances and waxes. Tablet formulations for extended release are also described in U.S. Pat. No. 5,942,244.

## Inflammatory Markers

Anatabine can be used to reduce elevated blood levels of inflammatory markers such as CRP. Thus, in some embodiments levels of inflammatory markers can be used to aid in determining doses of anatabine to be administered as well as to monitor treatment of various inflammatory disorders and to assist physicians in deciding on a course of a treatment for an individual at risk of an inflammatory disorder. These markers include, but are not limited to, C-reactive protein (CRP), soluble intercellular adhesion molecule (sICAM-1), ICAM 3, BL-CAM, LFA-2, VCAM-1, NCAM, PECAM, fibrinogen, serum amyloid A (SAA), TNF$\alpha$, lipoprotein associated phospholipase A2 (LpPlA2), sCD40 ligand, myeloperoxidase, interleukin-6 (IL-6), and interleukin-8 (IL-8).

The level of one or more inflammatory markers can be determined in a patient already receiving anatabine therapy or in an individual at risk for an inflammatory disorder or suspected of having an inflammatory disorder. The level is compared to a predetermined value, and the difference indicates whether the patient will benefit from anatabine administration or from continued anatabine administration. The level of inflammatory marker can be determined by any art recognized method. Typically, the level is determined by measuring the level of the marker in a body fluid, for example, blood, lymph, saliva, or urine. The level can be determined by ELISA, or immunoassays or other conventional techniques for determining the presence of the marker. Conventional methods include sending a sample(s) of a patient's body fluid to a commercial laboratory for measurement.

The predetermined value can take a variety of forms and will vary according to the inflammatory marker. The predetermined value can be single cut-off value, such as a median or a mean, or it can be a range. The predetermined value also can depend on the individual or particular inflammatory disorder. Appropriate ranges and categories can be selected by those of ordinary skill in the art using routine methods. See US 2006/0115903; US 2004/0175754.

Markers such as CRP, sICAM-1, ICAM 3, BL-CAM, LFA-2, VCAM-1, NCAM, PECAM, fibrinogen, SAA, TNF$\alpha$, lipoprotein associated phospholipase A2 (LpPlA2), sCD40 ligand, myeloperoxidase, IL-6, and IL-8 are useful markers for systemic inflammation. In some embodiments the inflammatory marker is CRP, which is associated both with cardiovascular disease (see US 2006/0115903) and cancer, such as colon cancer (Baron et al., N. Engl. J. Med. 348, 891-99,

2003). Elevated levels of CRP are also observed in patients with insulin-resistance (Visser et al., JAMA. 1999, 282(22):2131-5). Diabetic and insulin-resistant patients also have elevated levels of TNFα, IL-6, and IL-8 (Roytblat et al., Obes Res. 2000, 8(9):673-5; Straczkowski et al., J Clin Endocrinol Metab. 2002, 87(10):4602-6; Hotamisligil et al., Science. 1996, 271(5249):665-8; Sartipy P, Loskutoff D J. Proc Natl Acad Sci USA. 2003, 100(12):7265-70; Hotamisligil et al., J Clin Invest. 1995, 95(5):2409-15).

Each reference cited in this disclosure is incorporated herein in its entirety. The following examples illustrate but do not limit the scope of the disclosure set forth above.

**EXAMPLE 1**

**NFκB-mediated transcription assays; Cytotoxicity assays**

The effect of a range of doses of anatabine, nicotine, crude extract of smokeless tobacco, and alkaloid extract of smokeless tobacco was examined in an NFκB luciferase assay (inhibition of TNFa-induced NFκB activity). The smokeless tobacco used in these experiments was plain long-leaf Copenhagen tobacco purchased from a local vendor. Crude extract was extracted with methanol and water and clarified by centrifugation and filtration. The alkaloid extract was prepared from sodium hydroxide and methanol extraction, organic phase separation and purification. All treatment samples were prepared as a function of weight (μg/ml), and all samples were diluted in DMSO. Dilutions were made immediately before cell culture treatments and, in all cases, the final amount of DMSO did not exceed 1% in cell culture media.

Human endothelial kidney cells (HEK293) transfected with an NFκB luciferase reporter were challenged with TNFα for three hours, then samples were applied to the challenged cells. The results are shown in **FIGS. 1-3.**

Cytotoxicity assays using the supernatants from the treated cells were conducted using an LDH Cytotoxicity Detection Kit (Roche) according to the manufacturer's instructions. The results are shown in **FIGS. 4-6.**

As shown in **FIG. 1**, TNFα induces an increase in NFκB-mediated transcription of luciferase; administration of anatabine can reduce this transcription to control levels without cellular toxicity (**FIG. 4**). Crude extracts of smokeless tobacco, while not toxic to cells (**FIG. 5**), do not reduce TNFa-induced NFκB-mediated transcription (**FIG. 2**). Although not suitable for administration as pharmaceuticals, both nicotine and an alkaloid extract of smokeless tobacco reduce TNFα-induced NFκB-mediated transcription (**FIG. 3**); at higher doses, the alkaloid extract demonstrates pronounced cytotoxicity (**FIG. 6**).

**EXAMPLE 2**

**Materials and methods**

**Animals.** Male and female Sprague-Dawley rats (~ 200-250 grams) were obtained from Charles River Laboratories Inc., Wilmington, MA and used in compliance with the Animal Welfare Act, the Guide for the Care and Use of Laboratory Animals, and the Office of Laboratory Animal Welfare. Upon receipt at the vivarium, rats were examined by trained personnel to ensure acceptable health status. Rats were acclimated for at least 5 days prior to use.

Rats were housed 3 per cage. Cage size met or exceeded the requirements set forth by the Institute for Animal Laboratory Research Guide for the Care and Use of Laboratory Animals. The rats were kept in a room maintained at 64-84 °F (22-24 °C) with humidity set at 40-70 %. The room was illuminated with fluorescent lights timed to give a 12 hour-light, 12 hour-dark cycle. Standard rodent diet (PharmaServ lab diet 5001) and water were available for all rats. The feed was analyzed by the supplier, detailing nutritional information and levels of specified contaminants.

**Test compounds.** The following compounds were tested in the examples below:

(-) Nicotine hydrogen tartrate (Sigma Aldrich: N5260 Lot# 098K0676) 35.1% (w/w) nicotine;
(R, S) Anatabine tartrate (2:3) (Toronto Research Chemicals, A637510, Lot# 9-BHW-79-2) 41.6% (w/w) Anatabine;
(+/-)-nicotine-3'-d3 (Toronto Research Chemicals: N412423, Lot# 9-BCC-114-2);
(R,S)-Antabine-2,4,5,6-d4 (Toronto Research Chemicals: A637505, Lot# 6-SG-82-1); and
anatabine polacrilex (Emerson Resource Inc. lot # JK02-145); purity was 5.18 % as per Certificate of Analysis

Certificates of analyses for anatabine and nicotine indicated 98% and 100% purity, respectively. Anatabine was stored at 4 °C in a desiccated environment (silica), protected from light. Nicotine was stored at room temperature. Vehicle was sterile phosphate buffered saline (PBS) (Amresco lot#2819B188).

**Supplies.** The following were obtained from Becton Dickinson, Franklin Lakes, NJ: MICROTAINER® Brand Tubes ($K_2$) EDTA (lot# 9050883); serum separator blood collection tubes (lot# 9104015); and sodium citrate blood collection tubes (lot# 8310564). Ten percent neutral-buffered formalin was from Sigma Aldrich, St. Louis, MO (batch#

019K4386).

**EXAMPLE 3**

**Toxicokinetic evaluation of single doses of anatabine and nicotine in Sprague-Dawley Rats**

This example reports evaluation of the toxicokinetics of anatabine and nicotine following a single intravenous injection in Sprague-Dawley rats.

**Summary**

Anatabine was administered as a single intravenous (i.v.) injection at doses of 0.10, 0.75, or 1.0 mg/kg. Nicotine was administered as a single intravenous injection at a dose of 0.4 mg/kg. Six rats (3 males and 3 females) were dosed per dose group. Blood was collected for plasma at 15, 30, 60, 90, 120, 240, 360, 480, and 1440 minutes post i.v. administration. At the 1440 minute time point, animals were euthanized and perfused, and brains were removed and then homogenized. Plasma and brain homogenates were stored at -80 °C until analysis.

An additional 48 rats (24 males and 24 females) received a single intravenous dose via the tail vein at the same doses as mentioned above. At 30 and 360 minutes post administration, 6 rats (3 males and 3 females) per dose group, per time point were euthanized, bled via cardiac puncture, and perfused, and brains were collected. The brains were homogenized. Blood was spun, and plasma was collected. Plasma and brain homogenate were stored at -80 °C.

Both anatabine and nicotine can be measured in rat plasma and brain following a single bolus i.v. dose. The concentration of anatabine in plasma is dose-related. Both compounds are also rapidly cleared from plasma; however, the elimination half-life of anatabine is approximately 2- to 2.5-fold greater than that of nicotine ($t_{1/2}$, 1.64 to 1.68 hr for anatabine compared to 0.67 hr for nicotine). The apparent volume of distribution ($V_D$) for anatabine is also significantly greater than that of nicotine.

At all doses of anatabine the elimination half-life ($t_{1/2}$), mean residence time (MRT), and exposures ($AUC_{0\rightarrow\infty}$) tended to be higher for female rats compared to male rats; however, only at the highest dose of anatabine (1.0 mg/kg) was this difference statistically significant. At this dose level, the elimination half-life ($t_{1/2}$) of anatabine in females was 1.84 hr compared to 1.44 hr for males; mean residence time (MRT) was 2.80 hr for females compared to 2.18 hr for males; and exposure ($AUC_{0\rightarrow\infty}$) was 788.9 ng·hr/mL for females compared to 631.3 ng·hr/mL for males.

Anatabine and nicotine rapidly appear in brain tissue following i.v. administration, and the concentration of anatabine is dose-dependent. At each dose level the mean concentration of anatabine appeared to be higher in the brains of female animals compared to males; however, the differences were not statistically significant.

Anatabine tartrate (2:3) or nicotine bitartrate was dissolved to the appropriate concentrations in sterile PBS for the i.v. formulations (Table 2). The dosing solutions for each test compound were prepared on the basis of the relative content of the anatabine or nicotine base so that the final concentrations are reflective of the actual base concentration. Four aliquots of each dose level formulation were collected and stored at -80 °C. The test compound, corresponding dose level, number of animals, and sample collection times for Phase I of the study are shown in Table 3. The test compound, corresponding dose level, number of animals, and sample collection times for Phase II of the study are shown in Table 4. The physical signs of each animal were monitored following administration of the test compound.

The animals were weighed prior to dosing and received a single i.v. dose of either test compound at a volume of 5 mL/kg. Blood was collected via the venus plexus (retro-orbital) into tubes containing ($K_2$) EDTA. No more than 0.5 mL was collected per time point. For the 1440-minute time point of Phase I or the 30- and 360-minute time points of Phase II, the animals were euthanized, bled via cardiac puncture, and perfused. The brain was removed, weighed, homogenized in sterile 0.9% saline at a volume equal to its weight, and stored at -80 °C.

Plasma was separated according to the instructions for MICROTAINER® brand collection tubes (3 minutes, 2000x g). Plasma was decanted into microfuge tubes and stored at -80° C. Remaining test compound was stored at -80 °C.

**Analytical Methods**

The signal was optimized for each compound by electrospray ionization (ESI) positive or negative ionization mode. A single ion mode (SIM) scan was used to optimize the Fragmentor for the precursor ion and a product ion analysis was used to identify the best fragment for analysis and to optimize the collision energy. The fragment which gave the most sensitive and specific signal was chosen.

**Sample preparation.** Plasma and brain samples were treated with three volumes of methanol containing internal standard at 1 μM (either (+/-)-nicotine-3'-$d_3$ for nicotine or (*R*,*S*)-Antabine-2,4,5,6-$d_4$ for anatabine), incubated 10 min at 4 °C, and centrifuged. The amount of the test agent in the supernatant was determined by liquid chromatography tandem mass spectrometry (LC/MS/MS).

**Analysis.** Samples were analyzed by LC/MS/MS using an Agilent 6410 mass spectrometer coupled with an Agilent 1200 high pressure liquid chromatography (HPLC) and a CTC PAL chilled autosampler, all controlled by MassHunter software (Agilent). After separation on a hydrophilic interaction liquid chromatography (HILIC) HPLC column (Sepax) using an acetonitrile-ammonium acetate/acetic acid gradient system, peaks were analyzed by mass spectrometry (MS) using ESI ionization in multiple reaction monitoring (MRM) mode. MassHunter software was used to calculate the concentration of the test compounds in samples from the peak area using the appropriate calibration curves.

**Recovery.** Recovery standards were prepared by spiking blank matrix (plasma or brain homogenate) prior to deproteination or after with 23, 62, or 1667 ng/mL of test compound. Deproteination was done by adding 3 columns of methanol containing internal standard with centrifugation to pellet the precipitated protein. Recovery was calculated by dividing the area ratio (peak area of compound over internal standard of the precipitated sample over the recovery standard multiplied by 100. For example: area ratio of spiked plasma/area ratio of spiked deproteinated plasma x 100.

**Calibration samples.** Calibration curves were determined for both rat plasma and brain homogenate. Calibration samples were prepared by diluting a 50x stock solution of the test compound in PBS with blank matrix to the appropriate concentration and these samples were prepared as described above in the sample preparation. Stock solutions were prepared by serial dilution as shown in Table 5.

## Results

**Physical Signs.** All males and two females that received nicotine at 0.4 mg/kg experienced tremors immediately post dose and recovered within 2 to 4 minutes. One male (7C) and two females (8A and 8C) in this group also experienced labored breathing which lasted 2 to 4 minutes post dose. The same male (7C) was lethargic and recovered approximately 8 minutes post dose. All other animals in each dose group appeared normal following the administration of the test compounds.

**Method Development.** Table 6 shows the results of the LC/MS/MS method development for the determination of the appropriate ionization conditions and the mass to charge ratios (m/z) of the parent and product ions for anatabine and nicotine, and their deuterated analogues. The indicated product m/z ratios were used for the analysis of the relevant test samples.

The product ion spectra and sample chromatograms for each compound in Table 6 are shown in **FIGS. 12-19.** The limits of detection (LOD) of anatabine and nicotine and their lower (LLQ) and upper (ULQ) limits of quantitation were derived from the appropriate calibration curves for each test compound and are shown in Table 7.

Table 8 provides data on the per cent recovery of each test compound from either rat plasma or brain as a function of the given concentration. Except for the anatabine sample at the LOD and the nicotine samples in rat brain, recovery was generally greater than 90 per cent.

## Analysis of Dosing Solutions

Table 9 summarizes the analyses of the dosing solutions used in this study. The per cent differences between the actual and expected concentrations are shown. Except for the lowest dose of anatabine, which was 70 % of the expected concentration, the actual concentrations of test compounds were within 20 % of the expected levels.

## Plasma Pharmacokinetic Results & Analysis

Table 14 lists the plasma concentrations of anatabine and nicotine for all animals at each time point. Table 15 summarizes this data in terms of the mean plasma concentrations of the test compound at each time point for males, females and both genders combined. This data is presented graphically in **FIG. 7** and **FIG. 8** (semi-log plot). The 24-hr data points from all treatment groups were below the limits of quantitation. Between approximately 6 and 8 hours the plasma concentrations of nicotine and anatabine (0.1 mg/kg) were below the limits of quantitation.

Table 10 and Table 11 provide comparisons for several pharmacokinetic parameters between the different treatment groups and between male and female animals. Both nicotine and anatabine can be measured in rat plasma following a single i.v. bolus, and their concentrations appear to be dose-related. The elimination half-life ($t_{1/2}$) for each of the anatabine treatment groups was significantly greater than that for the nicotine treatment group (2.1x to 2.5x greater; 0.67 hr for nicotine compared to 1.44 to 1.68 hr for anatabine). The elimination half-lives were similar among the anatabine treatment groups. The longer half-life for anatabine is reflected in the longer mean residence times (MRT), which are about 2-fold longer for anatabine compared to nicotine. Finally, the apparent volume of distribution ($V_D$) was lower for the nicotine group compared to the anatabine treatment groups. Amongst the anatabine treatment groups, $V_D$ was significantly greater for the 0.1 mg/kg dose group compared to either of the two higher doses; however, it is not known whether this is a real difference or whether it is due to variability and the fewer number of measurable data points at the low dose.

Table 11 shows a comparison of these same parameters between male and female rats within each treatment group. There were no statistically significant differences between males and females except in the highest anatabine treatment group (1.0 mg/kg) where the females exhibited a longer elimination half-life and therefore, longer mean residence time than the males ($t_{1/2}$, 1.84 ± 0.16 hr and MRT, 2.80 ± 0.24 hr, females compared to $t_{1/2}$, 1.44 ± 0.08 and MRT, 2.18 ± 0.12 hr, males). This difference is apparent for all treatment groups, although it only achieved statistical significance in the highest anatabine group. The females in this treatment group also displayed a much greater overall exposure ($AUC_{0\rightarrow\infty}$) to anatabine than the male animals. This difference is depicted in **FIG. 9**, which shows the dose-exposure relationship for anatabine and nicotine. Overall, there appears to be a linear response between dose and exposure for anatabine; it is not possible to determine if the female animals display a non-linear response at high doses of anatabine.

**FIG. 11** shows the dose-concentration response for the 0.5-hour time point for males and females at each dose level. It appears that the brain levels of anatabine begin to level off between 0.75 mg/kg and 1.0 mg/kg.

Table 14 lists the concentrations of anatabine and nicotine in the brain extracts for all animals at each time point. Table 15 summarizes this data in terms of the mean concentrations of the test compound per gram of brain tissue at each time point for males, females and both genders combined. This data is presented graphically in **FIG. 10** and in tabular form in Table 12. After the 6-hour time point most concentrations were below the limits of quantitation; however, the test compound concentration was quantifiable in several samples at 24-hours.

**FIG. 11** shows the dose-concentration response for the 0.5-hour time point for males and females at each dose level. It appears that the brain levels of anatabine begin to level off between 0.75 mg/kg and 1.0 mg/kg.

**Discussion**

All males and two females that received nicotine at 0.4 mg/kg experienced tremors immediately post dose; however they recovered within 2 to 4 minutes. One male (7C) and two females (8A and 8C) in this group also experienced labored breathing which lasted 2 to 4 minutes post dose. The same male (7C) was lethargic and recovered approximately 8 minutes post dose. All animals in each of the anatabine dose groups appeared normal immediately following administration of the test compounds and no obvious adverse signs were observed.

Both nicotine and anatabine can be measured in rat plasma following a single, bolus, i.v. dose and their concentrations appear to be dose-related. The elimination half-life of anatabine is approximately 2- to 2.5-fold greater than that of nicotine, and this is also reflected in a longer mean residence time, which is approximately twice as long as that for nicotine. The 24-hr data points from all treatment groups were below the limits of quantitation and it appears that at the doses selected, the test compounds are cleared from rat plasma between 8 and 24 hours post-administration.

The apparent volume of distribution ($V_D$) was also significantly lower for the nicotine group compared to the anatabine treatment groups. Amongst the anatabine treatment groups, $V_D$ was significantly greater for the 0.1 mg/kg dose group compared to either of the two higher doses; however, it is not known whether this is a real difference or whether it is due to variability and the fewer number of measurable data points at the low dose.

When comparisons between male and female animals were conducted for these same parameters, within each treatment group, there were no statistically significant differences observed except for the highest anatabine treatment group (1.0 mg/kg) where the females exhibited a longer elimination half-life and therefore, longer mean residence time than the males ($t_{1/2}$, 1.84 ± 0.16 hr and MRT, 2.80 ± 0.24 hr, females compared to $t_{1/2}$, 1.44 ± 0.08 and MRT, 2.18 ± 0.12 hr, males). In fact, these differences between male and female animals were apparent for all treatment groups, although statistical significance was achieved only at the highest anatabine dose tested. The females in this treatment group also displayed a much greater overall exposure ($AUC_{0\rightarrow\infty}$) to anatabine than the male animals. Overall, there is a linear response between dose and plasma concentrations or exposure to anatabine in both male and female rats; although the response appears to be somewhat greater in female animals and is more pronounced at the higher dose levels. It is not possible to determine from the data if the female animals display a non-linear response at higher doses of anatabine.

Both anatabine and nicotine rapidly appear in brain tissue following i.v. administration. The concentrations of anatabine are dose-dependent but appear to level off between 0.75 mg/kg and 1.0 mg/kg. This observation is based on the levels measured only at the 0.5-hour time point and a greater number of time points are required for a more thorough evaluation. There were no statistically significant differences in the concentrations of either test compound in brain between male and female animals; however at each dose level the mean concentrations in the brains of females tended to be somewhat higher.

## EXAMPLE 4

### Toxicokinetic evaluation of single doses of anatabine and nicotine with a 14-day observation period

This example reports the evaluation of the toxicity of anatabine or nicotine for a period of fourteen days following a single intravenous injection in Sprague-Dawley rats. The toxicity of anatabine and nicotine was evaluated after a single intravenous (i.v.) injection in the rat. Anatabine was administered as a single intravenous injection at doses of 0.10, 0.75, or 1.5 mg/kg. Nicotine was administered as a single intravenous injection at a dose of 1.50 mg/kg. One control group of animals received a single i.v. dose of the vehicle at 5 mL/kg. Ten rats (5 males and 5 females) were dosed per group. Due to animal mortality in the nicotine-dosed group, the surviving animals were taken off study and a separate nicotine tolerability study was conducted. One female received a single i.v. dose of 1.25 mg/kg, and 3 females received a single i.v. dose of 1.0 mg/kg. Following the tolerability study, a group of 5 males and 5 females received a single i.v. dose of nicotine at 0.75 mg/kg.

All rats dosed with vehicle or anatabine, and the animals dosed with 0.75 mg/kg of nicotine were observed daily for 14 days. Body weight and food consumption was measured daily for 14 days. On day 15, urine was collected on all surviving animals. The animals were euthanized and bled via cardiac puncture, and blood was collected for analysis. Tissues were collected, weighed, evaluated for gross abnormalities, and stored in 10% neutral-buffered formalin.

All groups appeared normal immediately after dosing except for the animals dosed with 1.5 mg/kg of anatabine and those dosed with 1.5 mg/kg of nicotine. Both males and females dosed with 1.5 mg/kg of anatabine experienced tremors upon compound administration. The animals appeared normal by 15 minutes post dose. Upon completion of the 1.5 mg/kg dose of nicotine, tremors and rigidity were observed in all dosed animals. The tremors were more severe in the females. One male did not survive, whereas the other 4 appeared normal after 15 minutes. Three females were dosed and two died within 5 minutes of dosing; the remaining 2 females were not dosed due to the morbidity in the group. The surviving animals from this group were removed from study. These results suggest that both anatabine and nicotine affect both the peripheral and central nervous systems.

During the tolerability study, all rats (1 female dosed with 1.25 mg/kg of nicotine and 3 females dosed with 1.0 mg/kg of nicotine) experienced severe tremors upon completion of dosing, but all returned to normal by 20 minutes post dose. These animals were not included in the 14-day observation period.

Both males and females dosed with nicotine at 0.75 mg/kg experienced tremors upon compound administration but returned to normal within 15-20 minutes post dose. One male and two females died post dose. Surviving animals in all groups appeared normal throughout the 14-day observation period. The body weights for both male and female rats in the nicotine group were lower than those in the control and anatabine treatment groups; however, these were still within the study-specified range. Consequently body weight gain for this treatment group was also somewhat lower than the vehicle controls. Food consumption was similar among the groups over the 14-day period; however, consumption by males treated with 0.1 mg/kg or 1.5 mg/kg anatabine appeared to be somewhat higher than animals in the control group. This is not considered to be a treatment-related effect.

Hematology and blood chemistries for male and female animals were analyzed and evaluated for differences between the individual treatment groups and the relevant vehicle controls. All treatment groups showed no significant differences relative to the controls and/or the values were well within the normal ranges expected for this species. Similarly, no notable differences in any of the urinalysis parameters were observed between animals treated with either anatabine or nicotine, relative to the controls.

Anatabine or nicotine was dissolved to the appropriate concentrations in sterile PBS for the i.v. formulations (see Table 16). The dosing solutions for each test compound were prepared on the basis of the relative content of the anatabine or nicotine base so that the final concentrations reflect the actual base concentrations. Four aliquots of each dose formulation were collected and stored at -80 °C. The test compound, corresponding dose level, number of animals, and frequency of observations are shown in Table 17.

The animals were weighed prior to dosing and received a single i.v. dose via the lateral tail vein of either test compound or vehicle at a volume of 5 mL/kg. Due to animal mortality in the nicotine-dosed group (1.5 mg/kg), the surviving animals were taken off study and a separate nicotine tolerability study was conducted.

### Nicotine Tolerability Study

One female rat was dosed intravenously with 1.25 mg/kg of nicotine, and three females were received 1.0 mg/kg intravenously. Following the tolerability study, an additional group was added to the study. Five males and five females received a single intravenous dose of nicotine at 0.75 mg/kg. All animals were observed daily. Body weight and food consumption was measured daily, with any abnormal observations noted. Average daily body weights and food consumption was tabulated with standard deviation calculated.

On day 15, urine was collected on all surviving animals for urinalysis. The animals were euthanized and bled

via cardiac puncture. Blood was collected for hematology, clinical chemistry, and coagulation analysis. Tissues were collected, weighed, and stored in 10% neutral-buffered formalin for possible future analysis. The tests and tissues collected are summarized in Table 18.

## Results

### Dosing Solution Analysis

Table 19 summarizes the dosing solutions used during the conduct of this study. The per cent differences between the actual and expected concentrations of the test compounds are shown. The actual concentrations were within 20 per cent of the expected levels.

### General Observations

All groups appeared normal immediately after dosing except for the animals dosed with 1.5 mg/kg of anatabine and those dosed with 1.5 mg/kg of nicotine. Both males and females dosed with 1.5 mg/kg of anatabine experienced tremors upon compound administration. The animals appeared normal by 15 minutes post dose. Following administration of the 1.5 mg/kg dose of nicotine, tremors and rigidity were observed in all animals. The tremors were more severe in the females. One male did not survive, whereas the other 4 appeared normal after 15 minutes. Three females in this group were dosed and two died within 5 minutes of dosing; the remaining 2 females were not treated due to the observed morbidity in the group. The surviving animals from this group were removed from the study.

During the tolerability study, all rats (1 female dosed with 1.25 mg/kg of nicotine and 3 females dosed with 1.0 mg/kg of nicotine) experienced severe tremors upon completion of dosing, but all returned to normal by 20 minutes post dose. These animals were not included in the 14-day observation period.

Both males and females dosed with nicotine at 0.75 mg/kg experienced tremors upon compound administration, but returned to normal within 15-20 minutes post dose. One male and two females died post dose.

Surviving animals in all groups appeared normal throughout the 14-day observation period.

### Body Weights, Growth Rates and Food Consumption

The daily measured body weights for each animal are tabulated in Tables 28A-F and the average daily food consumption is summarized in Tables 29A, B. These data are summarized in Table 20 for the average weight gain over the 14-day observation period and the average daily food consumption, by treatment group and gender. FIG. 20 shows the mean body weights of animals in each treatment group on the day of dosing (Day 0) and for each day, thereafter.

The average weight gains for animals in each treatment group over the 14-day observation period were similar to those in the vehicle control group, except for the nicotine-dosed group of male animals that exhibited weight gains that were significantly lower than the controls. The mean increase in the weight of females of the nicotine-dosed group was also lower than that of the vehicle control, though not statistically significant at the 5 per cent level. It should be noted that the mean weights of the male and female animals in the nicotine-treated group at Day 0 were lower than their corresponding genders in the vehicle control. The difference for males was statistically significant (Vehicle: 234.6 $\pm$ 9.9 g *versus* Nicotine: 216.0 $\pm$ 6.2 g; $p$=0.014), although that for females was not (Vehicle: 209.8 $\pm$ 7.3 g *versus* Nicotine: 195.3 $\pm$ 10.4 g; $p$=0.058).

The average daily food consumption per animal was statistically higher in the males of the 0.1 mg/kg and 1.5 mg/kg anatabine treatment groups. This difference is not considered to be clinically significant or related to any treatment effects.

Overall, although some differences in the changes in weight and food consumption were statistically significant, they are not considered to be treatment-related.

### Necropsy Observations and Organ Weights

Upon necropsy and organ collection no noticeable differences or abnormalities were observed between the vehicle-dosed animals and the test compound-dosed animals. Individual organ weights can be found in Table 36. Several statistically significant differences in organ weights were noted (see Table 21 and Table 22); however, they do not appear to be dose-related and likely due to the small sample sizes and variability in the organ collection. In general, several organ weights tended to be lower in the nicotine-treated group, although this observation is likely related to the lower animal weights in this group relative to the controls.

### Hematology and Coagulation Parameters

Plasma samples collected for hematology were analyzed, and individual values for the various parameters for each animal are listed in Table 31 (normal ranges, Table 30) and these are summarized in terms of descriptive statistics in Table 23A, Table 23B, and Table 24. Also shown are statistical comparisons between the vehicle controls and the various treatment groups, subdivided by gender.

In general, there were few significant differences between the treatment groups and the vehicle control group for either gender. Female rats in 0.1 mg/kg anatabine group showed a small but statistically significant decrease in mean corpuscular hemoglobin concentration (MCHC) relative to the control; however, the values are still within the normal range for this species. Similarly, females in the 1.5 mg/kg anatabine and 0.75 mg/kg nicotine treatment groups showed small, but statistically significant decreases in mean corpuscular volume (MCV) and mean corpuscular hemoglobin (MCH), although these values were still within the normal range for this species as well.

Males and females in the 0.75 mg/kg and 1.5 mg/kg anatabine groups showed a statistically significant decrease in reticulocyte count compared to the control animals; however, these values are also well within the normal range for this parameter.

There were no notable differences in red blood cells, white blood cells, platelet counts, lymphocyte, monocyte, eosinophil and basophil counts, or neutrophil segmentation.

Individual values for the coagulation parameters activated partial thromboplastin (aPTT) and prothrombin times (PT) for each animal are listed in Table 34 (normal ranges, Table 30). These are summarized in terms of descriptive statistics in Table 25. Also shown are statistical comparisons between the vehicle controls and the various treatment groups, subdivided by gender. There were no significant differences in aPTT or PT between the vehicle control and each of the anatabine treatment groups; although the aPTT values for all these groups were outside the normal range. In both male and female animals of the nicotine group, however, aPTT was significantly lower relative to the vehicle control group, indicative of faster clotting times due to the intrinsic, contact activation pathway. The origin of this difference is not known, although the values are within the normal range for this species.

### Clinical Chemistry

Plasma samples collected for blood chemistries were analyzed, and individual values for the various parameters for each animal are listed in Table 33 (normal ranges, Table 32), and these are summarized in terms of descriptive statistics in Tables 26A, 26B, 27A, and 27B. Also shown are statistical comparisons between the vehicle controls and the various treatment groups, subdivided by gender.

Values for all clinical chemistry parameters were within the respective normal ranges. There were several parameters where statistically significant differences were noted between treatment groups and controls. Specifically, males treated with anatabine at 0.75 mg/kg and 1.5 mg/kg showed slight increases in albumin levels, as did females treated with 0.1 mg/kg and 0.75 mg/kg anatabine, but not at 1.5 mg/kg. Total protein was slightly increased in males in all anatabine treatment groups and the nicotine group relative to vehicles controls. In females, total protein was somewhat higher only in the 0.1 mg/kg anatabine and nicotine groups. Finally, as with total protein, globulins were marginally higher at all anatabine dose levels and the nicotine dose group in males. Globulins were also slightly higher in females in the 0.1 mg/kg anatabine and nicotine groups. The higher globulin levels, but not albumin, in the nicotine group is reflected in slightly lower A/G ratios, for both genders. Nevertheless, all the reported values for albumin, globulins and total protein were within the normal range for this species. There were small, but statistically significant differences noted for calcium levels in males in the nicotine-treated group and for sodium levels in males at 0.75 mg/kg and 1.5 mg/kg anatabine and females in the 1.5 mg/kg anatabine treatment groups. The values are well within normal ranges and therefore, not clinically significant.

### Urinalysis

Individual values of the urinalysis parameters for each animal are listed in Table 35. There were no notable differences between the active treatment groups and controls and the observations are all consistent with those expected for this species.

### Discussion

The toxicity of anatabine and nicotine was evaluated after a single intravenous (i.v.) injection in the rat. Anatabine was administered at doses of 0.10, 0.75, or 1.5 mg/kg. Nicotine was administered at a dose of 1.50 mg/kg, initially; however, due to mortality and significant adverse effects observed at this dose and at lower doses of 1.0 mg/kg and 1.25 mg/kg, a separate group was included in the study and dosed with nicotine at 0.75 mg/kg. One group of animals

received a single i.v. dose of the vehicle at 5 mL/kg. Ten rats (5 males and 5 females) were dosed per group.

All rats dosed with vehicle or anatabine, and the animals dosed with 0.75 mg/kg of nicotine were observed daily for 14 days. Body weight and food consumption was measured daily for 14 days. On day 15, urine was collected on all surviving animals. The animals were euthanized, bled via cardiac puncture, and blood was collected for analysis. Tissues were collected, weighed, any gross abnormalities were noted, and stored in 10% neutral-buffered formalin for possible future analysis.

All animals, at all dose levels of anatabine, survived the study; however, those in the 1.5 mg/kg anatabine group experienced tremors and shaking immediately after test compound administration, which lasted for approximately 15 minutes post-treatment. In the nicotine treatment group (0.75 mg/kg), one male animal and 2 females died following test compound administration, and all animals experienced tremors and shaking for up to 20 minutes post-administration. These results suggest that both anatabine and nicotine affect both the peripheral and central nervous systems.

The growth rates and food consumption in all anatabine treatment groups were similar to their appropriate male or female vehicle controls. Male rats in the nicotine treatment group had a slightly lower growth rate; however, this is unlikely to be related to the test compound. This group of animals began the study at a lower average weight than males in the control or anatabine treatment groups. The food consumption in males, in the 0.1 mg/kg and 1.5 mg/kg anatabine groups was somewhat higher than controls and although the result was statistically significant it is not likely to be related to an effect of the test compound.

At necropsy, no noticeable differences or gross abnormalities were observed in any of the organs collected between the vehicle-treated and the test compound-treated animals. Several statistically significant differences in organ weights were noted; however, they do not appear to be dose-related and are likely due to the small sample sizes and the inherent variability associated with organ collection. The weights of heart, liver and kidneys in males, and thymus and heart in females of the nicotine-treated group were significantly lower than those of the corresponding vehicle controls; however, this observation is likely related to the lower overall animal weights in this group relative to the controls.

The hematology parameters for all treatment groups and genders were within the normal ranges expected for this species or displayed no significant differences when compared to the vehicle controls. Activated partial thromboplastin and prothrombin times were similar for all anatabine treatment groups relative to the controls; however, they were higher than the expected normal range. Both males and females in the nicotine group displayed significantly shorter clotting times via the intrinsic or contact activation pathway (aPTT) compared to the relevant control animals; however, the values were within the normal ranges for this species. Clotting times via the extrinsic or tissue factor pathway as determined by prothrombin times (PT) were normal.

Values for all clinical chemistry parameters were within the respective normal ranges or showed no differences relative to the vehicle control group.

Evaluation of the individual urinalysis parameters for each animal showed no notable differences between the active treatment groups and controls.

## EXAMPLE 5

### Toxicokinetic evaluation in Sprague-Dawley rats of oral multi-dose administration of anatabine

This example reports the results of an evaluation of the pharmacokinetics of anatabine following multiple oral doses in Sprague-Dawley rats.

### Summary

The plasma pharmacokinetic profile of orally administered anatabine was investigated in the rat. This study consisted of two groups of 8 animals each, 4 males and 4 females. One group received a total of 0.6 mg anatabine per kilogram body weight (BW) and the second group received 6.0 mg anatabine per kilogram BW in three, divided, oral, doses of 0.2 mg/kg BW (0.6 mg total) or 2.0 mg/kg BW (6.0 mg total). The test compound was administered as anatabine polacrilex and each dose was administered at 0, 4, and 8 hours and was administered in a volume of 5 mL/kg BW. Blood was collected for plasma at 30, 60, 240, 270, 300, 480, 540, 600, 720 and 1440 minutes post initial dose.

All animals in both treatment groups appeared normal immediately following each administration of the test compound and no adverse signs were observed for the duration of the observation and plasma sampling period.

The mean time to maximal plasma concentration following the first two oral doses ranged from 0.50 to 0.88 $\pm$ 0.25 hr. There were no significant differences between gender or dose group. After the third dose of test compound, the mean time to maximal plasma concentration ranged from 1.00 to 2.00 $\pm$ 1.41 hr. Within each dose group there were no significant differences in $C_{p, max}$ between males and females and nor was there any significant change in this parameter over time. In females of the high dose group $C_{p, max}$ appeared to increase from 259.8 $\pm$ 35.4 ng/mL to 374.8 $\pm$ 122.9 ng/mL; however, the trend was not statistically significant.

There were two, observable, minima following the first two oral doses of anatabine polacrilex. In general, the minima were not significantly different from one another over time, except for females of the high dose group, which increased from 51.5 ± 26.0 ng/mL to 180 ± 31 ng/mL.

The total exposure, elimination half-lives, mean transit times and mean absorption times did not differ significantly between male and female rats within the two treatment groups. When these data are combined and grouped according to dose level the total exposure is significantly greater at the high dose as would be expected; however, the terminal elimination half-life is also significantly higher in the 6.0 mg/kg BW group compared to the 0.6 mg/kg BW dose group.

The overall elimination half-life of anatabine following the first oral dose was 1.93 ± 0.73 hr, the mean transit time was 3.01 ± 1.25 hr and the mean absorption time was 0.56 ± 1.25 hr. The mean absorption time of 0.56 compares favorably with the calculated $T_{max}$ values following the first two doses and indicates that the absorption of anatabine occurs within the first 30 to 60 minutes after oral administration.

Anatabine was stored at 4 °C, protected from light. The vehicle was sterile phosphate buffered saline (PBS) (Amresco). The test compound was formulated in sterile phosphate buffered saline (PBS) based on the content of anatabine base in the anatabine polacrilex. Two formulations were prepared; one for each of the two treatment groups. The test compound was formulated for each treatment group just prior to the first dose administration and constantly stirred until dosing was completed (Table 37). Four aliquots of each dose level formulation were collected and stored at - 80 °C. The test compound, corresponding dose level, and number of animals are shown in Table 38. The sample collection times are shown in Table 39.

The physical signs of each animal were monitored following administration of the test compound.

The animals were weighed prior to dosing and received three doses p.o. of test compound at a volume of 5 mL/kg. Blood was collected via the venus plexus (retro-orbital) into tubes containing ($K_2$) EDTA. No more than 0.5 mL was collected per time point. For the 1440-minute time point the animals were euthanized, and bled via cardiac puncture.

Plasma was separated as per package instructions for MICROTAINER® brand collection tubes (3 minutes, 2000x g). Plasma was decanted into microfuge tubes and stored at -80° C. Remaining test compound was placed at -80 °C .

Sample preparation. Plasma samples were treated with three volumes of methanol containing internal standard at 1 μM (*R*,*S*)-Antabine-2,4,5,6-$d_4$), incubated 10 min at 4 °C, and centrifuged. The amount of the test agent in the supernatant was determined by LC/MS/MS.

Analysis. Samples were analyzed by LC/MS/MS using an Agilent 6410 mass spectrometer coupled with an Agilent 1200 high pressure liquid chromatography (HPLC) and a CTC PAL chilled autosampler, all controlled by MassHunter software (Agilent). After separation on a Hydrophilic interaction liquid chromatography (HILIC) HPLC column (Sepax) using an acetonitrile-ammonium acetate/acetic acid gradient system, peaks were analyzed by mass spectrometry (MS) using ESI ionization in multiple reaction monitoring (MRM) mode. MassHunter software was used to calculate the concentration of the test compounds in samples from the peak area using the appropriate calibration curves.

**Calibration samples.** Calibration curves were determined in rat plasma. Calibration samples were prepared by diluting a 50x stock solution of the test compound in PBS with blank matrix to the appropriate concentration and these samples were prepared as described above in the sample preparation. Stock solutions were prepared by serial dilution as shown in Table 40.

**Data Analysis.** Descriptive statistics were calculated for all pharmacokinetic parameters. Elimination half-lives ($t_{1/2}$) were calculated by linear regression of logarithmically transformed plasma concentration data for each period between doses and following the final dose.

Total areas under the plasma concentration curves (AUC) and under the first moment curves (AUMC) were calculated using linear trapezoidal summation across all concentration time points as well as for intervals between each dose administration and following the final dose. For the interval following the first oral dose of anatabine polacrilex, mean transit times (MTT) were calculated from the corresponding ratio of AUMC to AUC. Mean absorption times (MAT) were calculated according to the following relation:

$$MAT = MTT - MRT,$$

where MRT represents the mean residence time. This was calculated from the mean residence times.

The statistical comparison of parameters between male and female animals was made using a two-tailed, unpaired, *t*-test with a 95 per cent confidence interval. Repeated-measures analysis of variance (ANOVA) was used for multiple comparisons of $C_{p,\,max}$ involving successive determinations on the same group of animals.

## Results

**Physical Signs.** No adverse events were observed.

**Method Development.** Table 41 shows the results of the LC/MS/MS method development for the determination of the appropriate ionization conditions and the mass to charge ratios (m/z) of the parent and product ions for anatabine and its deuterated analogue as determined above. The indicated product m/z ratios were used for the analysis of the relevant test samples.

See Example 3 for the product ion spectra and sample chromatograms for each compound in Table 41. The limits of detection (LOD), lower (LLQ), and upper (ULQ) limits of quantitation was derived from the calibration curve and are shown in Table 42.

**Analysis of Dosing Solutions.** Table 43 provides a summary of the analyses of the dosing solutions used during the conduct of this study. The per cent differences between the actual and expected concentrations are shown. The lowest dose of anatabine, which was 63% of the expected concentration and the high dose was 84% of the expected level.

**Plasma Pharmacokinetic Results & Analysis. FIG. 21A** and **FIG. 21B** show the mean plasma anatabine concentration-time curves for male and female rats in each of the two dose groups: 0.6 mg/kg (**FIG. 21A**) and 6.0 mg/kg BW (**FIG. 21B**). **FIG. 22A** and **FIG. 22B** show the same data with the values from both males and females combined. In each instance, three plasma concentration maxima can be observed corresponding to the administration of the three divided doses of anatabine polacrilex at 0, 4 and 8 hours. Similarly, two anatabine plasma concentration minima are found prior to administration of the final dose.

The mean maxima and minima anatabine plasma concentrations ($C_{p,\ max}$, $C_{p,\ min}$) for males and females in each dose group are recorded in Table 44 along with the mean time to maximal concentration following each of the three doses ($T_{max}$). Statistical comparisons between male and female animals within each dose group revealed no significant differences in any of the parameters, except for the second plasma concentration minimum ($C_{p,\ min(2)}$) in both treatment groups; 15.3 $\pm$ 5.5 ng/mL versus 7.5 $\pm$ 1.7 ng/mL in the 0.6 mg/kg BW treatment group, and 93 $\pm$ 16 ng/mL versus 180 $\pm$ 31 ng/mL in the 6.0 mg/kg BW treatment group. **FIG. 23A** and **FIG. 23B** show the data in Table 44 plotted as a function of time.

The times to reach maximal concentration generally occurred within 0.5 hr and 1.0 hr post administration in both treatment groups and for both genders, following doses one and two (see Table 45). After the third dose, $t_{max(3)}$ was generally between 1.0 and 2.0 hours post-administration; however, it should be noted that the earliest sampling point was at 1 hr following this dose.

Table 45 shows a comparison of the plasma concentration maxima and minima over time for male and female rats in both treatment groups. There were no statistically significant changes in any of these parameters except for the plasma concentration minima for female rats in the high dose group; $C_{p,\ min}$ increased from 51.5 $\pm$ 26.0 ng/mL to 180.0 $\pm$ 30.7 ng/mL.

The mean exposures (AUC), elimination half-lives ($t_{1/2}$), mean transit times (MTT) and mean absorption times (MAT) are reported in Table 46 for male and female animals in the two treatment groups. There are no significant differences between the genders in any parameter, at either dose level.

When the male and female data are combined, as shown in Table 47, there is a significant difference in total exposure as would be expected as a consequence of the two different dose levels ($AUC_{0\rightarrow\infty}$; 285 $\pm$ 77 ng·hr/mL versus 3496 $\pm$ 559 ng·hr/mL). There is also a significant difference in the terminal elimination half-life between the two treatment groups ($t_{1/2,\ terminal}$; 1.79 $\pm$ 0.64 hr versus 4.53 $\pm$ 1.77 hr), where $t_{1/2,\ terminal}$ refers to the elimination half-life following the final dose of anatabine polacrilex.

As there were no significant differences in the calculated elimination half-life, mean transit times and mean absorption times between treatment groups following the first dose of the test compound ($t_{1/2,\ 0\rightarrow 4}$, $MTT_{0\rightarrow 4}$, and $MAT_{0\rightarrow 4}$, respectively), the data at both dose levels were combined for males and females (see Table 48). There were no significant differences in these parameters between genders.

Table 49 provides animal weights and dosing times. Table 50 provides measured concentrations of anatabine in rat plasma samples at each time point. Table 51 provides mean concentration and description statistics of anatabine in plasma samples at each time point.

The data from both genders are also combined to give corresponding overall values. The calculated mean elimination half-life ($t_{1/2,\ 0\rightarrow 4}$) is 1.93 $\pm$ 0.73 hr, the mean transit time ($MTT_{0\rightarrow 4}$) is 3.01 $\pm$ 1.25 hr, and the mean absorption time ($MAT_{0\rightarrow 4}$) is 0.56 $\pm$ 1.25 hr.

## Discussion

This study evaluated the pharmacokinetics of anatabine in male and female Sprague-Dawley rats following the repeat-dose administration of anatabine polacrilex by oral gavage at two different dose levels. Anatabine was administered at 0.6 mg/kg BW in three, divided, doses of 0.2 mg/kg BW, or at 6.0 mg/kg BW in three, divided, doses of 2.0 mg/kg BW. Each dose was separated by an interval of four hours. All animals in both treatment groups appeared normal immediately following each administration of the test compound and no adverse signs were observed for the duration of the observation and plasma sampling period.

Anatabine concentrations can be measured in rat plasma following single and repeat oral dosing. The mean time to maximal plasma concentration following the first two oral doses ranged from 0.50 to 0.88 ± 0.25 hr. There were no significant differences between gender or dose group. After the third dose of test compound, the mean time to maximal plasma concentration ranged from 1.00 to 2.00 ± 1.41 hr; although in this instance the first time point measured was at one hour post-dose and therefore, it is possible that actual maximum occurred prior to this time. Within each dose group there were no significant differences in $C_{p,\ max}$ between males and females, nor was there any significant change in this parameter over time. In females of the high dose group $C_{p,\ max}$ appeared to increase from 259.8 ± 35.4 ng/mL to 374.8 ± 122.9 ng/mL; however, the trend was not statistically significant.

There were also two, observable, minima following the first two oral doses of anatabine polacrilex. In general, the minima were not significantly different from one another over time, except for females of the high dose group, which increased from 51.5 ± 26.0 ng/mL to 180 ± 31 ng/mL. Overall, these results suggest that with a 4-hour dosing interval, and after eight hours, near steady-state conditions appear have been achieved in male animals, whereas in females this may not yet be the case.

Within the two treatment groups, the total exposure, elimination half-lives, mean transit times and mean absorption times did not differ significantly between male and female rats. When these data are combined and grouped according to dose level the total exposure is significantly greater at the high dose as would be expected; however, the terminal elimination half-life is also significantly higher in the 6.0 mg/kg BW group compared to the 0.6 mg/kg BW dose group. The reason for this difference is not apparent since the mean transit times and mean absorption times did not differ significantly.

The elimination half-life, mean transit time and mean absorption time following the first oral dose of the test compound are the most reliable estimates of these parameters since the plasma concentration data are not confounded by carry-over amounts from a previous dose. The overall elimination half-life of anatabine following the first oral dose was 1.93 ± 0.73 hr, the mean transit time was 3.01 ± 1.25 hr and the mean absorption time was 0.56 ± 1.25 hr. The mean absorption time (also often called mean arrival time) of 0.56 compares favorably with the calculated $T_{max}$ values following the first two doses and indicates that the absorption of anatabine occurs within the first 30 to 60 minutes after oral administration.

**EXAMPLE 6**

**Treatment of thyroiditis**

This example illustrates administering anatabine for treating thyroiditis. A female patient, aged approximately 52, had been afflicted with Hashimoto's thyroiditis for approximately 5 years. The patient's condition had advanced to a state where the treating physician recommended a thyroid lobectomy. The patient orally ingested a tablet containing about 600 μg anatabine citrate, 20 times daily over a period of 30 days. At the conclusion of the treatment, inflammation of the thyroid was reduced to normal levels, such that the patient was no longer in need of a thyroid lobectomy. The patient continued the treatment for an additional 30 days, after which time the patient's voice distortion associated with thyroiditis was no longer present.

**Table 1.**

| | |
|---|---|
| headaches | Reuter et al. (2006) Headache 43: 426-427 |
| pain | Tegeder et al. (2004) Journal of Neuroscience 24: 1637-1645<br>Niederberger et al. (2004) Neurosci. 24, 1637-45<br>Niederberger et al. (2008) FASEB Journal 22:3432-3442 |
| complex regional pain syndrome | Hettne et al. (2007) Journal of Biomedical Discovery and Collaboration 2: 2 |
| cardiac hypertrophy | Purcell & Molkentin (2003) Circulation 108: 638-640<br>Freund et al. (2005) Circulation 111: 2319-2325<br>Sen & Roy (2005) American Journal of Physiology: Heart and Circulatory Physiology 289: H17-H19 |
| muscular dystrophy (type 2a) | Baghdiguian et al. (1999) Nature Medicine 5: 503-511 ; erratum in Nature Medicine 1999 Jul;5(7):849] |

(continued)

| | |
|---|---|
| headaches | Reuter et al. (2006) Headache 43: 426-427 |
| muscle wasting | Hasselgren (2007) Journal of Cellular Physiology 293: R1545-R1551 |
| catabolic disorders | Holmes-McNary (2002) Current Opinion in Clinical Nutrition and Metabolic Care 5: 255-263 |
| diabetes mellitus, type 1 | Ho & Bray (1999) Proceedings of the Society of Experimental Biology and Medicine 222: 205-213<br>Eldor et al. (2006) Proceedings of the National Academy of Sciences USA 103: 5072-5077 |
| diabetes mellitus, type 2 | Yuan et al. (2001) Science 293: 1673-1677<br>Lehrke et al. (2004) PLoS Medicine 1: e45<br>Chen F (2005) Biochemical and Biophysical Research Communications 332: 1-3 |
| obesity | Gil et al. (2007) British Journal of Nutition 98: S121-S126 |
| fetal growth retardation | Mammon et al. (2005) Review of Diabetic Studies 2 27-34 |
| hypercholesterolemia | Wilson et al. (2000) Atherosclerosis 148: 23-30 |
| atherosclerosis | Brand et al., J Clin Invest 1996;97:1715-1722.<br>Ross et al. (2001) American Journal of Clinical Pathology 116: S97-S107<br>Li & Gao (2005) Medical Hypotheses 64: 694-698 |
| heart disease | Valen et al. (2001) Journal of the American College of Cardiology 38: 307-314 |
| chronic heart failure | Frantz et al. (2003) Cardiovascular Research 57: 749-756 Gong et al. (2007) International Journal of Clinical Practice 61: 611-621 |
| ischemia/reperfusion | Toledo-Pereyra et al. (2004) Experimental and Clincal Transplantation 2: 174-177<br>Nichols (2004) Drug News and Perspectives 17: 99-104 Ridder & Schwaninger (2008) Neuroscience 158(3):995-1006 |
| stroke | Herrmann et al. (2005) Nature Medicine 11: 13322-1329 |
| cerebral aneurysm | Aoki et al. (2007) Circulation 116 2830-2840<br>Aoki et al. (2009) Arteriosclerosis, Thrombosis, and Vascular Biology 29: 1080-1086 |
| angina pectoris | Ritchie (1998) Circulation 98: 1707-1713 |
| pulmonary disease | Christman et al. (2000) Chest 117: 1482-1487 |
| cystic fibrosis | Bodas & Vij, Discovery Medicine, 9(47):346-356, April 2010<br>Pollard et al. (2005) Proteomics 5: 2210-2216 Carrabino et al. (2006) Journal of Cystic Fibrosis 5: 113-119<br>Rottner et al. (2007) FASEB Journal 21: 2939-2948 |
| acid-induced lung injury | Madjdpour et al. (2003) Anesthesiology 99: 1323-1332 |
| pulmonary hypertension | Sawada et al. (2007) Chest 132: 1265-1274 |
| chronic obstructive pulmonary disease (COPD) | Barnes (2002) Nature Reviews Drug Discovery 1: 437-446 Rahman & Kilty (2006) Current Drug Targets 7: 707-720 |
| adult respiratory distress syndrome | Schwartz et al., Crit Care Med 1996;24:1285-1292. |
| hyaline membrane disease | Cheah et al. (2005) Pediatric Research 57: 616-623 |
| kidney disease | Guijarro & Egido (2001) Kidney International 59: 415-424 Camici (2007) Medical Hypotheses 68: 900-905 Guzik & Harrison (2007) Circulation Research 101: 227-229 |

(continued)

| | |
|---|---|
| headaches | Reuter et al. (2006) Headache 43: 426-427 |
| glomerular disease | Zheng et al. (2005) Virchows Archiv 448: 172-183 |
| alcoholic liver disease | Zima & Kalousova (2005) Alcoholism, Clinical and Experimental Research 29 (Supplement): 110S-115S |
| leptospirosis renal disease | Yang et al. (2001) Nephrology Dialysis Transplantation 16 Suppl 5: 73-77 |
| gut diseases | Neurath et al. (1998) Gut 43: 856-860 |
| peritoneal endometriosis | Gonzalez-Ramos et al. (2007) Molecular Human Reproduction 13(7): 503-509 |
| skin diseases | Bell et al. (2003) Cell Signaling 15: 1-7 |
| nasal sinusitis | Xu et al. (2006) Neurochemistry Research 31: 1263-1269 |
| anhidrotic ecodermal dysplasia-id | Puel et al. (2005) Journal of Endotoxin Research 11: 220-224 |
| Behcet's disease | Todaro et al. (2005) Arthritis and Rheumatism 52: 2179-2191 |
| incontinentia pigmenti | Courtois & Israel (2000) Sci STKE Nov 14 (58): PE1 |
| tuberculosis | Zea et al. (2006) Journal of Infectious Diseases 194: 1385-1393 |
| asthma | Barnes, Am J Respir Crit Care Med 1996;154:S21-S27 Stacey et al., Biochem Biophys Res Commun 1997;236:522-526<br>Pahl & Szelenyi (2002) Inflammation Research 51: 273-282 |
| arthritis | Roshak et al. (2002) Current Opinion in Pharmacology 2: 316-321<br>Roman-Blas & Jimenez (2006) Osteoarthritis and Cartilage 14 839-848<br>Aud & Peng (2006) Nature Clinical Practice: Rheumatology 2: 434-442<br>Okamoto (2006) Endocrine, Metabolic & Immune Disorders: Drug Targets 6: 359-372 |
| rheumatoid arthritis | Handel et al., Arthritis Rheum 1995;38:1762-1770<br>Sakurada et al., Int Immunol 1996;8:1483-1493<br>Roshak et al., J Biol Chem 1996;271:31496- 31501. |
| Crohn's disease | Pena & Penate (2002) Revista Espanola d Enfermedades Digestivas 94: 351-360 |
| colitis | Chen et al. (2005) World Journal of Gastroenterology 11: 1508-1514 |
| ulcerative colitis | Barnes & Karin, NEJM 336, 1066-71, 1997 |
| ocular allergy | Bielory et al. (2002) Current Opinion in Allergy and Clinical Immunology 2: 435-445 |
| glaucoma | Zhou et al. (2005) Journal of Biological Chemistry 280: 31240-31248 |
| appendicitis | Pennington et al. (2000) American Surgery 66: 914-918 |
| Paget's disease | Lin et al. (2007) Journal of Dermatological Science 45: 187-192 |
| pancreatitis | Weber & Adler (2001) Pancreatology 1: 356-362 Gray et al. (2006) Pancreas 33: 260-267 |
| periodonitis | Nichols et al. (2001) Annals of Periodontology 6: 20-29 ; Ambili et al. (2005) Journal of Periodontology 76: 1148-1153 |
| endometriosis | Guo (2006) Gynecology and Obstetrics Investigations 63: 71-97<br>Celik et al. (2008) Human Reproduction 23: 2458-2465 |

(continued)

| | |
|---|---|
| headaches | Reuter et al. (2006) Headache 43: 426-427 |
| inflammatory bowel disease | Neurath et al., Nature Med 1996;2:998-1004<br>Dijkstra et al. (2002) Scandinavian Journal of Gastroenterology Supplement 236: 37-41<br>Atreya et al. (2008) Journal of Internal Medicine 263: 591-596 |
| inflammatory lung disease | Park & Christman (2006) Current Drug Targets 7: 661-668 |
| sepsis | Wratten et al. (2001) Contributions to Nephrology 132: 400-414 |
| septic shock | Mukaida et al., J Leukoc Res 1996;59:145-151 Bohrer et al., J Clin Invest 1997;100:972-985. |
| silica-induced diseases | Chen & Shi 2002) Molecular and Cellular Biochemistry 234-235: 169-176 |
| sleep apnea | Lavie (2003) Sleep Medicine Review 7: 35-51 |
| viral infection (e.g., rhinovirus, influenzavirus) | Barnes & Karin, NEJM 336, 1066-71, 1997 |
| aids (HIV-1) | Hiscott et al. (2001) Journal of Clinical Investigation 107: 143-151 |
| allograft rejection | Lee & Burckart, J. Clin. Pharmacol. 38, 981-93, 1998<br>Bach et al., Nature Med 13, 944-48, 1997A<br>Bach et al., Transplant Proc 29, 56-58, 1997B |
| autoimmunity | Hayashi & Faustman (2002) Diabetes Technology & Therapeutics 2: 415-428 |
| anti phospholipid syndrome | Lopez-Pedrera et al. (2005) Arthritis and Rheumatism 54: 301-311 |
| lupus | Kammer & Tsokos (2002) Current Directions in Autoimmunity 5: 131-150 |
| lupus nephritis | Zheng et al. (2006) Human Pathology 37: 637-647 |
| chronic disease syndrome | Maes et al. (2007) Neuro Endocinol Letters 28: 456-462 |
| familial Mediterranean fever | Onen (2006) Rheumatology International 26: 489-496 |
| hereditary periodic fever syndrome | Jeru et al. (2008) Proceedings of the National Academy of Sciences USA 105, 1614-1619 |
| psychosocial stress diseases | Bierhaus et al. (2004) Pediatic Nephrology 19: 1189-1191 |
| neuropathological diseases | Cechetto (2001) Progress in Brain Research 132: 391-404 |
| familial amyloidotic polyneuropathy | Mazzeo et al. (2004) Archives of Neurology 61: 1097-1102 |
| traumatic brain injury | Hang et al. (2005) World Journal of Gastroenterology 11: 1149-1154 |
| spinal cord injury | Brambilla et al. (2005) Journal of Experimental Medicine 202: 145-156 |
| Parkinson's disease | Soos et al. (2004) Neuroreport 15 1715-1718 |
| multiple sclerosis | Satoh et al. (2007) Neuroscience Letters 422: 30-33 |
| rheumatic disease | Okamoto T (2006) Endocrine, Metabolic & Immune Disorders: Drug Targets 6: 359-372 |
| Alzheimer's disease | Mattson & Camandola (2001) Journal of Clinical Investigation 107: 247-254<br>Kaltschmidt et al., Proc. Natl. Acad. Sci. USA 94:2642-2647, 1997<br>Lukiw, J. Biol. Chem. 2008 Nov 14;283(46):31315-22. Epub 2008 Sep 18 |
| amyotrophic lateral sclerosis | Xu et al. (2006) Neurochemistry Research 31: 1263-1269 |
| Huntington's disease | Khoshnan et al. (2004) Journal of Neuroscience 24: 7999-8008 |
| retinal disease | Kitaoka et al. (2004) Brain Research Molecular Brain Research 131: 8-16 |
| cataracts | Yang et al. (2006) Chinese Journal of Traumatology 9: 86-90 |
| hearing loss | Merchant et al. (2005) Otology and Neurotology 26: 151-160 |

(continued)

| | |
|---|---|
| headaches | Reuter et al. (2006) Headache 43: 426-427 |
| cancer | Gilmore et al. (2002) Cancer Letters 181: 1-9 |
| solid tumors (generally) | Pacifico & Leonardi (2006) Biochemical Pharmacology 72: 1142-1152 |
| breast cancer | akshatri et al. (1997) Molecular and Cellular Biology 17: 3629-3639 |
| cervical cancer | Nair et al. (2003) Oncogene 22: 50-58 |
| ovarian cancer | Lin et al., Clin. Cancer Res. 13, 3423-30, 2007<br>Samanta et al., J. Biol. Chem. 279, 7576-83, 2004 |
| adenocarcinoma | Dejardin et al. (1999) Oncogene 18: 2567-2577 |
| vulvar cancer | Seppanen & Vihko (2000) Immunology Letters 74: 103-109 |
| prostate cancer | Huang et al. (2001) Oncogene 20: 4188-4197 |
| kidney cancer | Oya et al. (2001) Oncogene 20: 3888-3896<br>Oya et al. (2003) Carcinogenesis 24: 377-384 |
| bladder cancer | Horiguchi et al. (2003) Expert Reviews in Anticancer Therapy 3: 793-798 |
| lung cancer | Tichelaar et al. (2004) Chest 125: 153S |
| mesothelioma | Bertino et al. (2007) International Journal of Cancer 121: 2766-2774 |
| non small-cell lung cancer | Zhang et al. (2006) Annals of Thoracic Surgery 82 243-248 |
| liver cancer | Tai et al. (2000) Cancer 89: 2274-2281 |
| pancreatic cancer | Wang et al. (1999) Clinical Cancer Research 5: 119-127<br>Zhang & Rigas (2006) International Journal of Oncology 29: 185-192 |
| esophageal/gastric cancer | Sutter et al. (2004) Onkologie 27: 17-21 Jackson & Evers (2006) Cancer Treatment and Research 130:39-65 |
| laryngeal cancer | Zhu et al. (2004) Lin Chuang Er Bi Yan Hou Ke Za Zhi 18: 745-6, 766 |
| stomach cancer | Sasaki et al. (2001) Clinical Cancer Research 7: 4136-4142 |
| colon cancer | Lind et al. (2001) Surgery 130: 363-369 |
| thyroid cancer | Visconti et al. (1997) Oncogene 15: 1987-1994 |
| parathyroid cancer | Corbetta et al. (2005) Endocrine Related Cancer 12: 929-937 |
| melanoma | Yang & Richmond (2001) Cancer Research 61: 4901-4909 Amiri KI and Richmond A (2005) Cancer Metastasis Reviews 24: 301-313 |
| squamous cell carcinoma | Loercher et al. (2004) Cancer Research 64: 6511-6523 |
| head and neck cancer | Ondrey et al. (1999) Molecular Carcinogenesis 26: 119-129<br>Jackson-Bernitsas et al. (2007) Oncogene 26: 1385-1397 |
| endometrial (uterine) cancer | Pallares et al. (2004) Journal of Pathology 204: 595-577 |
| cylindromatosis | Kovalenko et al. (2003) Nature 424: 801-805 |
| trichoepithelioma | Almeida et al. (2008) Dermatol. 127, 587-93 |
| Hilar cholangiocarcinoma | Chen et al. (2005) World Journal of Gastroenterology 11: 726-728 |
| oral carcinoma | Nakayama et al. (2001) Cancer 92: 3037-3044 Ruan et al. (2007) Phytotherapy Research 22, 407-15 |
| astrocytoma/glioblastoma | Hayashi et al. (2001) Neurologia Medico-Chirufica 41: 187-195<br>Smith et al. (2007) Molecular and Cellular Biochemistry 307:1-2, 141-147 |
| neuroblastoma | Bian et al. (2002) Journal of Biological Chemistry 277: 42144-42150 |
| glioblastoma | Raychaudhuri et al. (2007) Journal of Neurooncology 85: 39-47 |

(continued)

| | |
|---|---|
| headaches | Reuter et al. (2006) Headache 43: 426-427 |
| Hodgkin's lymphoma | Bargou et al. (1996) Blood 87: 4340-4347 Bargou et al. (1997) Journal of Clinical Investigation 100: 2961-2969 |
| acute lymphoblastic leukemia | Kordes et al. (2000) Leukemia 14: 399-402 |
| acute myelogenous leukemia | Guzman et al. (2001) Blood 98: 2301-2307 |
| acute T-cell leukemia (+/-HTLV-1) | Arima & Tei (2001) Leukemia and Lymphoma 40: 267-278 |
| acute non-lymphocytic leukemia | Lei & Zhao (2007) Zhongguo Shi Yan Xue Za Zhi 15 253-257 |
| chronic lymphocytic leukemia | Furman et al. (2000) Journal of Immunology 164: 2200-2206 |
| Burkitts lymphoma (EBV) | Knecht et al. (2001) Oncology 60: 289-302 |
| mantle cell lymphoma | Martinez (2003) Cancer Research 63: 8226-8232 |
| myelodysplastic syndrome | Fabre et al. (2007) Oncogene 26: 4071-4083 |
| multiple myeloma | Gilmore (2007) Cancer Cell 12 95-97 Berenson et al.(2001) Seminars in Oncology 28: 626-633 |
| diffuse large b-cell lymphoma | Davis et al. (2001) Journal of Experimental Medicine 194: 1861-1874 |
| MALT lymphoma | Sagaert et al. (2007) Leukemia 21: 389-396 |
| Waldenstrom macroglobulinemia | Leleu et al. (2008) Blood 111: 5068-5077 |
| osteoporosis | Ray & Cohn, J. Clin. Invest. 104, 985-993, 1999; Christman et al., Chest 117, 1482-1487, 2000 |

**Table 2. Dosing solutions**

| Test compound | Percentage content of anatabine or nicotine base | Dose level (mg/kg) | Test compound concentration(total) (mg/mL) | Test compound concentration (base) (mg/mL) | Injection volume (mL/kg) |
|---|---|---|---|---|---|
| Anatabine | 41.6 | 0.10 | 0.048 | 0.020 | 5 |
| Anatabine | 41.6 | 0.75 | 0.36 | 0.15 | 5 |
| Anatabine | 41.6 | 1.0 | 0.48 | 0.20 | 5 |
| Nicotine | 35.1 | 0.4 | 0.23 | 0.081 | 5 |

**Table 3. Phase I**

| Test compound | Route | Dose level (mg/kg) | Number of animals (M/F) | Collection times (minutes)[a] |
|---|---|---|---|---|
| Anatabine | i.v. | 0.10 | 3/3 | 15, 30, 60, 90, 120, 240, 360, 480, 1440 |
| Anatabine | i.v. | 0.75 | 3/3 | 15, 30, 60, 90, 120, 240, 360, 480, 1440 |
| Anatabine | i.v. | 1.0 | 3/3 | 15, 30, 60, 90, 120, 240, 360, 480, 1440 |
| Nicotine | i.v. | 0.4 | 3/3 | 15, 30, 60, 90, 120, 240, 360, 480, 1440 |
| [a] Plasma samples were collected at all time points,. Brain tissue was collected at 1440 minutes. | | | | |

**Table 4. Phase II**

| Test compound | Route | Dose level (mg/kg) | Number of animals per time point (M/F) | Collection times (minutes)[a] |
|---|---|---|---|---|
| Anatabine | i.v. | 0.10 | 3/3 | 30, 360 |

(continued)

| Test compound | Route | Dose level (mg/kg) | Number of animals per time point (M/F) | Collection times (minutes)[a] |
|---|---|---|---|---|
| Anatabine | i.v. | 0.75 | 3/3 | 30, 360 |
| Anatabine | i.v. | 1.0 | 3/3 | 30, 360 |
| Nicotine | i.v. | 0.4 | 3/3 | 30, 360 |
| [a] Plasma samples and brain tissue were collected at all time points. | | | | |

**Table 5. Calibration Curve Concentrations**

| nominal concentration (ng/mL) | stock concentration (μg/mL) |
|---|---|
| 5000 | 250 |
| 1667 | 83.3 |
| 555.5 | 27.8 |
| 185.2 | 9.3 |
| 61.7 | 3.1 |
| 20.6 | 1.0 |
| 6.9 | 0.34 |
| 2.3 | 0.11 |
| 0.76 | 0.038 |
| 0.25 | 0.013 |

**Table 6. LC/MS/MS ionization conditions and identity of parent and product ions**

| Compound | MW | Polarization | Precursor m/z | Product m/z | Collision energy (V) |
|---|---|---|---|---|---|
| Anatabine | 160.2 | Positive | 161.1 | 115.1 | 28 |
| Nicotine | 162.3 | Positive | 163.1 | 117.1 | 28 |
| (+/-)-nicotine-3'-$d_3$ | 165.25 | Positive | 166.1 | 118 | 28 |
| (*R*,*S*)-Antabine-2,4,5,6-$d_4$ | 164.24 | Positive | 165.1 | 148.1 | 20 |

**Table 7. Limits of Detection and Calibration Curves**

| Sample | Limit of Detection (LOD) (ng/mL) | Lower Limit of Quantitation (LLQ) (ng/mL) | Upper Limit of Quantitation (ULQ) (ng/mL) |
|---|---|---|---|
| Anatabine in rat plasma | 0.76 | 2.3 | 5000 |
| Anatabine in rat brain | 0.76 | 2.3 | 5000 |
| Nicotine in rat plasma | 0.76 | 2.3 | 5000 |
| Nicotine in rat brain | 0.76 | 2.3 | 5000 |

**Table 8. Recovery from Plasma**

| | % Recovery at Given Concentrations | | |
|---|---|---|---|
| Sample | 2.3 ng/mL | 62 ng/mL | 1667 ng/mL |
| Anatabine in rat plasma | 74 | 96 | ND[a] |
| Anatabine in rat brain | ND | 96 | 90 |
| Nicotine in rat plasma | ND | 105 | 104 |
| Nicotine in rat brain | ND | 78 | 84 |
| [a]ND - not determined; two points per condition were evaluated for measuring recovery. | | | |

**Table 9. Dosing Solution Analysis**

| Compound | Dose (mg/kg) | Expected Concentration (mg/mL) | Actual Concentration (mg/mL) | Actual Concentration relative to Expected (%) |
|---|---|---|---|---|
| Nicotine | 0.4 | 0.081 | 0.096 | 118.5 |
| Nicotine | 0.4 | 0.081 | 0.096 | 118.5 |
| Anatabine | 0.1 | 0.02 | 0.014 | 70 |
| Anatabine | 0.75 | 0.15 | 0.135 | 90 |
| Anatabine | 1 | 0.2 | 0.177 | 88.5 |
| Anatabine | 0.1 | 0.02 | 0.015 | 75 |
| Anatabine | 0.75 | 0.15 | 0.133 | 88.7 |
| Anatabine | 1 | 0.2 | 0.162 | 81 |

**Table 10. Statistical Comparison of the Pharmacokinetic Parameters**

<table>
<tr><th rowspan="2">Parameter</th><th colspan="2">Nicotine (0.4 mg/kg)</th><th colspan="2">Anatabine (0.1 mg/kg)</th><th colspan="2">Anatabine (0.75 mg/kg)</th><th>Anatabine (1.0 mg/kg)</th></tr>
<tr><th>M+F</th><th>p</th><th>M+F</th><th>p</th><th>M+F</th><th>p</th><th>M+F</th></tr>
<tr><td>$AUC_{0\to\infty}$ (ng·hr/mL)</td><td>156.5 ± 32.6</td><td></td><td>35.0 ± 11.6</td><td></td><td>504.4 ± 63.5</td><td></td><td>710.07 ± 88.4</td></tr>
<tr><td rowspan="3">$t_{1/2}$ (hr)</td><td rowspan="3">0.67 ± 0.07</td><td>0.003[a]</td><td rowspan="3">1.44 ± 0.48</td><td>0.25[d]</td><td rowspan="3">1.68 ± 0.09</td><td rowspan="2">0.69[f]</td><td rowspan="3">1.64 ± 0.25</td></tr>
<tr><td><0.001[b]</td><td>0.39[e]</td></tr>
<tr><td><0.001[c]</td><td></td><td></td></tr>
<tr><td rowspan="3">MRT (hr)</td><td rowspan="3">1.22 ± 0.13</td><td>0.004[a]</td><td rowspan="3">2.29 ± 0.07</td><td>0.35[d]</td><td rowspan="3">2.58 ± 0.14</td><td>0.63[f]</td><td rowspan="3">2.49 ± 0.38</td></tr>
<tr><td><0.001[b]</td><td>0.55[e]</td><td></td></tr>
<tr><td><0.001[c]</td><td></td><td></td></tr>
</table>

(continued)

| Parameter | Nicotine (0.4 mg/kg) | | Anatabine (0.1 mg/kg) | | Anatabine (0.75 mg/kg) | | Anatabine (1.0 mg/kg) |
|---|---|---|---|---|---|---|---|
| | M+F | p | M+F | p | M+F | p | M+F |
| $V_D$ (L/kg) | 2.06 ± 0.13 | <0.001[a] | 6.08 ± 0.45 | <0.001[d] | 3.33 ± 0.13 | 0.15[f] | 3.08 ± 0.16 |
| | | <0.001[b] | | <0.001[e] | | | |
| | | <0.001[c] | | | | | |

[a] Nicotine vs. Anatabine (0.1 mg/kg)
[b] Nicotine vs. Anatabine (0.75 mg/kg)
[c] Nicotine vs. Anatabine (1.0 mg/kg)
[d] Anatabine (0.1 mg/kg) vs. Anatabine (0.75 mg/kg)
[e] Anatabine (0.1 mg/kg) vs. Anatabine (1.0 mg/kg)
[f] Anatabine (0.75 mg/kg) vs. Anatabine (1.0 mg/kg)

**Table 11. Comparison of Pharmacokinetic Parameters (± Std Dev) between male and female animals in each Treatment Group**

| Parameter | Nicotine (0.4 mg/kg) | | | Anatabine (0.1 mg/kg) | | | Anatabine (0.75 mg/kg) | | | Anatabine (1.0 mg/kg) | | |
|---|---|---|---|---|---|---|---|---|---|---|---|---|
| | male | female | p | male | Female | p | male | female | p | male | female | p |
| $AUC_{0\to\infty}$ (ng·hr/mL) | 140.5 ± 5.8 | 172.6 ± 43.1 | 0.27 | 32.0 ± 7.7 | 37.9 ± 15.9 | 0.59 | 464.4 ± 36.2 | 544.5 ± 62.7 | 0.13 | 631.3 ± 28.2 | 788.9 + 9.3 | <0.001 |
| $t_{1/2}$ (hr) | 0.66 ± 0.11 | 0.68 ± 0.02 | 0.81 | 1.25 ± 0.32 | 1.64 ± 0.59 | 0.37 | 1.64 ± 0.12 | 1.73 ±0.03 | 0.28 | 1.44 ± 0.08 | 1.84 ± 0.16 | 0.02 |
| MRT (hr) | 1.21 ± 0.20 | 1.22 ± 0.05 | 0.90 | 2.03 ± 0.41 | 2.55 ±0.93 | 0.42 | 2.50 ± 0.19 | 2.65 ±0.01 | 0.25 | 2.18 ± 0.12 | 2.80 ± 0.24 | 0.02 |
| $V_D$ (L/kg) | 2.10 ± 0.20 | 1.91 ± 0.17 | 0.54 | 5.29 ± 0.49 | 6.52 ± 0.66 | 0.22 | 3.48 ± 0.18 | 3.19 ± 0.15 | 0.38 | 3.01 ±0.12 | 3.16 ± 0.15 | 0.29 |

**Table 12. Mean (ng/g ± Std Dev) concentrations of nicotine and anatabine in rat brain extracts following a single, bolus, i.v. dose**

| Treatment Group | Nicotine | | Anatabine | | Anatabine | | Anatabine | |
|---|---|---|---|---|---|---|---|---|
| | 0.4 mg/kg | | 0.1 mg/kg | | 0.75 mg/kg | | 1.0 mg/kg | |
| Time (hr) | Male | Female | Male | Female | Male | Female | Male | Female |
| 0.5 | 94.3 ± 20.6 | 120.0 ± 3.0 | 20.0 ± 1.4 | 29.7 ± 4.5 | 276.0 ± 37.0 | 314.7 ± 67.9 | 323.0 ± 35.9 | 346.0 ± 56.7 |
| 6 | 6.0 ± 1.4 | 5.0 | 3.0 | 3.7 ± 1.5 | 21.3 ± 5.1 | 21.0 ± 6.2 | 5.0 ± 2.6 | 6.0 ± 2.8 |
| 24 | 6.3 ± 2.3 | 7.0 | 3.0 | 2.0 | <LLQ | <LLQ | 2.5 ± 0.7 | <LLQ |

## Table 13A.

**Rat PK i.v. dose -Brain Collection**

Compound : Anatabine, Nicoti Dose : 5 mL/kg

Route : i.v., PBS

| | | | Dose | | 24 hr | | |
|---|---|---|---|---|---|---|---|
| Cmpnd | Rat | B.W. (g) | volume (mL) | time | time | Brain wt (g) | Volume (mL) |
| 1 MALE Anatabine 0.1 mg/kg | A | 245 | 1.23 | 8:17 | 8:17 | 1.74 | 1.74 |
| | B | 247 | 1.23 | 8:19 | 8:19 | 1.88 | 1.88 |
| | C | 238 | 1.20 | 8:21 | 8:21 | 1.80 | 1.80 |
| 2 FEMALE Anatabine 0.1 mg/kg | A | 205 | 1.03 | 8:23 | 8:23 | 1.68 | 1.68 |
| | B | 211 | 1.05 | 8:25 | 8:25 | 1.83 | 1.83 |
| | C | 202 | 1.00 | 8:27 | 8:27 | 1.66 | 1.66 |
| 3 MALE Anatabine 0.75 mg/kg | A | 255 | 1.28 | 8:29 | 8:29 | 1.90 | 1.90 |
| | B | 223 | 1.13 | 8:31 | 8:31 | 1.82 | 1.82 |
| | C | 242 | 1.20 | 8:33 | 8:33 | 1.84 | 1.84 |
| 4 FEMALE Anatabine 0.75 mg/kg | A | 204 | 1.03 | 8:35 | 8:35 | 1.82 | 1.82 |
| | B | 205 | 1.03 | 8:37 | 8:37 | 1.82 | 1.82 |
| | C | 210 | 1.05 | 8:39 | 8:39 | 1.71 | 1.71 |
| 5 MALE Anatabine 1.0 mg/kg | A | 242 | 1.20 | 8:41 | 8:41 | 1.83 | 1.83 |
| | B | 251 | 1.25 | 8:43 | 8:43 | 1.85 | 1.85 |
| | C | 246 | 1.23 | 8:45 | 8:45 | 1.90 | 1.90 |
| 6 FEMALE Anatabine 1.0 mg/kg | A | 213 | 1.08 | 8:47 | 8:47 | 1.95 | 1.95 |
| | B | 218 | 1.10 | 8:49 | 8:49 | 1.75 | 1.75 |
| | C | 219 | 1.10 | 8:51 | 8:51 | 1.91 | 1.91 |
| 7 MALE Nicotine 0.4 mg/kg | A | 242 | 1.20 | 8:54 | 8:54 | 2.03 | 2.03 |
| | B | 241 | 1.20 | 8:56 | 8:56 | 1.98 | 1.98 |
| | C | 252 | 1.25 | 8:58 | 8:58 | 1.86 | 1.86 |
| 8 FEMALE Nicotine 0.4 mg/kg | A | 219 | 1.10 | 9:00 | 9:00 | 1.82 | 1.82 |
| | B | 215 | 1.08 | 9:02 | 9:02 | 1.87 | 1.87 |
| | C | 220 | 1.10 | 9:04 | 9:04 | 1.87 | 1.87 |
| Control Male | 1 | N/A | N/A | N/A | N/A | 1.83 | 1.83 |
| | 2 | N/A | N/A | N/A | N/A | 1.94 | 1.94 |
| | 3 | N/A | N/A | N/A | N/A | 1.82 | 1.82 |

## Table 13B. Animal Weights and Dosing Times

**Rat PK i.v. dose -Brain Collection**

Dose : 5 mL/kg

Compound : Anatabine, Nicotine

Route : i.v., PBS

| | | | Dose | | 0.5 hour | | |
|---|---|---|---|---|---|---|---|
| Cmpnd | Rat | B.W. (g) | volume (mL) | time | time | Brain wt (g) | Volume (mL) |
| 1 | D | 264 | 1.30 | 12:38 | 13:08 | 1.58 | 1.58 |
| MALE | E | 270 | 1.35 | 12:41 | 13:11 | 1.54 | 1.54 |
| Anatabine 0.1 mg/kg | F | 252 | 1.26 | 12:45 | 13:15 | 1.69 | 1.69 |
| 2 | D | 220 | 1.10 | 12:46 | 13:16 | 1.78 | 1.78 |
| FEMALE | E | 206 | 1.03 | 12:50 | 13:20 | 1.54 | 1.54 |
| Anatabine 0.1 mg/kg | F | 214 | 1.08 | 12:52 | 13:22 | 1.69 | 1.69 |
| 3 | D | 259 | 1.30 | 12:56 | 13:26 | 1.70 | 1.70 |
| MALE | E | 266 | 1.33 | 12:58 | 13:28 | 1.66 | 1.66 |
| Anatabine 0.75 mg/kg | F | 264 | 1.33 | 13:03 | 13:33 | 1.46 | 1.46 |
| 4 | D | 208 | 1.05 | 13:05 | 13:35 | 1.81 | 1.81 |
| FEMALE | E | 219 | 1.10 | 13:09 | 13:39 | 1.84 | 1.84 |
| Anatabine 0.75 mg/kg | F | 223 | 1.13 | 13:12 | 13:42 | 1.69 | 1.69 |
| 5 | D | 276 | 1.38 | 13:16 | 13:46 | 2.00 | 2.00 |
| MALE | E | 252 | 1.25 | 13:19 | 13:49 | 1.84 | 1.84 |
| Anatabine 1.0 mg/kg | F | 255 | 1.28 | 13:22 | 13:52 | 1.68 | 1.68 |
| 6 | D | 212 | 1.05 | 13:25 | 13:55 | 1.66 | 1.66 |
| FEMALE | E | 225 | 1.13 | 13:29 | 13:59 | 1.56 | 1.56 |
| Anatabine 1.0 mg/kg | F | 236 | 1.18 | 13:32 | 14:02 | 1.79 | 1.79 |
| 7 | D | 273 | 1.38 | 13:36 | 14:06 | 1.76 | 1.76 |
| MALE | E | 256 | 1.28 | 13:39 | 14:09 | 1.72 | 1.72 |
| Nicotine 0.4 mg/kg | F | 263 | 1.33 | 13:44 | 14:14 | 1.77 | 1.77 |
| 8 | D | 212 | 1.05 | 13:48 | 14:18 | 1.75 | 1.75 |
| FEMALE | E | 213 | 1.05 | 13:52 | 14:22 | 1.81 | 1.81 |
| Nicotine 0.4 mg/kg | F | 231 | 1.15 | 13:59 | 14:29 | 1.90 | 1.90 |

**Table 14. Measured Concentrations of Anatabine and Nicotine in Rat Brain Extracts and Plasma Samples at Each Time Point**

| Compound | Dose Group | Tissue | Rat ID | Time Point (hr) | Concentration (ng/mL) |
|---|---|---|---|---|---|
| Nicotine | Nicotine 0.4 mg/kg | Rat Brain | 7D | 0.5 | 110 |
| Nicotine | Nicotine 0.4 mg/kg | Rat Brain | 7E | 0.5 | 71 |

(continued)

| Compound | Dose Group | Tissue | Rat ID | Time Point (hr) | Concentration (ng/mL) |
|---|---|---|---|---|---|
| Nicotine | Nicotine 0.4 mg/kg | Rat Brain | 7F | 0.5 | 102 |
| Nicotine | Nicotine 0.4 mg/kg | Rat Brain | 7G | 6 | 5 |
| Nicotine | Nicotine 0.4 mg/kg | Rat Brain | 7H | 6 | <LLQ |
| Nicotine | Nicotine 0.4 mg/kg | Rat Brain | 71 | 6 | 7 |
| Nicotine | Nicotine 0.4 mg/kg | Rat Brain | 7A | 24 | 5 |
| Nicotine | Nicotine 0.4 mg/kg | Rat Brain | 7B | 24 | 5 |
| Nicotine | Nicotine 0.4 mg/kg | Rat Brain | 7C | 24 | 9 |
| Nicotine | Nicotine 0.4 mg/kg | Rat Brain | 8D | 0.5 | 117 |
| Nicotine | Nicotine 0.4 mg/kg | Rat Brain | 8E | 0.5 | 120 |
| Nicotine | Nicotine 0.4 mg/kg | Rat Brain | 8F | 0.5 | 123 |
| Nicotine | Nicotine 0.4 mg/kg | Rat Brain | 8G | 6 | <LLQ |
| Nicotine | Nicotine 0.4 mg/kg | Rat Brain | 8H | 6 | <LLQ |
| Nicotine | Nicotine 0.4 mg/kg | Rat Brain | 81 | 6 | 5 |
| Nicotine | Nicotine 0.4 mg/kg | Rat Brain | 8A | 24 | <LLQ |
| Nicotine | Nicotine 0.4 mg/kg | Rat Brain | 8B | 24 | 7 |
| Nicotine | Nicotine 0.4 mg/kg | Rat Brain | 8C | 24 | <LLQ |
| | | | | | |
| Anatabine | Anatabine 0.1 mg/kg | Rat Brain | 1D | 0.5 | <LLQ |
| Anatabine | Anatabine 0.1 mg/kg | Rat Brain | 1E | 0.5 | 19 |
| Anatabine | Anatabine 0.1 mg/kg | Rat Brain | 1F | 0.5 | 21 |
| Anatabine | Anatabine 0.1 mg/kg | Rat Brain | 1G | 6 | 3 |
| Anatabine | Anatabine 0.1 mg/kg | Rat Brain | 1H | 6 | <LLQ |
| Anatabine | Anatabine 0.1 mg/kg | Rat Brain | 1I | 6 | <LLQ |
| Anatabine | Anatabine 0.1 mg/kg | Rat Brain | 1A | 24 | 3 |
| Anatabine | Anatabine 0.1 mg/kg | Rat Brain | 1B | 24 | <LLQ |
| Anatabine | Anatabine 0.1 mg/kg | Rat Brain | 1C | 24 | <LLQ |
| Anatabine | Anatabine 0.1 mg/kg | Rat Brain | 2D | 0.5 | 34 |
| Anatabine | Anatabine 0.1 mg/kg | Rat Brain | 2E | 0.5 | 25 |
| Anatabine | Anatabine 0.1 mg/kg | Rat Brain | 2F | 0.5 | 30 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Brain | 2G | 6 | 5 |
| Anatabine | Anatabine 0.1 mg/kg | Rat Brain | 2H | 6 | 4 |
| Anatabine | Anatabine 0.1 mg/kg | Rat Brain | 2I | 6 | 2 |
| Anatabine | Anatabine 0.1 mg/kg | Rat Brain | 2A | 24 | 2 |
| Anatabine | Anatabine 0.1 mg/kg | Rat Brain | 2B | 24 | <LLQ |
| Anatabine | Anatabine 0.1 mg/kg | Rat Brain | 2C | 24 | <LLQ |
| Anatabine | Anatabine 0.75 mg/kg | Rat Brain | 3D | 0.5 | 266 |
| Anatabine | Anatabine 0.75 mg/kg | Rat Brain | 3E | 0.5 | 317 |
| Anatabine | Anatabine 0.75 mg/kg | Rat Brain | 3F | 0.5 | 245 |
| Anatabine | Anatabine 0.75 mg/kg | Rat Brain | 3G | 6 | 17 |
| Anatabine | Anatabine 0.75 mg/kg | Rat Brain | 3H | 6 | 27 |
| Anatabine | Anatabine 0.75 mg/kg | Rat Brain | 3I | 6 | 20 |
| Anatabine | Anatabine 0.75 mg/kg | Rat Brain | 3A | 24 | <LLQ |
| Anatabine | Anatabine 0.75 mg/kg | Rat Brain | 3B | 24 | <LLQ |
| Anatabine | Anatabine 0.75 mg/kg | Rat Brain | 3C | 24 | <LLQ |
| Anatabine | Anatabine 0.75 mg/kg | Rat Brain | 4D | 0.5 | 393 |
| Anatabine | Anatabine 0.75 mg/kg | Rat Brain | 4E | 0.5 | 272 |
| Anatabine | Anatabine 0.75 mg/kg | Rat Brain | 4F | 0.5 | 279 |
| Anatabine | Anatabine 0.75 mg/kg | Rat Brain | 4G | 6 | 16 |
| Anatabine | Anatabine 0.75 mg/kg | Rat Brain | 4H | 6 | 28 |
| Anatabine | Anatabine 0.75 mg/kg | Rat Brain | 4I | 6 | 19 |
| Anatabine | Anatabine 0.75 mg/kg | Rat Brain | 4A | 24 | <LLQ |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Brain | 4B | 24 | <LLQ |
| Anatabine | Anatabine 0.75 mg/kg | Rat Brain | 4C | 24 | <LLQ |
| Anatabine | Anatabine 1.0 mg/kg | Rat Brain | 5D | 0.5 | 349 |
| Anatabine | Anatabine 1.0 mg/kg | Rat Brain | 5E | 0.5 | 338 |
| Anatabine | Anatabine 1.0 mg/kg | Rat Brain | 5F | 0.5 | 282 |
| Anatabine | Anatabine 1.0 mg/kg | Rat Brain | 5G | 6 | 3 |
| Anatabine | Anatabine 1.0 mg/kg | Rat Brain | 5H | 6 | 4 |
| Anatabine | Anatabine 1.0 mg/kg | Rat Brain | 5I | 6 | 8 |
| Anatabine | Anatabine 1.0 mg/kg | Rat Brain | 5A | 24 | 3 |
| Anatabine | Anatabine 1.0 mg/kg | Rat Brain | 5B | 24 | 2 |
| Anatabine | Anatabine 1.0 mg/kg | Rat Brain | 5C | 24 | <LLQ |
| Anatabine | Anatabine 1.0 mg/kg | Rat Brain | 6D | 0.5 | 362 |
| Anatabine | Anatabine 1.0 mg/kg | Rat Brain | 6E | 0.5 | 393 |
| Anatabine | Anatabine 1.0 mg/kg | Rat Brain | 6F | 0.5 | 283 |
| Anatabine | Anatabine 1.0 mg/kg | Rat Brain | 6G | 6 | <LLQ |
| Anatabine | Anatabine 1.0 mg/kg | Rat Brain | 6H | 6 | 8 |
| Anatabine | Anatabine 1.0 mg/kg | Rat Brain | 6I | 6 | 4 |
| Anatabine | Anatabine 1.0 mg/kg | Rat Brain | 6A | 24 | <LLQ |
| Anatabine | Anatabine 1.0 mg/kg | Rat Brain | 6B | 24 | <LLQ |
| Anatabine | Anatabine 1.0 mg/kg | Rat Brain | 6C | 24 | <LLQ |
| | | | | | |
| Nicotine | Nicotine 0.4 mg/kg | Rat Plasma | 7A | 0.25 | 194 |
| Nicotine | Nicotine 0.4 mg/kg | Rat Plasma | 7B | 0.25 | 156 |

(continued)

| Compound | Dose Group | Tissue | Rat ID | Time Point (hr) | Concentration (ng/mL) |
|---|---|---|---|---|---|
| Nicotine | Nicotine 0.4 mg/kg | Rat Plasma | 7C | 0.25 | 145 |
| Nicotine | Nicotine 0.4 mg/kg | Rat Plasma | 7A | 0.5 | 123 |
| Nicotine | Nicotine 0.4 mg/kg | Rat Plasma | 7B | 0.5 | 85 |
| Nicotine | Nicotine 0.4 mg/kg | Rat Plasma | 7C | 0.5 | 90 |
| Nicotine | Nicotine 0.4 mg/kg | Rat Plasma | 7D | 0.5 | 187 |
| Nicotine | Nicotine 0.4 mg/kg | Rat Plasma | 7E | 0.5 | 118 |
| Nicotine | Nicotine 0.4 mg/kg | Rat Plasma | 7F | 0.5 | 157 |
| Nicotine | Nicotine 0.4 mg/kg | Rat Plasma | 7A | 1 | 72 |
| Nicotine | Nicotine 0.4 mg/kg | Rat Plasma | 7B | 1 | 67 |
| Nicotine | Nicotine 0.4 mg/kg | Rat Plasma | 7C | 1 | 68 |
| Nicotine | Nicotine 0.4 mg/kg | Rat Plasma | 7A | 1.5 | 33 |
| Nicotine | Nicotine 0.4 mg/kg | Rat Plasma | 7B | 1.5 | 30 |
| Nicotine | Nicotine mg/kg | Rat Plasma | 7C | 1.5 | 44 |
| Nicotine | Nicotine 0.4 mg/kg | Rat Plasma | 7A | 2 | 21 |
| Nicotine | Nicotine 0.4 mg/kg | Rat Plasma | 7B | 2 | 32 |
| Nicotine | Nicotine 0.4 mg/kg | Rat Plasma | 7C | 2 | 21 |
| Nicotine | Nicotine 0.4 mg/kg | Rat Plasma | 7A | 4 | <LLQ |
| Nicotine | Nicotine 0.4 mg/kg | Rat Plasma | 7B | 4 | <LLQ |
| Nicotine | Nicotine 0.4 mg/kg | Rat Plasma | 7C | 4 | <LLQ |
| Nicotine | Nicotine 0.4 mg/kg | Rat Plasma | 7A | 6 | <LLQ |
| Nicotine | Nicotine 0.4 mg/kg | Rat Plasma | 7B | 6 | <LLQ |
| Nicotine | Nicotine 0.4 mg/kg | Rat Plasma | 7C | 6 | <LLQ |
| Nicotine | Nicotine 0.4 mg/kg | Rat Plasma | 7G | 6 | <LLQ |
| Nicotine | Nicotine 0.4 mg/kg | Rat Plasma | 7H | 6 | <LLQ |
| Nicotine | Nicotine 0.4 mg/kg | Rat Plasma | 7I | 6 | <LLQ |
| Nicotine | Nicotine 0.4 mg/kg | Rat Plasma | 7A | 8 | <LLQ |
| Nicotine | Nicotine 0.4 mg/kg | Rat Plasma | 7B | 8 | <LLQ |
| Nicotine | Nicotine 0.4 mg/kg | Rat Plasma | 7C | 8 | <LLQ |
| Nicotine | Nicotine 0.4 mg/kg | Rat Plasma | 7A | 24 | <LLQ |
| Nicotine | Nicotine 0.4 mg/kg | Rat Plasma | 7B | 24 | <LLQ |
| Nicotine | Nicotine 0.4 mg/kg | Rat Plasma | 7C | 24 | <LLQ |
| Nicotine | Nicotine 0.4 mg/kg | Rat Plasma | 8A | 0.25 | 175 |
| Nicotine | Nicotine 0.4 mg/kg | Rat Plasma | 8B | 0.25 | 145 |
| Nicotine | Nicotine 0.4 mg/kg | Rat Plasma | 8C | 0.25 | 184 |
| Nicotine | Nicotine 0.4 mg/kg | Rat Plasma | 8A | 0.5 | 111 |
| Nicotine | Nicotine 0.4 mg/kg | Rat Plasma | 8B | 0.5 | 95 |
| Nicotine | Nicotine 0.4 mg/kg | Rat Plasma | 8C | 0.5 | 125 |

(continued)

| Compound | Dose Group | Tissue | Rat ID | Time Point (hr) | Concentration (ng/mL) |
|---|---|---|---|---|---|
| Nicotine | Nicotine 0.4 mg/kg | Rat Plasma | 8D | 0.5 | 180 |
| Nicotine | Nicotine 0.4 mg/kg | Rat Plasma | 8E | 0.5 | 157 |
| Nicotine | Nicotine 0.4 mg/kg | Rat Plasma | 8F | 0.5 | 160 |
| Nicotine | Nicotine 0.4 mg/kg | Rat Plasma | 8A | 1 | 67 |
| Nicotine | Nicotine 0.4 mg/kg | Rat Plasma | 8B | 1 | 72 |
| Nicotine | Nicotine 0.4 mg/kg | Rat Plasma | 8C | 1 | 107 |
| Nicotine | Nicotine 0.4 mg/kg | Rat Plasma | 8A | 1.5 | 49 |
| Nicotine | Nicotine 0.4 mg/kg | Rat Plasma | 8B | 1.5 | 37 |
| Nicotine | Nicotine 0.4 mg/kg | Rat Plasma | 8C | 1.5 | 64 |
| Nicotine | Nicotine 0.4 mg/kg | Rat Plasma | 8A | 2 | 25 |
| Nicotine | Nicotine 0.4 mg/kg | Rat Plasma | 8B | 2 | 24 |
| Nicotine | Nicotine 0.4 mg/kg | Rat Plasma | 8C | 2 | 46 |
| Nicotine | Nicotine 0.4 mg/kg | Rat Plasma | 8A | 4 | 3 |
| Nicotine | Nicotine 0.4 mg/kg | Rat Plasma | 8B | 4 | <LLQ |
| Nicotine | Nicotine 0.4 mg/kg | Rat Plasma | 8C | 4 | 4 |
| Nicotine | Nicotine 0.4 mg/kg | Rat Plasma | 8A | 6 | <LLQ |
| Nicotine | Nicotine 0.4 mg/kg | Rat Plasma | 8B | 6 | <LLQ |
| Nicotine | Nicotine 0.4 mg/kg | Rat Plasma | 8C | 6 | <LLQ |
| Nicotine | Nicotine 0.4 mg/kg | Rat Plasma | 8G | 6 | <LLQ |
| Nicotine | Nicotine 0.4 mg/kg | Rat Plasma | 8H | 6 | <LLQ |
| Nicotine | Nicotine 0.4 mg/kg | Rat Plasma | 8I | 6 | <LLQ |
| Nicotine | Nicotine 0.4 mg/kg | Rat Plasma | 8A | 8 | <LLQ |
| Nicotine | Nicotine 0.4 mg/kg | Rat Plasma | 8B | 8 | <LLQ |
| Nicotine | Nicotine 0.4 mg/kg | Rat Plasma | 8C | 8 | <LLQ |
| Nicotine | Nicotine 0.4 mg/kg | Rat Plasma | 8A | 24 | <LLQ |
| Nicotine | Nicotine 0.4 mg/kg | Rat Plasma | 8B | 24 | <LLQ |
| Nicotine | Nicotine 0.4 mg/kg | Rat Plasma | 8C | 24 | <LLQ |
| | | | | | |
| Anatabine | Anatabine 0.1 mg/kg | Rat Plasma | 1A | 0.25 | 24 |
| Anatabine | Anatabine 0.1 mg/kg | Rat Plasma | 1B | 0.25 | 14 |
| Anatabine | Anatabine 0.1 mg/kg | Rat Plasma | 1C | 0.25 | 20 |
| Anatabine | Anatabine 0.1 mg/kg | Rat Plasma | 1A | 0.5 | 16 |
| Anatabine | Anatabine 0.1 mg/kg | Rat Plasma | 1B | 0.5 | 17 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Plasma | 1C | 0.5 | 13 |
| Anatabine | Anatabine 0.1 mg/kg | Rat Plasma | 1D | 0.5 | 30 |
| Anatabine | Anatabine 0.1 mg/kg | Rat Plasma | 1E | 0.5 | 32 |
| Anatabine | Anatabine 0.1 mg/kg | Rat Plasma | 1F | 0.5 | 33 |
| Anatabine | Anatabine 0.1 mg/kg | Rat Plasma | 1A | 1 | 10 |
| Anatabine | Anatabine 0.1 mg/kg | Rat Plasma | 1B | 1 | 16 |
| Anatabine | Anatabine 0.1 mg/kg | Rat Plasma | 1C | 1 | 11 |
| Anatabine | Anatabine 0.1 mg/kg | Rat Plasma | 1A | 1.5 | 7 |
| Anatabine | Anatabine 0.1 mg/kg | Rat Plasma | 1B | 1.5 | <LLQ |
| Anatabine | Anatabine 0.1 mg/kg | Rat Plasma | 1C | 1.5 | 6 |
| Anatabine | Anatabine 0.1 mg/kg | Rat Plasma | 1A | 2 | 6 |
| Anatabine | Anatabine 0.1 mg/kg | Rat Plasma | 1B | 2 | 8 |
| Anatabine | Anatabine 0.1 mg/kg | Rat Plasma | 1C | 2 | 5 |
| Anatabine | Anatabine 0.1 mg/kg | Rat Plasma | 1A | 4 | 3 |
| Anatabine | Anatabine 0.1 mg/kg | Rat Plasma | 1B | 4 | 3 |
| Anatabine | Anatabine 0.1 mg/kg | Rat Plasma | 1C | 4 | <LLQ |
| Anatabine | Anatabine 0.1 mg/kg | Rat Plasma | 1A | 6 | <LLQ |
| Anatabine | Anatabine 0.1 mg/kg | Rat Plasma | 1B | 6 | <LLQ |
| Anatabine | Anatabine 0.1 mg/kg | Rat Plasma | 1C | 6 | <LLQ |
| Anatabine | Anatabine 0.1 mg/kg | Rat Plasma | 1G | 6 | 7 |
| Anatabine | Anatabine 0.1 mg/kg | Rat Plasma | 1H | 6 | 4 |
| Anatabine | Anatabine 0.1 mg/kg | Rat Plasma | 1I | 6 | 4 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Plasma | 1A | 8 | <LLQ |
| Anatabine | Anatabine 0.1 mg/kg | Rat Plasma | 1B | 8 | <LLQ |
| Anatabine | Anatabine 0.1 mg/kg | Rat Plasma | 1C | 8 | <LLQ |
| Anatabine | Anatabine 0.1 mg/kg | Rat Plasma | 1A | 24 | <LLQ |
| Anatabine | Anatabine 0.1 mg/kg | Rat Plasma | 1B | 24 | <LLQ |
| Anatabine | Anatabine 0.1 mg/kg | Rat Plasma | 1C | 24 | <LLQ |
| Anatabine | Anatabine 0.1 mg/kg | Rat Plasma | 2D | 0.5 | 31 |
| Anatabine | Anatabine 0.1 mg/kg | Rat Plasma | 2E | 0.5 | 30 |
| Anatabine | Anatabine 0.1 mg/kg | Rat Plasma | 2F | 0.5 | 34 |
| Anatabine | Anatabine 0.1 mg/kg | Rat Plasma | 2G | 6 | 3 |
| Anatabine | Anatabine 0.1 mg/kg | Rat Plasma | 2H | 6 | 4 |
| Anatabine | Anatabine 0.1 mg/kg | Rat Plasma | 21 | 6 | 4 |
| Anatabine | Anatabine 0.1 mg/kg | Rat Plasma | 2A | 0.25 | 15 |
| Anatabine | Anatabine 0.1 mg/kg | Rat Plasma | 2B | 0.25 | 21 |
| Anatabine | Anatabine 0.1 mg/kg | Rat Plasma | 2C | 0.25 | 14 |
| Anatabine | Anatabine 0.1 mg/kg | Rat Plasma | 2A | 0.5 | 16 |
| Anatabine | Anatabine 0.1 mg/kg | Rat Plasma | 2B | 0.5 | 13 |
| Anatabine | Anatabine 0.1 mg/kg | Rat Plasma | 2C | 0.5 | 10 |
| Anatabine | Anatabine 0.1 mg/kg | Rat Plasma | 2A | 1 | 12 |
| Anatabine | Anatabine 0.1 mg/kg | Rat Plasma | 2B | 1 | 12 |
| Anatabine | Anatabine 0.1 mg/kg | Rat Plasma | 2C | 1 | 9 |
| Anatabine | Anatabine 0.1 mg/kg | Rat Plasma | 2A | 1.5 | 14 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Plasma | 2B | 1.5 | 12 |
| Anatabine | Anatabine 0.1 mg/kg | Rat Plasma | 2C | 1.5 | 7 |
| Anatabine | Anatabine 0.1 mg/kg | Rat Plasma | 2A | 2 | 8 |
| Anatabine | Anatabine 0.1 mg/kg | Rat Plasma | 2B | 2 | 8 |
| Anatabine | Anatabine 0.1 mg/kg | Rat Plasma | 2C | 2 | 4 |
| Anatabine | Anatabine 0.1 mg/kg | Rat Plasma | 2A | 4 | 3 |
| Anatabine | Anatabine 0.1 mg/kg | Rat Plasma | 2B | 4 | 5 |
| Anatabine | Anatabine 0.1 mg/kg | Rat Plasma | 2C | 4 | <LLQ |
| Anatabine | Anatabine 0.1 mg/kg | Rat Plasma | 2A | 6 | <LLQ |
| Anatabine | Anatabine 0.1 mg/kg | Rat Plasma | 2B | 6 | 3 |
| Anatabine | Anatabine 0.1 mg/kg | Rat Plasma | 2C | 6 | <LLQ |
| Anatabine | Anatabine 0.1 mg/kg | Rat Plasma | 2A | 8 | <LLQ |
| Anatabine | Anatabine 0.1 mg/kg | Rat Plasma | 2B | 8 | <LLQ |
| Anatabine | Anatabine 0.1 mg/kg | Rat Plasma | 2C | 8 | <LLQ |
| Anatabine | Anatabine 0.1 mg/kg | Rat Plasma | 2A | 24 | <LLQ |
| Anatabine | Anatabine 0.1 mg/kg | Rat Plasma | 2B | 24 | <LLQ |
| Anatabine | Anatabine 0.1 mg/kg | Rat Plasma | 2C | 24 | <LLQ |
| Anatabine | Anatabine 0.75 mg/kg | Rat Plasma | 3A | 0.25 | 216 |
| Anatabine | Anatabine 0.75 mg/kg | Rat Plasma | 3B | 0.25 | 162 |
| Anatabine | Anatabine 0.75 mg/kg | Rat Plasma | 3C | 0.25 | 223 |
| Anatabine | Anatabine 0.75 mg/kg | Rat Plasma | 3A | 0.5 | 176 |
| Anatabine | Anatabine 0.75 mg/kg | Rat Plasma | 3B | 0.5 | 176 |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Plasma | 3C | 0.5 | 190 |
| Anatabine | Anatabine 0.75 mg/kg | Rat Plasma | 3D | 0.5 | 342 |
| Anatabine | Anatabine 0.75 mg/kg | Rat Plasma | 3E | 0.5 | 271 |
| Anatabine | Anatabine 0.75 mg/kg | Rat Plasma | 3F | 0.5 | 292 |
| Anatabine | Anatabine 0.75 mg/kg | Rat Plasma | 3A | 1 | 153 |
| Anatabine | Anatabine 0.75 mg/kg | Rat Plasma | 3B | 1 | 146 |
| Anatabine | Anatabine 0.75 mg/kg | Rat Plasma | 3C | 1 | 156 |
| Anatabine | Anatabine 0.75 mg/kg | Rat Plasma | 3A | 1.5 | 120 |
| Anatabine | Anatabine 0.75 mg/kg | Rat Plasma | 3B | 1.5 | 124 |
| Anatabine | Anatabine 0.75 mg/kg | Rat Plasma | 3C | 1.5 | 136 |
| Anatabine | Anatabine 0.75 mg/kg | Rat Plasma | 3A | 2 | 73 |
| Anatabine | Anatabine 0.75 mg/kg | Rat Plasma | 3B | 2 | 74 |
| Anatabine | Anatabine 0.75 mg/kg | Rat Plasma | 3C | 2 | 104 |
| Anatabine | Anatabine 0.75 mg/kg | Rat Plasma | 3A | 4 | 29 |
| Anatabine | Anatabine 0.75 mg/kg | Rat Plasma | 3B | 4 | 36 |
| Anatabine | Anatabine 0.75 mg/kg | Rat Plasma | 3C | 4 | 39 |
| Anatabine | Anatabine 0.75 mg/kg | Rat Plasma | 3A | 6 | 15 |
| Anatabine | Anatabine 0.75 mg/kg | Rat Plasma | 3B | 6 | 14 |
| Anatabine | Anatabine 0.75 mg/kg | Rat Plasma | 3C | 6 | 13 |
| Anatabine | Anatabine 0.75 mg/kg | Rat Plasma | 3G | 6 | 15 |
| Anatabine | Anatabine 0.75 mg/kg | Rat Plasma | 3H | 6 | 31 |
| Anatabine | Anatabine 0.75 mg/kg | Rat Plasma | 3I | 6 | 20 |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Plasma | 3A | 8 | 8 |
| Anatabine | Anatabine 0.75 mg/kg | Rat Plasma | 3B | 8 | 11 |
| Anatabine | Anatabine 0.75 mg/kg | Rat Plasma | 3C | 8 | 8 |
| Anatabine | Anatabine 0.75 mg/kg | Rat Plasma | 3A | 24 | <LLQ |
| Anatabine | Anatabine 0.75 mg/kg | Rat Plasma | 3B | 24 | <LLQ |
| Anatabine | Anatabine 0.75 mg/kg | Rat Plasma | 3C | 24 | <LLQ |
| Anatabine | Anatabine 0.75 mg/kg | Rat Plasma | 4A | 0.25 | 204 |
| Anatabine | Anatabine 0.75 mg/kg | Rat Plasma | 4B | 0.25 | 226 |
| Anatabine | Anatabine 0.75 mg/kg | Rat Plasma | 4C | 0.25 | 207 |
| Anatabine | Anatabine 0.75 mg/kg | Rat Plasma | 4A | 0.5 | 166 |
| Anatabine | Anatabine 0.75 mg/kg | Rat Plasma | 4B | 0.5 | 229 |
| Anatabine | Anatabine 0.75 mg/kg | Rat Plasma | 4C | 0.5 | 175 |
| Anatabine | Anatabine 0.75 mg/kg | Rat Plasma | 4D | 0.5 | 307 |
| Anatabine | Anatabine 0.75 mg/kg | Rat Plasma | 4E | 0.5 | 359 |
| Anatabine | Anatabine 0.75 mg/kg | Rat Plasma | 4F | 0.5 | 396 |
| Anatabine | Anatabine 0.75 mg/kg | Rat Plasma | 4A | 1 | 146 |
| Anatabine | Anatabine 0.75 mg/kg | Rat Plasma | 4B | 1 | 207 |
| Anatabine | Anatabine 0.75 mg/kg | Rat Plasma | 4C | 1 | 165 |
| Anatabine | Anatabine 0.75 mg/kg | Rat Plasma | 4A | 1.5 | 136 |
| Anatabine | Anatabine 0.75 mg/kg | Rat Plasma | 4B | 1.5 | 134 |
| Anatabine | Anatabine 0.75 mg/kg | Rat Plasma | 4C | 1.5 | 150 |
| Anatabine | Anatabine 0.75 mg/kg | Rat Plasma | 4A | 2 | 89 |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Plasma | 4B | 2 | 113 |
| Anatabine | Anatabine 0.75 mg/kg | Rat Plasma | 4C | 2 | 97 |
| Anatabine | Anatabine 0.75 mg/kg | Rat Plasma | 4A | 4 | 45 |
| Anatabine | Anatabine 0.75 mg/kg | Rat Plasma | 4B | 4 | 50 |
| Anatabine | Anatabine 0.75 mg/kg | Rat Plasma | 4C | 4 | 41 |
| Anatabine | Anatabine 0.75 mg/kg | Rat Plasma | 4A | 6 | 14 |
| Anatabine | Anatabine 0.75 mg/kg | Rat Plasma | 4B | 6 | 23 |
| Anatabine | Anatabine 0.75 mg/kg | Rat Plasma | 4C | 6 | 18 |
| Anatabine | Anatabine 0.75 mg/kg | Rat Plasma | 4G | 6 | 15 |
| Anatabine | Anatabine 0.75 mg/kg | Rat Plasma | 4H | 6 | 38 |
| Anatabine | Anatabine 0.75 mg/kg | Rat Plasma | 41 | 6 | 16 |
| Anatabine | Anatabine 0.75 mg/kg | Rat Plasma | 4A | 8 | 10 |
| Anatabine | Anatabine 0.75 mg/kg | Rat Plasma | 4B | 8 | 12 |
| Anatabine | Anatabine 0.75 mg/kg | Rat Plasma | 4C | 8 | 11 |
| Anatabine | Anatabine 0.75 mg/kg | Rat Plasma | 4A | 24 | <LLQ |
| Anatabine | Anatabine 0.75 mg/kg | Rat Plasma | 4B | 24 | <LLQ |
| Anatabine | Anatabine 0.75 mg/kg | Rat Plasma | 4C | 24 | <LLQ |
| Anatabine | Anatabine 1.0 mg/kg | Rat Plasma | 5A | 0.25 | 296 |
| Anatabine | Anatabine 1.0 mg/kg | Rat Plasma | 5B | 0.25 | 291 |
| Anatabine | Anatabine 1.0 mg/kg | Rat Plasma | 5C | 0.25 | 270 |
| Anatabine | Anatabine 1.0 mg/kg | Rat Plasma | 5A | 0.5 | 288 |
| Anatabine | Anatabine 1.0 mg/kg | Rat Plasma | 5B | 0.5 | 264 |

(continued)

| Compound | Dose Group | Tissue | Rat ID | Time Point (hr) | Concentration (ng/mL) |
|---|---|---|---|---|---|
| Anatabine | Anatabine 1.0 mg/kg | Rat Plasma | 5C | 0.5 | 265 |
| Anatabine | Anatabine 1.0 mg/kg | Rat Plasma | 5D | 0.5 | 447 |
| Anatabine | Anatabine 1.0 mg/kg | Rat Plasma | 5E | 0.5 | 384 |
| Anatabine | Anatabine 1.0 mg/kg | Rat Plasma | 5F | 0.5 | 366 |
| Anatabine | Anatabine 1.0 mg/kg | Rat Plasma | 5A | 1 | 257 |
| Anatabine | Anatabine 1.0 mg/kg | Rat Plasma | 5B | 1 | 225 |
| Anatabine | Anatabine 1.0 mg/kg | Rat Plasma | 5C | 1 | 219 |
| Anatabine | Anatabine 1.0 mg/kg | Rat Plasma | 5A | 1.5 | 163 |
| Anatabine | Anatabine 1.0 mg/kg | Rat Plasma | 5B | 1.5 | 148 |
| Anatabine | Anatabine 1.0 mg/kg | Rat Plasma | 5C | 1.5 | 160 |
| Anatabine | Anatabine 1.0 mg/kg | Rat Plasma | 5A | 2 | 121 |
| Anatabine | Anatabine 1.0 mg/kg | Rat Plasma | 5B | 2 | 129 |
| Anatabine | Anatabine 1.0 mg/kg | Rat Plasma | 5C | 2 | 119 |
| Anatabine | Anatabine 1.0 mg/kg | Rat Plasma | 5A | 4 | 36 |
| Anatabine | Anatabine 1.0 mg/kg | Rat Plasma | 5B | 4 | 51 |
| Anatabine | Anatabine 1.0 mg/kg | Rat Plasma | 5C | 4 | 40 |
| Anatabine | Anatabine 1.0 mg/kg | Rat Plasma | 5A | 6 | 20 |
| Anatabine | Anatabine 1.0 mg/kg | Rat Plasma | 5B | 6 | 19 |
| Anatabine | Anatabine 1.0 mg/kg | Rat Plasma | 5C | 6 | 15 |
| Anatabine | Anatabine 1.0 mg/kg | Rat Plasma | 5G | 6 | 26 |
| Anatabine | Anatabine 1.0 mg/kg | Rat Plasma | 5H | 6 | 38 |
| Anatabine | Anatabine 1.0 mg/kg | Rat Plasma | 5I | 6 | 17 |

(continued)

| Compound | Dose Group | Tissue | Rat ID | Time Point (hr) | Concentration (ng/mL) |
|---|---|---|---|---|---|
| Anatabine | Anatabine 1.0 mg/kg | Rat Plasma | 5A | 8 | 8 |
| Anatabine | Anatabine 1.0 mg/kg | Rat Plasma | 5B | 8 | 9 |
| Anatabine | Anatabine 1.0 mg/kg | Rat Plasma | 5C | 8 | 6 |
| Anatabine | Anatabine 1.0 mg/kg | Rat Plasma | 5A | 24 | <LLQ |
| Anatabine | Anatabine 1.0 mg/kg | Rat Plasma | 5B | 24 | <LLQ |
| Anatabine | Anatabine 1.0 mg/kg | Rat Plasma | 5C | 24 | <LLQ |
| Anatabine | Anatabine 1.0 mg/kg | Rat Plasma | 6A | 0.25 | 293 |
| Anatabine | Anatabine 1.0 mg/kg | Rat Plasma | 6B | 0.25 | 271 |
| Anatabine | Anatabine 1.0 mg/kg | Rat Plasma | 6C | 0.25 | 302 |
| Anatabine | Anatabine 1.0 mg/kg | Rat Plasma | 6A | 0.5 | 222 |
| Anatabine | Anatabine 1.0 mg/kg | Rat Plasma | 6B | 0.5 | 253 |
| Anatabine | Anatabine 1.0 mg/kg | Rat Plasma | 6C | 0.5 | 236 |
| Anatabine | Anatabine 1.0 mg/kg | Rat Plasma | 6D | 0.5 | 347 |
| Anatabine | Anatabine 1.0 mg/kg | Rat Plasma | 6E | 0.5 | 362 |
| Anatabine | Anatabine 1.0 mg/kg | Rat Plasma | 6F | 0.5 | 395 |
| Anatabine | Anatabine 1.0 mg/kg | Rat Plasma | 6A | 1 | 218 |
| Anatabine | Anatabine 1.0 mg/kg | Rat Plasma | 6B | 1 | 225 |
| Anatabine | Anatabine 1.0 mg/kg | Rat Plasma | 6C | 1 | 244 |
| Anatabine | Anatabine 1.0 mg/kg | Rat Plasma | 6A | 1.5 | 196 |
| Anatabine | Anatabine 1.0 mg/kg | Rat Plasma | 6B | 1.5 | 192 |
| Anatabine | Anatabine 1.0 mg/kg | Rat Plasma | 6C | 1.5 | 211 |
| Anatabine | Anatabine 1.0 mg/kg | Rat Plasma | 6A | 2 | 147 |

(continued)

| Compound | Dose Group | Tissue | Rat ID | Time Point (hr) | Concentration (ng/mL) |
|---|---|---|---|---|---|
| Anatabine | Anatabine 1.0 mg/kg | Rat Plasma | 6B | 2 | 170 |
| Anatabine | Anatabine 1.0 mg/kg | Rat Plasma | 6C | 2 | 174 |
| Anatabine | Anatabine 1.0 mg/kg | Rat Plasma | 6A | 4 | 73 |
| Anatabine | Anatabine 1.0 mg/kg | Rat Plasma | 6B | 4 | 52 |
| Anatabine | Anatabine 1.0 mg/kg | Rat Plasma | 6C | 4 | 57 |
| Anatabine | Anatabine 1.0 mg/kg | Rat Plasma | 6A | 6 | 33 |
| Anatabine | Anatabine 1.0 mg/kg | Rat Plasma | 6B | 6 | 34 |
| Anatabine | Anatabine 1.0 mg/kg | Rat Plasma | 6C | 6 | 21 |
| Anatabine | Anatabine 1.0 mg/kg | Rat Plasma | 6G | 6 | 27 |
| Anatabine | Anatabine 1.0 mg/kg | Rat Plasma | 6H | 6 | 24 |
| Anatabine | Anatabine 1.0 mg/kg | Rat Plasma | 61 | 6 | 18 |
| Anatabine | Anatabine 1.0 mg/kg | Rat Plasma | 6A | 8 | 19 |
| Anatabine | Anatabine 1.0 mg/kg | Rat Plasma | 6B | 8 | 19 |
| Anatabine | Anatabine 1.0 mg/kg | Rat Plasma | 6C | 8 | 15 |
| Anatabine | Anatabine 1.0 mg/kg | Rat Plasma | 6A | 24 | <LLQ |
| Anatabine | Anatabine 1.0 mg/kg | Rat Plasma | 6B | 24 | <LLQ |
| Anatabine | Anatabine 1.0 mg/kg | Rat Plasma | 6C | 24 | <LLQ |

Table 15. Mean Concentrations and Descriptive Statistics of Anatabine and Nicotine in Rat Brain Extracts and Plasma Samples at Each Time Point

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Nicotine | Nicotine 0.4 mg/kg | Rat Brain | male | 0.5 | 3 | 94.3 | 20.6 | 11.9 | 107.2 | 6 | 19.3 | 7.9 |
| Nicotine | Nicotine 0.4 mg/kg | Rat Brain | male | 6 | 2 | 6.0 | 1.4 | 1.0 | 5.7 | 3 | 1.2 | 0.7 |
| Nicotine | Nicotine 0.4 mg/kg | Rat Brain | male | 24 | 3 | 6.3 | 2.3 | 1.3 | 6.5 | 4 | 1.9 | 1.0 |
| Nicotine | Nicotine 0.4 mg/kg | Rat Brain | female | 0.5 | 3 | 120.0 | 3.0 | 1.7 | | | | |
| Nicotine | Nicotine 0.4 mg/kg | Rat Brain | female | 6 | 1 | 5.0 | ND | ND | | | | |
| Nicotine | Nicotine 0.4 mg/kg | Rat Brain | female | 24 | 1 | 7.0 | ND | ND | | | | |
| Anatabine | Anatabine 0.1 mg/kg | Rat Brain | male | 0.5 | 2 | 20.0 | 1.4 | 1.0 | 25.8 | 5 | 6.2 | 2.8 |
| Anatabine | Anatabine 0.1 mg/kg | Rat Brain | male | 6 | 1 | 3.0 | ND | ND | 3.5 | 4 | 1.3 | 0.6 |
| Anatabine | Anatabine 0.1 mg/kg | Rat Brain | male | 24 | 1 | 3.0 | ND | ND | 2.5 | 2 | 0.7 | 0.5 |
| Anatabine | Anatabine 0.1 mg/kg | Rat Brain | female | 0.5 | 3 | 29.7 | 4.5 | 2.6 | | | | |
| Anatabine | Anatabine 0.1 mg/kg | Rat Brain | female | 6 | 3 | 3.7 | 1.5 | 0.9 | | | | |
| Anatabine | Anatabine 0.1 mg/kg | Rat Brain | female | 24 | 1 | 2.0 | ND | ND | | | | |
| Anatabine | Anatabine 0.75 mg/kg | Rat Brain | male | 0.5 | 3 | 276.0 | 37.0 | 21.4 | 295.3 | 6 | 53.3 | 21.8 |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 0.75 mg/kg | Rat Brain | male | 6 | 3 | 21.3 | 5.1 | 3.0 | 21.2 | 6 | 5.1 | 2.1 |
| Anatabine | Anatabine 0.75 mg/kg | Rat Brain | male | 24 | 0 | <LLQ | ND | ND | <LLQ | 0 | ND | ND |
| Anatabine | Anatabine 0.75 mg/kg | Rat Brain | female | 0.5 | 3 | 314.7 | 67.9 | 39.2 | | | | |
| Anatabine | Anatabine 0.75 mg/kg | Rat Brain | female | 6 | 3 | 21.0 | 6.2 | 3.6 | | | | |
| Anatabine | Anatabine 0.75 mg/kg | Rat Brain | female | 24 | 0 | <LLQ | ND | ND | | | | |
| Anatabine | Anatabine 1.0 mg/kg | Rat Brain | male | 0.5 | 3 | 323.0 | 35.9 | 20.7 | 334.5 | 6 | 44.3 | 18.1 |
| Anatabine | Anatabine 1.0 mg/kg | Rat Brain | male | 6 | 3 | 5.0 | 2.6 | 1.5 | 5.4 | 5 | 2.4 | 1.1 |
| Anatabine | Anatabine 1.0 mg/kg | Rat Brain | male | 24 | 2 | 2.5 | 0.7 | 0.5 | 2.5 | 2 | 0.7 | 0.5 |
| Anatabine | Anatabine 1.0 mg/kg | Rat Brain | female | 0.5 | 3 | 346.0 | 56.7 | 32.7 | | | | |
| Anatabine | Anatabine 1.0 mg/kg | Rat Brain | female | 6 | 2 | 6.0 | 2.8 | 2.0 | | | | |
| Anatabine | Anatabine 1.0 mg/kg | Rat Brain | female | 24 | 0 | <LLQ | ND | ND | | | | |
| Nicotine | Nicotine 0.4 mg/kg | Rat Plasma | male | 0.25 | 3 | 165.0 | 25.7 | 14.8 | 166.5 | 6 | 20.8 | 8.5 |
| Nicotine | Nicotine 0.4 mg/kg | Rat Plasma | male | 0.5 | 3 | 99.3 | 20.6 | 11.9 | 104.8 | 6 | 17.2 | 7.0 |

(continued)

| Compound | Dose Group | Tissue | Sex | Time Point (hr) | Male or Female | | | | Combined Male and Female | | | |
|---|---|---|---|---|---|---|---|---|---|---|---|---|
| | | | | | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Nicotine | Nicotine 0.4 mg/kg | Rat Plasma | male | 0.5 | 3 | 154.0 | 34.6 | 20.0 | 159.8 | 6 | 24.1 | 9.9 |
| Nicotine | Nicotine 0.4 mg/kg | Rat Plasma | male | 1 | 3 | 69.0 | 2.6 | 1.5 | 75.5 | 6 | 15.6 | 6.4 |
| Nicotine | Nicotine 0.4 mg/kg | Rat Plasma | male | 1.5 | 3 | 35.7 | 7.4 | 4.3 | 42.8 | 6 | 12.5 | 5.1 |
| Nicotine | Nicotine 0.4 mg/kg | Rat Plasma | male | 2 | 3 | 24.7 | 6.4 | 3.7 | 28.2 | 6 | 9.6 | 3.9 |
| Nicotine | Nicotine 0.4 mg/kg | Rat Plasma | male | 4 | 0 | <LLQ | ND | ND | 3.5 | 2 | 0.7 | 0.5 |
| Nicotine | Nicotine 0.4 mg/kg | Rat Plasma | male | 6 | 0 | <LLQ | ND | ND | <LLQ | 0 | ND | ND |
| Nicotine | Nicotine 0.4 mg/kg | Rat Plasma | male | 8 | 0 | <LLQ | ND | ND | <LLQ | 0 | ND | ND! |
| Nicotine | Nicotine 0.4 mg/kg | Rat Plasma | male | 24 | 0 | <LLQ | ND | ND | <LLQ | 0 | ND | ND |
| Nicotine | Nicotine 0.4 mg/kg | Rat Plasma | female | 0.25 | 3 | 168.0 | 20.4 | 11.8 | 108.4 | 5 | 83.0 | 37.1 |
| Nicotine | Nicotine 0.4 mg/kg | Rat Plasma | female | 0.5 | 3 | 110.3 | 15.0 | 8.7 | 79.7 | 12 | 65.0 | 18.8 |
| Nicotine | Nicotine 0.4 mg/kg | Rat Plasma | female | 0.5 | 3 | 165.7 | 12.5 | 7.2 | 95.3 | 6 | 77.7 | 31.7 |
| Nicotine | Nicotine 0.4 mg/kg | Rat Plasma | female | 1 | 3 | 82.0 | 21.8 | 12.6 | 50.8 | 6 | 37.6 | 15.3 |
| Nicotine | Nicotine 0.4 mg/kg | Rat Plasma | female | 1.5 | 3 | 50.0 | 13.5 | 7.8 | 33.6 | 5 | 24.4 | 10.9 |

(continued)

| | | | | | Male or Female | | | | Combined Male and Female | | | |
|---|---|---|---|---|---|---|---|---|---|---|---|---|
| Compound | Dose Group | Tissue | Sex | Time Point (hr) | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Nicotine | Nicotine 0.4 mg/kg | Rat Plasma | female | 2 | 3 | 31.7 | 12.4 | 7.2 | 19.2 | 6 | 15.8 | 6.5 |
| Nicotine | Nicotine 0.4 mg/kg | Rat Plasma | female | 4 | 2 | 3.5 | 0.7 | 0.5 | 3.6 | 5 | 0.9 | 0.4 |
| Nicotine | Nicotine 0.4 mg/kg | Rat Plasma | female | 6 | 0 | <LLQ | ND | ND | 5.5 | 2 | 2.1 | 1.5 |
| Nicotine | Nicotine 0.4 mg/kg | Rat Plasma | female | 6 | 0 | <LLQ | ND | ND | 5.5 | 2 | 2.1 | 1.5 |
| Nicotine | Nicotine 0.4 mg/kg | Rat Plasma | female | 8 | 0 | <LLQ | ND | ND | 4.0 | 1 | ND | ND |
| Nicotine | Nicotine 0.4 mg/kg | Rat Plasma | female | 24 | 0 | <LLQ | ND | ND | <LLQ | 0 | ND | ND |
| Anatabine | Anatabine 0.1 mg/kg | Rat Plasma | male | 0.25 | 3 | 19.3 | 5.0 | 2.9 | 18.0 | 6 | 4.2 | 1.7 |
| Anatabine | Anatabine 0.1 mg/kg | Rat Plasma | male | 0.5 | 3 | 15.3 | 2.1 | 1.2 | 22.9 | 12 | 9.4 | 2.7 |
| Anatabine | Anatabine 0.1 mg/kg | Rat Plasma | male | 0.5 | 3 | 31.7 | 1.5 | 0.9 | 31.7 | 6 | 1.6 | 0.7 |
| Anatabine | Anatabine 0.1 mg/kg | Rat Plasma | male | 1 | 3 | 12.3 | 3.2 | 1.9 | 8.0 | 6 | 5.2 | 2.1 |
| Anatabine | Anatabine 0.1 mg/kg | Rat Plasma | male | 1.5 | 2 | 6.5 | 0.7 | 0.5 | 9.2 | 5 | 2.8 | 1.2 |
| Anatabine | Anatabine 0.1 mg/kg | Rat Plasma | male | 2 | 3 | 6.3 | 1.5 | 0.9 | 8.7 | 6 | 3.6 | 1.5 |
| Anatabine | Anatabine 0.1 mg/kg | Rat Plasma | male | 4 | 2 | 3.0 | 0.0 | 0.0 | 5.2 | 5 | 2.6 | 1.2 |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 0.1 mg/kg | Rat Plasma | male | 6 | 0 | <LLQ | ND | ND | 4.3 | 6 | 1.5 | 0.6 |
| Anatabine | Anatabine 0.1 mg/kg | Rat Plasma | male | 6 | 3 | 5.0 | 1.7 | 1.0 | 4.5 | 4 | 1.7 | 0.9 |
| Anatabine | Anatabine 0.1 mg/kg | Rat Plasma | male | 8 | 0 | <LLQ | ND | ND | <LLQ | 0 | ND | ND |
| Anatabine | Anatabine 0.1 mg/kg | Rat Plasma | male | 24 | 0 | <LLQ | ND | ND | <LLQ | 0 | ND | ND |
| Anatabine | Anatabine 0.1 mg/kg | Rat Plasma | female | 0.25 | 3 | 16.7 | 3.8 | 2.2 | | | | |
| Anatabine | Anatabine 0.1 mg/kg | Rat Plasma | female | 0.5 | 3 | 13.0 | 3.0 | 1.7 | | | | |
| Anatabine | Anatabine 0.1 mg/kg | Rat Plasma | female | 0.5 | 3 | 31.7 | 2.1 | 1.2 | | | | |
| Anatabine | Anatabine 0.1 mg/kg | Rat Plasma | female | 6 | 3 | 3.7 | 0.6 | 0.3 | | | | |
| Anatabine | Anatabine 0.1 mg/kg | Rat Plasma | female | 1 | 3 | 11.0 | 1.7 | 1.0 | | | | |
| Anatabine | Anatabine 0.1 mg/kg | Rat Plasma | female | 1.5 | 3 | 11.0 | 3.6 | 2.1 | | | | |
| Anatabine | Anatabine 0.1 mg/kg | Rat Plasma | female | 2 | 3 | 6.7 | 2.3 | 1.3 | | | | |
| Anatabine | Anatabine 0.1 mg/kg | Rat Plasma | female | 4 | 2 | 4.0 | 1.4 | 1.0 | | | | |
| Anatabine | Anatabine 0.1 mg/kg | Rat Plasma | female | 6 | 1 | 3.0 | ND | ND | | | | |

(continued)

| Compound | Dose Group | Tissue | Sex | Time Point (hr) | Male or Female | | | | Combined Male and Female | | | |
|---|---|---|---|---|---|---|---|---|---|---|---|---|
| | | | | | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Anatabine | Anatabine 0.1 mg/kg | Rat Plasma | female | 8 | 0 | <LLQ | ND | ND | | | | |
| Anatabine | Anatabine 0.1 mg/kg | Rat Plasma | female | 24 | 0 | <LLQ | ND | ND | | | | |
| Anatabine | Anatabine 0.75 mg/kg | Rat Plasma | male | 0.25 | 3 | 200.3 | 33.4 | 19.3 | 206.3 | 6 | 23.4 | 9.5 |
| Anatabine | Anatabine 0.75 mg/kg | Rat Plasma | male | 0.5 | 3 | 180.7 | 8.1 | 4.7 | 256.6 | 12 | 82.2 | 23.7 |
| Anatabine | Anatabine 0.75 mg/kg | Rat Plasma | male | 0.5 | 3 | 301.7 | 36.5 | 21.1 | 327.8 | 6 | 46.4 | 18.9 |
| Anatabine | Anatabine 0.75 mg/kg | Rat Plasma | male | 1 | 3 | 151.7 | 5.1 | 3.0 | 162.2 | 6 | 23.1 | 9.4 |
| Anatabine | Anatabine 0.75 mg/kg | Rat Plasma | male | 1.5 | 3 | 126.7 | 8.3 | 4.8 | 133.3 | 6 | 10.6 | 4.3 |
| Anatabine | Anatabine 0.75 mg/kg | Rat Plasma | male | 2 | 3 | 83.7 | 17.6 | 10.2 | 91.7 | 6 | 16.1 | 6.6 |
| Anatabine | Anatabine 0.75 mg/kg | Rat Plasma | male | 4 | 3 | 34.7 | 5.1 | 3.0 | 40.0 | 6 | 7.3 | 3.0 |
| Anatabine | Anatabine 0.75 mg/kg | Rat Plasma | male | 6 | 3 | 14.0 | 1.0 | 0.6 | 19.3 | 12 | 7.8 | 2.2 |
| Anatabine | Anatabine 0.75 mg/kg | Rat Plasma | male | 6 | 3 | 22.0 | 8.2 | 4.7 | 22.5 | 6 | 9.7 | 4.0 |
| Anatabine | Anatabine 0.75 mg/kg | Rat Plasma | male | 8 | 3 | 9.0 | 1.7 | 1.0 | 10.0 | 6 | 1.7 | 0.7 |
| Anatabine | Anatabine 0.75 mg/kg | Rat Plasma | male | 24 | 0 | <LLQ | ND | ND | <LLQ | 0 | ND | ND |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 0.75 mg/kg | Rat Plasma | female | 0.25 | 3 | 212.3 | 11.9 | 6.9 | | | | |
| Anatabine | Anatabine 0.75 mg/kg | Rat Plasma | female | 0.5 | 3 | 190.0 | 34.1 | 19.7 | | | | |
| Anatabine | Anatabine 0.75 mg/kg | Rat Plasma | female | 0.5 | 3 | 354.0 | 44.7 | 25.8 | | | | |
| Anatabine | Anatabine 0.75 mg/kg | Rat Plasma | female | 1 | 3 | 172.7 | 31.2 | 18.0 | | | | |
| Anatabine | Anatabine 0.75 mg/kg | Rat Plasma | female | 1.5 | 3 | 140.0 | 8.7 | 5.0 | | | | |
| Anatabine | Anatabine 0.75 mg/kg | Rat Plasma | female | 2 | 3 | 99.7 | 12.2 | 7.1 | | | | |
| Anatabine | Anatabine 0.75 mg/kg | Rat Plasma | female | 4 | 3 | 45.3 | 4.5 | 2.6 | | | | |
| Anatabine | Anatabine 0.75 mg/kg | Rat Plasma | female | 6 | 3 | 18.3 | 4.5 | 2.6 | | | | |
| Anatabine | Anatabine 0.75 mg/kg | Rat Plasma | female | 6 | 3 | 23.0 | 13.0 | 7.5 | | | | |
| Anatabine | Anatabine 0.75 mg/kg | Rat Plasma | female | 8 | 3 | 11.0 | 1.0 | 0.6 | | | | |
| Anatabine | Anatabine 0.75 mg/kg | Rat Plasma | female | 24 | 0 | <LLQ | ND | ND | | | | |
| Anatabine | Anatabine 1.0 mg/kg | Rat Plasma | male | 0.25 | 3 | 285.7 | 13.8 | 8.0 | 287.2 | 6 | 13.4 | 5.5 |
| Anatabine | Anatabine 1.0 mg/kg | Rat Plasma | male | 0.5 | 3 | 272.3 | 13.6 | 7.8 | 319.1 | 12 | 73.1 | 21.1 |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 1.0 mg/kg | Rat Plasma | male | 0.5 | 3 | 399.0 | 42.5 | 24.6 | 383.5 | 6 | 35.4 | 14.5 |
| Anatabine | Anatabine 1.0 mg/kg | Rat Plasma | male | 1 | 3 | 233.7 | 20.4 | 11.8 | 231.3 | 6 | 15.7 | 6.4 |
| Anatabine | Anatabine 1.0 mg/kg | Rat Plasma | male | 1.5 | 3 | 157.0 | 7.9 | 4.6 | 178.3 | 6 | 24.7 | 10.1 |
| Anatabine | Anatabine 1.0 mg/kg | Rat Plasma | male | 2 | 3 | 123.0 | 5.3 | 3.1 | 143.3 | 6 | 24.3 | 9.9 |
| Anatabine | Anatabine 1.0 mg/kg | Rat Plasma | male | 4 | 3 | 42.3 | 7.8 | 4.5 | 51.5 | 6 | 13.2 | 5.4 |
| Anatabine | Anatabine 1.0 mg/kg | Rat Plasma | male | 6 | 3 | 18.0 | 2.6 | 1.5 | 24.3 | 12 | 7.4 | 2.1 |
| Anatabine | Anatabine 1.0 mg/kg | Rat Plasma | male | 6 | 3 | 27.0 | 10.5 | 6.1 | 25.0 | 6 | 7.6 | 3.1 |
| Anatabine | Anatabine 1.0 mg/kg | Rat Plasma | male | 8 | 3 | 7.7 | 1.5 | 0.9 | 12.7 | 6 | 5.8 | 2.3 |
| Anatabine | Anatabine 1.0 mg/kg | Rat Plasma | male | 24 | 0 | <LLQ | ND | ND | <LLQ | 0 | ND | ND |
| Anatabine | Anatabine 1.0 mg/kg | Rat Plasma | female | 0.25 | 3 | 288.7 | 15.9 | 9.2 | | | | |
| Anatabine | Anatabine 1.0 mg/kg | Rat Plasma | female | 0.5 | 3 | 237.0 | 15.5 | 9.0 | | | | |
| Anatabine | Anatabine 1.0 mg/kg | Rat Plasma | female | 0.5 | 3 | 368.0 | 24.6 | 14.2 | | | | |
| Anatabine | Anatabine 1.0 mg/kg | Rat Plasma | female | 1 | 3 | 229.0 | 13.5 | 7.8 | | | | |

(continued)

| Compound | Dose Group | Tissue | Sex | Time Point (hr) | Male or Female | | | | Combined Male and Female | | | |
|---|---|---|---|---|---|---|---|---|---|---|---|---|
| | | | | | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Anatabine | Anatabine 1.0 mg/kg | Rat Plasma | female | 1.5 | 3 | 199.7 | 10.0 | 5.8 | | | | |
| Anatabine | Anatabine 1.0 mg/kg | Rat Plasma | female | 2 | 3 | 163.7 | 14.6 | 8.4 | | | | |
| Anatabine | Anatabine 1.0 mg/kg | Rat Plasma | female | 4 | 3 | 60.7 | 11.0 | 6.3 | | | | |
| Anatabine | Anatabine 1.0 mg/kg | Rat Plasma | female | 6 | 3 | 29.3 | 7.2 | 4.2 | | | | |
| Anatabine | Anatabine 1.0 mg/kg | Rat Plasma | female | 6 | 3 | 23.0 | 4.6 | 2.6 | | | | |
| Anatabine | Anatabine 1.0 mg/kg | Rat Plasma | female | 8 | 3 | 17.7 | 2.3 | 1.3 | | | | |
| Anatabine | Anatabine 1.0 mg/kg | Rat Plasma | female | 24 | 0 | <LLQ | ND | ND | | | | |

Table 16. Dosing solutions

| Test compound | Percentage content of anatabine or nicotine base | Dose level (mg/kg) | Test compound base concentration (mg/mL) | Injection volume (mL/kg) |
|---|---|---|---|---|
| Anatabine | 41.6 | 0.10 | 0.048 | 5 |
| Anatabine | 41.6 | 0.75 | 0.36 | 5 |
| Anatabine | 41.6 | 1.5 | 0.72 | 5 |
| Nicotine | 35.1 | 0.75 | 0.36 | 5 |

Table 17. Study Outline

| Test compound | Route | Dose level (mg/kg) | Number of animals (M/F) | Observations, body weight and food consumption frequency |
|---|---|---|---|---|
| Vehicle | i.v. | 0.0 | 5/5 | Daily for 14 days |
| Anatabine | i.v. | 0.10 | 5/5 | Daily for 14 days |
| Anatabine | i.v. | 0.75 | 5/5 | Daily for 14 days |
| Anatabine | i.v. | 1.5 | 5/5 | Daily for 14 days |
| Nicotine | i.v. | 1.5 | 5/5 | Not applicable |
| Nicotine[1] | i.v. | 0.75 | 5/5 | Daily for 14 days |

Table 18. Test Performed and Tissues Collected

| Parameter | Tests performed/tissues collected |
|---|---|
| Hematology | Hematocrit, Hemoglobin, MCH, MCH Concentration, MCV, RBC, Reticulocyte count, Platelet count, WBC, WBC differential, blood smear evaluation |
| Clinical Chemistry | A/G ratio (calculated), ALT, Albumin, Alkaline phosphatase, AST. Bilirubin, Calcium, Chloride, Cholesterol (total), Creatinine, Globulin (calculated), Glucose, Phosphorus (inorganic), Potassium, Sodium, Total protein, BUN |
| Coagulation | Activated partial thromboplastin time, Prothrombin time |
| Urinalysis | Bilirubin, Blood, color and appearance, Glucose, Ketones, pH, Protein, Specific gravity, Urobilinogen, Volume |
| Necropsy | Adrenal glands[1], Brain, Heart, Kidneys, Liver, Lungs, Ovaries and Uterus, Pituitary gland[1]. Prostate gland, Spleen, Testes, Thymus, Thyroid and Parathyroid glands[1], section of small intestines |
| [1] Not weighed; placed in cassettes. | |

Table 19. Dosing Solution Analysis

| Compound | Dosing Date | Dose (mg/kg) | Expected Conc. (mg/ml) | Actual Concentration (mg/mL) | Actual Concentration relative to Expected % |
|---|---|---|---|---|---|
| Nicotine | Jun 16 | 0.4 | 0.081 | 0.096 | 118.5 |
| Anatabine | Jun 09 | 0.1 | 0.02 | 0.016 | 80 |
| Anatabine | Jun 09 | 0.75 | 0.15 | 0.127 | 85 |
| Anatabine | Jun 09 | 1.5 | 0.2 | 0.239 | 119.5 |

**Table 20. Mean Increase in Body Weights and Daily Food Consumption (descriptive statistics)**

| Test Compound | Dose (mg/kg) | Gender | Number of animals | Mean weight increase by day 14 (g) | Standard Deviation | *p* (comparison to Vehicle) | Mean food consumption/ day (g) | Standard Deviation | *p* (comparison to Vehicle) |
|---|---|---|---|---|---|---|---|---|---|
| Vehicle | - | Male | 5 | 130.6 | 16.8 | | 27.7 | 2.8 | |
| | | Female | 5 | 49.7 | 6.4 | | 20.3 | 2.7 | |
| Anatabine | 0.1 | Male | 5 | 132.5 | 17.1 | 0.864 | 31.0 | 4.0 | **0.001** |
| | | Female | 5 | 39.0 | 10.3 | 0.083 | 20.8 | 3.6 | 0.535 |
| Anatabine | 0.75 | Male | 5 | 111.6 | 8.9 | 0.055 | 27.8 | 3.2 | 0.936 |
| | | Female | 5 | 43.1 | 9.3 | 0.229 | 19.7 | 2.8 | 0.434 |
| Anatabine | 1.5 | Male | 5 | 137.9 | 14.0 | 0.475 | 30.8 | 4.0 | **0.001** |
| | | Female | 5 | 38.6 | 13.0 | 0.125 | 19.8 | 3.0 | 0.504 |
| Nicotine | 0.75 | Male | 4 | 98.5 | 15.8 | **0.0001** | 26.4 | 3.2 | 0.098 |
| | | Female | 3 | 40.8 | 3.4 | 0.071 | 20.6 | 2.6 | 0.598 |

**Table 21. Mean Organ Weights (descriptive statistics) for Male Animals by Treatment Group**

| Test Compound | Dose (mg/kg) | | Organ Weight (g) Thymus | Heart | Lungs | Liver | Kidneys | Spleen | Small intestine | Prostate | Testes | Brain |
|---|---|---|---|---|---|---|---|---|---|---|---|---|
| Vehicle | - n=5 | Mean | 0.77 | 1.66 | 1.90 | 15.81 | 3.53 | 1.03 | 0.64 | 0.49 | 3.39 | 2.02 |
| | | Std Dev | 0.04 | 0.18 | 0.21 | 1.44 | 0.30 | 0.24 | 0.43 | 0.11 | 0.07 | 0.12 |
| Anatabine | 0.1 n=5 | Mean | 0.75 | 1.74 | 1.98 | 16.81 | 3.57 | 0.82 | 0.66 | 0.59 | 3.42 | 2.15 |
| | | Std Dev | 0.04 | 0.16 | 0.22 | 0.96 | 0.29 | 0.11 | 0.26 | 0.12 | 0.24 | 0.18 |
| | | *p*[a] | ns[b] | ns | ns | ns | ns | ns | ns | ns | ns | ns |
| Anatabine | 0.75 n=5 | Mean | 0.67 | 1.43 | 1.82 | 14.50 | 3.15 | 0.74 | 0.68 | 0.51 | 3.21 | 2.07 |
| | | Std Dev | 0.22 | 0.11 | 0.16 | 0.50 | 0.32 | 0.12 | 0.41 | 0.08 | 0.42 | 0.08 |
| | | *p* | ns | **0.04** | ns | ns | ns | **0.04** | ns | ns | ns | ns |
| Anatabine | 1.5 n=5 | Mean | 0.72 | 1.52 | 1.94 | 16.55 | 3.45 | 0.86 | 0.68 | 0.47 | 3.24 | 1.93 |
| | | Std Dev | 0.14 | 0.06 | 0.11 | 1.55 | 0.28 | 0.10 | 0.22 | 0.03 | 0.28 | 0.14 |
| | | *p* | ns | ns | ns | ns | ns | ns | ns | ns | ns | ns |
| Nicotine | 0.75 n=4 | Mean | 0.58 | 1.25 | 1.65 | 12.69 | 3.03 | 0.77 | 0.55 | 0.36 | 3.03 | 1.94 |
| | | Std Dev | 0.13 | 0.11 | 0.14 | 1.04 | 0.13 | 0.10 | 0.13 | 0.09 | 0.09 | 0.09 |
| | | *p* | ns | **0.006** | ns | **0.008** | **0.017** | ns | ns | ns | ns | ns |

[a] *p*, probability relative to Vehicle control; [b] ns, not significant

**Table 22 Mean Organ Weights (descriptive statistics) for Female Animals by Treatment Group**

| Test Compound | Dose (mg/kg) | | Organ Weight (g) | | | | | | | | |
|---|---|---|---|---|---|---|---|---|---|---|---|
| | | | Thymus | Heart | Lungs | Liver | Kidneys | Spleen | Small intestine | Ovaries/ uterus | Brain |
| Vehicle | - n=5 | Mean | 0.69 | 1.27 | 1.52 | 10.94 | 2.34 | 0.61 | 0.82 | 1.10 | 1.93 |
| | | Std Dev | 0.10 | 0.09 | 0.13 | 1.18 | 0.40 | 0.09 | 0.39 | 0.53 | 0.15 |
| Anatabine | 0.1 n=5 | Mean | 0.64 | 1.13 | 1.41 | 10.84 | 2.18 | 0.65 | 0.65 | 1.14 | 1.86 |
| | | Std Dev | 0.10 | 0.14 | 0.17 | 1.38 | 0.18 | 0.10 | 0.15 | 0.45 | 0.06 |
| | | *p*[a] | ns[b] | ns | ns | ns | ns | ns | ns | ns | ns |
| Anatabine | 0.75 n=5 | Mean | 0.56 | 1.11 | 1.45 | 10.42 | 2.30 | 0.60 | 0.75 | 1.58 | 1.90 |
| | | Std Dev | 0.04 | 0.11 | 0.12 | 1.09 | ns | 0.09 | 0.38 | 1.20 | 0.13 |
| | | *p* | **0.026** | **0.033** | ns | ns | ns | ns | ns | ns | ns |
| Anatabine | 1.5 n=5 | Mean | 0.57 | 1.00 | 1.46 | 10.04 | 2.28 | 0.60 | 0.70 | 1.03 | 1.95 |
| | | Std Dev | 0.05 | 0.09 | 0.14 | 1.16 | 0.29 | 0.08 | 0.23 | 0.24 | 0.08 |
| | | *p* | ns | **0.001** | ns | ns | ns | ns | ns | ns | ns |
| Nicotine | 0.75 n=3 | Mean | 0.50 | 0.99 | 1.32 | 9.59 | 2.31 | 0.56 | 0.36 | 1.28 | 1.86 |
| | | Std Dev | 0.03 | 0.14 | 0.14 | 0.47 | 0.03 | 0.17 | 0.07 | 0.34 | 0.08 |
| | | *p* | **0.022** | **0.013** | ns | ns | ns | ns | ns | ns | ns |

[a] *p*, probability relative to Vehicle control
[b] ns, not significant

**Table 23A. Hematology Parameters (descriptive statistics) (Part 1) by Treatment Group and Gender**

| Treatment Group | Gender | | WBC x $10^3/\mu L$ | RBC x $10^6/\mu L$ | HGB g/dL | HCT % | MCV fL | MCH pg | MCHC % | RETICULOCYTE COUNT % | PLATELET COUNT x $10^3/\mu L$ |
|---|---|---|---|---|---|---|---|---|---|---|---|
| Vehicle | M | n | 5 | 5 | 5 | 5 | 5 | 5 | 5 | 5 | 4 |
| | | Mean | 10.5 | 7.3 | 15.2 | 45.4 | 62.2 | 20.7 | 33.4 | 6.1 | 1287.3 |
| | | Std Dev | 1.4 | 0.2 | 0.5 | 1.5 | 1.5 | 0.6 | 0.4 | 1.0 | 210.8 |
| | F | N | 5 | 5 | 5 | 5 | 5 | 5 | 5 | 5 | 5 |
| | | Mean | 9.3 | 7.8 | 16.4 | 47.3 | 61.0 | 21.0 | 34.5 | 5.0 | 1198.4 |
| | | Std Dev | 2.8 | 0.7 | 1.7 | 4.7 | 1.2 | 0.6 | 0.8 | 1.1 | 200.2 |
| Anatabine 0.1 mg/kg | M | n | 5 | 5 | 5 | 5 | 5 | 5 | 5 | 5 | 5 |
| | | Mean | 15.6 | 7.4 | 15.8 | 47.1 | 63.4 | 21.2 | 33.5 | 5.5 | 1248.6 |
| | | Std Dev | 4.7 | 0.4 | 1.2 | 2.9 | 1.8 | 0.6 | 0.9 | 0.5 | 358.1 |
| | | *p*[a] | ns[b] | ns | ns | ns | ns | ns | ns | ns | ns |
| | F | n | 5 | 5 | 5 | 5 | 5 | 5 | 5 | 5 | 3 |
| | | Mean | 9.3 | 7.8 | 15.6 | 46.4 | 60.0 | 20.2 | 33.7 | 4.5 | 997.3 |
| | | Std Dev | 3.4 | 0.7 | 1.0 | 3.2 | 2.0 | 0.8 | 0.3 | 1.5 | 165.8 |
| | | *p* | ns | ns | ns | ns | ns | ns | **0.042**[c] | ns | ns |
| Anatabine 0.75 mg/kg | M | n | 5 | 5 | 5 | 5 | 5 | 5 | 5 | 5 | 4 |
| | | Mean | 12.1 | 7.5 | 15.6 | 45.8 | 61.4 | 20.9 | 34.0 | 4.4 | 1325.8 |
| | | Std Dev | 5.7 | 0.7 | 1.5 | 4.0 | 1.5 | 0.8 | 0.7 | 0.6 | 193.7 |
| | | *p* | ns | ns | ns | ns | ns | ns | ns | **0.012**[c] | ns |
| | F | n | 5 | 5 | 5 | 5 | 5 | 5 | 5 | 5 | 5 |
| | | Mean | 10.3 | 7.5 | 15.4 | 45.2 | 59.8 | 20.4 | 34.1 | 3.3 | 1269.6 |
| | | Std Dev | 4.0 | 0.3 | 0.5 | 1.5 | 2.3 | 0.8 | 0.3 | 0.9 | 356.4 |
| | | *p* | ns | ns | ns | ns | ns | ns | ns | **0.022**[c] | ns |

**Table 23B. Hematology Parameters (descriptive statistics) (Part 1) by Treatment Group and Gender – cont'd**

| Treatment Group | Gender | | WBC x $10^3/\mu L$ | RBC x $10^6/\mu L$ | HGB g/dL | HCT % | MCV fL | MCH pg | MCHC % | RETICULOCYTE COUNT % | PLATELET COUNT x $10^3/\mu L$ |
|---|---|---|---|---|---|---|---|---|---|---|---|
| Anatabine 1.5 mg/kg | M | n | 5 | 5 | 5 | 5 | 5 | 5 | 5 | 5 | 5 |
| | | Mean | 13.1 | 7.5 | 15.4 | 46.1 | 62.0 | 20.6 | 33.4 | 4.2 | 1222.5 |
| | | Std Dev | 3.1 | 0.2 | 0.6 | 2.2 | 1.9 | 0.5 | 0.4 | 0.8 | 248.4 |
| | | *p* | ns | ns | ns | ns | ns | ns | ns | **0.011[c]** | ns |
| | F | n | 5 | 5 | 5 | 5 | 5 | 5 | 5 | 5 | 5 |
| | | Mean | 9.2 | 7.7 | 15.2 | 45.4 | 58.8 | 19.6 | 33.4 | 3.2 | 1127.4 |
| | | Std Dev | 2.5 | 0.4 | 0.7 | 2.7 | 0.8 | 0.4 | 0.6 | 0.9 | 503.7 |
| | | *p* | ns | ns | ns | ns | **0.011[c]** | **0.002[c]** | **0.036[c]** | **0.021[c]** | ns |
| Nicotine 0.75 mg/kg | M | n | 4 | 4 | 4 | 4 | 4 | 4 | 4 | 4 | 4 |
| | | Mean | 11.4 | 7.6 | 15.8 | 46.6 | 61.5 | 20.9 | 34.0 | 5.2 | 1355.8 |
| | | Std Dev | 3.2 | 0.5 | 0.7 | 2.4 | 1.0 | 0.6 | 0.5 | 0.8 | 83.0 |
| | | *p* | ns | ns | ns | ns | ns | ns | ns | ns | ns |
| | F | n | 3 | 3 | 3 | 3 | 3 | 3 | 3 | 3 | 2 |
| | | Mean | 10.2 | 8.0 | 16.0 | 46.7 | 58.7 | 19.9 | 34.2 | 5.1 | 1363.0 |
| | | Std Dev | 3.1 | 0.5 | 0.7 | 2.2 | 1.2 | 0.5 | 0.2 | 0.7 | 248.9 |
| | | *p* | ns | ns | ns | ns | **0.038[c]** | **0.038[c]** | ns | ns | ns |

[a] *p*, probability relative to Vehicle control
[b] ns, not significant
[c] Mean within normal range

**Table 24. Hematology Parameters (descriptive statistics) (Part 2) by Treatment Group and Gender**

| Treatment Group | Gender | | NEUTROPHIL SEG % | LYMPHOCYTE % | MONOCYTE % | EOSINOPHIL % | BASOPHIL % |
|---|---|---|---|---|---|---|---|
| Vehicle | M | n | 5 | 5 | 5 | 5 | 5 |
| | | Mean | 8.8 | 89.6 | 1.6 | 0.0 | 0.0 |
| | | Std Dev | 2.2 | 2.3 | 1.5 | 0.0 | 0.0 |
| | F | N | 5 | 5 | 5 | 5 | 5 |
| | | Mean | 12.6 | 85.2 | 2.2 | 0.0 | 0.0 |
| | | Std | 2.6 | 2.9 | 0.4 | 0.0 | 0.0 |
| Anatabine 0.1 mg/kg | M | n | 5 | 5 | 5 | 5 | 5 |
| | | Mean | 8.6 | 89.2 | 2.0 | 0.2 | 0.0 |
| | | Std Dev | 3.6 | 2.9 | 1.0 | 0.4 | 0.0 |
| | | *p*[a] | ns[b] | ns | ns | ns | ns |
| | F | n | 5 | 5 | 5 | 5 | 5 |
| | | Mean | 8.0 | 90.2 | 1.8 | 0.0 | 0.0 |
| | | Std Dev | 1.9 | 1.9 | 0.8 | 0.0 | 0.0 |
| | | *p* | ns | ns | ns | ns | ns |
| Anatabine 0.75 mg/kg | M | n | 5 | 5 | 5 | 5 | 5 |
| | | Mean | 9.8 | 88.8 | 1.4 | 0.0 | 0.0 |
| | | Std Dev | 2.5 | 2.2 | 0.5 | 0.0 | 0.0 |
| | | *p* | ns | ns | ns | ns | ns |
| | F | n | 5 | 5 | 5 | 5 | 5 |
| | | Mean | 8.6 | 90.6 | 0.8 | 0.0 | 0.0 |
| | | Std Dev | 2.3 | 2.6 | 0.8 | 0.0 | 0.0 |
| | | *p* | ns | ns | ns | ns | ns |
| Anatabine 1.5 mg/kg | M | n | 5 | 5 | 5 | 5 | 5 |
| | | Mean | 14.6 | 83.8 | 1.6 | 0.0 | 0.0 |
| | | Std Dev | 7.7 | 8.0 | 0.9 | 0.0 | 0.0 |
| | | *p* | ns | ns | ns | ns | ns |
| | F | n | 5 | 5 | 5 | 5 | 5 |
| | | Mean | 12.6 | 86.6 | 0.8 | 0.0 | 0.0 |
| | | Std Dev | 4.4 | 3.8 | 0.8 | 0.0 | 0.0 |
| | | *p* | ns | ns | ns | ns | ns |
| Nicotine 0.75 mg/kg | M | n | 4 | 4 | 4 | 4 | 4 |
| | | Mean | 11.8 | 85.8 | 2.5 | 0.0 | 0.0 |
| | | Std Dev | 3.3 | 4.2 | 1.0 | 0.0 | 0.0 |
| | | *p* | ns | ns | ns | ns | ns |
| | F | n | 3 | 3 | 3 | 3 | 3 |
| | | Mean | 12.7 | 86.3 | 1.0 | 0.0 | 0.0 |
| | | Std Dev | 2.1 | 3.1 | 1.0 | 0.0 | 0.0 |
| | | *p* | ns | ns | ns | ns | ns |

[a] *p*, probability relative to Vehicle control
[b] ns, not significant

**Table 25. Coagulation Parameters (descriptive statistics) by Treatment Group and Gender**

| Treatment Group | Gender | | ACTIVATED PARTIAL THROMBOPLASTIN TIME (seconds) | PROTHROMBIN TIME (seconds) |
|---|---|---|---|---|
| Vehicle | M | n | 5 | 5 |
| | | Mean | 32.0 | 12.9 |
| | | Std Dev | 2.2 | 0.5 |
| | F | n | 5 | 5 |
| | | Mean | 35.4 | 12.7 |
| | | Std Dev | 4.1 | 0.3 |
| Anatabine 0.1 mg/kg | M | n | 5 | 5 |
| | | Mean | 32.4 | 12.8 |
| | | Std Dev | 3.3 | 0.7 |
| | | *p*[a] | ns[b] | ns |
| | F | n | 5 | 5 |
| | | Mean | 29.4 | 12.1 |
| | | Std Dev | 3.4 | 0.4 |
| | | *p* | ns | ns |
| Anatabine 0.75 mg/kg | M | n | 5 | 5 |
| | | Mean | 38.7 | 13.3 |
| | | Std Dev | 9.1 | 2.6 |
| | | *p* | ns | ns |
| | F | n | 5 | 5 |
| | | Mean | 33.3 | 14.1 |
| | | Std Dev | 2.8 | 5.5 |
| | | *p* | ns | ns |
| Anatabine 1.5 mg/kg | M | n | 5 | 5 |
| | | Mean | 30.6 | 13.2 |
| | | Std Dev | 3.1 | 0.4 |
| | | *p* | ns | ns |
| | F | n | 5 | 5 |
| | | Mean | 32.5 | 13.3 |
| | | Std Dev | 2.9 | 0.2 |
| | | *p* | ns | ns |
| Nicotine 0.75 mg/kg | M | n | 4 | 4 |
| | | Mean | 16.3 | 13.6 |
| | | Std Dev | 1.1 | 0.6 |
| | | *p* | **<0.001**[c] | ns |
| | F | n | 2 | 2 |
| | | Mean | 16.9 | 14.1 |
| | | Std Dev | 2.6 | 0.2 |
| | | *p* | **0.002**[c] | ns |

[a] *p*, probability relative to Vehicle control
[b] ns, not significant
[c] Mean within normal range

**Table 26A. Clinical Chemistry Parameters (descriptive statistics) (Part 1) by Treatment Group and Gender**

| Treatment Group | Gender | | ALK PHOSPHATASE IU/L | ALT IU/L | AST IU/L | ALBUMIN g/dL | TOT PROTEIN g/dL | GLOBULIN g/dL | TOT BILIRUBIN mg/dL | DIR BILIRUBIN mg/dL | BUN mg/dL |
|---|---|---|---|---|---|---|---|---|---|---|---|
| Vehicle | M | n | 5 | 5 | 5 | 5 | 5 | 5 | 5 | 5 | 5 |
| | | Mean | 370.80 | 59.40 | 99.00 | 3.12 | 5.62 | 2.50 | 0.00 | 0.00 | 18.60 |
| | | Std Dev | 72.47 | 2.07 | 22.86 | 0.04 | 0.04 | 0.00 | 0.00 | 0.00 | 1.14 |
| | F | N | 5 | 5 | 5 | 5 | 5 | 5 | 5 | 5 | 5 |
| | | Mean | 230.20 | 58.20 | 80.80 | 3.40 | 6.24 | 2.84 | 0.00 | 0.00 | 18.20 |
| | | Std Dev | 54.56 | 4.44 | 11.32 | 0.07 | 0.09 | 0.11 | 0.00 | 0.00 | 4.44 |
| Anatabine 0.1 mg/kg | M | n | 5 | 5 | 5 | 5 | 5 | 5 | 5 | 5 | 5 |
| | | Mean | 407.40 | 70.60 | 96.00 | 3.24 | 6.10 | 2.86 | 0.00 | 0.00 | 18.00 |
| | | Std Dev | 145.89 | 17.77 | 7.75 | 0.15 | 0.23 | 0.18 | 0.00 | 0.00 | 2.55 |
| | | *p*[a] | ns[b] | ns | ns | ns | **0.002**[c] | **0.002**[c] | ns | ns | ns |
| | F | n | 5 | 5 | 5 | 5 | 5 | 5 | 5 | 5 | 5 |
| | | Mean | 248.40 | 49.60 | 77.00 | 3.62 | 6.64 | 3.02 | 0.00 | 0.00 | 19.80 |
| | | Std Dev | 101.28 | 9.63 | 7.42 | 0.11 | 0.22 | 0.13 | 0.00 | 0.00 | 1.10 |
| | | *p* | ns | ns | ns | **0.005**[c] | **0.005**[c] | **0.049**[c] | ns | ns | ns |
| Anatabine 0.75 mg/kg | M | n | 5 | 5 | 5 | 5 | 5 | 5 | 5 | 5 | 5 |
| | | Mean | 370.80 | 66.80 | 89.60 | 3.28 | 6.04 | 2.76 | 0.02 | 0.02 | 16.60 |
| | | Std Dev | 74.89 | 14.64 | 14.12 | 0.13 | 0.24 | 0.11 | 0.04 | 0.04 | 3.36 |
| | | *p* | ns | ns | ns | **0.032**[c] | **0.005**[c] | **0.001**[c] | ns | ns | ns |
| | F | n | 5 | 5 | 5 | 5 | 5 | 5 | 5 | 5 | 5 |
| | | Mean | 158.00 | 54.60 | 73.20 | 3.52 | 6.38 | 2.86 | 0.00 | 0.00 | 15.40 |
| | | Std Dev | 45.27 | 5.90 | 14.75 | 0.08 | 0.16 | 0.13 | 0.00 | 0.00 | 0.55 |
| | | *p* | ns | ns | ns | **0.040**[c] | ns | ns | ns | ns | ns |

**Table 26B. Clinical Chemistry Parameters (descriptive statistics) (Part 1) by Treatment Group and Gender – cont'd**

| Treatment Group | Gender | | ALK PHOSPHATASE IU/L | ALT IU/L | AST IU/L | ALBUMIN g/dL | TOT PROTEIN g/dL | GLOBULIN g/dL | TOT BILIRUBIN mg/dL | DIR BILIRUBIN mg/dL | BUN mg/dL |
|---|---|---|---|---|---|---|---|---|---|---|---|
| Anatabine 1.5 mg/kg | M | n | 5 | 5 | 5 | 5 | 5 | 5 | 5 | 5 | 5 |
| | | Mean | 411.40 | 70.40 | 97.00 | 3.32 | 6.00 | 2.68 | 0.00 | 0.00 | 17.20 |
| | | Std Dev | 133.64 | 20.61 | 26.88 | 0.13 | 0.25 | 0.13 | 0.00 | 0.00 | 1.30 |
| | | *p* | ns | ns | ns | **0.012**[c] | **0.011**[c] | **0.015**[c] | ns | ns | ns |
| | F | n | 5 | 5 | 5 | 5 | 5 | 5 | 5 | 5 | 5 |
| | | Mean | 194.00 | 49.20 | 76.60 | 3.48 | 6.36 | 2.88 | 0.02 | 0.02 | 16.20 |
| | | Std Dev | 58.97 | 6.18 | 9.21 | 0.15 | 0.28 | 0.16 | 0.04 | 0.04 | 2.17 |
| | | *p* | ns | ns | ns | ns | ns | ns | ns | ns | ns |
| Nicotine 0.75 mg/kg | M | n | 4 | 4 | 4 | 4 | 4 | 4 | 4 | 4 | 4 |
| | | Mean | 291.00 | 55.75 | 82.25 | 3.08 | 6.00 | 2.93 | 0.05 | 0.00 | 17.50 |
| | | Std Dev | 51.59 | 8.46 | 7.63 | 0.05 | 0.16 | 0.17 | 0.06 | 0.00 | 1.73 |
| | | *p* | ns | ns | ns | ns | **0.001**[c] | **0.001**[c] | ns | ns | ns |
| | F | n | 3 | 3 | 3 | 3 | 3 | 3 | 3 | 3 | 3 |
| | | Mean | 188.67 | 68.33 | 103.00 | 3.30 | 6.50 | 3.20 | 0.10 | 0.03 | 19.00 |
| | | Std Dev | 74.33 | 24.83 | 29.05 | 0.00 | 0.10 | 0.10 | 0.00 | 0.06 | 1.73 |
| | | *p* | ns | ns | ns | ns | **0.009**[c] | **0.004**[c] | ns | ns | ns |

[a] *p*, probability relative to Vehicle control
[b] ns, not significant
[c] Mean within normal range

**Table 27A. Clinical Chemistry Parameters (descriptive statistics) (Part 2) by Treatment Group and Gender**

| Treatment Group | Gender | | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RATIO |
|---|---|---|---|---|---|---|---|---|---|---|---|
| Vehicle | M | n | 5 | 5 | 5 | 5 | 5 | 5 | 5 | 5 | 5 |
| | | Mean | 0.36 | 60.00 | 222.00 | 12.12 | 10.82 | 98.60 | 6.30 | 145.40 | 1.22 |
| | | Std Dev | 0.05 | 4.06 | 47.00 | 0.31 | 0.51 | 1.52 | 0.29 | 1.14 | 0.04 |
| | F | n | 5 | 5 | 5 | 5 | 5 | 5 | 5 | 5 | 5 |
| | | Mean | 0.42 | 67.40 | 198.20 | 11.66 | 9.06 | 99.80 | 5.92 | 145.60 | 1.18 |
| | | Std Dev | 0.04 | 11.48 | 13.03 | 0.34 | 0.61 | 0.45 | 0.50 | 0.55 | 0.08 |
| Anatabine 0.1 mg/kg | M | n | 5 | 5 | 5 | 5 | 5 | 5 | 5 | 5 | 5 |
| | | Mean | 0.36 | 67.20 | 219.00 | 12.00 | 10.82 | 98.00 | 6.40 | 146.20 | 1.14 |
| | | Std Dev | 0.05 | 8.35 | 23.73 | 0.39 | 0.54 | 1.00 | 0.40 | 1.48 | 0.09 |
| | | *p*[a] | ns[b] | ns | ns | ns | ns | ns | ns | ns | ns |
| | F | n | 5 | 5 | 5 | 5 | 5 | 5 | 5 | 5 | 5 |
| | | Mean | 0.44 | 76.40 | 207.60 | 11.72 | 8.08 | 100.20 | 5.82 | 146.80 | 1.22 |
| | | Std Dev | 0.05 | 11.63 | 24.11 | 0.58 | 0.48 | 2.17 | 0.65 | 1.30 | 0.04 |
| | | *p* | ns | ns | ns | ns | ns | ns | ns | ns | ns |
| Anatabine 0.75 mg/kg | M | n | 5 | 5 | 5 | 5 | 5 | 5 | 5 | 5 | 5 |
| | | Mean | 0.36 | 62.80 | 202.00 | 12.04 | 10.92 | 98.60 | 6.12 | 147.80 | 1.20 |
| | | Std Dev | 0.05 | 6.38 | 25.25 | 0.38 | 0.85 | 1.14 | 0.45 | 1.48 | 0.00 |
| | | *p* | ns | ns | ns | ns | ns | ns | ns | **0.021**[c] | ns |
| | F | n | 5 | 5 | 5 | 5 | 5 | 5 | 5 | 5 | 5 |
| | | Mean | 0.44 | 69.80 | 220.80 | 11.68 | 8.64 | 98.00 | 5.88 | 145.00 | 1.24 |
| | | Std Dev | 0.05 | 9.88 | 21.73 | 0.29 | 1.22 | 2.00 | 0.30 | 1.22 | 0.05 |
| | | *p* | ns | ns | ns | ns | ns | ns | ns | ns | ns |

**Table 27B. Clinical Chemistry Parameters (descriptive statistics) (Part 2) by Treatment Group and Gender – cont'd**

| Treatment Group | Gender | | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RATIO |
|---|---|---|---|---|---|---|---|---|---|---|---|
| Anatabine 1.5 mg/kg | M | n | 5 | 5 | 5 | 5 | 5 | 5 | 5 | 5 | 5 |
| | | Mean | 0.36 | 62.40 | 205.60 | 11.88 | 11.00 | 99.80 | 6.02 | 147.80 | 1.24 |
| | | Std Dev | 0.05 | 4.39 | 6.11 | 0.51 | 0.83 | 0.84 | 0.77 | 1.30 | 0.05 |
| | | *p* | ns | ns | ns | ns | ns | ns | ns | **0.015 [c]** | ns |
| | F | n | 5 | 5 | 5 | 5 | 5 | 5 | 5 | 5 | 5 |
| | | Mean | 0.40 | 70.00 | 188.40 | 11.36 | 8.24 | 100.80 | 5.74 | 147.20 | 1.24 |
| | | Std Dev | 0.00 | 9.92 | 9.07 | 0.35 | 0.59 | 1.92 | 0.59 | 0.84 | 0.05 |
| | | *p* | ns | ns | ns | ns | ns | ns | ns | **0.007 [c]** | ns |
| Nicotine 0.75 mg/kg | M | n | 4 | 4 | 4 | 4 | 4 | 4 | 4 | 4 | 4 |
| | | Mean | 0.35 | 61.00 | 201.00 | 11.05 | 10.38 | 99.75 | 6.20 | 146.75 | 1.05 |
| | | Std Dev | 0.06 | 8.49 | 10.42 | 0.06 | 0.58 | 0.96 | 0.50 | 0.96 | 0.06 |
| | | *p* | ns | ns | ns | **<0.001 [c]** | ns | ns | ns | ns | **0.002 [c]** |
| | F | n | 3 | 3 | 3 | 3 | 3 | 3 | 3 | 3 | 3 |
| | | Mean | 0.43 | 65.00 | 238.67 | 11.03 | 8.37 | 98.67 | 6.27 | 145.00 | 1.03 |
| | | Std Dev | 0.06 | 14.93 | 53.72 | 0.21 | 1.02 | 1.53 | 0.50 | 1.73 | 0.06 |
| | | *p* | ns | ns | ns | ns | ns | ns | ns | ns | **0.038 [c]** |

[a] *p*, probability relative to Vehicle control
[b] ns, not significant
[c] Mean within normal range

Table 28A. Dosing Calculations and Body Weights, days 1-5 (anatabine)

| Group | Rat | M/F | B.W. (g) | Dose volume (mL) | time of dosing | day 1 B. W. (g) | day 2 B. W. (g) | day 3 B. W. (g) | day 4 B. W. (g) | day 5 B. W. (g) |
|---|---|---|---|---|---|---|---|---|---|---|
| **A-1** | 1 | M | 250 | 1.25 | 10:39 | 258.0 | 270.0 | 280.3 | 296.0 | 306.6 |
| vehicle | 2 | M | 228 | 1.15 | 10:41 | 238.0 | 251.0 | 257.8 | 273.7 | 286.0 |
| 5 mL/kg | 3 | M | 235 | 1.18 | 10:43 | 241.0 | 250.0 | 260.2 | 278.0 | 287.0 |
| **A-2** | 4 | M | 224 | 1.13 | 10:44 | 230.0 | 239.0 | 247.6 | 262.6 | 267.0 |
| | 5 | M | 236 | 1.18 | 10:45 | 241.0 | 253.1 | 261.7 | 281.0 | 289.0 |
| **A-3** | 6 | F | 207 | 1.03 | 10:49 | 210.2 | 220.5 | 218.1 | 224.2 | 227.0 |
| | 7 | F | 221 | 1.1 | 10:50 | 221.9 | 222.0 | 225.1 | 229.2 | 235.7 |
| | 8 | F | 209 | 1.05 | 10:51 | 210.0 | 214.0 | 216.9 | 219.5 | 220.0 |
| **A-4** | 9 | F | 201 | 1 | 10:51 | 200.0 | 200.1 | 205.0 | 216.7 | 212.0 |
| | 10 | F | 211 | 1.05 | 10:52 | 208.4 | 215.0 | 216.1 | 227.9 | 225.0 |
| **B-1** | 1 | M | 237 | 1.18 | 10:48 | 245.0 | 257.0 | 270.0 | 286.0 | 296.5 |
| Anatabine | 2 | M | 227 | 1.13 | 10:49 | 233.7 | 235.1 | 251.0 | 270.0 | 273.8 |
| 0.1 mg/kg | 3 | M | 230 | 1.15 | 10:50 | 235.0 | 231.0 | 252.0 | 271.2 | 278.5 |
| **B-2** | 4 | M | 243 | 1.23 | 10:52 | 243.5 | 253.5 | 259.4 | 280.5 | 288.3 |
| | 5 | M | 235 | 1.18 | 10:55 | 239.9 | 247.3 | 256.6 | 273.7 | 286.6 |
| **B-3** | 6 | F | 226 | 1.13 | 10:56 | 223.0 | 223.9 | 230.8 | 242.3 | 246.9 |
| | 7 | F | 212 | 1.05 | 10:57 | 211.1 | 215.1 | 213.2 | 222.4 | 226.4 |
| | 8 | F | 207 | 1.03 | 10:58 | 207.0 | 208.1 | 209.1 | 220.7 | 219.3 |
| **B-4** | 9 | F | 205 | 1.03 | 10:58 | 201.0 | 208.8 | 209.1 | 211.4 | 221.0 |
| | 10 | F | 215 | 1.08 | 10:59 | 210.9 | 212.8 | 219.3 | 224.7 | 229.5 |

| Group | Rat | M/F | B.W. (g) | Dose volume (mL) | time | day 1 B. W. (g) | day 2 B. W. (g) | day 3 B. W. (g) | day 4 B. W. (g) | day 4 B. W. (g) |
|---|---|---|---|---|---|---|---|---|---|---|
| **C-1** | 1 | M | 239 | 1.2 | 10:58 | 242.7 | 248.9 | 261.1 | 275.7 | 282.3 |
| Anatabine | 2 | M | 241 | 1.2 | 11:00 | 251.4 | 259.7 | 269.1 | 286.0 | 287.6 |
| 0.75 mg/kg | 3 | M | 229 | 1.15 | 11:02 | 231.9 | 240.0 | 252.0 | 268.0 | 269.9 |
| C-2 | 4 | M | 223 | 1.13 | 11:04 | 226.8 | 232.1 | 243.0 | 256.3 | 262.0 |
| | 5 | M | 240 | 1.2 | 11:06 | 239.8 | 247.2 | 256.1 | 271.2 | 275.7 |
| C-3 | 6 | F | 214 | 1.08 | 11:01 | 208.9 | 206.2 | 214.0 | 230.0 | 225.4 |
| | 7 | F | 206 | 1.03 | 11:02 | 207.7 | 210.0 | 214.0 | 214.7 | 219.3 |
| | 8 | F | 215 | 1.08 | 11:03 | 219.1 | 225.5 | 224.8 | 232.4 | 237.1 |
| C-4 | 9 | F | 207 | 1.03 | 11:05 | 196.7 | 210.0 | 213.6 | 222.1 | 220.8 |
| | 10 | F | 212 | 1.05 | 11:06 | 199.5 | 210.1 | 210.8 | 220.0 | 227.6 |
| **D-1** | 1 | M | 230 | 1.15 | 11:07 | 234.0 | 240.4 | 258.4 | 244.4 | 286.3 |
| Anatabine | 2 | M | 248 | 1.25 | 11:21 | 242.6 | 254.0 | 267.9 | 255.1 | 268.1 |
| 1.5 mg/kg | 3 | M | 228 | 1.15 | 11:23 | 231.0 | 240.3 | 252.5 | 247.3 | 269.8 |
| **D-2** | 4 | M | 239 | 1.2 | 11:26 | 245.3 | 257.0 | 272.5 | 290.2 | 295.8 |
| | 5 | M | 227 | 1.15 | 11:28 | 227.8 | 237.3 | 250.1 | 262.0 | 270.5 |
| **D-3** | 6 | F | 216 | 1.08 | 11:08 | 213.9 | 212.0 | 220.0 | 226.2 | 228.0 |
| | 7 | F | 206 | 1.03 | 11:09 | 204.0 | 206.2 | 210.3 | 216.8 | 217.5 |
| | 8 | F | 219 | 1.1 | 11:20 | 219.6 | 221.6 | 224.3 | 234.7 | 230.9 |
| **D-4** | 9 | F | 231 | 1.15 | 11:22 | 229.5 | 233.9 | 237.0 | 248.1 | 247.5 |
| | 10 | F | 206 | 1.03 | 11:23 | 206.1 | 208.6 | 211.4 | 221.8 | 218.9 |

Table 28B. Dosing Calculations and Body Weights, days 6-12 (anatabine)

| Group | Rat | M/F | day 6 B. W. (g) | day 7 B. W. (g) | day 8 B. W. (g) | day 9 B. W. (g) | day 10 B. W. (g) | day 11 B. W. (g) | day 12 B. W. (g) |
|---|---|---|---|---|---|---|---|---|---|
| **A-1** | 1 | M | 308 | 317.0 | 332.0 | 338.0 | 348.7 | 364.2 | 369.5 |
| vehicle | 2 | M | 283.7 | 300.0 | 310.0 | 315.0 | 327.2 | 342.6 | 342.6 |
| 5 mL/kg | 3 | M | 287.9 | 300.0 | 314.0 | 317.0 | 332.0 | 349.1 | 354.1 |
| A-2 | 4 | M | 268.8 | 273.5 | 286.0 | 286.9 | 296.1 | 312.7 | 316.7 |
| | 5 | M | 294.6 | 301.1 | 315.0 | 315.2 | 332.3 | 339.7 | 351.0 |
| A-3 | 6 | F | 228.2 | 231.7 | 235.5 | 237.4 | 246.7 | 253.8 | 248.9 |
| | 7 | F | 235.3 | 236.8 | 260.8 | 249.8 | 252.8 | 261.3 | 256.3 |
| | 8 | F | 221.9 | 225.9 | 226.4 | 230.1 | 236.4 | 248.0 | 240.9 |
| A-4 | 9 | F | 216 | 217.4 | 218.0 | 224.8 | 228.5 | 237.1 | 238.8 |
| | 10 | F | 232.4 | 232.5 | 233.0 | 238.3 | 241.9 | 256.7 | 256.9 |
| **B-1** | 1 | M | 301.8 | 318.2 | 327.0 | 336.8 | 346.4 | 365.7 | 359.9 |
| Anatabine | 2 | M | 278 | 293.8 | 302.0 | 303.4 | 312.9 | 331.0 | 320.0 |
| 0.1 mg/kg | 3 | M | 284.3 | 297.6 | 309.0 | 310.0 | 319.9 | 343.8 | 341.9 |
| B-2 | 4 | M | 288.3 | 301.2 | 310.0 | 318.7 | 322.3 | 346.9 | 347.3 |
| | 5 | M | 219.6 | 300.7 | 311.0 | 317.4 | 330.4 | 348.8 | 360.9 |
| B-3 | 6 | F | 241.1 | 246.5 | 252.0 | 255.1 | 261.9 | 274.8 | 277.2 |
| | 7 | F | 235.5 | 226.4 | 232.0 | 239.9 | 234.5 | 238.5 | 246.3 |
| | 8 | F | 220.1 | 221.7 | 227.0 | 224.1 | 226.0 | 233.9 | 242.0 |
| B-4 | 9 | F | 218.8 | 222.1 | 223.0 | 236.0 | 232.5 | 245.6 | 240.0 |
| | 10 | F | 222.9 | 232.1 | 235.0 | 232.0 | 233.7 | 245.4 | 241.6 |

| Group | Rat | M/F | day 6 B. W. (g) | day 7 B. W. (g) | day 8 B. W. (g) | day 9 B. W. (g) | day 10 B. W. (g) | day 11 B. W. (g) | day 12 B. W. (g) |
|---|---|---|---|---|---|---|---|---|---|
| **C-1** | 1 | M | 282.4 | 294.5 | 300.0 | 307.6 | 317.2 | 334.6 | 327.6 |
| Anatabine | 2 | M | 287.6 | 302.9 | 310.6 | 319.8 | 331.9 | 339.8 | 325.4 |
| 0.75 mg/kg | 3 | M | 268.2 | 281.8 | 293.4 | 298.8 | 307.9 | 329.4 | 322.5 |
| C-2 | 4 | M | 268.2 | 274.1 | 284.2 | 296.2 | 303.8 | 316.1 | 324.8 |
| | 5 | M | 281.3 | 287.1 | 292.0 | 298.5 | 306.8 | 321.9 | 331.1 |
| C-3 | 6 | F | 224.4 | 227.4 | 232.0 | 237.3 | 239.3 | 250.9 | 249.7 |
| | 7 | F | 217.8 | 223 | 220.0 | 226.3 | 232.2 | 239.1 | 233.5 |
| | 8 | F | 236.2 | 243.2 | 243.0 | 247.6 | 252.7 | 268.8 | 259.6 |
| C-4 | 9 | F | 214.7 | 222 | 224.6 | 229.1 | 230.9 | 240.9 | 241.6 |
| | 10 | F | 221.4 | 219.3 | 227.4 | 230.6 | 233.5 | 244.1 | 247.9 |
| **D-1** | 1 | M | 279.1 | 292 | 300.0 | 302.1 | 317.1 | 333.0 | 341.1 |
| Anatabine | 2 | M | 296.9 | 305.6 | 319.0 | 323.1 | 331.8 | 359.6 | 360.3 |
| 1.5 mg/kg | 3 | M | 280.4 | 288.2 | 298.4 | 303.8 | 318.3 | 335.4 | 327.9 |
| D-2 | 4 | M | 302.3 | 316.7 | 326.0 | 337.3 | 347.0 | 369.5 | 380.4 |
| | 5 | M | 274.8 | 283.5 | 297.0 | 301.9 | 310.0 | 332.6 | 336.3 |
| D-3 | 6 | F | 221.2 | 229 | 229.6 | 232.8 | 232.8 | 242.1 | 236.9 |
| | 7 | F | 204.6 | 208.1 | 216.4 | 218.7 | 229.0 | 233.0 | 227.4 |
| | 8 | F | 229.3 | 235.7 | 244.0 | 243.9 | 252.1 | 259.7 | 257.1 |
| D-4 | 9 | F | 246 | 249.9 | 256.7 | 258.3 | 260.9 | 266.9 | 276.3 |
| | 10 | F | 216.9 | 224 | 225.0 | 225.8 | 234.5 | 243.5 | 240.9 |

Table 28C. Dosing Calculations and Body Weights, days 13-14 (anatabine)

| | | day 13 | day 14 | |
|---|---|---|---|---|
| Group | Rat | M/F | B. W. (g) | B. W. (g) |
| **A-1** | 1 | M | 379.1 | 389.0 |
| vehicle | 2 | M | 349 | 363.0 |
| 5 mL/kg | 3 | M | 356 | 377.0 |
| A-2 | 4 | M | 317 | 325.0 |
| | 5 | M | 356 | 371.9 |
| A-3 | 6 | F | 258 | 265.2 |
| | 7 | F | 249.8 | 268.2 |
| | 8 | F | 245.6 | 252.7 |
| A-4 | 9 | F | 235 | 245.8 |
| | 10 | F | 253 | 265.7 |
| **B-1** | 1 | M | 367 | 391.0 |
| Anatabine | 2 | M | 331 | 347.3 |
| 0.1 mg/kg | 3 | M | 349 | 368.8 |
| B-2 | 4 | M | 334.4 | 353.5 |
| | 5 | M | 360 | 373.8 |
| B-3 | 6 | F | 271.8 | 272.7 |
| | 7 | F | 244 | 248.5 |
| | 8 | F | 241 | 243.9 |
| B-4 | 9 | F | 242.1 | 255.6 |
| | 10 | F | 244 | 239.2 |

| Group | Rat | M/F | day 13<br>B. W. (g) | day 14<br>B. W. (g) |
|---|---|---|---|---|
| **C-1** Anatabine 0.75 mg/kg | 1 | M | 335.6 | 351.9 |
| | 2 | M | 338 | 357.9 |
| | 3 | M | 330 | 345.1 |
| C-2 | 4 | M | 326 | 339 |
| | 5 | M | 326 | 335.9 |
| C-3 | 6 | F | 248 | 251.5 |
| | 7 | F | 237.2 | 242.8 |
| | 8 | F | 268 | 274.1 |
| C-4 | 9 | F | 234 | 250.7 |
| | 10 | F | 243 | 250.6 |

| | | | | |
|---|---|---|---|---|
| **D-1** Anatabine 1.5 mg/kg | 1 | M | 339.8 | 359.8 |
| | 2 | M | 370 | 389.7 |
| | 3 | M | 340 | 357.1 |
| D-2 | 4 | M | 379 | 399.9 |
| | 5 | M | 337 | 355 |
| D-3 | 6 | F | 242.4 | 249.9 |
| | 7 | F | 223 | 224.4 |
| | 8 | F | 257 | 264.7 |
| D-4 | 9 | F | 275.8 | 283.1 |
| | 10 | F | 240.3 | 248.8 |

Table 28D. Dosing Calculations and Body Weights, days 1-5 (nicotine)

| Group | Rat | M/F | B.W. (g) | Dose volume (mL) | time | day 1 B. W. (g) | day 2 B. W. (g) | day 3 B. W. (g) | day 4 B. W. (g) | day 5 B. W. (g) |
|---|---|---|---|---|---|---|---|---|---|---|
| **E-1** | 1 | M | 218 | 1.1 | 8:45 | 228 | 239.1 | 245.1 | 263.4 | 269.1 |
| Nicotine | 2 | M | 218 | 1.1 | 8:48 | 223 | 229.5 | 241.1 | 257.6 | 260.7 |
| 0.75 mg/kg | 3 | M | 207 | 1.03 | 8:50 | 219 | 229.1 | 238.4 | 252.9 | 258.7 |
| **E-2** | 4 | M | 214 | 1.08 | 8:51 | | | | | |
| | 5 | M | 221 | 1.1 | 8:53 | 227 | 234.9 | 243 | 260.5 | 272.1 |
| **E-3** | 6 | F | 187 | 0.93 | 8:55 | 191.6 | 190.5 | 198 | 207.8 | 212.3 |
| | 7 | F | 195 | 0.98 | 8:57 | | | | | |
| | 8 | F | 193 | 0.98 | 8:59 | | | | | |
| **E-4** | 9 | F | 207 | 1.05 | 9:01 | 206.1 | 213.4 | 217.1 | 223.8 | 226.3 |
| | 10 | F | 192 | 0.95 | 9:03 | 192.8 | 195.7 | 199.1 | 205.6 | 206.4 |

Table 28E. Dosing Calculations and Body Weights, days 6-12 (nicotine)

| Group | Rat | M/F | day 6 B. W. (g) | day 7 B. W. (g) | day 8 B. W. (g) | day 9 B. W. (g) | day 10 B. W. (g) | day 11 B. W. (g) | day 12 B. W. (g) |
|---|---|---|---|---|---|---|---|---|---|
| **E-1** | 1 | M | 270 | 288 | 285.6 | 285.6 | 301 | 300.1 | 303 |
| Nicotine | 2 | M | 262.8 | 277.8 | 278 | 274.6 | 283.4 | 291.5 | 287.3 |
| 0.75 mg/kg | 3 | M | 259 | 279.9 | 284 | 274.9 | 286.1 | 292.2 | 290.7 |
| E-2 | 4 | M | | | | | | | |
| | 5 | M | 275.4 | 295.8 | 293.7 | 303.5 | 314.2 | 317.7 | 329.5 |
| E-3 | 6 | F | 207 | 213 | 218 | 219.1 | 221.8 | 222.8 | 232.1 |
| | 7 | F | | | | | | | |
| | 8 | F | | | | | | | |
| E-4 | 9 | F | 228.9 | 240.1 | 237.4 | 236.4 | 240.1 | 244.5 | 248.9 |
| | 10 | F | 205.5 | 218.3 | 217.4 | 213.2 | 220.5 | 226 | 227.7 |

Table 28F. Dosing Calculations and Body Weights, days 13-14 (nicotine)

| Group | Rat | M/F | day 13 B. W. (g) | day 14 B. W. (g) |
|---|---|---|---|---|
| E-1 | 1 | M | 312.3 | 316.1 |
| Nicotine | 2 | M | 292.4 | 297.6 |
| 0.75 mg/kg | 3 | M | 299.4 | 305 |
| E-2 | 4 | M | [redacted] | [redacted] |
| | 5 | M | 330 | 339.2 |
| E-3 | 6 | F | 232.6 | 230.4 |
| | 7 | F | [redacted] | [redacted] |
| | 8 | F | [redacted] | [redacted] |
| E-4 | 9 | F | 251.8 | 249 |
| | 10 | F | 227.3 | 229 |

Table 29A: **Average Daily Food Consumption per Rat (grams)**

| | | | **A- Vehicle** | | **B - 0.1 mg/kg** | | **Anatabine C - 0.75 mg/kg** | | **D - 1.5 mg/kg** | |
|---|---|---|---|---|---|---|---|---|---|---|
| Date | cage# | # of rats | Male | Female | Male | Female | Male | Female | Male | Female |
| day 1 | 1 | 3 | 25.1 | 14.1 | 25.8 | 16.4 | 23.9 | 17.1 | 22.7 | 16.1 |
| | 2 | 2 | 21.1 | 15.4 | 28.0 | 14.4 | 19.2 | 17.1 | 21.4 | 13.6 |
| day 2 | 1 | 3 | 27.4 | 16.3 | 33.8 | 20.2 | 26.3 | 22.5 | 24.2 | 20.5 |
| | 2 | 2 | 24.5 | 20.4 | 29.1 | 20.4 | 29.5 | 11.3 | 30.2 | 19.8 |
| day 3 | 1 | 3 | 25.4 | 16.8 | 24.8 | 19.4 | 28.1 | 21.0 | 30.1 | 19.6 |
| | 2 | 2 | 25.3 | 20.3 | 22.0 | 18.2 | 26.3 | 17.4 | 31.2 | 20.9 |

(continued)

| | | | Anatabine | | | | | | | |
|---|---|---|---|---|---|---|---|---|---|---|
| | | | A- Vehicle | | B - 0.1 mg/kg | | C - 0.75 mg/kg | | D - 1.5 mg/kg | |
| Date | cage# | # of rats | Male | Female | Male | Female | Male | Female | Male | Female |
| day 4 | 1 | 3 | 26.8 | 17.6 | 34.1 | 23.2 | 28.3 | 21.0 | 22.1 | 20.8 |
| | 2 | 2 | 26.4 | 20.2 | 28.4 | 18.0 | 29.8 | 18.7 | 30.0 | 21.6 |
| day 5 | 1 | 3 | 28.5 | 17.2 | 30.6 | 22.4 | 24.4 | 19.1 | 30.0 | 18.5 |
| | 2 | 2 | 28.3 | 22.4 | 28.6 | 21.4 | 27.0 | 21.7 | 29.0 | 18.5 |
| day 6 | 1 | 3 | 26.7 | 20.6 | 33.6 | 26.8 | 25.6 | 18.8 | 33.6 | 14.2 |
| | 2 | 2 | 26.0 | 19.2 | 28.0 | 18.2 | 29.5 | 16.4 | 35.4 | 18.4 |
| day 7 | 1 | 3 | 30.1 | 21.6 | 35.7 | 21.7 | 29.0 | 24.0 | 34.1 | 21.2 |
| | 2 | 2 | 27.9 | 20.5 | 31.8 | 22.4 | 31.5 | 17.4 | 33.5 | 28.8 |
| day 8 | 1 | 3 | 33.2 | 23.7 | 33.2 | 26.5 | 31.3 | 23.2 | 31.7 | 22.7 |
| | 2 | 2 | 31.5 | 23.8 | 31.9 | 21.3 | 32.6 | 22.8 | 34.4 | 19.8 |
| day 9 | 1 | 3 | 29.1 | 24.1 | 35.5 | 21.1 | 29.1 | 23.7 | 33.9 | 21.7 |
| | 2 | 2 | 27.7 | 24.9 | 36.8 | 23.6 | 30.3 | 23.0 | 34.5 | 23.1 |
| day 10 | 1 | 3 | 30.4 | 22.3 | 34.7 | 20.4 | 28.7 | 20.0 | 33.5 | 21.7 |
| | 2 | 2 | 29.4 | 20.5 | 26.7 | 16.9 | 28.0 | 21.0 | 32.4 | 21.7 |
| day 11 | 1 | 3 | 28.0 | 19.9 | 29.1 | 19.0 | 24.4 | 20.5 | 28.3 | 15.6 |
| | 2 | 2 | 24.4 | 22.9 | 30.6 | 19.1 | 26.7 | 18.6 | 29.9 | 19.0 |
| day 12 | 1 | 3 | 27.4 | 20.3 | 31.6 | 27.9 | 21.7 | 16.6 | 30.1 | 18.4 |
| | 2 | 2 | 25.6 | 18.8 | 34.9 | 16.7 | 31.0 | 20.4 | 33.1 | 20.8 |
| day 13 | 1 | 3 | 33.6 | 22.4 | 39.9 | 29.6 | 33.9 | 22.0 | 36.8 | 19.6 |
| | 2 | 2 | 30.8 | 20.9 | 27.1 | 19.3 | 27.2 | 17.7 | 34.7 | 20.5 |
| day 14 | 1 | 3 | 28.7 | 20.9 | 32.3 | 18.8 | 27.8 | 17.6 | 29.9 | 17.1 |
| | 2 | 2 | 26.4 | 19.6 | 28.8 | 19.1 | 26.4 | 20.7 | 31.4 | 19.2 |

Table 29B: **Average Daily Food Consumption per Rat (grams)**

| | | E- Nicotine 0.75 mg/kg | | | |
|---|---|---|---|---|---|
| | | Males | | Female | |
| Date | cage# | # of rats | grams | # of rats | grams |
| day 1 | 1 | 3 | 20.8 | 1 | 23.7 |
| | 2 | 1 | 19.3 | 2 | 14.8 |
| day 2 | 1 | 3 | 23.5 | 1 | 24.9 |
| | 2 | 1 | 26.2 | 2 | 18.4 |
| day 3 | 1 | 3 | 26.6 | 1 | 22.2 |
| | 2 | 1 | 26.2 | 2 | 22.3 |
| day 4 | 1 | 3 | 26.2 | 1 | 19.4 |
| | 2 | 1 | 23.7 | 2 | 15.8 |

(continued)

| | | E- Nicotine 0.75 mg/kg | | | |
|---|---|---|---|---|---|
| | | Males | | Female | |
| Date | cage# | # of rats | grams | # of rats | grams |
| day 5 | 1 | 3 | 25.1 | 1 | 18.7 |
| | 2 | 1 | 21.9 | 2 | 16.2 |
| day 6 | 1 | 3 | 23.8 | 1 | 20.0 |
| | 2 | 1 | 25.9 | 2 | 17.2 |
| day 7 | 1 | 3 | 26.5 | 1 | 19.4 |
| | 2 | 1 | 27.9 | 2 | 21.7 |
| day 8 | 1 | 3 | 24.0 | 1 | 20.1 |
| | 2 | 1 | 28.2 | 2 | 20.6 |
| day 9 | 1 | 3 | 27.3 | 1 | 24.4 |
| | 2 | 1 | 26.1 | 2 | 21.5 |
| day 10 | 1 | 3 | 29.6 | 1 | 22.6 |
| | 2 | 1 | 30.9 | 2 | 20.4 |
| day 11 | 1 | 3 | 30.1 | 1 | 20.4 |
| | 2 | 1 | 29.1 | 2 | 20.3 |
| day 12 | 1 | 3 | 26.5 | 1 | 22.5 |
| | 2 | 1 | 23.4 | 2 | 21.7 |
| day 13 | 1 | 3 | 31.9 | 1 | 23.7 |
| | 2 | 1 | 28.4 | 2 | 21.2 |
| day 14 | 1 | 3 | 32.7 | 1 | 24.4 |
| | 2 | 1 | 26.4 | 2 | 19.6 |

Table 30. Hematology/Coagulation Parameters: Normal Ranges in the Rat

| | **Unit B Measure** | **Range** |
|---|---|---|
| WBC | x $10^3/\mu L$ | 3.0 - 17.0 |
| RBC | x $10^6/\mu L$ | 5 - 10 |
| HGB | g/dL | 11 - 19 |
| HCT | % | 35 - 57 |
| MCV | fL | 46 - 65 |
| MCH | pg | 18 - 23 |
| MCHC | g/dL | 31 - 40 |
| RETICULOCYTE COUNT | % | 0 - 25 |
| NEUTROPHIL SEG | % | 7 - 15 |
| LYMPHOCYTE | % | 77 - 89 |
| MONOCYTE | % | 0 - 5 |
| EOSINOPHIL | % | 0 - 4 |
| BASOPHIL | % | 0 - 1 |

(continued)

| | Unit B Measure | Range |
|---|---|---|
| PLATELET COUNT | x $10^3/\mu L$ | 200 - 1500 |
| aPTT | sec | 13.2 - 22.4 |
| PT | sec | 11.0 - 15.6 |

Table 31. Hematology Parameters

| Animal ID | Sex | Anatabine Dose (mg/kg) | WBC | RBC | HGB | HCT | MCV | MCH | MCHC | RETICULOCYTE COUNT | PLATELET COUNT |
|---|---|---|---|---|---|---|---|---|---|---|---|
| | | | $x\ 10^3/\mu L$ | $x\ 10^6/\mu L$ | gm/dL | % | $U^3$ | UUG | % | % | $x\ 10^3/\mu L$ |
| A1 | M | 0 | 9.4 | 7.33 | 14.5 | 43.6 | 60 | 19.7 | 33.1 | 4.6 | 1494 |
| A2 | M | 0 | 11.8 | 7.22 | 15.1 | 44.9 | 62 | 20.9 | 33.6 | 6.1 | 1124 |
| A3 | M | 0 | 12.2 | 7.26 | 15.4 | 45.5 | 63 | 21.2 | 33.9 | 6.8 | 1088 |
| A4 | M | 0 | 9.9 | 7.69 | 15.8 | 47.8 | 62 | 20.5 | 33 | 5.9 | TNP[1] |
| A5 | M | 0 | 9.4 | 7.13 | 15.2 | 45.4 | 64 | 21.3 | 33.4 | 7.3 | 1443 |
| A6 | F | 0 | 5.8 | 8.4 | 18.3 | 51 | 61 | 21.7 | 35.8 | 5.6 | 1500 |
| A7 | F | 0 | 11.6 | 7.2 | 15.6 | 45 | 63 | 21.6 | 34.6 | 3.4 | 1057 |
| A8 | F | 0 | 12 | 7.35 | 15.2 | 44.3 | 60 | 20.6 | 34.2 | 6.3 | 1263 |
| A9 | F | 0 | 7 | 8.71 | 18.1 | 53.5 | 61 | 20.8 | 33.8 | 4.7 | 986 |
| A10 | F | 0 | 10.1 | 7.16 | 14.6 | 42.7 | 60 | 20.4 | 34.3 | 5.2 | 1186 |
| B1 | M | 0.1 | 23.5 | 8.05 | 17.6 | 52.1 | 65 | 21.9 | 33.8 | 6.1 | 701 |
| B2 | M | 0.1 | 15 | 7.38 | 15.5 | 45.7 | 62 | 21.1 | 34 | 5.4 | 1339 |
| B3 | M | 0.1 | 11.1 | 7.4 | 16.1 | 47.5 | 64 | 21.8 | 34 | 4.7 | 1601 |
| B4 | M | 0.1 | 13.2 | 7.42 | 15.2 | 45 | 61 | 20.5 | 33.8 | 5.6 | 1107 |
| B5 | M | 0.1 | 15.4 | 6.96 | 14.5 | 45.3 | 65 | 20.8 | 32 | 5.9 | 1495 |
| B6 | F | 0.1 | 4.7 | 7.7 | 15.8 | 46.7 | 61 | 20.5 | 33.8 | 4.8 | 1145 |
| B7 | F | 0.1 | 10.7 | 8.71 | 17 | 51.3 | 59 | 19.6 | 33.2 | 5.4 | TNP[1] |
| B8 | F | 0.1 | 8 | 7.1 | 14.8 | 44 | 62 | 20.9 | 33.6 | 6.1 | TNP[1] |
| B9 | F | 0.1 | 14 | 7.06 | 14.6 | 43.1 | 61 | 20.8 | 34 | 3.7 | 1029 |
| B10 | F | 0.1 | 9.2 | 8.22 | 15.8 | 46.8 | 57 | 19.2 | 33.7 | 2.4 | 818 |

| Animal ID | Sex | Anatabine Dose (mg/kg) | WBC | RBC | HGB | HCT | MCV | MCH | MCHC | RETICULOCYTE COUNT | PLATELET COUNT |
|---|---|---|---|---|---|---|---|---|---|---|---|
| | | | Hematology Parameters | | | | | | | | |
| | | | x $10^3/\mu L$ | x $10^6/\mu L$ | gm/dL | % | $U^3$ | UUG | % | % | x $10^3/\mu L$ |
| C1 | M | 0.75 | 9.9 | 8.23 | 16.8 | 49.6 | 60 | 20.3 | 33.8 | 3.6 | 1446 |
| C2 | M | 0.75 | 11.1 | 7.51 | 15.8 | 45.5 | 61 | 21 | 34.7 | 4.2 | 1478 |
| C3 | M | 0.75 | 22.1 | 7.63 | 16.8 | 48.4 | 63 | 22 | 34.7 | 4.1 | 1327 |
| C4 | M | 0.75 | 7.5 | 6.23 | 13.1 | 39.2 | 63 | 21.1 | 33.5 | 4.7 | TNP[1] |
| C5 | M | 0.75 | 9.9 | 7.73 | 15.4 | 46.3 | 60 | 19.9 | 33.2 | 5.3 | 1052 |
| C6 | F | 0.75 | 13.5 | 7.42 | 15 | 44.7 | 60 | 20.2 | 33.6 | 3.4 | 653 |
| C7 | F | 0.75 | 14.5 | 7.78 | 14.9 | 43.9 | 56 | 19.2 | 34.1 | 1.8 | 1475 |
| C8 | F | 0.75 | 10.9 | 7.11 | 15.1 | 44.2 | 62 | 21.2 | 34.2 | 3.2 | 1304 |
| C9 | F | 0.75 | 8 | 7.58 | 15.6 | 45.4 | 60 | 20.5 | 34.3 | 3.8 | 1540 |
| C10 | F | 0.75 | 4.8 | 7.81 | 16.2 | 47.6 | 61 | 20.8 | 34.1 | 4.1 | 1376 |
| D1 | M | 1.5 | 12.4 | 7.82 | 15.8 | 48 | 62 | 20.2 | 32.9 | 4.4 | TNP2 |
| D2 | M | 1.5 | 15.3 | 7.43 | 15.4 | 46.1 | 62 | 20.7 | 33.4 | 3.3 | 1350 |
| D3 | M | 1.5 | 8.4 | 7.49 | 16.1 | 48.5 | 65 | 21.5 | 33.1 | 3.6 | 1407 |
| D4 | M | 1.5 | 13 | 7.16 | 14.6 | 43.1 | 60 | 20.4 | 33.8 | 5.1 | 1274 |
| D5 | M | 1.5 | 16.4 | 7.37 | 15 | 44.8 | 61 | 20.4 | 33.6 | 4.8 | 859 |
| D6 | F | 1.5 | 6.4 | 8.17 | 15.8 | 47.1 | 58 | 19.3 | 33.6 | 3.2 | 1399 |
| D7 | F | 1.5 | 7 | 7.77 | 15.1 | 45.9 | 59 | 19.4 | 32.8 | 2.8 | 1346 |
| D8 | F | 1.5 | 12.1 | 7.65 | 14.8 | 44.3 | 58 | 19.3 | 33.4 | 4.6 | 1312 |
| D9 | F | 1.5 | 10.9 | 8.01 | 15.9 | 48.2 | 60 | 19.8 | 32.9 | 3.4 | 228 |
| D10 | F | 1.5 | 9.5 | 7.05 | 14.2 | 41.4 | 59 | 20.2 | 34.4 | 2.1 | 1352 |

**Hematology Parameters**

| Animal ID | Sex | Anatabine Dose (mg/kg) | WBC x $10^3/\mu L$ | RBC x $10^6/\mu L$ | HGB gm/dL | HCT % | MCV $U^3$ | MCH UUG | MCHC % | RETICULOCYTE COUNT % | PLATELET COUNT x $10^3/\mu L$ |
|---|---|---|---|---|---|---|---|---|---|---|---|
| E1 | M | 0.75[1] | 9.2 | 7.43 | 15.2 | 45.6 | 61 | 20.5 | 33.4 | 4.3 | 1458 |
| E2 | M | 0.75[1] | 13.6 | 7.61 | 15.9 | 46.2 | 61 | 20.8 | 34.4 | 4.8 | 1343 |
| E3 | M | 0.75[1] | 8.2 | 8.26 | 16.8 | 50 | 61 | 20.4 | 33.7 | 5.6 | 1366 |
| E5 | M | 0.75[1] | 14.6 | 7.04 | 15.3 | 44.5 | 63 | 21.7 | 34.3 | 6.1 | 1256 |
| E6 | F | 0.75[1] | 11.2 | 8.25 | 16.2 | 47.7 | 58 | 19.6 | 34 | 5.8 | 1539 |
| E9 | F | 0.75[1] | 6.7 | 7.41 | 15.2 | 44.2 | 60 | 20.5 | 34.4 | 4.9 | 1187 |
| E10 | F | 0.75[1] | 12.7 | 8.36 | 16.5 | 48.3 | 58 | 19.7 | 34.1 | 4.5 | TNP[2] |

1. Dose is 0.75 mg/kg nicotine
2. TNP: Test not performed due to clot in EDTA tube

| | | | Hematology Parameters | | | | | | | |
|---|---|---|---|---|---|---|---|---|---|---|
| | | Anatabine | NEUTROPHIL SEG | LYMPHOCYTE | MONOCYTE | EOSINOPHIL | BASOPHIL | PLATELET EST | POLYCHROMASIA | ANISOCYTOSIS |
| Animal ID | Sex | Dose (mg/kg) | % | % | % | % | % | | | |
| A1 | M | 0 | 9 | 90 | 1 | 0 | 0 | ADEQUATE | SLIGHT | SLIGHT |
| A2 | M | 0 | 9 | 87 | 4 | 0 | 0 | ADEQUATE | SLIGHT | SLIGHT |
| A3 | M | 0 | 12 | 88 | 0 | 0 | 0 | ADEQUATE | SLIGHT | SLIGHT |
| A4 | M | 0 | 6 | 93 | 1 | 0 | 0 | DECREASED | SLIGHT | SLIGHT |
| A5 | M | 0 | 8 | 90 | 2 | 0 | 0 | ADEQUATE | SLIGHT | SLIGHT |
| A6 | F | 0 | 9 | 89 | 2 | 0 | 0 | INCREASED | SLIGHT | SLIGHT |
| A7 | F | 0 | 12 | 86 | 2 | 0 | 0 | ADEQUATE | SLIGHT | SLIGHT |
| A8 | F | 0 | 14 | 84 | 2 | 0 | 0 | ADEQUATE | DNR | DNR |
| A9 | F | 0 | 12 | 86 | 2 | 0 | 0 | ADEQUATE | SLIGHT | SLIGHT |
| A10 | F | 0 | 16 | 81 | 3 | 0 | 0 | ADEQUATE | SLIGHT | SLIGHT |
| B1 | M | 0.1 | 12 | 87 | 1 | 0 | 0 | ADEQUATE | SLIGHT | SLIGHT |
| B2 | M | 0.1 | 3 | 94 | 3 | 0 | 0 | ADEQUATE | SLIGHT | SLIGHT |
| B3 | M | 0.1 | 7 | 90 | 3 | 0 | 0 | INCREASED | SLIGHT | SLIGHT |
| B4 | M | 0.1 | 10 | 87 | 2 | 1 | 0 | ADEQUATE | SLIGHT | SLIGHT |
| B5 | M | 0.1 | 11 | 88 | 1 | 0 | 0 | ADEQUATE | SLIGHT | SLIGHT |
| B6 | F | 0.1 | 8 | 89 | 3 | 0 | 0 | ADEQUATE | SLIGHT | SLIGHT |
| B7 | F | 0.1 | 8 | 90 | 2 | 0 | 0 | ADEQUATE | SLIGHT | SLIGHT |
| B8 | F | 0.1 | 7 | 91 | 2 | 0 | 0 | DECREASED | SLIGHT | SLIGHT |
| B9 | F | 0.1 | 6 | 93 | 1 | 0 | 0 | ADEQUATE | SLIGHT | SLIGHT |
| B10 | F | 0.1 | 11 | 88 | 1 | 0 | 0 | ADEQUATE | SLIGHT | SLIGHT |

DNR: Did not report – insufficient sample

| | | | Hematology Parameters | | | | | | | |
|---|---|---|---|---|---|---|---|---|---|---|
| | | Anatabine | NEUTROPHIL SEG | LYMPHOCYTE | MONOCYTE | EOSINOPHIL | BASOPHIL | PLATELET EST | POLYCHROMASIA | ANISOCYTOSIS |
| Animal ID | Sex | Dose (mg/kg) | % | % | % | % | % | | | |
| C1 | M | 0.75 | 13 | 86 | 1 | 0 | 0 | ADEQUATE | SLIGHT | SLIGHT |
| C2 | M | 0.75 | 10 | 89 | 1 | 0 | 0 | ADEQUATE | SLIGHT | SLIGHT |
| C3 | M | 0.75 | 6 | 92 | 2 | 0 | 0 | ADEQUATE | SLIGHT | SLIGHT |
| C4 | M | 0.75 | 10 | 89 | 1 | 0 | 0 | DECREASED | SLIGHT | SLIGHT |
| C5 | M | 0.75 | 10 | 88 | 2 | 0 | 0 | ADEQUATE | DNR | DNR |
| C6 | F | 0.75 | 6 | 93 | 1 | 0 | 0 | ADEQUATE | SLIGHT | SLIGHT |
| C7 | F | 0.75 | 12 | 87 | 1 | 0 | 0 | ADEQUATE | SLIGHT | SLIGHT |
| C8 | F | 0.75 | 9 | 91 | 0 | 0 | 0 | ADEQUATE | SLIGHT | SLIGHT |
| C9 | F | 0.75 | 9 | 89 | 2 | 0 | 0 | INCREASED | SLIGHT | SLIGHT |
| C10 | F | 0.75 | 7 | 93 | 0 | 0 | 0 | ADEQUATE | SLIGHT | SLIGHT |
| D1 | M | 1.5 | 20 | 77 | 3 | 0 | 0 | DECREASED | SLIGHT | SLIGHT |
| D2 | M | 1.5 | 6 | 93 | 1 | 0 | 0 | ADEQUATE | SLIGHT | SLIGHT |
| D3 | M | 1.5 | 12 | 86 | 2 | 0 | 0 | ADEQUATE | SLIGHT | SLIGHT |
| D4 | M | 1.5 | 25 | 74 | 1 | 0 | 0 | ADEQUATE | SLIGHT | SLIGHT |
| D5 | M | 1.5 | 10 | 89 | 1 | 0 | 0 | ADEQUATE | SLIGHT | SLIGHT |
| D6 | F | 1.5 | 11 | 88 | 1 | 0 | 0 | ADEQUATE | SLIGHT | SLIGHT |
| D7 | F | 1.5 | 13 | 87 | 0 | 0 | 0 | ADEQUATE | SLIGHT | SLIGHT |
| D8 | F | 1.5 | 20 | 80 | 0 | 0 | 0 | ADEQUATE | SLIGHT | SLIGHT |
| D9 | F | 1.5 | 10 | 89 | 1 | 0 | 0 | ADEQUATE | SLIGHT | SLIGHT |
| D10 | F | 1.5 | 9 | 89 | 2 | 0 | 0 | ADEQUATE | SLIGHT | SLIGHT |

DNR: Did not report- insufficient sample

| | | | Hematology Parameters | | | | | | | | |
|---|---|---|---|---|---|---|---|---|---|---|---|
| | | Anatabine | NEUTROPHIL SEG | LYMPHOCYTE | MONOCYTE | EOSINOPHIL | BASOPHIL | PLATELET EST | POLYCHROMASIA | ANISOCYTOSIS |
| Animal ID | Sex | Dose (mg/kg) | % | % | % | % | % | | | |
| E1 | M | 0.75[1] | 12 | 86 | 2 | 0 | 0 | ADEQUATE | SLIGHT | SLIGHT |
| E2 | M | 0.75[1] | 11 | 87 | 2 | 0 | 0 | ADEQUATE | SLIGHT | SLIGHT |
| E3 | M | 0.75[1] | 16 | 80 | 4 | 0 | 0 | ADEQUATE | SLIGHT | SLIGHT |
| E5 | M | 0.75[1] | 8 | 90 | 2 | 0 | 0 | ADEQUATE | SLIGHT | SLIGHT |
| E6 | F | 0.75[1] | 15 | 83 | 2 | 0 | 0 | INCREASED | SLIGHT | SLIGHT |
| E9 | F | 0.75[1] | 12 | 87 | 1 | 0 | 0 | ADEQUATE | SLIGHT | SLIGHT |
| E10 | F | 0.75[1] | 11 | 89 | 0 | 0 | 0 | DECREASED | SLIGHT | SLIGHT |

1. Dose is 0.75 mg/kg nicotine

**Table 32. Clinical Chemistry Parameters: Normal Ranges in the Rat**

| | Unit of Measure | Range |
|---|---|---|
| ALKALINE PHOSPHATASE | IU/L | 160 - 500 |
| ALT (SGPT) | IU/L | 35 - 80 |
| AST (SGOT) | IU/L | 33 - 53 |
| GLOBULIN | g/dL | 1.4 - 5.0 |
| ALBUMIN | g/dL | 2.9 - 5.9 |
| TOTAL PROTEIN | g/dL | 4.5 - 8.4 |
| TOTAL BILIRUBIN | mg/dL | 0 - 0.64 |
| BLOOD UREA NITROGEN (BUN) | mg/dL | 11 - 23 |
| CREATININE | mg/dL | 0.4 - 3.8 |
| CHOLESTEROL | mg/dL | 35 - 75 |
| GLUCOSE | mg/dL | 80 - 300 |
| CALCIUM | mg/dL | 9.1 - 15.1 |
| PHOSPHORUS | mg/dL | 4.7 - 16.0 |
| CHLORIDE | mEq/L | 79 - 111 |
| POTASSIUM | mEq/L | 3.6 - 9.2 |
| SODIUM | mEq/L | 142 - 154 |

Table 33. Clinical Chemistry Parameters

| | | | Clinical Chemistry Parameters | | | | | | | | |
|---|---|---|---|---|---|---|---|---|---|---|---|
| | | Anatabine | ALK PHOSPHATASE | ALT | AST | ALBUMIN | TOT PROTEIN | GLOBULIN | TOT BILIRUBIN | DIR BILIRUBIN | BUN |
| Animal ID | Sex | Dose (mg/kg) | IU/L | IU/L | IU/L | g/dL | g/dL | g/dL | mg/dL | mg/dL | mg/dL |
| A1 | M | 0 | 463 | 62 | 85 | 3.1 | 5.6 | 2.5 | 0 | 0 | 19 |
| A2 | M | 0 | 398 | 59 | 78 | 3.1 | 5.6 | 2.5 | 0 | 0 | 17 |
| A3 | M | 0 | 275 | 61 | 96 | 3.1 | 5.6 | 2.5 | 0 | 0 | 19 |
| A4 | M | 0 | 393 | 57 | 137 | 3.2 | 5.7 | 2.5 | 0 | 0 | 18 |
| A5 | M | 0 | 325 | 58 | 99 | 3.1 | 5.6 | 2.5 | 0 | 0 | 20 |
| A6 | F | 0 | 248 | 55 | 71 | 3.4 | 6.4 | 3 | 0 | 0 | 17 |
| A7 | F | 0 | 265 | 61 | 81 | 3.3 | 6.2 | 2.9 | 0 | 0 | 14 |
| A8 | F | 0 | 292 | 62 | 75 | 3.4 | 6.2 | 2.8 | 0 | 0 | 20 |
| A9 | F | 0 | 176 | 52 | 77 | 3.5 | 6.2 | 2.7 | 0 | 0 | 15 |
| A10 | F | 0 | 170 | 61 | 100 | 3.4 | 6.2 | 2.8 | 0 | 0 | 25 |
| | | | | | | | | | | | |
| B1 | M | 0.1 | 470 | 92 | 97 | 3.4 | 6.3 | 2.9 | 0 | 0 | 18 |
| B2 | M | 0.1 | 227 | 65 | 99 | 3.2 | 5.8 | 2.6 | 0 | 0 | 18 |
| B3 | M | 0.1 | 364 | 74 | 93 | 3.1 | 5.9 | 2.8 | 0 | 0 | 21 |
| B4 | M | 0.1 | 358 | 44 | 85 | 3.1 | 6.2 | 3.1 | 0 | 0 | 14 |
| B5 | M | 0.1 | 618 | 78 | 106 | 3.4 | 6.3 | 2.9 | 0 | 0 | 19 |
| B6 | F | 0.1 | 261 | 57 | 78 | 3.6 | 6.7 | 3.1 | 0 | 0 | 21 |
| B7 | F | 0.1 | 140 | 43 | 69 | 3.5 | 6.3 | 2.8 | 0 | 0 | 19 |
| B8 | F | 0.1 | 209 | 47 | 70 | 3.6 | 6.7 | 3.1 | 0 | 0 | 21 |
| B9 | F | 0.1 | 412 | 62 | 86 | 3.8 | 6.9 | 3.1 | 0 | 0 | 19 |
| B10 | F | 0.1 | 220 | 39 | 82 | 3.6 | 6.6 | 3 | 0 | 0 | 19 |

| | | | | Clinical Chemistry Parameters | | | | | | | | |
|---|---|---|---|---|---|---|---|---|---|---|---|---|
| | | Anatabine | | ALK PHOSPHATASE | ALT | AST | ALBUMIN | TOT PROTEIN | GLOBULIN | TOT BILIRUBIN | DIR BILIRUBIN | BUN |
| Animal ID | Sex | Dose (mg/kg) | | IU/L | IU/L | IU/L | g/dL | g/dL | g/dL | mg/dL | mg/dL | mg/dL |
| | | | | | | | | | | | | |
| C1 | M | 0.75 | | 479 | 75 | 103 | 3.4 | 6.2 | 2.8 | 0 | 0 | 15 |
| C2 | M | 0.75 | | 297 | 57 | 66 | 3.1 | 5.7 | 2.6 | 0 | 0 | 18 |
| C3 | M | 0.75 | | 325 | 65 | 97 | 3.2 | 5.9 | 2.7 | 0 | 0 | 12 |
| C4 | M | 0.75 | | 337 | 50 | 90 | 3.3 | 6.1 | 2.8 | 0 | 0 | 17 |
| C5 | M | 0.75 | | 416 | 87 | 92 | 3.4 | 6.3 | 2.9 | 0.1 | 0.1 | 21 |
| C6 | F | 0.75 | | 123 | 63 | 95 | 3.5 | 6.3 | 2.8 | 0 | 0 | 15 |
| C7 | F | 0.75 | | 142 | 49 | 69 | 3.6 | 6.3 | 2.7 | 0 | 0 | 16 |
| C8 | F | 0.75 | | 235 | 55 | 76 | 3.4 | 6.2 | 2.8 | 0 | 0 | 15 |
| C9 | F | 0.75 | | 130 | 49 | 54 | 3.6 | 6.6 | 3 | 0 | 0 | 16 |
| C10 | F | 0.75 | | 160 | 57 | 72 | 3.5 | 6.5 | 3 | 0 | 0 | 15 |
| | | | | | | | | | | | | |
| D1 | M | 1.5 | | 389 | 107 | 144 | 3.4 | 6.2 | 2.8 | 0 | 0 | 18 |
| D2 | M | 1.5 | | 644 | 61 | 83 | 3.2 | 5.7 | 2.5 | 0 | 0 | 17 |
| D3 | M | 1.5 | | 304 | 65 | 91 | 3.5 | 6.3 | 2.8 | 0 | 0 | 18 |
| D4 | M | 1.5 | | 357 | 58 | 77 | 3.2 | 5.8 | 2.6 | 0 | 0 | 18 |
| D5 | M | 1.5 | | 363 | 61 | 90 | 3.3 | 6 | 2.7 | 0 | 0 | 15 |
| D6 | F | 1.5 | | 189 | 47 | 78 | 3.7 | 6.6 | 2.9 | 0 | 0 | 18 |
| D7 | F | 1.5 | | 218 | 53 | 82 | 3.5 | 6.5 | 3 | 0 | 0 | 17 |
| D8 | F | 1.5 | | 181 | 43 | 70 | 3.5 | 6.5 | 3 | 0 | 0 | 15 |
| D9 | F | 1.5 | | 272 | 58 | 88 | 3.4 | 6.3 | 2.9 | 0.1 | 0.1 | 18 |
| D10 | F | 1.5 | | 110 | 45 | 65 | 3.3 | 5.9 | 2.6 | 0 | 0 | 13 |
| | | | | | | | | | | | | |

| | | | Clinical Chemistry Parameters | | | | | | | | |
|---|---|---|---|---|---|---|---|---|---|---|---|
| | | Anatabine | ALK PHOSPHATASE | ALT | AST | ALBUMIN | TOT PROTEIN | GLOBULIN | TOT BILIRUBIN | DIR BILIRUBIN | BUN |
| Animal ID | Sex | Dose (mg/kg) | IU/L | IU/L | IU/L | g/dL | g/dL | g/dL | mg/dL | mg/dL | mg/dL |
| E1 | M | 0.75[1] | 241 | 54 | 89 | 3.1 | 6 | 2.9 | 0.1 | 0 | 15 |
| E2 | M | 0.75[1] | 257 | 59 | 81 | 3.1 | 6.2 | 3.1 | 0 | 0 | 18 |
| E3 | M | 0.75[1] | 313 | 45 | 72 | 3.1 | 5.8 | 2.7 | 0 | 0 | 19 |
| E5 | M | 0.75[1] | 353 | 65 | 87 | 3 | 6 | 3 | 0.1 | 0 | 18 |
| E6 | F | 0.75[1] | 274 | 97 | 133 | 3.3 | 6.4 | 3.1 | 0.1 | 0 | 20 |
| E9 | F | 0.75[1] | 138 | 54 | 75 | 3.3 | 6.6 | 3.3 | 0.1 | 0.1 | 17 |
| E10 | F | 0.75[1] | 154 | 54 | 101 | 3.3 | 6.5 | 3.2 | 0.1 | 0 | 20 |

1. Dose is 0.75 mg/kg nicotine

| Animal ID | Sex | Anatabine Dose (mg/kg) | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RATIO |
|---|---|---|---|---|---|---|---|---|---|---|---|
| | | | Clinical Chemistry Parameters | | | | | | | | |
| A1 | M | 0 | 0.3 | 54 | 183 | 12.3 | 10.9 | 97 | 6.8 | 144 | 1.2 |
| A2 | M | 0 | 0.4 | 62 | 215 | 12.1 | 11 | 97 | 6.1 | 145 | 1.2 |
| A3 | M | 0 | 0.4 | 60 | 217 | 12.2 | 10.8 | 99 | 6.2 | 147 | 1.2 |
| A4 | M | 0 | 0.3 | 65 | 302 | 12.4 | 11.4 | 100 | 6.3 | 145 | 1.3 |
| A5 | M | 0 | 0.4 | 59 | 193 | 11.6 | 10 | 100 | 6.1 | 146 | 1.2 |
| A6 | F | 0 | 0.4 | 85 | 205 | 11.9 | 10 | 100 | 5.5 | 145 | 1.1 |
| A7 | F | 0 | 0.4 | 72 | 176 | 11.3 | 9 | 99 | 5.5 | 146 | 1.1 |
| A8 | F | 0 | 0.4 | 56 | 206 | 11.4 | 9.1 | 100 | 5.8 | 146 | 1.2 |
| A9 | F | 0 | 0.4 | 64 | 197 | 11.6 | 8.9 | 100 | 6.7 | 146 | 1.3 |
| A10 | F | 0 | 0.5 | 60 | 207 | 12.1 | 8.3 | 100 | 6.1 | 145 | 1.2 |
| B1 | M | 0.1 | 0.4 | 65 | 258 | 12.7 | 10.3 | 98 | 5.9 | 148 | 1.2 |
| B2 | M | 0.1 | 0.4 | 64 | 219 | 11.8 | 10.3 | 97 | 6.5 | 144 | 1.2 |
| B3 | M | 0.1 | 0.4 | 75 | 214 | 11.9 | 11.2 | 99 | 6.6 | 146 | 1.1 |
| B4 | M | 0.1 | 0.3 | 56 | 194 | 11.8 | 11.5 | 97 | 6.9 | 147 | 1 |
| B5 | M | 0.1 | 0.3 | 76 | 210 | 11.8 | 10.8 | 99 | 6.1 | 146 | 1.2 |
| B6 | F | 0.1 | 0.4 | 90 | 224 | 12 | 8.5 | 99 | 5.4 | 147 | 1.2 |
| B7 | F | 0.1 | 0.5 | 74 | 170 | 11.4 | 8.2 | 102 | 6.3 | 148 | 1.3 |
| B8 | F | 0.1 | 0.5 | 65 | 227 | 11.9 | 7.4 | 102 | 5.2 | 148 | 1.2 |
| B9 | F | 0.1 | 0.4 | 66 | 220 | 12.4 | 8.5 | 97 | 5.5 | 145 | 1.2 |
| B10 | F | 0.1 | 0.4 | 87 | 197 | 10.9 | 7.8 | 101 | 6.7 | 146 | 1.2 |

Clinical Chemistry Parameters

| Animal ID | Sex | Anatabine Dose (mg/kg) | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RATIO |
|---|---|---|---|---|---|---|---|---|---|---|---|
| C1 | M | 0.75 | 0.4 | 69 | 211 | 12.3 | 11 | 98 | 6.3 | 148 | 1.2 |
| C2 | M | 0.75 | 0.4 | 60 | 222 | 12.2 | 10.7 | 99 | 6.3 | 146 | 1.2 |
| C3 | M | 0.75 | 0.3 | 54 | 219 | 11.8 | 10 | 100 | 5.4 | 147 | 1.2 |
| C4 | M | 0.75 | 0.3 | 62 | 160 | 11.5 | 10.6 | 99 | 6 | 150 | 1.2 |
| C5 | M | 0.75 | 0.4 | 69 | 198 | 12.4 | 12.3 | 97 | 6.6 | 148 | 1.2 |
| C6 | F | 0.75 | 0.5 | 66 | 237 | 11.8 | 9.7 | 97 | 5.5 | 145 | 1.3 |
| C7 | F | 0.75 | 0.4 | 64 | 247 | 11.3 | 7.7 | 96 | 5.9 | 144 | 1.3 |
| C8 | F | 0.75 | 0.4 | 63 | 192 | 11.6 | 9.6 | 99 | 6 | 145 | 1.2 |
| C9 | F | 0.75 | 0.4 | 69 | 211 | 12.1 | 9.2 | 97 | 5.7 | 144 | 1.2 |
| C10 | F | 0.75 | 0.5 | 87 | 217 | 11.6 | 7 | 101 | 6.3 | 147 | 1.2 |
| D1 | M | 1.5 | 0.4 | 57 | 209 | 12.2 | 10.9 | 99 | 7.2 | 146 | 1.2 |
| D2 | M | 1.5 | 0.4 | 68 | 204 | 11 | 9.6 | 100 | 5.4 | 149 | 1.3 |
| D3 | M | 1.5 | 0.4 | 64 | 207 | 12.2 | 11.6 | 99 | 6.3 | 148 | 1.3 |
| D4 | M | 1.5 | 0.3 | 64 | 196 | 12.1 | 11.6 | 100 | 5.3 | 149 | 1.2 |
| D5 | M | 1.5 | 0.3 | 59 | 212 | 11.9 | 11.3 | 101 | 5.9 | 147 | 1.2 |
| D6 | F | 1.5 | 0.4 | 68 | 196 | 11.8 | 8.4 | 102 | 6.1 | 148 | 1.3 |
| D7 | F | 1.5 | 0.4 | 78 | 173 | 11.6 | 8.5 | 100 | 6.3 | 147 | 1.2 |
| D8 | F | 1.5 | 0.4 | 81 | 192 | 11.3 | 7.2 | 101 | 5.2 | 148 | 1.2 |
| D9 | F | 1.5 | 0.4 | 67 | 193 | 11.2 | 8.6 | 98 | 5 | 147 | 1.2 |
| D10 | F | 1.5 | 0.4 | 56 | 188 | 10.9 | 8.5 | 103 | 6.1 | 146 | 1.3 |

| | | | Clinical Chemistry Parameters | | | | | | | | | |
|---|---|---|---|---|---|---|---|---|---|---|---|---|
| Animal ID | Sex | Anatabine Dose (mg/kg) | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RATIO |
| E1 | M | 0.75[1] | 0.3 | 53 | 199 | 11.1 | 10.5 | 100 | 6.7 | 147 | 1.1 |
| E2 | M | 0.75[1] | 0.3 | 71 | 204 | 11.1 | 10.4 | 99 | 6 | 148 | 1 |
| E3 | M | 0.75[1] | 0.4 | 55 | 213 | 11 | 9.6 | 99 | 5.6 | 146 | 1.1 |
| E5 | M | 0.75[1] | 0.4 | 65 | 188 | 11 | 11 | 101 | 6.5 | 146 | 1 |
| E6 | F | 0.75[1] | 0.5 | 76 | 299 | 11.1 | 8.8 | 97 | 6.8 | 144 | 1.1 |
| E9 | F | 0.75[1] | 0.4 | 71 | 196 | 11.2 | 9.1 | 99 | 6.2 | 144 | 1 |
| E10 | F | 0.75[1] | 0.4 | 48 | 221 | 10.8 | 7.2 | 100 | 5.8 | 147 | 1 |

1. Dose is 0.75 mg/kg nicotine

**Table 34. Coagulation Parameters**

| Animal ID | Sex | Anatabine Dose (mg/kg) | Coagulation Parameters<br>ACTIVE PARTIAL THROMBOPLASTIN TIME<br>seconds | PROTHROMBIN TIME<br>seconds |
|---|---|---|---|---|
| A1 | M | 0 | 33.5 | 13.3 |
| A2 | M | 0 | 31.7 | 13.3 |
| A3 | M | 0 | 29.6 | 12.5 |
| A4 | M | 0 | 30.4 | 12.3 |
| A5 | M | 0 | 34.9 | 13.1 |
| A6 | F | 0 | 36.7 | 12.8 |
| A7 | F | 0 | 36.2 | 12.7 |
| A8 | F | 0 | 40.6 | 13.2 |
| A9 | F | 0 | 34.5 | 12.5 |
| A10 | F | 0 | 29.2 | 12.5 |
| B1 | M | 0.1 | 34.9 | 13.6 |
| B2 | M | 0.1 | 36.4 | 13.4 |
| B3 | M | 0.1 | 29.6 | 12.5 |
| B4 | M | 0.1 | 28.9 | 12.3 |
| B5 | M | 0.1 | 32 | 12.1 |
| B6 | F | 0.1 | 31.1 | 12.3 |
| B7 | F | 0.1 | 34.2 | 11.3 |
| B8 | F | 0.1 | 26 | 12.2 |
| B9 | F | 0.1 | 26.7 | 12.3 |
| B10 | F | 0.1 | 28.9 | 12.3 |
| C1 | M | 0.75 | 39.7 | 12.4 |
| C2 | M | 0.75 | 43.9 | 12.1 |
| C3 | M | 0.75 | 27.9 | 12.1 |
| C4 | M | 0.75 | 50.4 | 17.9 |
| C5 | M | 0.75 | 31.7 | 12.1 |
| C6 | F | 0.75 | 31.4 | 11.4 |
| C7 | F | 0.75 | 32.5 | 12.1 |
| C8 | F | 0.75 | 31.5 | 11.6 |
| C9 | F | 0.75 | 32.7 | 11.4 |
| C10 | F | 0.75 | 38.2 | 23.8 |
| D1 | M | 1.5 | 34.1 | 13.8 |
| D2 | M | 1.5 | 33.8 | 13.1 |
| D3 | M | 1.5 | 28.3 | 12.9 |
| D4 | M | 1.5 | 27.9 | 12.9 |
| D5 | M | 1.5 | 29.1 | 13.5 |
| D6 | F | 1.5 | 37.2 | 13.2 |
| D7 | F | 1.5 | 31.7 | 13.2 |
| D8 | F | 1.5 | 29.3 | 13 |
| D9 | F | 1.5 | 32.3 | 13.6 |
| D10 | F | 1.5 | 32 | 13.3 |
| E1 | M | 0.75[1] | 15 | 13.8 |
| E2 | M | 0.75[1] | 16.3 | 13.2 |
| E3 | M | 0.75[1] | 17.6 | 14.2 |

(continued)

| | | | Coagulation Parameters | |
|---|---|---|---|---|
| Animal ID | Sex | Anatabine Dose (mg/kg) | ACTIVE PARTIAL THROMBOPLASTIN TIME | PROTHROMBIN TIME |
| | | | seconds | seconds |
| E5 | M | 0.75[1] | 16.2 | 13 |
| E6 | F | 0.75[1] | 18.7 | 13.9 |
| E9 | F | 0.75[1] | 15 | 14.2 |
| E10 | F | 0.75[1] | UNABLE TO OBTAIN RESULTS DUE TO FIBRIN CLOTS | UNABLE TO OBTAIN RESULTS DUE TO FIBRIN CLOTS |

1. Dose is 0.75 mg/kg nicotine

**Table 35. Urinalysis Results**

| Animal ID | Sex | Anatabine Dose (mg/kg) | Urinalysis VOLUME | COLOR | CLARITY | SPEC GRAVITY | pH | PROTEIN | GLUCOSE | KETONES | UROBILINOGEN | BILIRUBIN | BLOOD |
|---|---|---|---|---|---|---|---|---|---|---|---|---|---|
| A1 | M | 0 | <0.5 mL | YELLOW | HAZY | 1.046 | 7 | TRACE | NEGATIVE | 1+ | NORMAL | NEGATIVE | NEGATIVE |
| A2 | M | 0 | <0.5 mL | YELLOW | HAZY | 1.046 | 7 | TRACE | NEGATIVE | 1+ | NORMAL | NEGATIVE | NEGATIVE |
| A3 | M | 0 | <0.5 mL | YELLOW | HAZY | 1.046 | 7.5 | TRACE | NEGATIVE | 1+ | NORMAL | 1+ | NEGATIVE |
| A4 | M | 0 | <0.5 mL | YELLOW | HAZY | 1.031 | 7.5 | TRACE | NEGATIVE | NEGATIVE | NORMAL | NEGATIVE | 2+ |
| A5 | M | 0 | <0.5 mL | YELLOW | HAZY | 1.026 | 7.5 | TRACE | NEGATIVE | NEGATIVE | NORMAL | NEGATIVE | TRACE |
| A6 | F | 0 | <0.5 mL | YELLOW | HAZY | 1.034 | 6.5 | TRACE | NEGATIVE | NEGATIVE | NORMAL | NEGATIVE | TRACE |
| A7 | F | 0 | <0.5 mL | YELLOW | HAZY | 1.034 | 7 | TRACE | NEGATIVE | NEGATIVE | NORMAL | NEGATIVE | TRACE |
| A8 | F | 0 | <0.5 mL | YELLOW | HAZY | 1.024 | 8.5 | A | NEGATIVE | NEGATIVE | NORMAL | NEGATIVE | NEGATIVE |
| A9 | F | 0 | <0.5 mL | YELLOW | HAZY | 1.021 | 8 | 1+ | NEGATIVE | NEGATIVE | NORMAL | NEGATIVE | NEGATIVE |
| A10 | F | 0 | <0.25 mL | YELLOW | HAZY | 1.047 | 7 | A | NEGATIVE | NEGATIVE | NORMAL | NEGATIVE | TRACE |
| B1 | M | 0.1 | <0.5 mL | YELLOW | HAZY | 1.027 | 8.5 | TRACE | NEGATIVE | 1+ | NORMAL | NEGATIVE | TRACE |
| B2 | M | 0.1 | A | DNR | DNR | DNR | DNR | DNR | DNR | DNR | DNR | DNR | DNR |
| B3 | M | 0.1 | <0.5 mL | YELLOW | HAZY | 1.039 | 7.5 | TRACE | NEGATIVE | NEGATIVE | NORMAL | NEGATIVE | TRACE |
| B4 | M | 0.1 | <0.5 mL | YELLOW | HAZY | 1.027 | 7 | NEGATIVE | NEGATIVE | 1+ | NORMAL | NEGATIVE | NEGATIVE |
| B5 | M | 0.1 | <0.5 mL | YELLOW | HAZY | 1.048 | 7.5 | TRACE | NEGATIVE | 1+ | NORMAL | NEGATIVE | 1+ |
| B6 | F | 0.1 | <0.5 mL | YELLOW | HAZY | 1.039 | 7.5 | A | NEGATIVE | NEGATIVE | NORMAL | NEGATIVE | NEGATIVE |
| B7 | F | 0.1 | <0.5 mL | YELLOW | HAZY | 1.036 | 7.5 | A | NEGATIVE | 1+ | NORMAL | NEGATIVE | TRACE |
| B8 | F | 0.1 | <0.5 mL | YELLOW | HAZY | 1.045 | 7 | NEGATIVE | NEGATIVE | 1+ | NORMAL | 1+ | NEGATIVE |
| B9 | F | 0.1 | 0.5 | YELLOW | HAZY | 1.017 | 8 | NEGATIVE | NEGATIVE | NEGATIVE | NORMAL | NEGATIVE | NEGATIVE |
| B10 | F | 0.1 | A | DNR | DNR | DNR | DNR | DNR | DNR | DNR | DNR | DNR | DNR |
| C1 | M | 0.75 | | | | | | **No Urine Sample Submitted** | | | | | |
| C2 | M | 0.75 | <0.5 mL | YELLOW | HAZY | 1.05 | 7 | TRACE | NEGATIVE | 1+ | NORMAL | NEGATIVE | TRACE |
| C3 | M | 0.75 | <0.5 mL | YELLOW | HAZY | 1.051 | 7 | TRACE | NEGATIVE | 1+ | NORMAL | NEGATIVE | NEGATIVE |
| C4 | M | 0.75 | <0.5 mL | YELLOW | HAZY | 1.043 | 8 | TRACE | NEGATIVE | NEGATIVE | NORMAL | 1+ | NEGATIVE |
| C5 | M | 0.75 | <0.5 mL | YELLOW | HAZY | 1.049 | 7 | TRACE | NEGATIVE | 1+ | NORMAL | NEGATIVE | NEGATIVE |
| C6 | F | 0.75 | <0.5 mL | YELLOW | HAZY | 1.016 | 7.5 | A | NEGATIVE | NEGATIVE | NORMAL | NEGATIVE | 3+ |
| C7 | F | 0.75 | <0.5 mL | YELLOW | HAZY | 1.039 | 6 | A | NEGATIVE | NEGATIVE | NORMAL | NEGATIVE | TRACE |
| C8 | F | 0.75 | <0.5 mL | YELLOW | HAZY | 1.036 | 8 | A | NEGATIVE | 1+ | NORMAL | NEGATIVE | NEGATIVE |
| C9 | F | 0.75 | <0.25 mL | YELLOW | HAZY | A | DNR | DNR | NEGATIVE | 1+ | DNR | NEGATIVE | DNR |

(continued)

| Animal ID | Sex | Anatabine Dose (mg/kg) | Urinalysis VOLUME | COLOR | CLARITY | SPEC GRAVITY | pH | PROTEIN | GLUCOSE | KETONES | UROBILINOGEN | BILIRUBIN | BLOOD |
|---|---|---|---|---|---|---|---|---|---|---|---|---|---|
| C10 | F | 0.75 | <0.5 mL | YELLOW | HAZY | 1.014 | 8 | 1+ | NEGATIVE | NEGATIVE | NORMAL | NEGATIVE | 2+ |
| D1 | M | 1.5 | <0.25 mL | YELLOW | HAZY | 1.031 | 8 | A | NEGATIVE | 1+ | NORMAL | NEGATIVE | 2+ |
| D2 | M | 1.5 | <0.5 mL | STRAW | CLOUDY | 1.034 | 7.5 | A | NEGATIVE | 1+ | NORMAL | NEGATIVE | 2+ |
| D3 | M | 1.5 | <0.25 mL | YELLOW | HAZY | 1.034 | 7.5 | A | NEGATIVE | 1+ | NORMAL | NEGATIVE | 2+ |
| D4 | M | 1.5 | <0.5 mL | YELLOW | HAZY | 1.045 | 7.5 | 2+ | NEGATIVE | 1+ | NORMAL | NEGATIVE | 2+ |
| D5 | M | 1.5 | <0.5 mL | YELLOW | HAZY | 1.047 | 8.5 | TRACE | NEGATIVE | 1+ | NORMAL | NEGATIVE | NEGATIVE |
| D6 | F | 1.5 | <0.5 mL | YELLOW | HAZY | 1.039 | 8 | 1+ | NEGATIVE | NEGATIVE | NORMAL | NEGATIVE | TRACE |
| D7 | F | 1.5 | <0.5 mL | YELLOW | HAZY | 1.038 | 8.5 | NEGATIVE | NEGATIVE | 1+ | NORMAL | NEGATIVE | NEGATIVE |
| D8 | F | 1.5 | <0.5 mL | YELLOW | HAZY | 1.039 | 7 | NEGATIVE | NEGATIVE | NEGATIVE | NORMAL | NEGATIVE | NEGATIVE |
| D9 | F | 1.5 | | | | | | **No Urine Sample Submitted** | | | | | |
| D10 | F | 1.5 | | | | | | **No Urine Sample Submitted** | | | | | |
| E1 | M | 0.75[1] | <0.5 mL | YELLOW | HAZY | 1.054 | 6 | NEGATIVE | NEGATIVE | NEGATIVE | NORMAL | NEGATIVE | NEGATIVE |
| E2 | M | 0.75[1] | <0.5 mL | YELLOW | HAZY | 1.058 | 6.5 | TRACE | NEGATIVE | NEGATIVE | NORMAL | NEGATIVE | 1+ |
| E3 | M | 0.75[1] | | | | | | **No Urine Sample Submitted** | | | | | |
| E5 | M | 0.75[1] | <0.5 mL | YELLOW | HAZY | 1.046 | 7 | NEGATIVE | NEGATIVE | NEGATIVE | NORMAL | NEGATIVE | NEGATIVE |
| E6 | F | 0.75[1] | 10UL | YELLOW | HAZY | 1.047 | 6.5 | NEGATIVE | NEGATIVE | NEGATIVE | NORMAL | NEGATIVE | 2+ |
| E9 | F | 0.75[1] | <0.5 mL | YELLOW | HAZY | 1.052 | 7.5 | NEGATIVE | NEGATIVE | NEGATIVE | NORMAL | NEGATIVE | NEGATIVE |
| E10 | F | 0.75[1] | | | | | | **No Urine Sample Submitted** | | | | | |

**DNR - did not report - insufficient sample**

1. Dose is 0.75 mg/kg nicotine

A. Sample quantity was not sufficient for complete testing: DNR did not report due to insufficient sample

Table 36. Tissue Collection Weights (g)

## Group A: Vehicle (Males)

| 1 | | 2 | | 3 | | 4 | | 5 | |
|---|---|---|---|---|---|---|---|---|---|
| Tissue | weights | Tissue | weights | Tissue | weights | Tissue | weights | Tissue | weights |
| Thymus | 0.80 | Thymus | 0.80 | Thymus | 0.80 | Thymus | 0.70 | Thymus | 0.76 |
| Heart | 1.65 | Heart | 1.78 | Heart | 1.87 | Heart | 1.39 | Heart | 1.62 |
| Lungs | 2.02 | Lungs | 1.67 | Lungs | 2.16 | Lungs | 1.70 | Lungs | 1.97 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 17.19 | Liver | 17.01 | Liver | 15.67 | Liver | 13.61 | Liver | 15.58 |
| Adrenals | Cass | Adrenals | Cass | Adrenals | Cass | Adrenals | Cass | Adrenals | Cass |
| Kidneys | 3.86 | Kidneys | 3.51 | Kidneys | 3.67 | Kidneys | 3.06 | Kidneys | 3.54 |
| Spleen | 1.12 | Spleen | 0.86 | Spleen | 1.41 | Spleen | 0.89 | Spleen | 0.88 |
| Small intestine | 0.53 | Small intestine | 1.4 | Small intestine | 0.47 | Small intestine | 0.41 | Small intestine | 0.41 |
| Prostate | 0.31 | Prostate | 0.56 | Prostate | 0.56 | Prostate | 0.57 | Prostate | 0.43 |
| Testes | 3.40 | Testes | 3.33 | Testes | 3.31 | Testes | 3.46 | Testes | 3.47 |
| Brain | 1.82 | Brain | 2.04 | Brain | 2.04 | Brain | 2.04 | Brain | 2.14 |
| Pituitary | Cass | Pituitary | cass | Pituitary | cass | Pituitary | Cass | Pituitary | Cass |
| Marrow | √ | Marrow | √ | Marrow | √ | Marrow | √ | Marrow | √ |

## Group A Vehicle (Females)

| 6 | | 7 | | 8 | | 9 | | 10 | |
|---|---|---|---|---|---|---|---|---|---|
| Tissue | weights | Tissue | weights | Tissue | weights | Tissue | weights | Tissue | weights |
| Thymus | 0.66 | Thymus | 0.79 | Thymus | 0.78 | Thymus | 0.55 | Thymus | 0.66 |
| Heart | 1.33 | Heart | 1.31 | Heart | 1.34 | Heart | 1.14 | Heart | 1.22 |
| Lungs | 1.67 | Lungs | 1.60 | Lungs | 1.36 | Lungs | 1.41 | Lungs | 1.56 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 12.40 | Liver | 11.24 | Liver | 10.19 | Liver | 9.36 | Liver | 11.49 |
| Adrenals | Cass | Adrenals | Cass | Adrenals | Cass | Adrenals | Cass | Adrenals | Cass |
| Kidneys | 2.66 | Kidneys | 2.72 | Kidneys | 2.14 | Kidneys | 1.75 | Kidneys | 2.42 |
| Spleen | 0.72 | Spleen | 0.68 | Spleen | 0.56 | Spleen | 0.50 | Spleen | 0.61 |
| Small intestine | 1.33 | Small intestine | 0.41 | Small intestine | 0.90 | Small intestine | 0.44 | Small intestine | 1.02 |
| Ovaries/ uterus | 0.76 | Ovaries/ uterus | 1.42 | Ovaries/ uterus | 0.83 | Ovaries/ uterus | 1.88 | Ovaries/ uterus | 0.61 |
| Brain | 1.87 | Brain | 2.06 | Brain | 1.85 | Brain | 2.12 | Brain | 1.76 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group B: Anatabine 0.1 mg/kg (Males)

| 1 | |
|---|---|
| **Tissue** | **weights** |
| Thymus | 0.74 |
| Heart | 1.71 |
| Lungs | 2.31 |
| Thyroid/ para thyroid | Cass |
| Liver | 17.92 |
| Adrenals | Cass |
| Kidneys | 3.55 |
| Spleen | 0.91 |
| Small intestine | 0.61 |
| Prostate | 0.72 |
| Testes | 3.69 |
| Brain | 2.35 |
| Pituitary | cass |
| Marrow | √ |

| 2 | |
|---|---|
| **Tissue** | **weights** |
| Thymus | 0.74 |
| Heart | 1.59 |
| Lungs | 1.77 |
| Thyroid/ para thyroid | Cass |
| Liver | 15.45 |
| Adrenals | Cass |
| Kidneys | 3.33 |
| Spleen | 0.66 |
| Small intestine | 0.96 |
| Prostate | 0.49 |
| Testes | 3.64 |
| Brain | 2.16 |
| Pituitary | cass |
| Marrow | √ |

| 3 | |
|---|---|
| **Tissue** | **weights** |
| Thymus | 0.76 |
| Heart | 1.97 |
| Lungs | 2.08 |
| Thyroid/ para thyroid | Cass |
| Liver | 16.98 |
| Adrenals | Cass |
| Kidneys | 3.70 |
| Spleen | 0.82 |
| Small intestine | 0.88 |
| Prostate | 0.73 |
| Testes | 3.14 |
| Brain | 2.24 |
| Pituitary | cass |
| Marrow | √ |

| 4 | |
|---|---|
| **Tissue** | **weights** |
| Thymus | 0.71 |
| Heart | 1.60 |
| Lungs | 1.91 |
| Thyroid/ para thyroid | Cass |
| Liver | 16.32 |
| Adrenals | Cass |
| Kidneys | 3.27 |
| Spleen | 0.93 |
| Small intestine | 0.50 |
| Prostate | 0.53 |
| Testes | 3.35 |
| Brain | 2.11 |
| Pituitary | cass |
| Marrow | √ |

| 5 | |
|---|---|
| **Tissue** | **weights** |
| Thymus | 0.81 |
| Heart | 1.85 |
| Lungs | 1.83 |
| Thyroid/ para thyroid | Cass |
| Liver | 17.38 |
| Adrenals | Cass |
| Kidneys | 3.99 |
| Spleen | 0.76 |
| Small intestine | 0.35 |
| Prostate | 0.48 |
| Testes | 3.29 |
| Brain | 1.88 |
| Pituitary | cass |
| Marrow | √ |

## Group B: Anatabine 0.1 mg/kg (Females)

| 6 | | 7 | | 8 | | 9 | | 10 | |
|---|---|---|---|---|---|---|---|---|---|
| Tissue | weights | Tissue | weights | Tissue | weights | Tissue | weights | Tissue | weights |
| Thymus | 0.68 | Thymus | 0.59 | Thymus | 0.49 | Thymus | 0.71 | Thymus | 0.73 |
| Heart | 1.29 | Heart | 1.12 | Heart | 0.95 | Heart | 1.05 | Heart | 1.25 |
| Lungs | 1.42 | Lungs | 1.39 | Lungs | 1.15 | Lungs | 1.52 | Lungs | 1.59 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 11.54 | Liver | 10.09 | Liver | 9.94 | Liver | 12.94 | Liver | 9.67 |
| Adrenals | Cass | Adrenals | Cass | Adrenals | Cass | Adrenals | Cass | Adrenals | Cass |
| Kidneys | 2.46 | Kidneys | 2.02 | Kidneys | 2.02 | Kidneys | 2.14 | Kidneys | 2.25 |
| Spleen | 0.79 | Spleen | 0.62 | Spleen | 0.51 | Spleen | 0.67 | Spleen | 0.65 |
| Small intestine | 0.87 | Small intestine | 0.71 | Small intestine | 0.55 | Small intestine | 0.49 | Small intestine | 0.63 |
| Ovaries/ uterus | 0.96 | Ovaries/ uterus | 1.79 | Ovaries/ uterus | 0.84 | Ovaries/ uterus | 1.42 | Ovaries/ uterus | 0.70 |
| Brain | 1.91 | Brain | 1.89 | Brain | 1.75 | Brain | 1.86 | Brain | 1.89 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group C: Anatabine 0.75 mg/kg (Males)

| 1 | | 2 | | 3 | | 4 | | 5 | |
|---|---|---|---|---|---|---|---|---|---|
| Tissue | weights | Tissue | weights | Tissue | weights | Tissue | weights | Tissue | weights |
| Thymus | 1.02 | Thymus | 0.52 | Thymus | 0.67 | Thymus | 0.69 | Thymus | 0.47 |
| Heart | 1.52 | Heart | 1.38 | Heart | 1.56 | Heart | 1.39 | Heart | 1.29 |
| Lungs | 1.86 | Lungs | 1.97 | Lungs | 1.72 | Lungs | 1.95 | Lungs | 1.58 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 14.92 | Liver | 13.91 | Liver | 14.70 | Liver | 14.01 | Liver | 14.94 |
| Adrenals | Cass | Adrenals | Cass | Adrenals | Cass | Adrenals | Cass | Adrenals | Cass |
| Kidneys | 3.45 | Kidneys | 2.77 | Kidneys | 3.49 | Kidneys | 3.16 | Kidneys | 2.89 |
| Spleen | 0.71 | Spleen | 0.63 | Spleen | 0.79 | Spleen | 0.93 | Spleen | 0.64 |
| Small intestine | 0.54 | Small intestine | 1.41 | Small intestine | 0.56 | Small intestine | 0.41 | Small intestine | 0.47 |
| Prostate | 0.49 | Prostate | 0.49 | Prostate | 0.65 | Prostate | 0.46 | Prostate | 0.45 |
| Testes | 3.19 | Testes | 3.84 | Testes | 3.03 | Testes | 3.28 | Testes | 2.70 |
| Brain | 2.01 | Brain | 2.19 | Brain | 2.12 | Brain | 2.00 | Brain | 2.02 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group C: Anatabine 0.75 mg/kg (Females)

| 6 | |
|---|---|
| Tissue | weights |
| Thymus | 0.56 |
| Heart | 1.09 |
| Lungs | 1.46 |
| Thyroid/ para thyroid | Cass |
| Liver | 10.71 |
| Adrenals | Cass |
| Kidneys | 2.08 |
| Spleen | 0.70 |
| Small intestine | 0.72 |
| Ovaries/ uterus | 0.81 |
| Brain | 1.99 |
| Pituitary | cass |
| Marrow | √ |

| 7 | |
|---|---|
| Tissue | weights |
| Thymus | 0.53 |
| Heart | 1.21 |
| Lungs | 1.39 |
| Thyroid/ para thyroid | Cass |
| Liver | 8.65 |
| Adrenals | Cass |
| Kidneys | 2.20 |
| Spleen | 0.65 |
| Small intestine | 0.37 |
| Ovaries/ uterus | 1.50 |
| Brain | 1.93 |
| Pituitary | cass |
| Marrow | √ |

| 8 | |
|---|---|
| Tissue | weights |
| Thymus | 0.63 |
| Heart | 1.23 |
| Lungs | 1.62 |
| Thyroid/ para thyroid | Cass |
| Liver | 11.37 |
| Adrenals | Cass |
| Kidneys | 2.68 |
| Spleen | 0.65 |
| Small intestine | 0.85 |
| Ovaries/ uterus | 0.72 |
| Brain | 1.73 |
| Pituitary | cass |
| Marrow | √ |

| 9 | |
|---|---|
| Tissue | weights |
| Thymus | 0.52 |
| Heart | 1.03 |
| Lungs | 1.30 |
| Thyroid/ para thyroid | Cass |
| Liver | 11.20 |
| Adrenals | Cass |
| Kidneys | 2.18 |
| Spleen | 0.51 |
| Small intestine | 0.47 |
| Ovaries/ uterus | 1.23 |
| Brain | 2.06 |
| Pituitary | cass |
| Marrow | √ |

| 10 | |
|---|---|
| Tissue | weights |
| Thymus | 0.54 |
| Heart | 0.99 |
| Lungs | 1.49 |
| Thyroid/ para thyroid | Cass |
| Liver | 10.19 |
| Adrenals | Cass |
| Kidneys | 2.36 |
| Spleen | 0.49 |
| Small intestine | 1.34 |
| Ovaries/ uterus | 3.65 |
| Brain | 1.81 |
| Pituitary | cass |
| Marrow | √ |

## Group D: Anatabine 1.5 mg/kg (Males)

| 1 | | 2 | | 3 | | 4 | | 5 | |
|---|---|---|---|---|---|---|---|---|---|
| Tissue | weights | Tissue | weights | Tissue | weights | Tissue | weights | Tissue | weights |
| Thymus | 0.83 | Thymus | 0.77 | Thymus | 0.54 | Thymus | 0.86 | Thymus | 0.59 |
| Heart | 1.50 | Heart | 1.53 | Heart | 1.47 | Heart | 1.61 | Heart | 1.47 |
| Lungs | 1.77 | Lungs | 2.06 | Lungs | 1.93 | Lungs | 2.02 | Lungs | 1.90 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 15.86 | Liver | 17.03 | Liver | 17.35 | Liver | 18.27 | Liver | 14.26 |
| Adrenals | Cass | Adrenals | Cass | Adrenals | Cass | Adrenals | Cass | Adrenals | Cass |
| Kidneys | 3.48 | Kidneys | 3.54 | Kidneys | 3.02 | Kidneys | 3.79 | Kidneys | 3.43 |
| Spleen | 0.71 | Spleen | 0.83 | Spleen | 0.86 | Spleen | 0.97 | Spleen | 0.92 |
| Small intestine | 0.43 | Small intestine | 0.77 | Small intestine | 0.56 | Small intestine | 1.00 | Small intestine | 0.66 |
| Prostate | 0.50 | Prostate | 0.45 | Prostate | | Prostate | 0.49 | Prostate | 0.45 |
| Testes | 2.78 | Testes | 3.28 | Testes | 3.51 | Testes | 3.41 | Testes | 3.23 |
| Brain | 1.75 | Brain | 1.81 | Brain | 2.02 | Brain | 2.00 | Brain | 2.08 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group D: Anatabine 1.5 mg/kg (Females)

| 6 | | 7 | | 8 | | 9 | | 10 | |
|---|---|---|---|---|---|---|---|---|---|
| Tissue | weights | Tissue | weights | Tissue | weights | Tissue | weights | Tissue | weights |
| Thymus | 0.59 | Thymus | 0.63 | Thymus | 0.52 | Thymus | 0.61 | Thymus | 0.51 |
| Heart | 1.12 | Heart | 0.88 | Heart | 1.04 | Heart | 0.97 | Heart | 0.98 |
| Lungs | 1.29 | Lungs | 1.46 | Lungs | 1.37 | Lungs | 1.60 | Lungs | 1.59 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 9.65 | Liver | 8.83 | Liver | 11.46 | Liver | 11.05 | Liver | 9.21 |
| Adrenals | Cass | Adrenals | Cass | Adrenals | Cass | Adrenals | Cass | Adrenals | Cass |
| Kidneys | 2.17 | Kidneys | 2.08 | Kidneys | 2.52 | Kidneys | 2.64 | Kidneys | 1.97 |
| Spleen | 0.53 | Spleen | 0.65 | Spleen | 0.64 | Spleen | 0.68 | Spleen | 0.51 |
| Small intestine | 0.49 | Small intestine | 0.60 | Small intestine | 1.07 | Small intestine | 0.56 | Small intestine | 0.77 |
| Ovaries/ uterus | 0.84 | Ovaries/ uterus | 1.20 | Ovaries/ uterus | 0.98 | Ovaries/ uterus | 1.34 | Ovaries/ uterus | 0.78 |
| Brain | 1.90 | Brain | 1.91 | Brain | 1.97 | Brain | 2.08 | Brain | 1.91 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group E: Nicotine 0.75 mg/kg (Males)

| 1 | | 2 | | 3 | | 5 | |
|---|---|---|---|---|---|---|---|
| Tissue | weights | Tissue | weights | Tissue | weights | Tissue | weights |
| Thymus | 0.54 | Thymus | 0.46 | Thymus | 0.56 | Thymus | 0.76 |
| Heart | 1.20 | Heart | 1.17 | Heart | 1.21 | Heart | 1.42 |
| Lungs | 1.84 | Lungs | 1.49 | Lungs | 1.62 | Lungs | 1.66 |
| Thyroid/ para thyroid | Cass | Thyroid/ para thyroid | Cass | Thyroid/ para thyroid | Cass | Thyroid/ para thyroid | Cass |
| Liver | 11.97 | Liver | 12.52 | Liver | 12.06 | Liver | 14.20 |
| Adrenals | Cass | Adrenals | Cass | Adrenals | Cass | Adrenals | Cass |
| Kidneys | 3.19 | Kidneys | 3.04 | Kidneys | 2.87 | Kidneys | 3.01 |
| Spleen | 0.81 | Spleen | 0.62 | Spleen | 0.78 | Spleen | 0.86 |
| Small intestine | 0.71 | Small intestine | 0.56 | Small intestine | 0.53 | Small intestine | 0.40 |
| Prostate | 0.41 | Prostate | 0.28 | Prostate | 0.45 | Prostate | 0.28 |
| Testes | 3.05 | Testes | 3.13 | Testes | 3.02 | Testes | 2.92 |
| Brain | 2.06 | Brain | 1.90 | Brain | 1.84 | Brain | 1.94 |
| Pituitary | Cass | Pituitary | Cass | Pituitary | Cass | Pituitary | Cass |
| Marrow | √ | Marrow | √ | Marrow | √ | Marrow | √ |

## Group E: Nicotine 0.75 mg/kg (Females)

| 6 | | 9 | | 10 | |
|---|---|---|---|---|---|
| Tissue | weights | Tissue | weights | Tissue | weights |
| Thymus | 0.54 | Thymus | 0.48 | Thymus | 0.48 |
| Heart | 1.09 | Heart | 1.06 | Heart | 0.83 |
| Lungs | 1.20 | Lungs | 1.48 | Lungs | 1.28 |
| Thyroid/ para thyroid | Cass | Thyroid/ para thyroid | Cass | Thyroid/ para thyroid | Cass |
| Liver | 9.48 | Liver | 10.11 | Liver | 9.18 |
| Adrenals | Cass | Adrenals | Cass | Adrenals | Cass |
| Kidneys | 2.34 | Kidneys | 2.29 | Kidneys | 2.29 |
| Spleen | 0.46 | Spleen | 0.76 | Spleen | 0.46 |
| Small intestine | 0.41 | Small intestine | 0.40 | Small intestine | 0.28 |
| Ovaries/ uterus | 1.59 | Ovaries/ uterus | 0.92 | Ovaries/ uterus | 1.32 |
| Brain | 1.77 | Brain | 1.93 | Brain | 1.87 |
| Pituitary | Cass | Pituitary | Cass | Pituitary | Cass |
| Marrow | √ | Marrow | √ | Marrow | √ |

**Table 37. Dosing solutions**

| Test compound | Percentage content of anatabine | Dose level (mg/kg) | Test compound concentration (total) (mg/mL) | Test compound concentration (base) (mg/mL) | Injection volume (mL/kg) |
|---|---|---|---|---|---|
| Anatabine | 5.18 | 0.20 | 0.772 | 0.04 | 5 |
| Anatabine | 5.18 | 2.0 | 7.72 | 0.4 | 5 |

**Table 38. Dosing**

| Test compound | Route | Sex | Concentration mg/kg/ dose | # of animals | Dosing times (minutes) |
|---|---|---|---|---|---|
| Anatabine | p.o. | M | 0.2 | 4 | 0, 240, 480 |
| | | F | 0.2 | 4 | |
| | | M | 2.0 | 4 | |
| | | F | 2.0 | 4 | |

**Table 39. Blood Collection Times**

| Test compound | Route | Sex | Concentration mg/kg/ dose | # of animals | Blood collection (minutes) |
|---|---|---|---|---|---|
| Anatabine | p.o. | M | 0.2 | 4 | 30,60,235 (pre-dose), 270, 300, 475 (pre-dose), 540, 600, 720, 1440 |
| | | F | 0.2 | 4 | |
| | | M | 2.0 | 4 | |
| | | F | 2.0 | 4 | |

**Table 40. Calibration Curve Concentrations**

| nominal concentration (nM) | stock concentration (μM) |
|---|---|
| 5000 | 250 |
| 1667 | 83.3 |
| 555.5 | 27.8 |
| 185.2 | 9.3 |
| 61.7 | 3.1 |
| 20.6 | 1.0 |
| 6.9 | 0.34 |
| 2.3 | 0.11 |
| 0.76 | 0.038 |
| 0.25 | 0.013 |

**Table 41. LC/MS/MS ionization conditions and identity of parent and productions.**

| Compound | MW | Polarization | Precursor m/z | Product m/z | Collision energy (V) |
|---|---|---|---|---|---|
| **Anatabine** | 160.2 | Positive | 161.1 | 115.1 | 28 |
| **(*R*,*S*)-Antabine-2,4,5,6-$d_4$** | 164.24 | Positive | 165.1 | 148.1 | 20 |

**Table 42. Limits of Detection and Calibration Curves**

| Sample | Limit of Detection (LOD) (ng/mL) | Lower Limit of Quantitation (LLQ) (ng/mL) | Upper Limit of Quantitation (ULQ) (ng/mL) |
|---|---|---|---|
| **Anatabine in rat plasma** | 0.37 | 1.1 | ≥801 |

Table 43. Dosing Solution Analysis

| Compound | Dose (mg/kg) | Expected Concentration (mg/mL) | Actual Concentration (mg/mL) | Actual Concentration relative to Expected (%) |
|---|---|---|---|---|
| **anatabine** | 0.2 | 0.04 | 0.025 | 63% |
| **anatabine** | 2.0 | 0.4 | 0.335 | 84% |

**Table 44. Comparison of pharmacokinetic parameters ($T_{max}$, $C_{p, max}$ and $C_{p, min}$) between male and female rats in each of the two treatment groups.**

| Parameter | Anatabine (0.6 mg/kg) | | | | | | | Anatabine (6.0 mg/kg) | | | | | | |
|---|---|---|---|---|---|---|---|---|---|---|---|---|---|---|
| | Male | | | Female | | | *p* | Male | | | Female | | | *p* |
| | n | Mean | SD | n | Mean | SD | | n | Mean | SD | n | Mean | SD | |
| $T_{max(1)}$ (hr) | 4 | 0.75 | 0.29 | 4 | 0.63 | 0.25 | 0.537 | 4 | 0.63 | 0.25 | 4 | 0.50 | 0.00 | 0.356 |
| $T_{max (2)}$ (hr) | 4 | 0.63 | 0.25 | 4 | 0.88 | 0.25 | 0.207 | 4 | 0.63 | 0.25 | 4 | 0.63 | 0.25 | 1.000 |
| $T_{max (3)}$ (hr) | 4 | 1.00 | 0 | 4 | 1.25 | 0.50 | 0.356 | 4 | 2.00 | 1.41 | 4 | 1.25 | 0.50 | 0.356 |
| $C_{p, max (1)}$ (ng/mL) | 4 | 34.3 | 2.1 | 4 | 38.3 | 6.1 | 0.262 | 4 | 244 | 86 | 4 | 260 | 35 | 0.738 |
| $C_{p, max (2)}$ (ng/mL) | 4 | 30.3 | 3.8 | 4 | 31.3 | 14.2 | 0.896 | 4 | 305 | 53 | 4 | 312 | 77 | 0.889 |
| $C_{p, max (3)}$ (ng/mL) | 3 | 37.3 | 8.1 | 4 | 35.5 | 10.6 | 0.814 | 4 | 283 | 94 | 4 | 375 | 123 | 0.281 |
| $C_{p, min (1)}$ (ng/mL) | 3 | 11.0 | 2.0 | 4 | 10.3 | 6.4 | 0.856 | 4 | 75 | 26 | 4 | 52 | 26 | 0.254 |
| Cp, min (2) (ng/mL) | 3 | 15.3 | 5.5 | 4 | 7.5 | 1.7 | 0.040 | 4 | 93 | 16 | 4 | 180 | 31 | 0.002 |

Table 45. Comparison of pharmacokinetic parameters ($C_{p, max}$ and $C_{p, min}$) over time for male and female rats in each of the two treatment groups.

| Parameter | Anatabine (0.6 mg/kg) | | | | | | Anatabine (6.0 mg/kg) | | | | | |
|---|---|---|---|---|---|---|---|---|---|---|---|---|
| | Male | | | Female | | | Male | | | Female | | |
| | Mean | SD | *p* | Mean | SD | *p* | Mean | SD | *p* | Mean | SD | *p* |
| $C_{p, max (1)}$ (ng/mL) | 34.3 | 2.1 | | 38.3 | 6.1 | | 287 | 11 | | 259.8 | 35.4 | |
| $C_{p, max (2)}$ (ng/mL) | 30.3 | 3.8 | 0.195 | 31.3 | 14.2 | 0.570 | 305 | 53 | 0.897 | 312.0 | 76.8 | 0.142 |
| $C_{p, max (3)}$ (ng/mL) | 37.3 | 8.1 | | 35.5 | 10.6 | | 283 | 94 | | 374.8 | 122.9 | |
| $C_{p, min (1)}$ (ng/mL) | 11.0 | 2.0 | 0.177 | 10.3 | 6.4 | 0.428 | 75 | 26 | 0.131 | 51.5 | 26.0 | 0.015 |
| $C_{p, min (2)}$ (ng/mL) | 15.3 | 5.5 | | 7.5 | 1.7 | | 93 | 16 | | 180.0 | 30.7 | |

**Table 46. Comparison of pharmacokinetic parameters ($AUC_{0\rightarrow\infty}$, $t_{1/2,\ 0\rightarrow 4}$, $t_{1/2,\ terminal}$, $MTT_{0\rightarrow 4}$ and $MAT_{0\rightarrow 4}$) between male and female rats in each of the two treatment groups.**

| Parameter | Anatabine (0.6 mg/kg) | | | | | | | Anatabine (6.0 mg/kg) | | | | | | |
|---|---|---|---|---|---|---|---|---|---|---|---|---|---|---|
| | Male | | | Female | | | *p* | Male | | | Female | | | *p* |
| | n | Mean | SD | n | Mean | SD | | n | Mean | SD | n | Mean | SD | |
| $AUC_{0\rightarrow\infty}$ (ng·hr/mL) | 3 | 280 | 102 | 4 | 290 | 71 | 0.880 | 4 | 3257 | 480 | 4 | 3735 | 587 | 0.255 |
| $t_{1/2,\ 0\rightarrow 4}$ (hr) | 4 | 2.05 | 0.43 | 4 | 2.06 | 1.20 | 0.986 | 3 | 1.76 | 0.39 | 4 | 1.82 | 0.81 | 0.918 |
| $t_{1/2,\ terminal}$ (hr) | 3 | 1.78 | 0.68 | 4 | 1.80 | 0.72 | 0.970 | 4 | 5.07 | 2.05 | 4 | 3.99 | 1.52 | 0.430 |
| $MTT_{0\rightarrow 4}$ (hr) | 3 | 3.17 | 0.51 | 4 | 2.98 | 1.84 | 0.873 | 4 | 3.18 | 1.29 | 4 | 2.76 | 1.39 | 0.668 |
| $MAT_{0\rightarrow 4}$ (hr) | 3 | 0.71 | 1.34 | 4 | 0.53 | 1.84 | 0.873 | 4 | 0.73 | 1.29 | 4 | 0.30 | 1.39 | 0.668 |

**Table 47. Comparison of pharmacokinetic parameters ($AUC_{0\to\infty}$, $t_{1/2,\ 0\to4}$, $t_{1/2,\ terminal}$, $MTT_{0\to4}$ and $MAT_{0\to4}$) between treatment groups.**

| Parameter | Anatabine (0.6 mg/kg) | | | Anatabine(6.0 mg/kg) | | | *p* |
|---|---|---|---|---|---|---|---|
| | n | Mean | SD | n | Mean | SD | |
| $AUC_{0\to\infty}$ (ng·hr/mL) | 7 | 285 | 77 | 8 | 3496 | 559 | <0.001 |
| $t_{1/2,\ 0\to4}$ (hr) | 8 | 2.05 | 0.83 | 7 | 1.79 | 0.62 | 0.514 |
| $t_{1/2,\ terminal}$ (hr) | 7 | 1.79 | 0.64 | 8 | 4.53 | 1.77 | 0.002 |
| $MTT_{0\to4}$ (hr) | 7 | 3.06 | 1.34 | 8 | 2.97 | 1.26 | 0.898 |
| $MAT_{0\to4}$ (hr) | 7 | 0.61 | 1.34 | 8 | 0.52 | 1.26 | 0.898 |

**Table 48. Comparison of pharmacokinetic parameters ($t_{1/2,\ 0\to4}$, $MTT_{0\to4}$ and $MAT_{0\to4}$) between male and female rats in both treatment groups, combined, and all data combined.**

| Parameter | Male | | | Female | | | *p* | Overall | | |
|---|---|---|---|---|---|---|---|---|---|---|
| | n | Mean | SD | n | Mean | SD | | n | Mean | SD |
| $t_{1/2,\ 0\to4}$ (hr) | 7 | 1.92 | 0.41 | 8 | 1.94 | 0.96 | 0.973 | 15 | 1.93 | 0.73 |
| $MTT_{0\to4}$ (hr) | 7 | 3.18 | 0.96 | 8 | 2.87 | 1.52 | 0.650 | 15 | 3.01 | 1.25 |
| $MAT_{0\to4}$ (hr) | 7 | 0.72 | 0.96 | 8 | 0.42 | 1.52 | 0.650 | 15 | 0.56 | 1.25 |

**Table 49. Animal Weights and Dosing Times**

| Cmpnd | Rat | B.W. (g) | Dose volume (ml) | Dose time | Time Points for Samples (hrs) - retro-orbital 0.5 | 1 | 4 (pre) | dose (2) | 4.5 | 5 | 8 (pre) | dose (3) | 9 | 10 | 12 | 24 |
|---|---|---|---|---|---|---|---|---|---|---|---|---|---|---|---|---|
| 1 | A | 246 | 1.2 | 6:06 | 6:36 | 7:06 | 10:01 | 10:06 | 10:36 | 11:06 | 2:01 | 2:06 | 3:06 | 4:06 | 6:06 | 6:06 |
| MALE | B | 231 | 1.2 | 6:07 | 6:37 | 7:07 | 10:02 | 10:07 | 10:37 | 11:07 | 2:02 | 2:07 | 3:07 | 4:07 | 6:07 | 6:07 |
| | C | 244 | 1.2 | 6:08 | 6:38 | 7:08 | 10:03 | 10:08 | 10:38 | 11:08 | 2:03 | 2:08 | 3:08 | 4:08 | 6:08 | 6:08 |
| Anatabine 0.2 MPK/ dose | D | 242 | 1.2 | 6:09 | 6:39 | 7:09 | 10:04 | 10:09 | 10:39 | 11:09 | 2:04 | 2:09 | 3:09 | 4:09 | 6:09 | 6:09 |
| 2 | A | 203 | 1.0 | 6:10 | 6:40 | 7:10 | 10:05 | 10:10 | 10:40 | 11:10 | 2:05 | 2:10 | 3:10 | 4:10 | 6:10 | 6:10 |
| FEMALE | B | 200 | 1.0 | 6:11 | 6:41 | 7:11 | 10:06 | 10:11 | 10:41 | 11:11 | 2:06 | 2:11 | 3:11 | 4:11 | 6:11 | 6:11 |
| | C | 212 | 1.1 | 6:12 | 6:42 | 7:12 | 10:07 | 10:12 | 10:42 | 11:12 | 2:07 | 2:12 | 3:12 | 4:12 | 6:12 | 6:12 |
| Anatabine 0.2 MPK/dose | D | 201 | 1.0 | 6:13 | 6:43 | 7:13 | 10:08 | 10:13 | 10:43 | 11:13 | 2:08 | 2:13 | 3:13 | 4:13 | 6:13 | 6:13 |
| 3 | A | 240 | 1.2 | 6:14 | 6:44 | 7:14 | 10:09 | 10:14 | 10:44 | 11:14 | 2:09 | 2:14 | 3:14 | 4:14 | 6:14 | 6:14 |
| MALE | B | 239 | 1.2 | 6:15 | 6:45 | 7:15 | 10:10 | 10:15 | 10:45 | 11:15 | 2:10 | 2:15 | 3:15 | 4:15 | 6:15 | 6:15 |
| | C | 241 | 1.2 | 6:16 | 6:46 | 7:16 | 10:11 | 10:16 | 10:46 | 11:16 | 2:11 | 2:16 | 3:16 | 4:16 | 6:16 | 6:16 |
| Anatabine 2.0 MPK/dose | D | 240 | 1.2 | 6:17 | 6:47 | 7:17 | 10:12 | 10:17 | 10:47 | 11:17 | 2:12 | 2:17 | 3:17 | 4:17 | 6:17 | 6:17 |
| 4 | A | 208 | 1.0 | 6:18 | 6:48 | 7:18 | 10:13 | 10:18 | 10:48 | 11:18 | 2:13 | 2:18 | 3:18 | 4:18 | 6:18 | 6:18 |
| FEMALE | B | 215 | 1.1 | 6:19 | 6:49 | 7:19 | 10:14 | 10:19 | 10:49 | 11:19 | 2:14 | 2:19 | 3:19 | 4:19 | 6:19 | 6:19 |
| | C | 207 | 1.0 | 6:20 | 6:50 | 7:20 | 10:15 | 10:20 | 10:50 | 11:20 | 2:15 | 2:20 | 3:20 | 4:20 | 6:20 | 6:20 |
| Anatabine 2.0 MPK/dose | D | 201 | 1.00 | 6:21 | 6:51 | 7:21 | 10:16 | 10:21 | 10:51 | 11:21 | 2:16 | 2:21 | 3:21 | 4:21 | 6:21 | 6:21 |

**Table 50. Measured Concentrations of Anatabine in Rat Plasma Samples at Each Time Point**

| Dose (mg/kg) | Animal ID | Sex | Time Point (min) | Anatabine Concentration (ng/mL) |
|---|---|---|---|---|
| 0.2 | 1A | male | 30 | 34 |
| | | | 60 | 26 |
| | | | 235 | 11 |
| | | | 270 | 35 |
| | | | 300 | 22 |
| | | | 475 | 9 |
| | | | 540 | 4 |
| | | | 600 | <LOQ |
| | | | 720 | <LOQ |
| | | | 1440 | <LOQ |
| | 1B | male | 30 | <LOQ |
| | | | 60 | 32 |
| | | | 235 | <LOQ |
| | | | 270 | 31 |
| | | | 300 | 26 |
| | | | 475 | <LOQ |
| | | | 540 | 28 |
| | | | 600 | 17 |
| | | | 720 | 5 |
| | | | 1440 | <LOQ |
| | 1C | male | 30 | 29 |
| | | | 60 | 34 |
| | | | 235 | 13 |
| | | | 270 | 20 |
| | | | 300 | 29 |
| | | | 475 | 18 |
| | | | 540 | 41 |
| | | | 600 | 35 |
| | | | 720 | 12 |
| | | | 1440 | <LOQ |

(continued)

| Dose (mg/kg) | Animal ID | Sex | Time Point (min) | Anatabine Concentration (ng/mL) |
|---|---|---|---|---|
| | 1D | male | 30 | 37 |
| | | | 60 | 24 |
| | | | 235 | 9 |
| | | | 270 | 26 |
| | | | 300 | 22 |
| | | | 475 | 19 |
| | | | 540 | 43 |
| | | | 600 | 34 |
| | | | 720 | 19 |
| | | | 1440 | <LOQ |
| | 2A | female | 30 | 34 |
| | | | 60 | 29 |
| | | | 235 | 4 |
| | | | 270 | 14 |
| | | | 300 | 18 |
| | | | 475 | 7 |
| | | | 540 | 24 |
| | | | 600 | 12 |
| | | | 720 | 18 |
| | | | 1440 | <LOQ |
| | 2B | female | 30 | 34 |
| | | | 60 | 33 |
| | | | 235 | 18 |
| | | | 270 | 11 |
| | | | 300 | 31 |
| | | | 475 | 7 |
| | | | 540 | 44 |
| | | | 600 | 18 |
| | | | 720 | 15 |
| | | | 1440 | <LOQ |

(continued)

| Dose (mg/kg) | Animal ID | Sex | Time Point (min) | Anatabine Concentration (ng/mL) |
|---|---|---|---|---|
| | 2C | female | 30 | 42 |
| | | | 60 | 47 |
| | | | 235 | 6 |
| | | | 270 | 17 |
| | | | 300 | 25 |
| | | | 475 | 6 |
| | | | 540 | 29 |
| | | | 600 | 20 |
| | | | 720 | 7 |
| | | | 1440 | <LOQ |
| | 2D | female | 30 | 38 |
| | | | 60 | 25 |
| | | | 235 | 13 |
| | | | 270 | 51 |
| | | | 300 | 44 |
| | | | 475 | 10 |
| | | | 540 | 29 |
| | | | 600 | 45 |
| | | | 720 | 15 |
| | | | 1440 | <LOQ |
| 2 | 3A | male | 30 | 298 |
| | | | 60 | 153 |
| | | | 235 | 84 |
| | | | 270 | 131 |
| | | | 300 | 312 |
| | | | 475 | 82 |
| | | | 540 | 223 |
| | | | 600 | 269 |
| | | | 720 | 133 |
| | | | 1440 | 12 |

(continued)

| Dose (mg/kg) | Animal ID | Sex | Time Point (min) | Anatabine Concentration (ng/mL) |
|---|---|---|---|---|
| | 3B | male | 30 | 288 |
| | | | 60 | 106 |
| | | | 235 | 46 |
| | | | 270 | 236 |
| | | | 300 | 232 |
| | | | 475 | 79 |
| | | | 540 | 214 |
| | | | 600 | 173 |
| | | | 720 | 401 |
| | | | 1440 | <LOQ |
| | 3C | male | 30 | 269 |
| | | | 60 | 272 |
| | | | 235 | 63 |
| | | | 270 | 364 |
| | | | 300 | 116 |
| | | | 475 | 97 |
| | | | 540 | 290 |
| | | | 600 | 130 |
| | | | 720 | 137 |
| | | | 1440 | 42 |
| | 3D | male | 30 | 116 |
| | | | 60 | 116 |
| | | | 235 | 105 |
| | | | 270 | 309 |
| | | | 300 | 202 |
| | | | 475 | 114 |
| | | | 540 | 173 |
| | | | 600 | 150 |
| | | | 720 | 71 |
| | | | 1440 | 36 |

(continued)

| Dose (mg/kg) | Animal ID | Sex | Time Point (min) | Anatabine Concentration (ng/mL) |
|---|---|---|---|---|
| | 4A | female | 30 | 245 |
| | | | 60 | 81 |
| | | | 235 | 75 |
| | | | 270 | 237 |
| | | | 300 | 216 |
| | | | 475 | 144 |
| | | | 540 | 231 |
| | | | 600 | 197 |
| | | | 720 | 186 |
| | | | 1440 | 42 |
| | 4B | female | 30 | 78 |
| | | | 60 | 95 |
| | | | 235 | 36 |
| | | | 270 | 324 |
| | | | 300 | 97 |
| | | | 475 | 219 |
| | | | 540 | 314 |
| | | | 600 | 207 |
| | | | 720 | 165 |
| | | | 1440 | 8 |
| | 4C | female | 30 | 218 |
| | | | 60 | 127 |
| | | | 235 | 23 |
| | | | 270 | 273 |
| | | | 300 | 191 |
| | | | 475 | 178 |
| | | | 540 | 369 |
| | | | 600 | 480 |
| | | | 720 | 244 |
| | | | 1440 | 36 |

(continued)

| Dose (mg/kg) | Animal ID | Sex | Time Point (min) | Anatabine Concentration (ng/mL) |
|---|---|---|---|---|
| | 4D | female | 30 | 98 |
| | | | 60 | 165 |
| | | | 235 | 72 |
| | | | 270 | 350 |
| | | | 300 | 414 |
| | | | 475 | 179 |
| | | | 540 | 474 |
| | | | 600 | 288 |
| | | | 720 | 217 |
| | | | 1440 | <LOQ |

**Table 51. Mean Concentrations and Descriptive Statistics of Anatabine in Plasma Samples at Each Time Point**

| Dose Group | Sex | Time Point (min) | Male or Female | | | | Combined Male and Female | | | |
|---|---|---|---|---|---|---|---|---|---|---|
| | | | N= | Avg. Conc. (ng/ml) | ±SD | ±SEM | Male and Female Avg. Conc. (ng/ml) | n= | ±SD | ±SEM |
| 0.2 | male | 30 | 3 | 33 | 4.2 | 2.4 | 36 | 7 | 4.3 | 1.6 |
| | | 60 | 4 | 30 | 4.6 | 2.3 | 31 | 8 | 7.5 | 2.6 |
| | | 235 | 3 | 11 | 1.6 | 0.9 | 10 | 7 | 4.7 | 1.8 |
| | | 270 | 4 | 26 | 6.9 | 3.4 | 26 | 8 | 13.3 | 4.7 |
| | | 300 | 4 | 26 | 3.0 | 1.5 | 27 | 8 | 7.9 | 2.8 |
| | | 475 | 3 | 18 | 5.3 | 3.0 | 11 | 7 | 5.4 | 2.0 |
| | | 540 | 4 | 38 | 17.9 | 8.9 | 30 | 8 | 13.1 | 4.6 |
| | | 600 | 3 | 29 | 10.4 | 6.0 | 26 | 7 | 12.3 | 4.3 |
| | | 720 | 3 | 12 | 6.5 | 3.8 | 13 | 7 | 5.2 | 1.8 |
| | | 1440 | 0 | n/a | n/a | n/a | n/a | 0 | n/a | n/a |
| 0.2 | female | 30 | 4 | 37 | 3.9 | 2.0 | | | | |
| | | 60 | 4 | 35 | 9.7 | 4.9 | | | | |
| | | 235 | 4 | 12 | 6.4 | 3.2 | | | | |
| | | 270 | 4 | 26 | 18.7 | 9.4 | | | | |
| | | 300 | 4 | 34 | 10.9 | 5.5 | | | | |
| | | 475 | 4 | 8 | 1.7 | 0.9 | | | | |
| | | 540 | 4 | 34 | 8.7 | 4.4 | | | | |
| | | 600 | 4 | 28 | 14.7 | 7.3 | | | | |
| | | 720 | 4 | 12 | 4.8 | 2.4 | | | | |
| | | 1440 | 0 | n/a | n/a | n/a | | | | |
| 2 | male | 30 | 4 | 243 | 85.6 | 42.8 | 201 | 8 | 90.1 | 31.9 |

(continued)

| Dose Group | Sex | Time Point (min) | Male or Female | | | | Combined Male and Female | | | |
|---|---|---|---|---|---|---|---|---|---|---|
| | | | N= | Avg. Conc. (ng/ml) | ±SD | ±SEM | Male and Female Avg. Conc. (ng/ml) | n= | ±SD | ±SEM |
| | | 60 | 4 | 165 | 76.1 | 38.0 | 139 | 8 | 60.4 | 21.3 |
| | | 235 | 4 | 72 | 25.7 | 12.9 | 63 | 8 | 26.9 | 9.5 |
| | | 270 | 4 | 303 | 100.9 | 50.5 | 278 | 8 | 76.4 | 27.0 |
| | | 300 | 4 | 183 | 80.7 | 40.3 | 222 | 8 | 102.3 | 36.2 |
| | | 475 | 4 | 97 | 16.2 | 8.1 | 136 | 8 | 51.7 | 18.3 |
| | | 540 | 4 | 226 | 48.3 | 24.1 | 286 | 8 | 98.7 | 34.9 |
| | | 600 | 4 | 151 | 61.7 | 30.8 | 237 | 8 | 112.2 | 39.7 |
| | | 720 | 4 | 203 | 146.6 | 73.3 | 194 | 8 | 99.1 | 35.0 |
| | | 1440 | 3 | 39 | 15.9 | 9.2 | 29 | 6 | 15.5 | 6.3 |
| 2 | female | 30 | 4 | 160 | 83.8 | 41.9 | | | | |
| | | 60 | 4 | 129 | 37.3 | 18.6 | | | | |
| | | 235 | 4 | 44 | 25.8 | 12.9 | | | | |
| | | 270 | 4 | 316 | 50.8 | 25.4 | | | | |
| | | 300 | 4 | 234 | 133.3 | 66.6 | | | | |
| | | 475 | 4 | 192 | 30.7 | 15.3 | | | | |
| | | 540 | 4 | 386 | 102.1 | 51.1 | | | | |
| | | 600 | 4 | 325 | 131.0 | 65.5 | | | | |
| | | 720 | 4 | 208 | 34.7 | 17.3 | | | | |
| | | 1440 | 3 | 22 | 18.6 | 10.7 | | | | |

**EXAMPLE 7**

**Effect of S-(-)-anatabine on TNFa-induced NFKB activity *in vitro***

The effect of S-(-)-anatabine on TNFa-induced NFκB activity *in vitro* was determined as described in Example 1. NFkB activity was stimulated with 20ng/ml of TNF$\alpha$, then varying doses of a racemic mixture of anatabine or S(-)-anatabine were applied to the challenged cells. The data were plotted as a percentage of the TNFa-induced NFkB activity and are shown in FIG. 24. In this assay the $IC_{50}$ for the racemic mixture of anatabine is approximately 600$\mu$g/ml, whereas the $IC_{50}$ for the (S)-enantiomer is approximately 330$\mu$g/ml.

**EXAMPLE 8**

**Step 1. Preparation of Formula I**

3-aminomethylpyridine was added to neat benzophenoneimine (1 eq). The reaction was allowed to proceed for 6 hours at 50 °C providing Formula I as the product.

**EXAMPLE 9**

**Steps 2i to 2iii: Conversion of Formula I to Anatabine using Potassium Tert Butoxide ($K^tOBu$)**

Step 2i: To Formula I of Example 1 was added $K^tOBu$ (1.5 eq) in THF at -78 °C to -45 °C and the reaction was

incubated for 30 min. Cis-1,4-dichloro-2-butene in 3 vol of THF at -78°C was added, and the temperature was allowed to warm to -45°C. The reaction was allowed to proceed at -45°C for 1-2 hours, providing Formula II. Optionally, the starting material may be added to the $K^tOBu$.

Step 2ii: 10% aqueous hydrochloric acid was added to the solution containing Formula II for 10-20 minutes to provide Formula III.

Step 2iii: The Formula III solution was basified by adding $K_2CO_3$ then treated with a 40% aqueous KOH solution to provide anatabine.

Results from different batches are show in Table 52.

**Table 52**

| Batch No. and amount | Reaction Conditions | Yield | Comments |
|---|---|---|---|
| A (2g) | i) $K^tOBu$ (1.1eq), THF,-78°C to -45°C for 30 min<br>ii)cis-1,4-dichloro-2-butene -45°C for 1-2h<br>iii) 10% aq HCl, 10-20 min, ether wash.<br>iv) $K_2CO_3$, 40% aqueous KOH | 0.7g (crude) | SM was added to $K^tOBu$.<br>64.52 % of Step 2i product<br>Anatabine 65.12% pure by HPLC |
| B (2g) | i) $K^tOBu$ (1.5eq), THF,-78°C to -45°C for 30 min<br>ii)cis-1,4-dichloro-2-butene -45°C for 1-2h<br>iii) 10% aq HCl, 10-20 min, ether wash.<br>iv) $K_2CO_3$, 40% aqueous KOH | 0.68g (crude) | Note that 1.5 eq of $K^tOBu$ was used.<br>70.32 % of Step 2i product<br>Anatabine 67.06% pure by HPLC |
| C (25g) | i) $K^tOBu$ (1.1eq), THF,-78°C to -45°C for 30 min<br>ii)cis-1,4-dichloro-2-butene -45°C for 1-2h<br>iii) 10% aq HCl, 10-20 min, ether wash.<br>iv) $K_2CO_3$, 40% aqueous KOH | 8.5g (crude) | 50 % of Step 2i product<br>Anatabine 57.7% pure by HPLC |

**EXAMPLE 10**

**Steps 2i to 2iii alternate route: Conversion of Formula I to Anatabine using LDA**

Formula I was prepared according to Example 1. LDA was added to Formula I at -10 to 0 °C. Cis-1,4-dichloro-2-butene was added at -78 °C to -45 °C. Steps 2ii and 2iii were performed according to Example 9.

**Table 53**

| Batch No. and amount | Reaction Conditions | Yield | Comments |
|---|---|---|---|
| D (25g) | i) LDA (1.2eq), THF, -30 °C to 0 °C for 30 min<br>ii)cis-1,4-dichloro-2-butene -78 °C to -45 °C for 1-2h<br>iii) 10% aq HCl, 10-20 min, ether wash.<br>iv) $K_2CO_3$, 40% aqueous KOH | 6.9g (crude) | 71 % conversion in IPC.<br>23.14% SM was seen in Step 2i.<br>72.64% purity by HPLC. |
| E (5g) | i) LDA (1.5eq) -30 °C to 0°C for 30 min<br>ii)cis-1,4-dichloro-2-butene -78 °C to -45°C for 1-2h<br>iii) 10% aq HCl, 10-20 min, ether wash.<br>iv) $K_2CO_3$, 40% aqueous KOH | - | 80 % conversion in IPC<br>Anatabine 70.6% pure by HPLC |

(continued)

| Batch No. and amount | Reaction Conditions | Yield | Comments |
|---|---|---|---|
| F (25g) | i) LDA (2.0eq), -30 °C to 0 °C for 30 min<br>ii) cis-1,4-dichloro-2-butene -78 °C to -45°C for 1-2h<br>iii) 10% aq HCl, 10-20 min, ether wash.<br>iv) $K_2CO_3$, 40% aqueous KOH | - | 67 % conversion in IPC<br>Anatabine 76.1% pure by HPLC |
| G (25g) | i) LDA (1.5eq), -30 °C to 0 °C for 30 min<br>ii) cis-1,4-dichloro-2-butene -78 °C to -45°C for 1-2h<br>iii) 10% aq HCl, 10-20 min, ether wash.<br>iv) $K_2CO_3$, 40% aqueous KOH | | Anatabine 73.7% pure by HPLC |

**EXAMPLE 10A**

**Recovery/Purification of Anatabine Using MTBE and Distillation**

The product of Example 9, Step 2iii was extracted with methyl t-butyl ether (MTBE), followed by distillation of the solvent and product distillation using a glass distillation assembly. Optionally, for scale-up, wiped film or thin film evaporation may be used. Yield of step 2 was 40%; overall yield was 26%.

**COMPARATIVE EXAMPLE 10A**

**Recovery/Purification of Anatabine Using Chloroform Column Chromatography**

The product of Step 2iii was treated with chloroform extraction as described in Deo et al. Chloroform extraction resulted in an anatabine yield of 10%.

EXAMPLE 11

**Stability analysis of Formula I**

Several batches of Formula I produced by reacting benzophenoneimine with aminomethylpyridine in the absence of benzene were stored refrigerated and also at room temperature. Testing by HPLC shows that purity was constant when refrigerated and only deviated 1-2% at room temperature.

**Table 54**

| Days | Purity (%) (stored at 2-8°C) | Purity (%) (stored at 25-28°C) |
|---|---|---|
| 1 | 88.61 | 88.93 |
| 2 | 88.83 | 85.49 |
| 3 | 88.30 | 88.02 |
| 6 | 88.83 | 86.62 |
| 12 | 88.75 | 87.47 |
| 18 | 88.46 | 87.25 |

**EXAMPLE 12**

**Stability Analysis of Anatabine Base With and Without BHT as a Preservative**

Anatabine base stability was analyzed with and without BHT (3,5-di-tert-butyl-4-hydroxytoluene). The initial purity of BHT-free anatabine base was 94.95%. The initial purity of anatabine base with BHT was 94.87%. Anatabine

stability, with and without BHT is reported in Table 55.

**Table 55**

| Day | Storage Temperature | Purity (%) (with BHT) | Purity (%) (without BHT) |
|---|---|---|---|
| 1 | 25-28 °C | 93.27 | 89.63 |
| | 2-8 °C | 93.26 | 94.44 |
| | -20 °C | 94.26 | 93.90 |
| 2 | 25-28 °C | 92.73 | 87.59 |
| | 2-8 °C | 94.83 | 94.05 |
| | -20 °C | 94.73 | 94.62 |
| 3 | 25-28 °C | 87.44 | 80.74 |
| | 2-8 °C | 94.26 | 91.65 |
| | -20 °C | 94.62 | 93.87 |
| 12 | 25-28 °C | 85.10 | 79.59 |
| | 2-8 °C | 94.18 | 90.12 |
| | -20 °C | 94.74 | 94.01 |

**EXAMPLE 13**

**Preparation of Anatabine Salts**

To a solution of anatabine (4.0 g, 24.9 mmol) in acetone (20 ml), L-(+)-tartaric acid (3.37, 22.4 mmol) was added at room temperature. The reaction mass was warmed to 50 °C for 16 hours. The supernatant was decanted and the solid was triturated with diethyl ether (20 ml), filtered and dried under vacuum.

**Table 56**

| Solvent Used | Anatabine Tartrate (% Purity by HPLC) |
|---|---|
| Isopropyl alcohol | > 94% |
| Acetone | > 99% |

To a solution of anatabine (0.47g, 2.9mmol) in acetone (3 ml), citric acid (0.5g, 2.6mmol) was added at room temperature under a nitrogen atmosphere. The reaction mass was warmed to 50 °C for 16 hours. The supernatant was decanted and the solid was triturated with diethyl ether (20 ml), filtered and dried under vacuum.

**Table 57**

| Solvent Used | Anatabine Citrate (% Purity by HPLC) |
|---|---|
| Acetone | > 97% |

While particular embodiments have been described and illustrated, it should be understood that the invention is not limited thereto since modifications may be made by persons skilled in the art. The present application contemplates any and all modifications that fall within the spirit and scope of the underlying invention disclosed and claimed herein.
Further aspects of the present disclosure are set forth in the following numbered paragraphs:

1. Use of an isolated form of anatabine or a pharmaceutically acceptable salt thereof in the manufacture of a medicament for reducing a symptom in an individual of a disorder comprising an NFKB-mediated inflammatory component.

2. Use of an isolated form of anatabine or a pharmaceutically acceptable salt thereof in the manufacture of a

medicament for reducing a risk in an individual of developing a disorder comprising an NFKB-mediated inflammatory component.

3. Use according to paragraph 1 or paragraph 2 wherein the NFKB-mediated inflammatory component is chronic inflammation.

4. Use according to paragraph 1 or paragraph 2 wherein the disorder is selected from the group consisting of thyroiditis, cancer, arthritis, Alzheimer's disease, and multiple sclerosis.

5. Use according to paragraph 1 or paragraph 2 wherein the medicament comprises an extended release formulation.

6. Use of an isolated form of anatabine or a pharmaceutically acceptable salt thereof in the manufacture of a medicament for treating alopecia areata or other disorders associated with hair loss.

7. Use of an isolated form of anatabine or a pharmaceutically acceptable salt thereof in the manufacture of a medicament for reducing blood serum levels of C-reactive protein (CRP) in an individual.

8. Use according to paragraph 7 wherein the individual is at risk for a disorder comprising an NFKB-mediated inflammatory component.

9. Use according to paragraph 7 wherein the individual has a disorder comprising an NFKB-mediated inflammatory component.

10. Use according to paragraph 7 wherein the NFKB-mediated inflammatory component is chronic inflammation.

11. Use according to paragraph 7 wherein the individual is at risk for a disorder selected from the group consisting of cardiovascular disease, cancer, and diabetes.

12. Use according to paragraph 7 wherein the individual has a disorder selected from the group consisting of cardiovascular disease, cancer, and diabetes.

13. Use according to any preceding paragraph, wherein anatabine is present as an isolated form of S-(-)-anatabine or a pharmaceutically acceptable salt thereof.

14. Use according to any of paragraphs 1 to 12, wherein anatabine is present as an isolated form of R-(+)-anatabine or a pharmaceutically acceptable salt thereof.

15. A bottled water product comprising about 100 ml to about 2,000 ml purified water and from about 0.00001 to about 0.0001 wt% of a water-soluble salt of anatabine.

16. The bottled water product of paragraph 15 wherein anatabine is present as a water-soluble salt of S-(-)-anatabine.

17. The bottled water product of paragraph 15 wherein anatabine is present as a water-soluble salt of R-(+)-anatabine.

18. A method of preparing benzylhydrylidene-pyridin-3-yl-methyl-amine, comprising reacting 3-aminomethyl pyridine with benzophenoneimine to obtain a compound of Formula I:

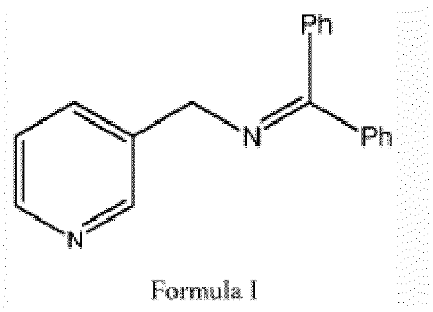

Formula I

19. The method of paragraph 18 wherein the benzophenoneimine comprises one or more substituents on either or both aromatic rings, wherein the substituents are selected from the group consisting of: nitrogen, a halogen, and an alkyl group.

20. The method of paragraph 18 further comprising the step of reacting the compound of Formula I with a non-nucleophilic base and a dielectrophile to obtain a compound of Formula II:

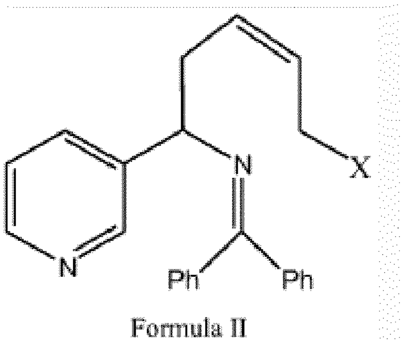

Formula II

wherein X is a halogen.

21. The method of paragraph 19 wherein the non-nucleophilic base is lithium diisopropylamide (LDA).

22. The method of paragraph 19 wherein the non-nucleophilic base is selected from the group consisting of sodium hydride, sodamide, alkyllithum reagents, and potassium tert-butoxide.

23. The method of paragraph 19 wherein the dielectrophile is cis-I,4-dichloro-2-butene.

24. A method of synthesizing anatabine comprising the steps of:

(a) reacting 3-aminomethyl pyridine with benzophenoneimine to obtain a compound of Formula I:

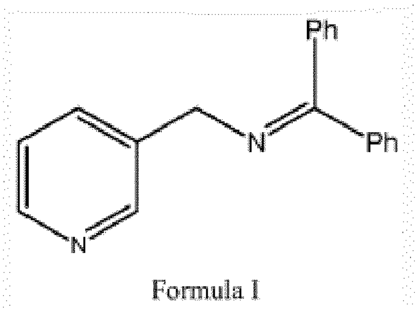

Formula I

(b) reacting the compound of Formula I with a non-nucleophilic base and a dielectrophile to obtain a compound of Formula II:

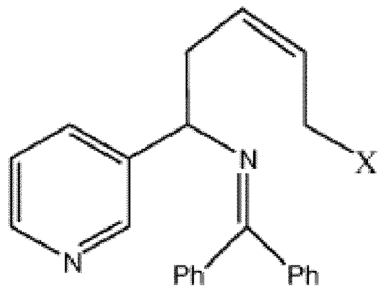

Formula II

wherein X is a halogen,

(c) treating the compound of Formula II with an acid to obtain a compound of Formula III:

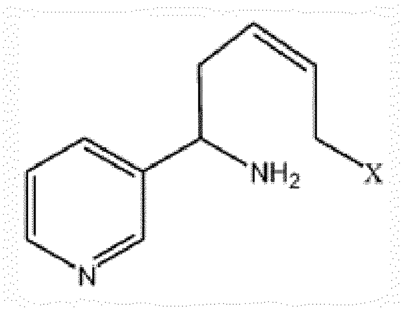

Formula III

wherein X is a halogen; and

(d) treating the compound of Formula III with at least one base to obtain anatabine.

25. A method of recovering anatabine from a reaction product, comprising contacting the reaction product with methyl tert-butyl ether (MTBE) under distillation.

26. A method of synthesizing an alkaloid comprising:

(a) reacting 3-aminomethyl pyridine with benzophenoneimine to obtain a compound of Formula I:

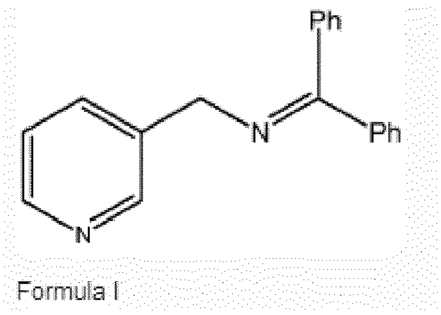

Formula I

(b) reacting the compound of Formula I with a non-nucleophilic base dielectrophile to obtain a compound of Formula IIa:

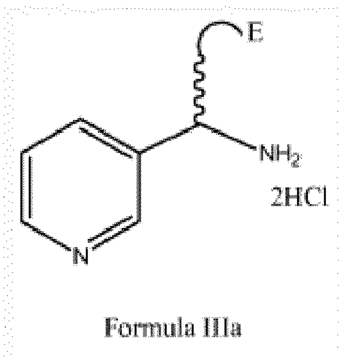

Formula IIIa

(c) treating the compound of Formula IIa with an acid to obtain a compound of Formula IIIa:

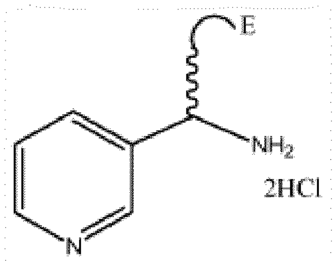

Formula IIIa

and

(d) treating the compound of Formula Ilia with at least one base to obtain the alkaloid.

27. The method of paragraph 26 wherein the alkaloid is selected from the group consisting of anabasine, nornicotine, N-methylanabasine, and anabaseine.

28. An acceptable pharmaceutical, dietary supplement or food grade salt of anatabine.

29. The salt of paragraph 28 which is anatabine citrate.

30. A composition comprising a salt of paragraph 28 and an acceptable pharmaceutical, dietary supplement or food grade carrier, excipient, or diluent.

31. A composition comprising a salt of paragraph 29 and a pharmaceutical, dietary supplement or food grade carrier, excipient, or diluent.

32. A method of reducing a symptom in an individual of a disorder comprising an NFKB-mediated inflammatory component, comprising administering to the individual a pharmaceutical composition comprising a therapeutically effective dose of an isolated form of anatabine or a pharmaceutically acceptable salt thereof.

33. A method of reducing a risk in an individual of developing a disorder comprising an NFKB-mediated inflammatory component, comprising administering to the individual a composition comprising a therapeutically effective dose of an isolated form of anatabine or a pharmaceutically acceptable salt thereof.

34. The method of paragraph 32 or paragraph 33 wherein the NFKB-mediated inflammatory component is chronic inflammation.

35. The method of paragraph 32 or paragraph 33 wherein the composition comprises a pharmaceutically acceptable salt of anatabine.

36. The method of paragraph 32 or paragraph 33 wherein the disorder is selected from the group consisting of thyroiditis, cancer, arthritis, Alzheimer's disease, and multiple sclerosis.

37. The method of paragraph 32 or paragraph 33 wherein the dose is in an extended release formulation.

38. The method of paragraph 32 or paragraph 33 wherein the dose is from about 0.1 to about 1.5 mg/kg body weight.

39. A method of treating alopecia areata or other disorders associated with hair loss comprising administering to the individual a pharmaceutical composition comprising a therapeutically effective dose of an isolated form of anatabine or a pharmaceutically acceptable salt thereof.

40. A method of reducing blood serum levels of C-reactive protein (CRP) in an individual, comprising administering to the individual a pharmaceutical composition comprising a therapeutically effective dose of an isolated form of anatabine or a pharmaceutically acceptable salt thereof.

41. The method of paragraph 40 wherein the individual is at risk for a disorder comprising an NFKB-mediated inflammatory component.

42. The method of paragraph 40 wherein the individual has a disorder comprising an NFKB-mediated inflammatory component.

43. The method of paragraph 40 wherein the NFKB-mediated inflammatory component is chronic inflammation.

44. The method of paragraph 40 wherein the composition comprises a pharmaceutically acceptable salt of anatabine.

45. The method of paragraph 40 wherein the individual is at risk for a disorder selected from the group consisting of cardiovascular disease, cancer, and diabetes.

46. The method of paragraph 40 wherein the individual has a disorder selected from the group consisting of cardiovascular disease, cancer, and diabetes.

47. The method of paragraph 40 wherein the dose is in an extended release formulation.

48. The method of paragraph 40 wherein the dose is from about 0.1 to about 1.5 mg/kg body weight.

49. A method of reducing or reversing cellular senescence in an individual comprising administering to the individual a pharmaceutical composition comprising a therapeutically effective dose of an isolated form of anatabine or a pharmaceutically acceptable salt thereof.

50. The method according to any of paragraphs 32 to 49, wherein anatabine is present as an isolated form of S-(-)-anatabine or a pharmaceutically acceptable salt thereof.

51. The method according to any of paragraphs 32 to 49, wherein anatabine is present as an isolated form of R-(+)-anatabine or a pharmaceutically acceptable salt thereof.

52. Use of an isolated form of anatabine or a pharmaceutically acceptable salt thereof in the manufacture of a medicament for reducing or reversing cellular senescence in an in dividual.

53. Use according to paragraph 52, wherein anatabine is present as an isolated form of S-(-)-anatabine or a pharmaceutically acceptable salt thereof.

54. Use according to paragraph 52, wherein anatabine is present as an isolated form of R-(+)-anatabine or a pharmaceutically acceptable salt thereof.

## Claims

**1.** Anatabine glutarate.

2. The anatabine glutarate according to claim 1, wherein the anatabine glutarate is S-(-)-anatabine glutarate or R-(+)-anatabine glutarate.

3. The anatabine glutarate according to claim 1 or claim 2, wherein the anatabine glutarate is a pharmaceutically acceptable grade or food grade.

4. The anatabine glutarate according to any of the preceding claims, wherein the anatabine glutarate is in purified form; preferably, wherein the anatabine glutarate has a purity of at least 95%.

5. A composition comprising the anatabine glutarate of any of the preceding claims and an acceptable pharmaceutical or food grade carrier or excipient; preferably, wherein the acceptable pharmaceutical or food grade carrier or excipient is a non-toxic, inert solid, semi-solid or liquid filler, diluent, encapsulating material or formulation auxiliary; preferably, wherein the nontoxic, inert solid, semi-solid or liquid filler, diluent, encapsulating material or formulation auxiliary is selected from the group consisting of: sugars, starches, cellulose and its derivatives, powdered tragacanth, malt, gelatin, talc, cocoa butter and suppository waxes, oils, glycols, esters, agar, buffering agents, alginic acid; pyrogen-free water; isotonic saline; Ringer's solution; ethyl alcohol, phosphate buffer solutions, compatible lubricants, colouring agents, releasing agents, coating agents, sweetening agents, flavouring agents, perfuming agents, preservatives and antioxidants.

6. An elixir, a beverage, a chew, a tablet, a lozenge or a gum comprising the anatabine glutarate according to any of claims 1 to 5.

7. A method for preparing a pharmaceutical composition comprising: (i) combining the anatabine glutarate according to any of claims 1 to 5 with excipients and a binder; and (ii) granulating the combined preparation from step (i) to form the pharmaceutical composition.

8. The method according to claim 10, wherein the granulation is dry-blended with any remaining ingredients and compressed into a solid form.

9. Anatabine glutarate for use as a medicament.

10. Anatabine glutarate according to claim 9 use as a medicament to reduce a symptom in an individual of a disorder comprising an NFKB-mediated inflammatory component or reducing a risk in an individual of developing a disorder comprising an NFKB-mediated inflammatory component.

11. Anatabine glutarate according to claim 10, wherein the NFKB-mediated inflammatory component is chronic inflammation; or wherein the disorder is selected from the group consisting of thyroiditis, cancer, Alzheimer's disease, and multiple sclerosis.

12. Anatabine glutarate according to claim 12, for use as a medicament to treat a disorder in an individual selected from the group consisting of an immune or autoimmune disorder, arthritis and myopathy; or for use as a medicament to treat a disorder in an individual selected from the group consisting of diabetes and a gastrointestinal inflammatory disorder; or for use as a medicament to treat a disorder in an individual selected from the group consisting of a vascular inflammatory disease, associated vascular pathologies, atherosclerosis, angiopathy, inflammation- induced atherosclerotic or thromboembolic macroangiopathy, coronary artery disease, cerebrovascular disease, peripheral vascular disease, cardiovascular circulatory disease such as ischemia/reperfusion, peripheral vascular disease, restenosis following angioplasty, inflammatory aortic aneurysm, vasculitis, stroke, spinal cord injury, congestive heart failure, hemorrhagic shock, ischemic heart disease/reperfusion injury, vasospasm following subarachnoid hemorrhage, vasospasm following cerebrovascular accident, pleuritis, pericarditis, inflammation- induced myocarditis, or a cardiovascular complication of diabetes; or for use as a medicament to treat a disorder in an individual selected from the group consisting of brain swelling, a neurodegenerative disease, inflammation related to a kidney disease, nephritis, glomerulonephritis, dialysis, peritoneal dialysis, pericarditis, chronic prostatitis, vasculitis, gout, or pancreatitis.

13. Anatabine glutarate according to any of claims 10 to 12, wherein the individual is a mammal.

14. Anatabine glutarate according to any of claims 10 to 13, wherein the dose to be administered to the individual is from about 1 $\mu$g/kg to about 7 mg/kg body weight of the individual.

**15.** Anatabine glutarate according to any of claims 9 to 14, wherein the anatabine glutarate is in the form of a medicament comprising an extended release formulation.

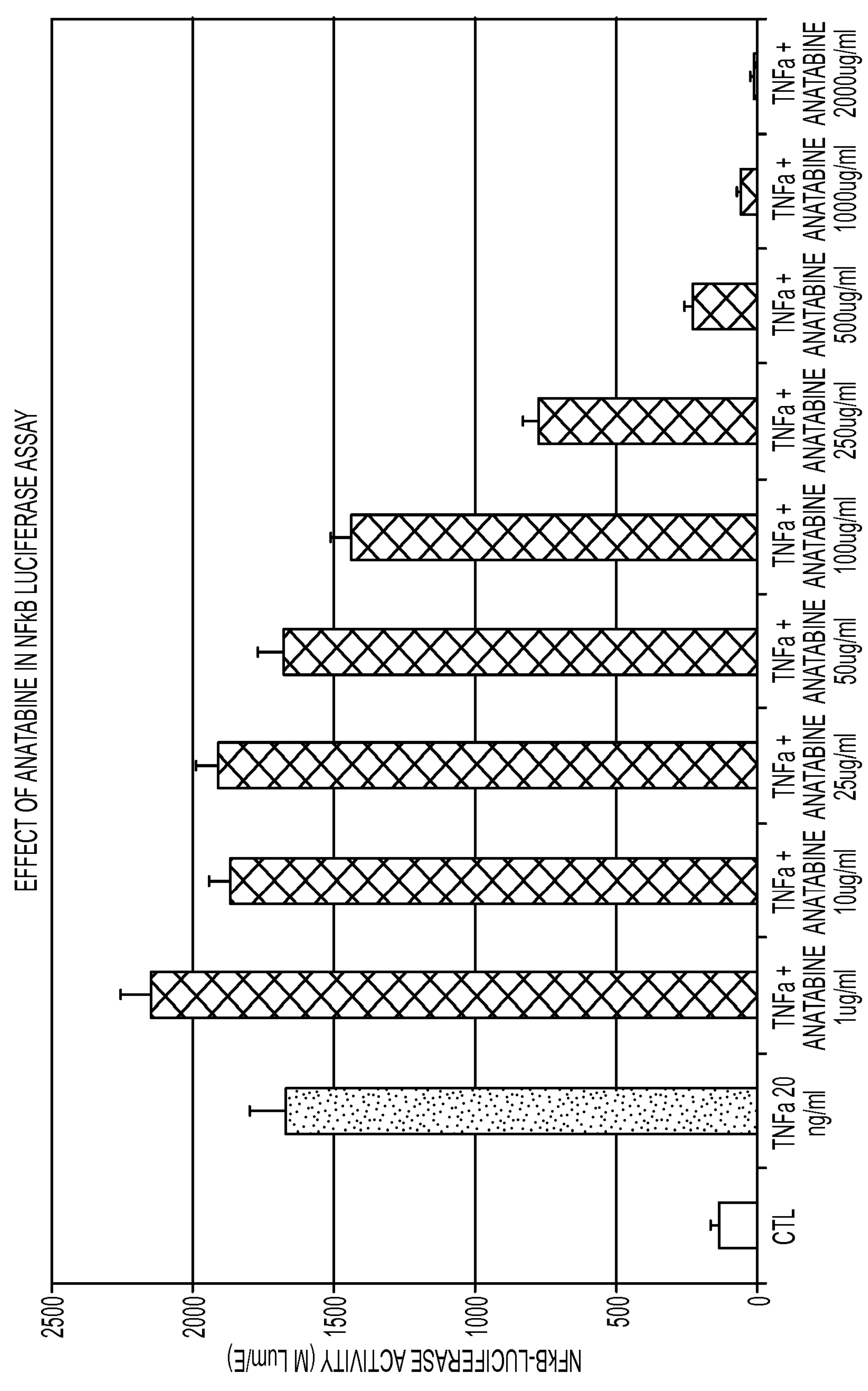
EFFECT OF ANATABINE IN NFkB LUCIFERASE ASSAY
NFkB-LUCIFERASE ACTIVITY (M Lum/E)
0
500
1000
1500
2000
2500
CTL
TNFa 20 ng/ml
TNFa + ANATABINE 1ug/ml
TNFa + ANATABINE 10ug/ml
TNFa + ANATABINE 25ug/ml
TNFa + ANATABINE 50ug/ml
TNFa + ANATABINE 100ug/ml
TNFa + ANATABINE 250ug/ml
TNFa + ANATABINE 500ug/ml
TNFa + ANATABINE 1000ug/ml
TNFa + ANATABINE 2000ug/ml

FIG. 1

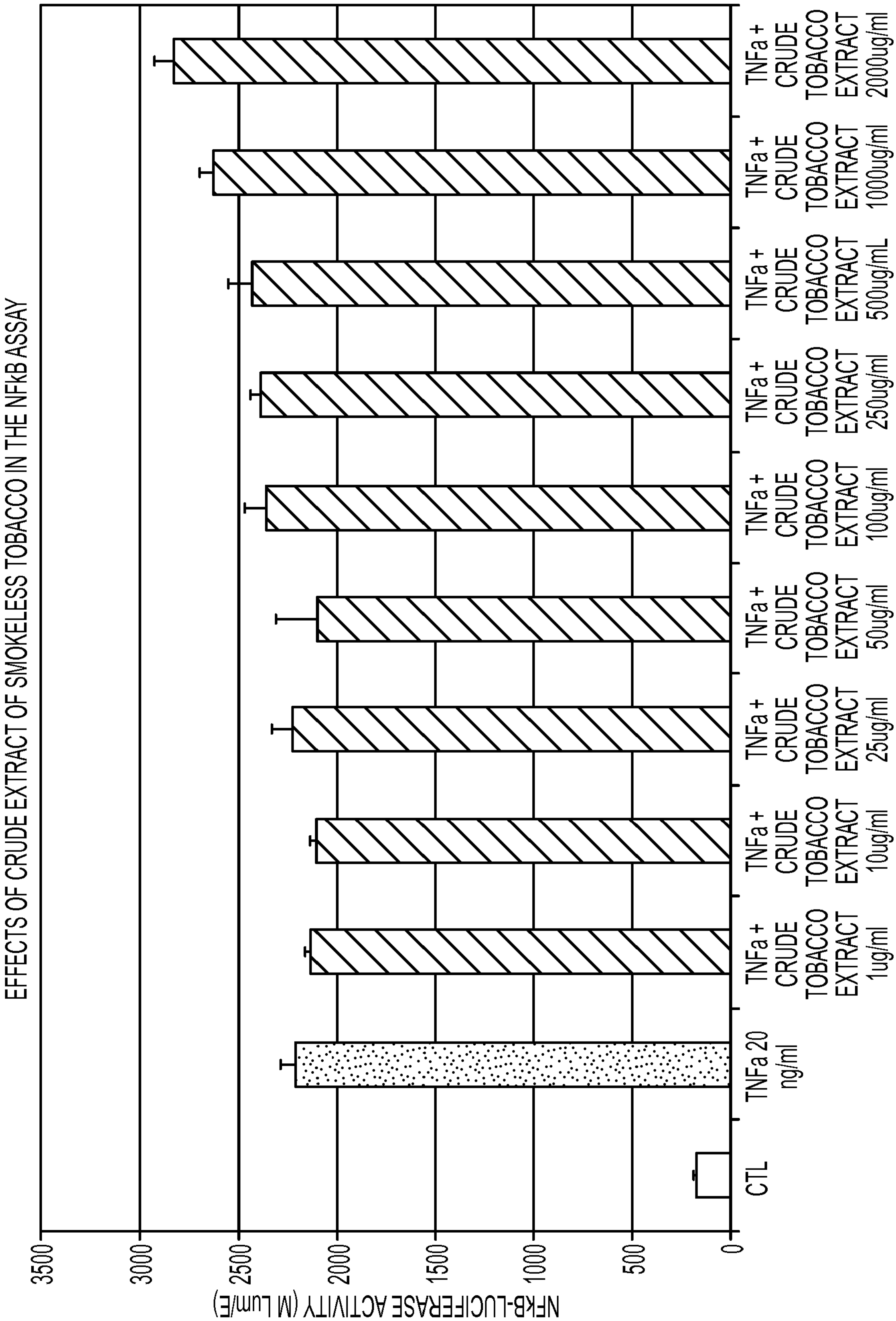
EFFECTS OF CRUDE EXTRACT OF SMOKELESS TOBACCO IN THE NFkB ASSAY
NFkB-LUCIFERASE ACTIVITY (M Lum/E)
0
500
1000
1500
2000
2500
3000
3500
CTL
TNFa 20 ng/ml
TNFa + CRUDE TOBACCO EXTRACT 1ug/ml
TNFa + CRUDE TOBACCO EXTRACT 10ug/ml
TNFa + CRUDE TOBACCO EXTRACT 25ug/ml
TNFa + CRUDE TOBACCO EXTRACT 50ug/ml
TNFa + CRUDE TOBACCO EXTRACT 100ug/ml
TNFa + CRUDE TOBACCO EXTRACT 250ug/ml
TNFa + CRUDE TOBACCO EXTRACT 500ug/mL
TNFa + CRUDE TOBACCO EXTRACT 1000ug/ml
TNFa + CRUDE TOBACCO EXTRACT 2000ug/ml

FIG. 2

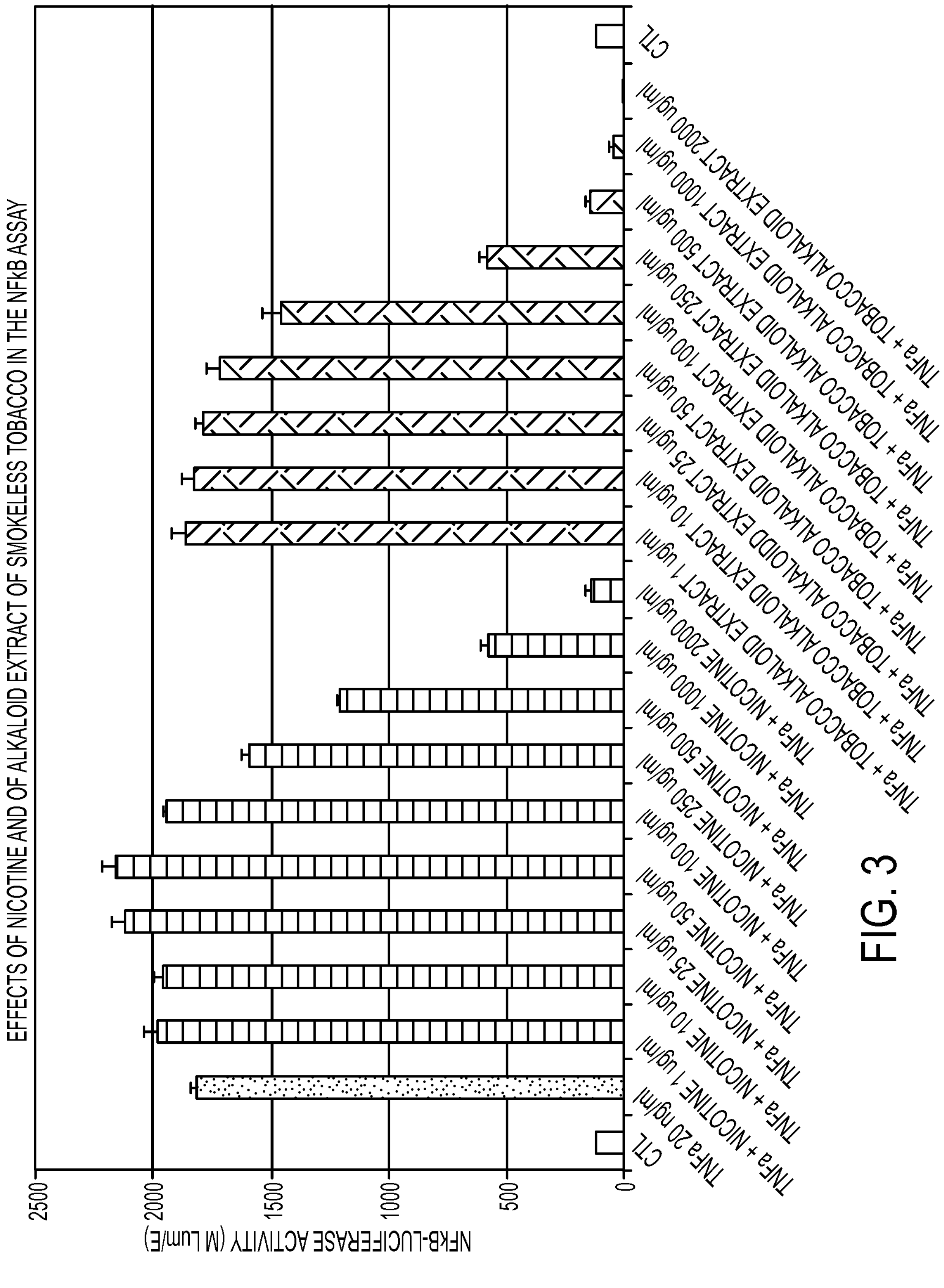
EFFECTS OF NICOTINE AND OF ALKALOID EXTRACT OF SMOKELESS TOBACCO IN THE NFkB ASSAY
NFKB-LUCIFERASE ACTIVITY (M Lum/E)
0
500
1000
1500
2000
2500
CTL
TNFa 20 ng/ml
TNFa + NICOTINE 1 ug/ml
TNFa + NICOTINE 10 ug/ml
TNFa + NICOTINE 25 ug/ml
TNFa + NICOTINE 50 ug/ml
TNFa + NICOTINE 100 ug/ml
TNFa + NICOTINE 250 ug/ml
TNFa + NICOTINE 500 ug/ml
TNFa + NICOTINE 1000 ug/ml
TNFa + NICOTINE 2000 ug/ml
TNFa + TOBACCO ALKALOID EXTRACT 1 ug/ml
TNFa + TOBACCO ALKALOID EXTRACT 10 ug/ml
TNFa + TOBACCO ALKALOID EXTRACT 25 ug/ml
TNFa + TOBACCO ALKALOID EXTRACT 50 ug/ml
TNFa + TOBACCO ALKALOID EXTRACT 100 ug/ml
TNFa + TOBACCO ALKALOID EXTRACT 250 ug/ml
TNFa + TOBACCO ALKALOID EXTRACT 500 ug/ml
TNFa + TOBACCO ALKALOID EXTRACT 1000 ug/ml
TNFa + TOBACCO ALKALOID EXTRACT 2000 ug/ml
CTL

FIG. 3

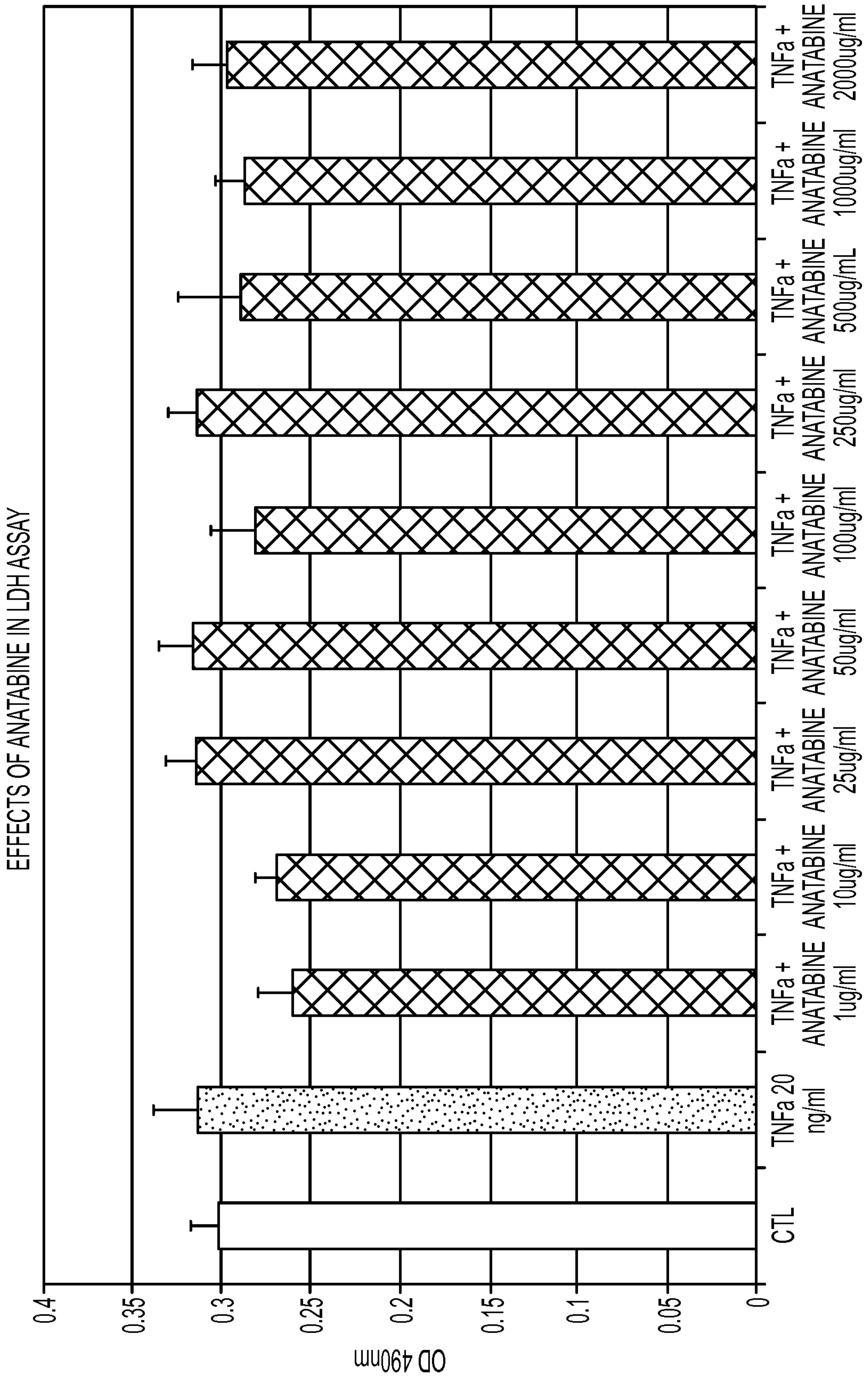
EFFECTS OF ANATABINE IN LDH ASSAY
OD 490nm
0
0.05
0.1
0.15
0.2
0.25
0.3
0.35
0.4
CTL
TNFa 20 ng/ml
TNFa + ANATABINE 1ug/ml
TNFa + ANATABINE 10ug/ml
TNFa + ANATABINE 25ug/ml
TNFa + ANATABINE 50ug/ml
TNFa + ANATABINE 100ug/ml
TNFa + ANATABINE 250ug/ml
TNFa + ANATABINE 500ug/mL
TNFa + ANATABINE 1000ug/ml
TNFa + ANATABINE 2000ug/ml

FIG. 4

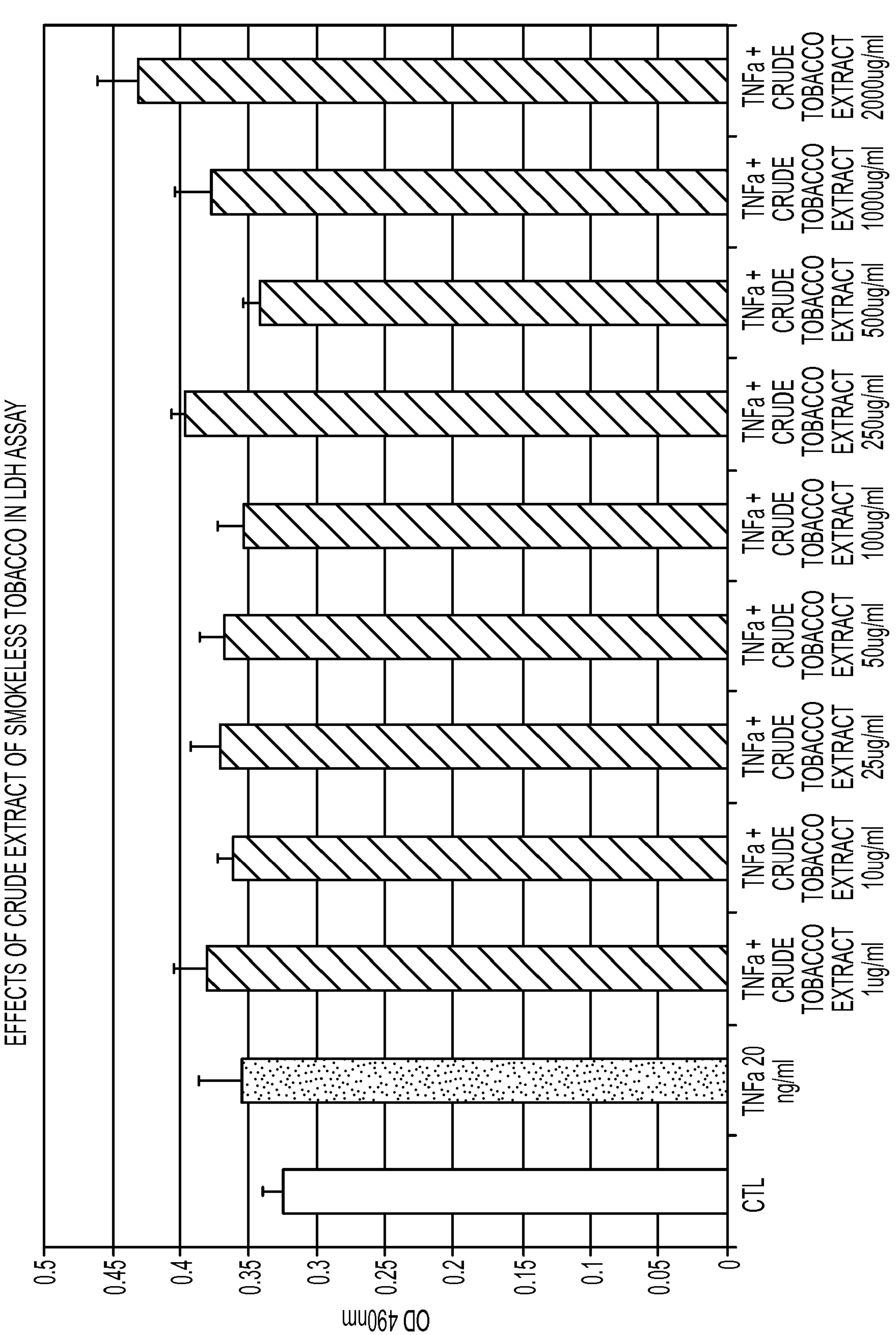
EFFECTS OF CRUDE EXTRACT OF SMOKELESS TOBACCO IN LDH ASSAY
OD 490nm
0
0.05
0.1
0.15
0.2
0.25
0.3
0.35
0.4
0.45
0.5
CTL
TNFa 20 ng/ml
TNFa + CRUDE TOBACCO EXTRACT 1ug/ml
TNFa + CRUDE TOBACCO EXTRACT 10ug/ml
TNFa + CRUDE TOBACCO EXTRACT 25ug/ml
TNFa + CRUDE TOBACCO EXTRACT 50ug/ml
TNFa + CRUDE TOBACCO EXTRACT 100ug/ml
TNFa + CRUDE TOBACCO EXTRACT 250ug/ml
TNFa + CRUDE TOBACCO EXTRACT 500ug/ml
TNFa + CRUDE TOBACCO EXTRACT 1000ug/ml
TNFa + CRUDE TOBACCO EXTRACT 2000ug/ml

FIG. 5

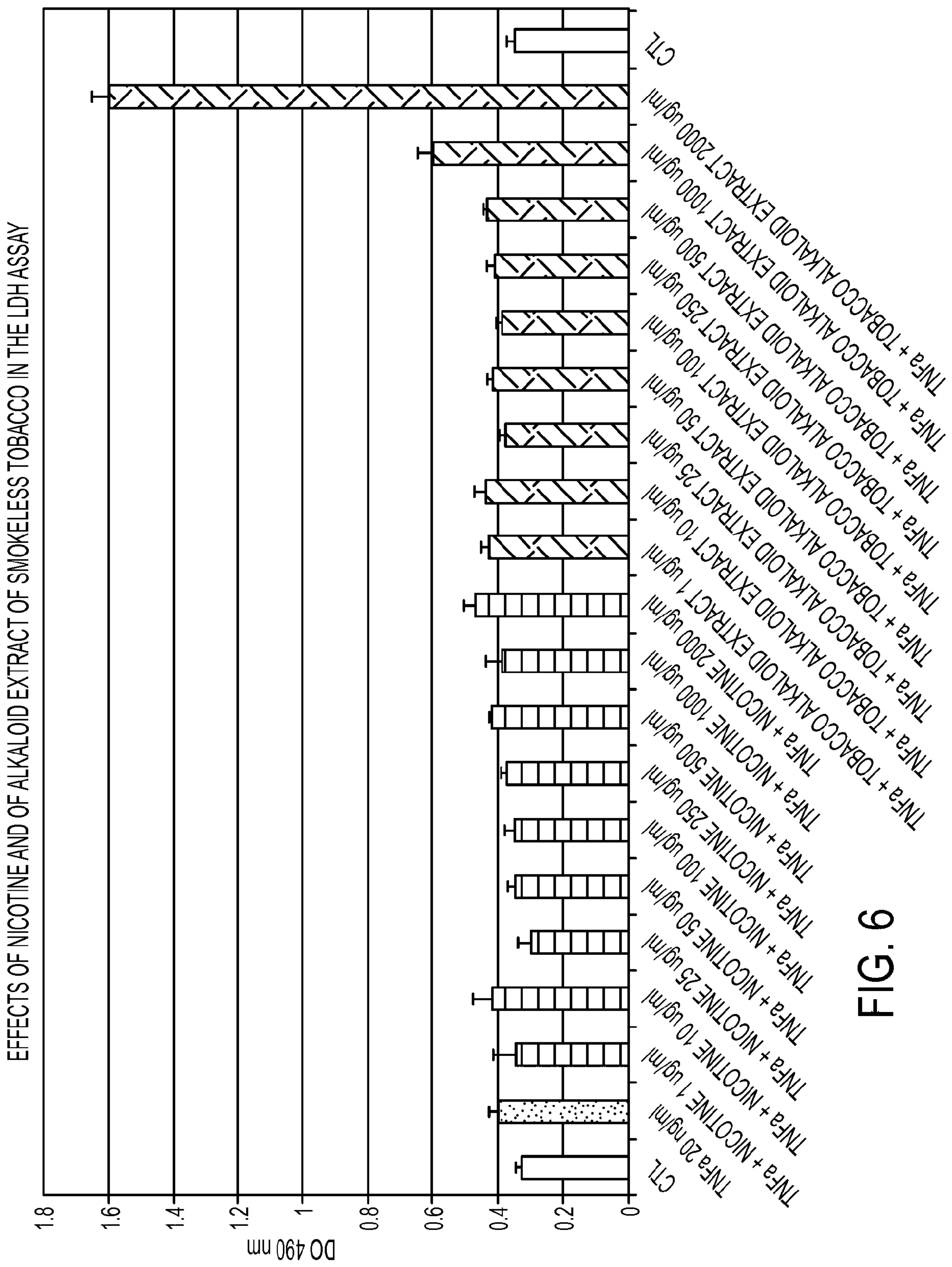
EFFECTS OF NICOTINE AND OF ALKALOID EXTRACT OF SMOKELESS TOBACCO IN THE LDH ASSAY
DO 490 nm
0
0.2
0.4
0.6
0.8
1
1.2
1.4
1.6
1.8
CTL
TNFa 20 ng/ml
TNFa + NICOTINE 1 ug/ml
TNFa + NICOTINE 10 ug/ml
TNFa + NICOTINE 25 ug/ml
TNFa + NICOTINE 50 ug/ml
TNFa + NICOTINE 100 ug/ml
TNFa + NICOTINE 250 ug/ml
TNFa + NICOTINE 500 ug/ml
TNFa + NICOTINE 1000 ug/ml
TNFa + NICOTINE 2000 ug/ml
TNFa + TOBACCO ALKALOID EXTRACT 1 ug/ml
TNFa + TOBACCO ALKALOID EXTRACT 10 ug/ml
TNFa + TOBACCO ALKALOID EXTRACT 25 ug/ml
TNFa + TOBACCO ALKALOID EXTRACT 50 ug/ml
TNFa + TOBACCO ALKALOID EXTRACT 100 ug/ml
TNFa + TOBACCO ALKALOID EXTRACT 250 ug/ml
TNFa + TOBACCO ALKALOID EXTRACT 500 ug/ml
TNFa + TOBACCO ALKALOID EXTRACT 1000 ug/ml
TNFa + TOBACCO ALKALOID EXTRACT 2000 ug/ml
CTL

FIG. 6

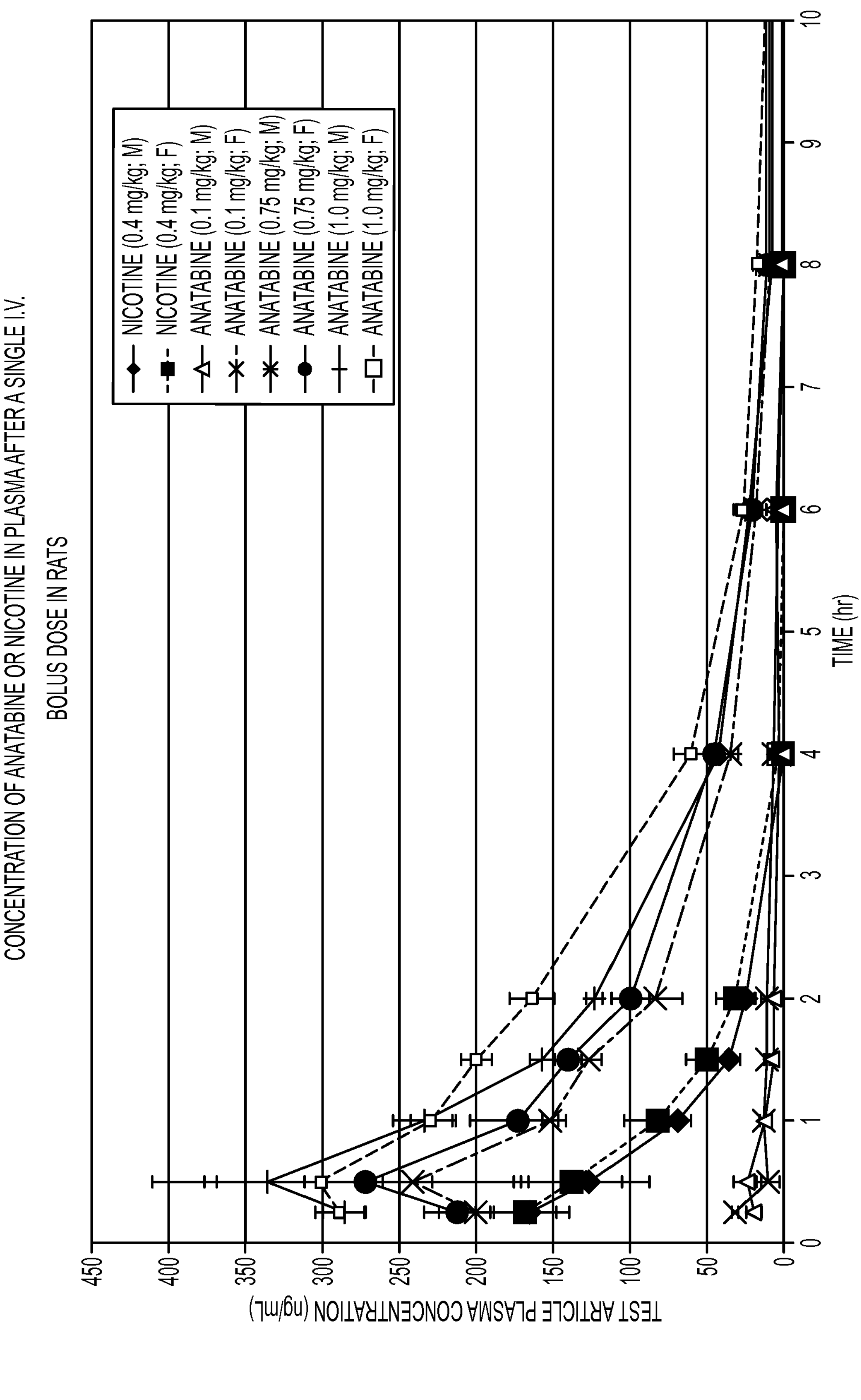
CONCENTRATION OF ANATABINE OR NICOTINE IN PLASMA AFTER A SINGLE I.V.
BOLUS DOSE IN RATS
NICOTINE (0.4 mg/kg; M)
NICOTINE (0.4 mg/kg; F)
ANATABINE (0.1 mg/kg; M)
ANATABINE (0.1 mg/kg; F)
ANATABINE (0.75 mg/kg; M)
ANATABINE (0.75 mg/kg; F)
ANATABINE (1.0 mg/kg; M)
ANATABINE (1.0 mg/kg; F)
TEST ARTICLE PLASMA CONCENTRATION (ng/mL)
0
50
100
150
200
250
300
350
400
450
TIME (hr)
0
1
2
3
4
5
6
7
8
9
10

FIG. 7

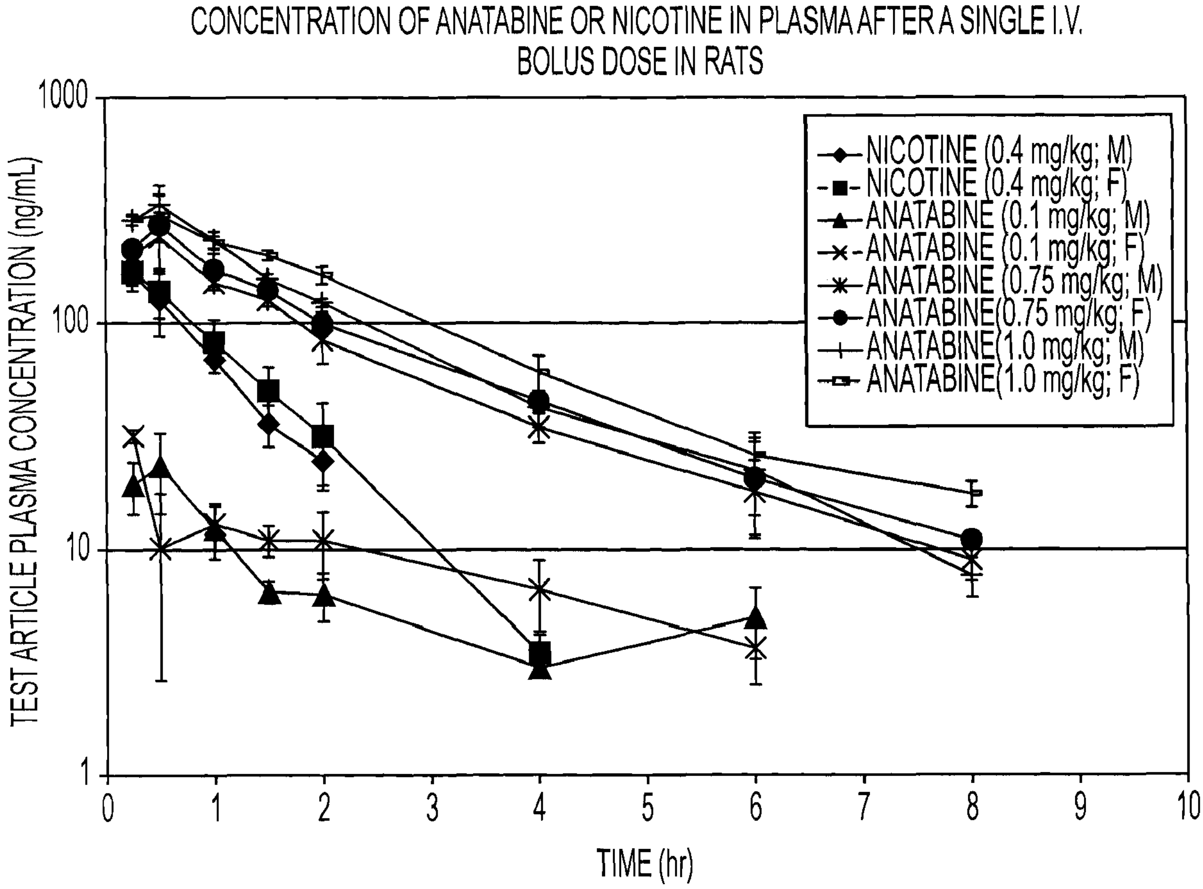
CONCENTRATION OF ANATABINE OR NICOTINE IN PLASMA AFTER A SINGLE I.V. BOLUS DOSE IN RATS
TEST ARTICLE PLASMA CONCENTRATION (ng/mL)
1000
100
10
1
0
1
2
3
4
5
6
7
8
9
10
TIME (hr)
NICOTINE (0.4 mg/kg; M)
NICOTINE (0.4 mg/kg; F)
ANATABINE (0.1 mg/kg; M)
ANATABINE (0.1 mg/kg; F)
ANATABINE (0.75 mg/kg; M)
ANATABINE(0.75 mg/kg; F)
ANATABINE(1.0 mg/kg; M)
ANATABINE(1.0 mg/kg; F)

FIG. 8

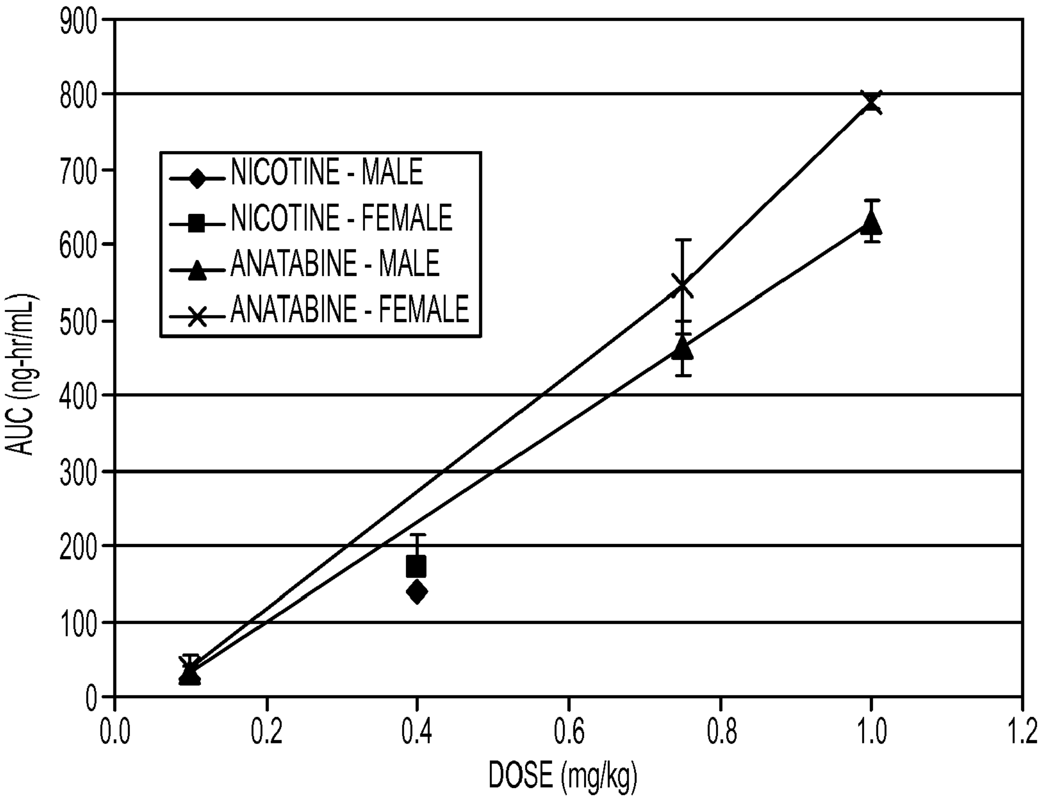
NICOTINE - MALE
NICOTINE - FEMALE
ANATABINE - MALE
ANATABINE - FEMALE
AUC (ng-hr/mL)
900
800
700
600
500
400
300
200
100
0
0.0
0.2
0.4
0.6
0.8
1.0
1.2
DOSE (mg/kg)

FIG. 9

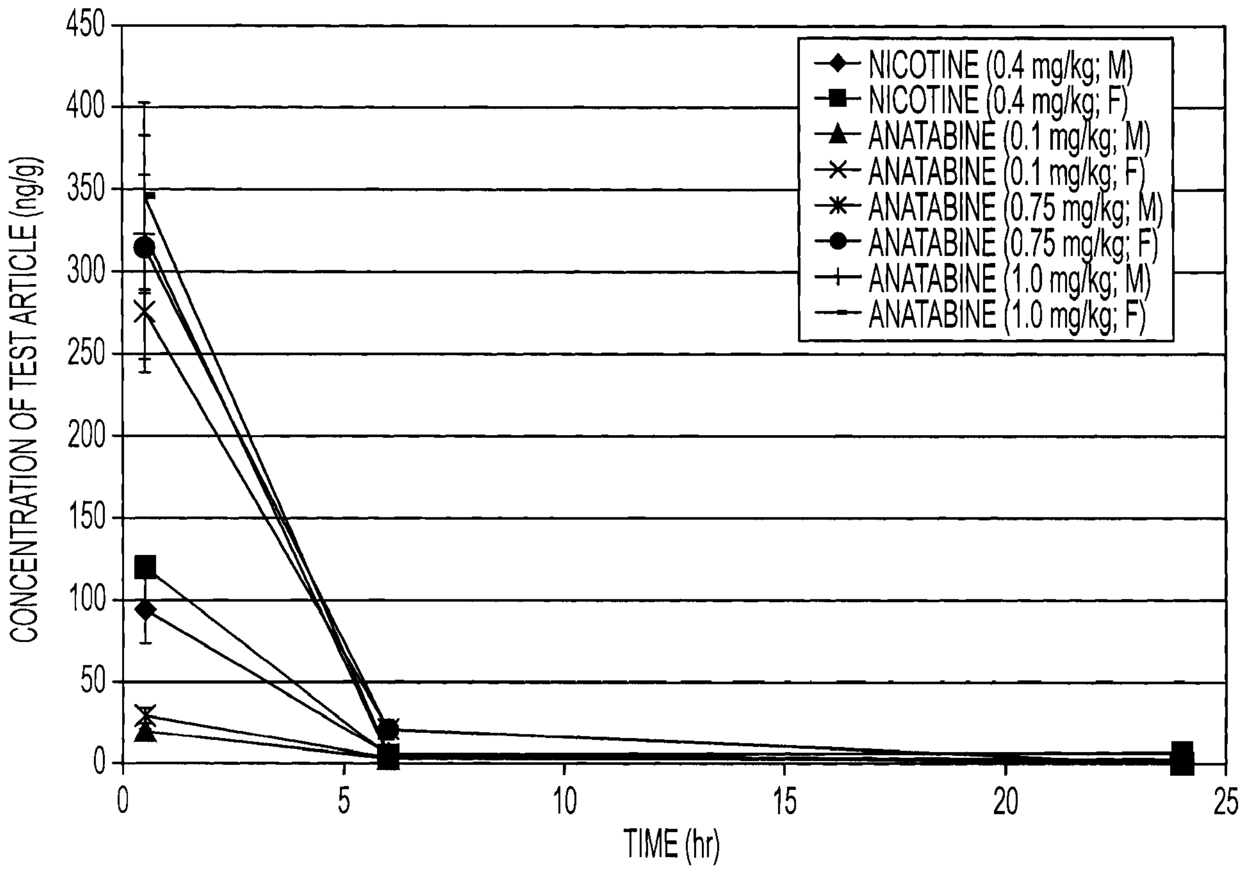

CONCENTRATION OF TEST ARTICLE (ng/g)
450
400
350
300
250
200
150
100
50
0
0
5
10
15
20
25
TIME (hr)
NICOTINE (0.4 mg/kg; M)
NICOTINE (0.4 mg/kg; F)
ANATABINE (0.1 mg/kg; M)
ANATABINE (0.1 mg/kg; F)
ANATABINE (0.75 mg/kg; M)
ANATABINE (0.75 mg/kg; F)
ANATABINE (1.0 mg/kg; M)
ANATABINE (1.0 mg/kg; F)


FIG. 10

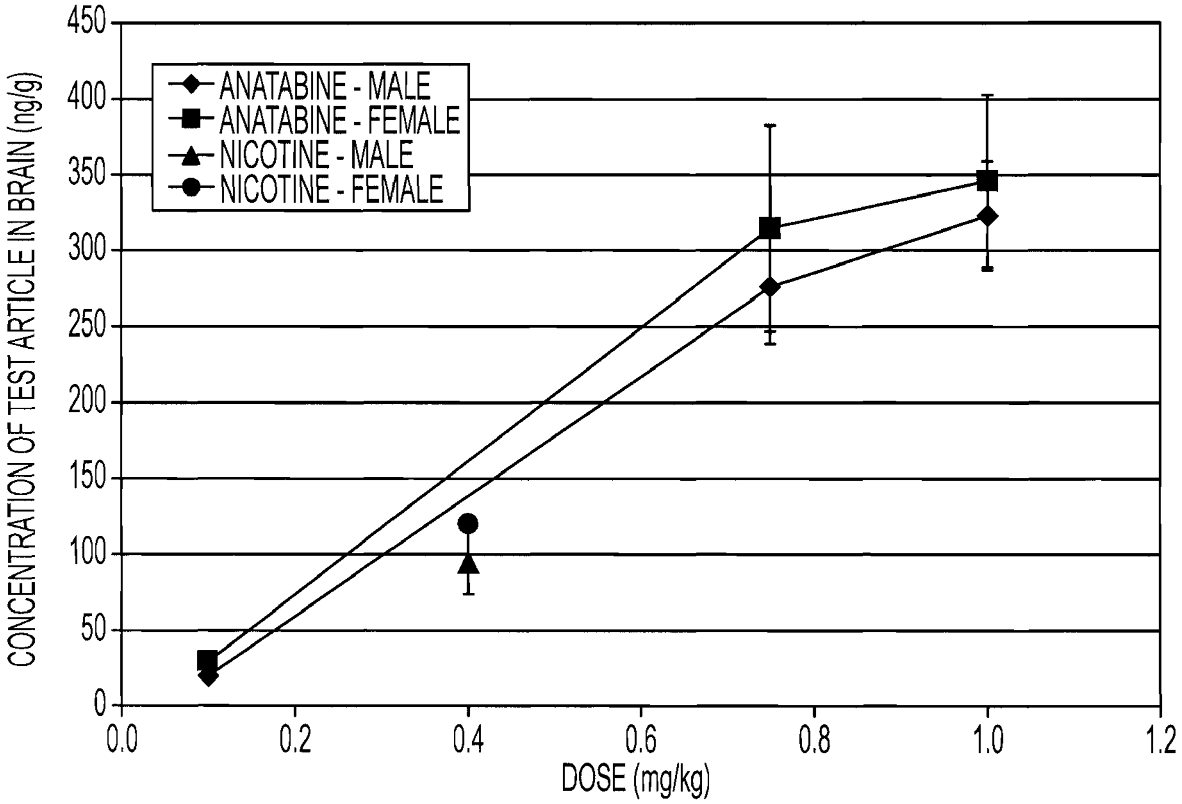
450
400
350
300
250
200
150
100
50
0
CONCENTRATION OF TEST ARTICLE IN BRAIN (ng/g)
ANATABINE - MALE
ANATABINE - FEMALE
NICOTINE - MALE
NICOTINE - FEMALE
0.0
0.2
0.4
0.6
0.8
1.0
1.2
DOSE (mg/kg)

FIG. 11

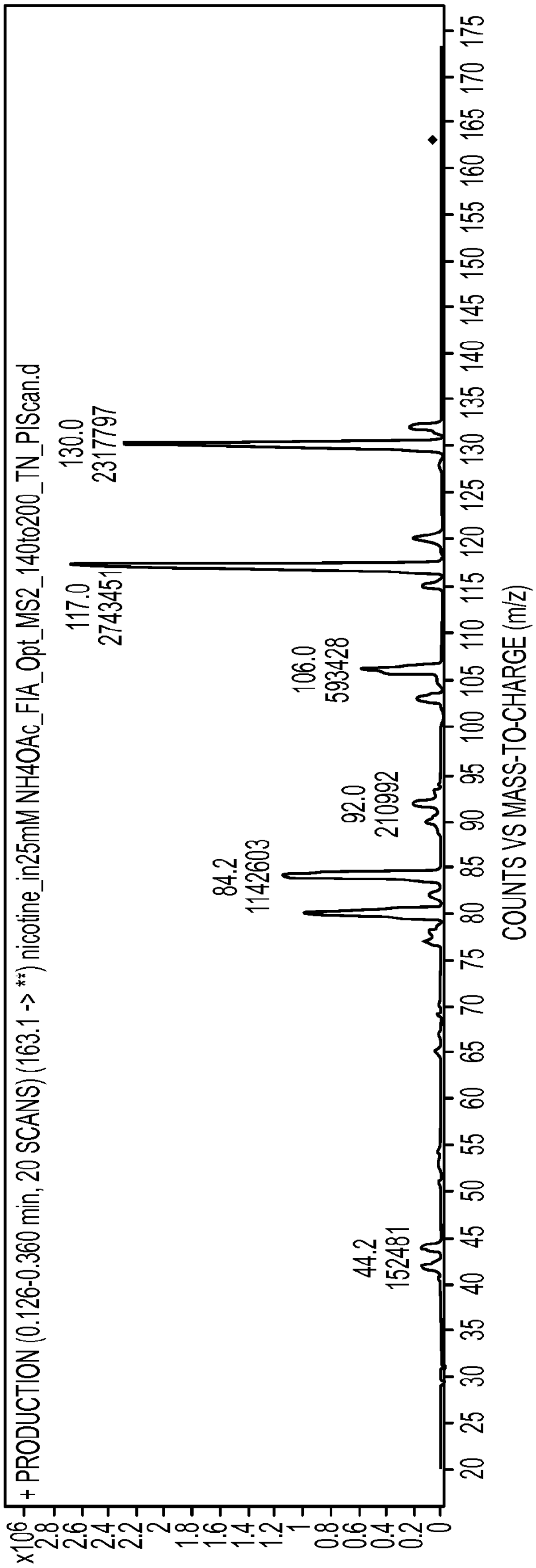
+ PRODUCTION (0.126-0.360 min, 20 SCANS) (163.1 -> **) nicotine_in25mM NH4OAc_FIA_Opt_MS2_140to200_TN_PIScan.d
x10^6
130.0
2317797
117.0
2743451
106.0
593428
92.0
210992
84.2
1142603
44.2
152481
COUNTS VS MASS-TO-CHARGE (m/z)

FIG. 12

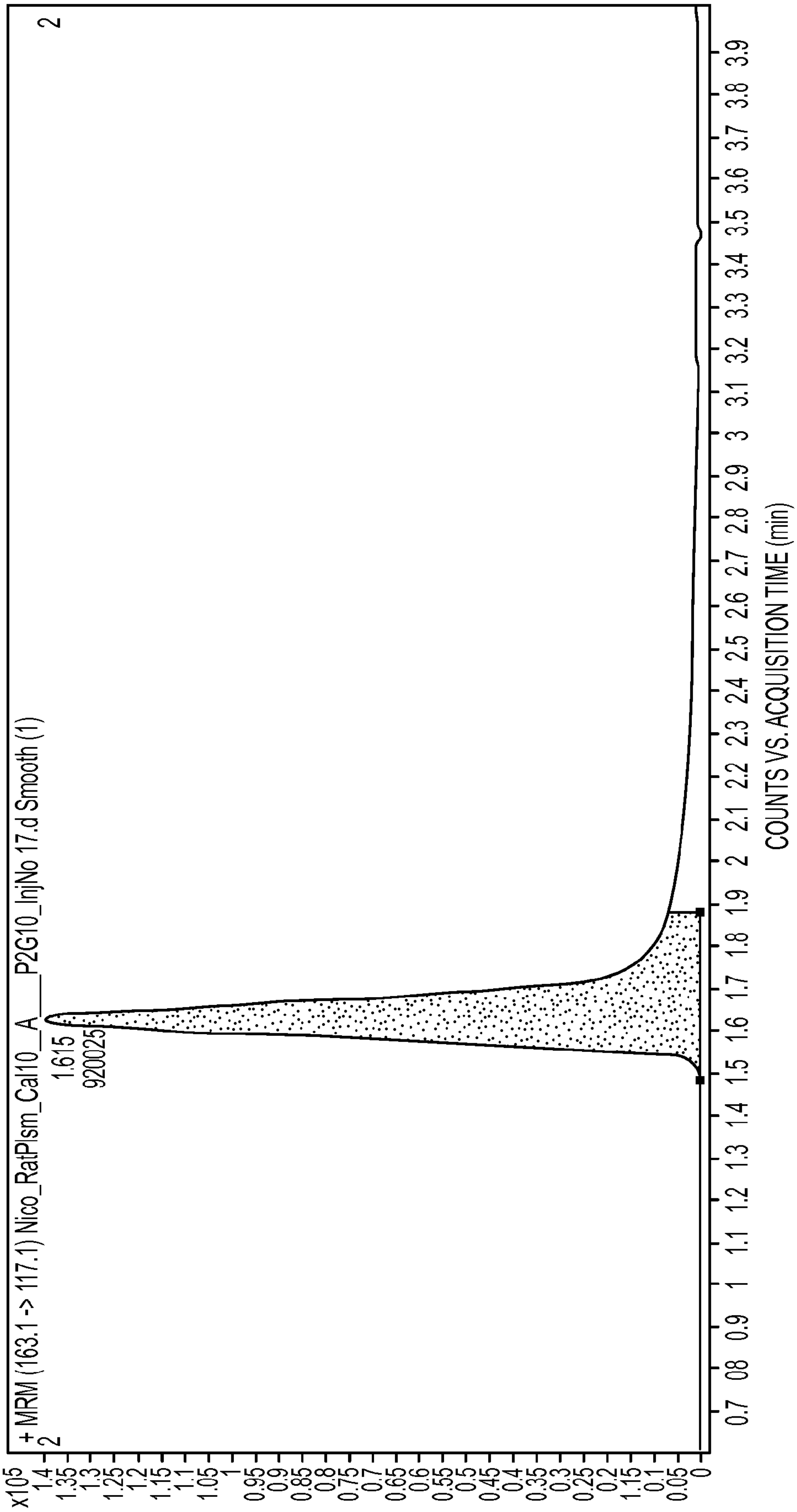
x10^5
+ MRM (163.1 -> 117.1) Nico_RatPlsm_Cal10__A___P2G10_InjNo 17.d Smooth (1)
1.615
920025
COUNTS VS. ACQUISITION TIME (min)

FIG. 13

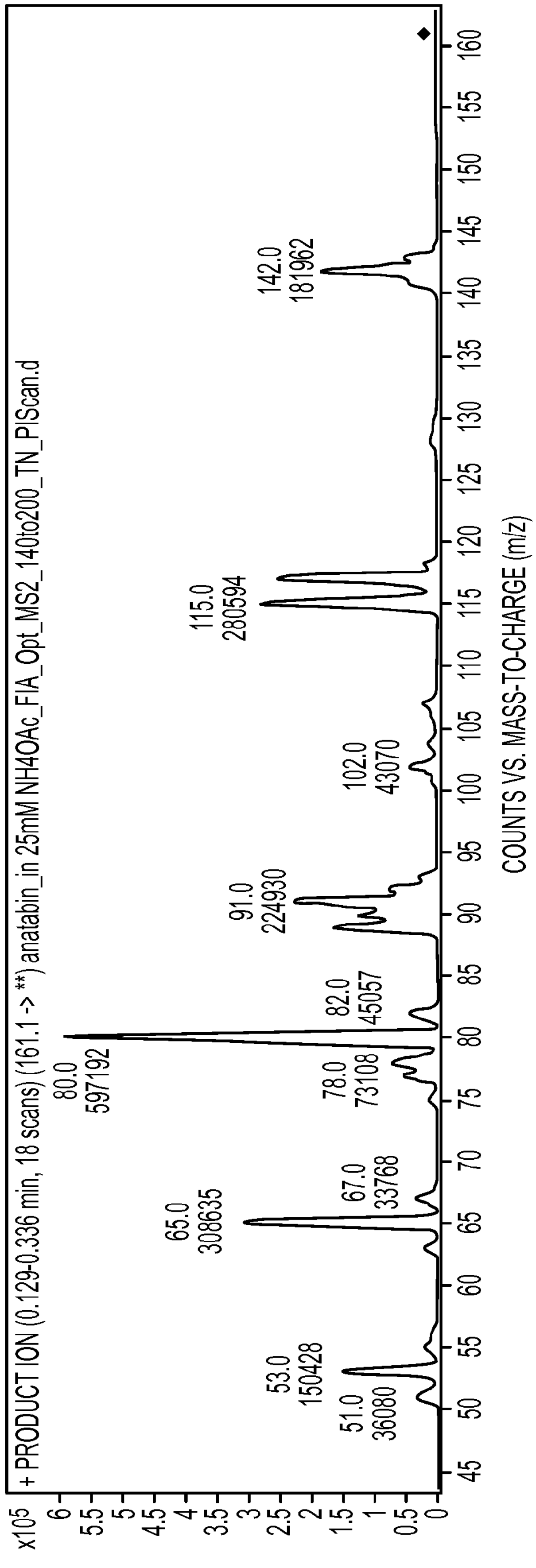
+ PRODUCT ION (0.129-0.336 min, 18 scans) (161.1 -> **) anatabin_in 25mM NH4OAc_FIA_Opt_MS2_140to200_TN_PIScan.d
x10^5
6
5.5
5
4.5
4
3.5
3
2.5
2
1.5
1
0.5
0
51.0
36080
53.0
150428
65.0
308635
67.0
33768
80.0
597192
78.0
73108
82.0
45057
91.0
224930
102.0
43070
115.0
280594
142.0
181962
45
50
55
60
65
70
75
80
85
90
95
100
105
110
115
120
125
130
135
140
145
150
155
160
COUNTS VS. MASS-TO-CHARGE (m/z)

FIG. 14

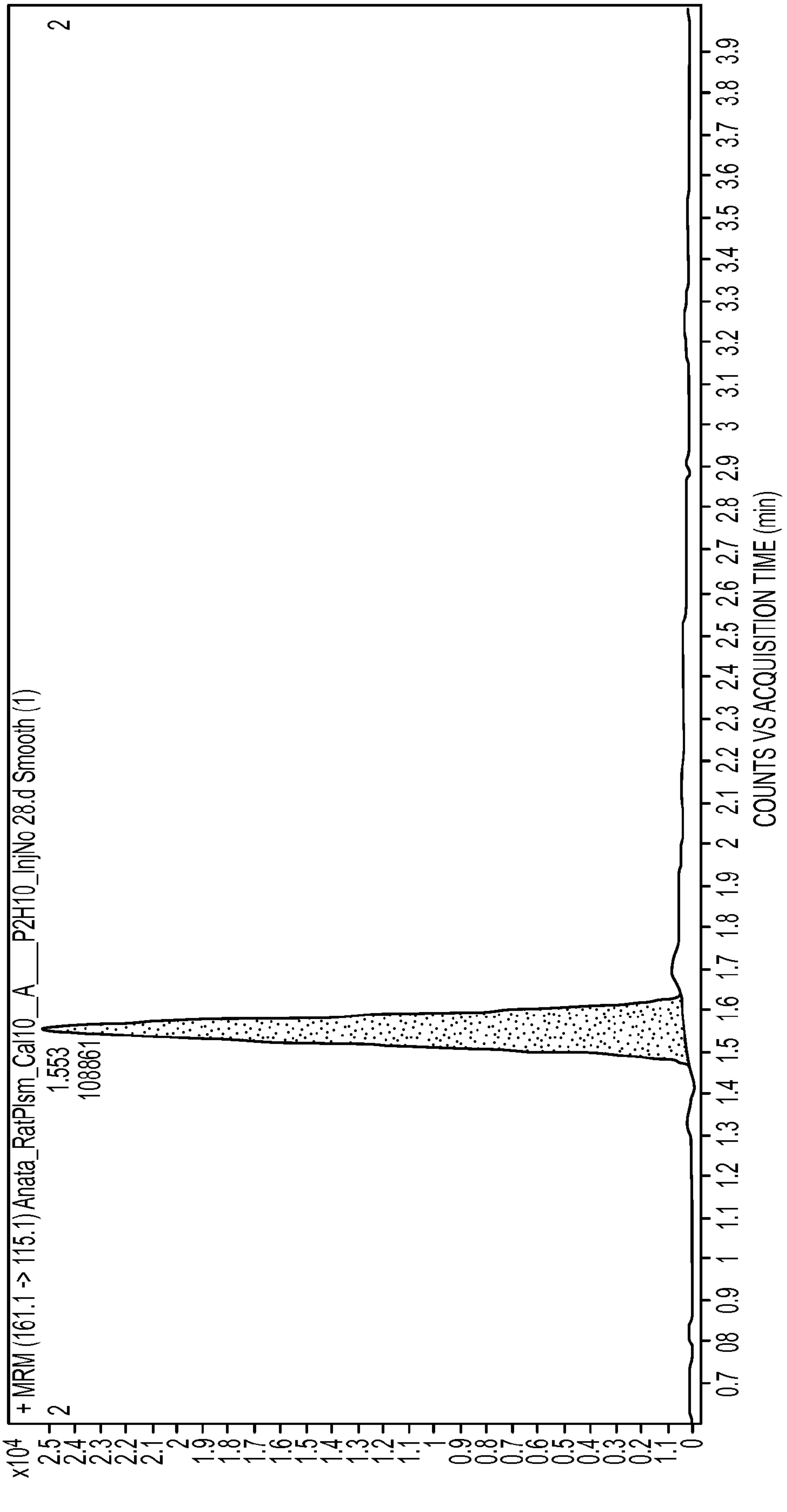
+ MRM (161.1 -> 115.1) Anata_RatPlsm_Cal10__A___P2H10_InjNo 28.d Smooth (1)
x10^4
1.553
108861
2
2
COUNTS VS ACQUISITION TIME (min)

FIG. 15

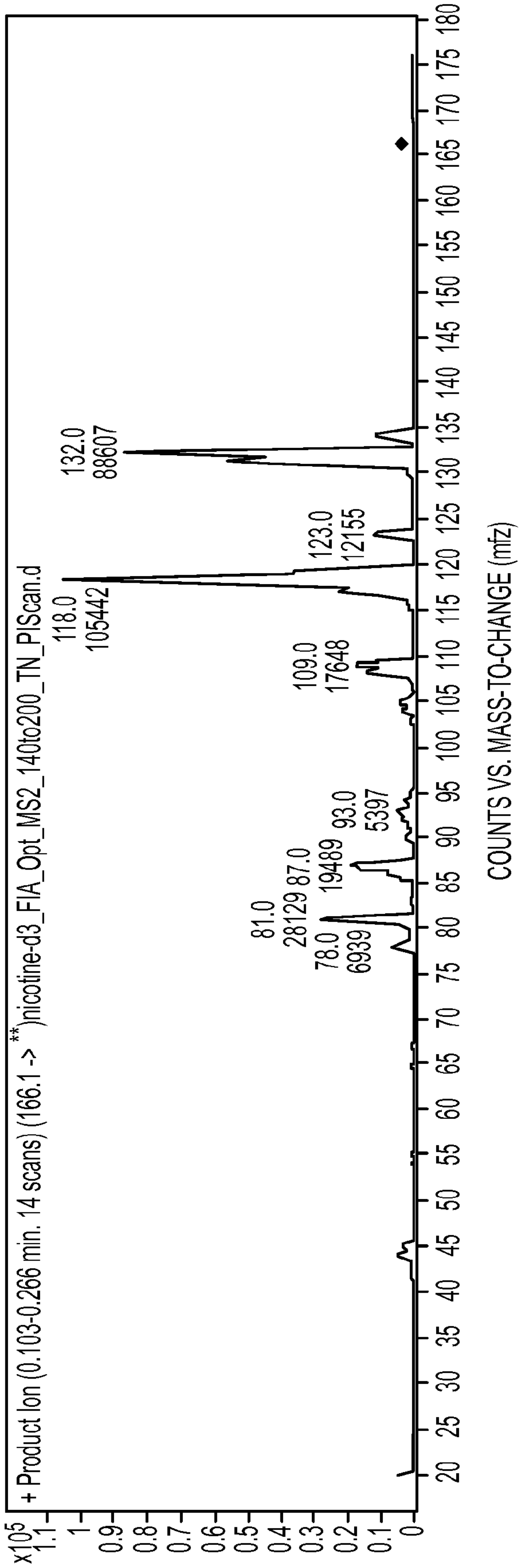
+ Product Ion (0.103-0.266 min. 14 scans) (166.1 -> **)nicotine-d3_FIA_Opt_MS2_140to200_TN_PIScan.d
x10^5
1.1
1
0.9
0.8
0.7
0.6
0.5
0.4
0.3
0.2
0.1
0
20 25 30 35 40 45 50 55 60 65 70 75 80 85 90 95 100 105 110 115 120 125 130 135 140 145 150 155 160 165 170 175 180
78.0
6939
81.0
28129
87.0
19489
93.0
5397
109.0
17648
118.0
105442
123.0
12155
132.0
88607
COUNTS VS. MASS-TO-CHANGE (m/z)

FIG. 16

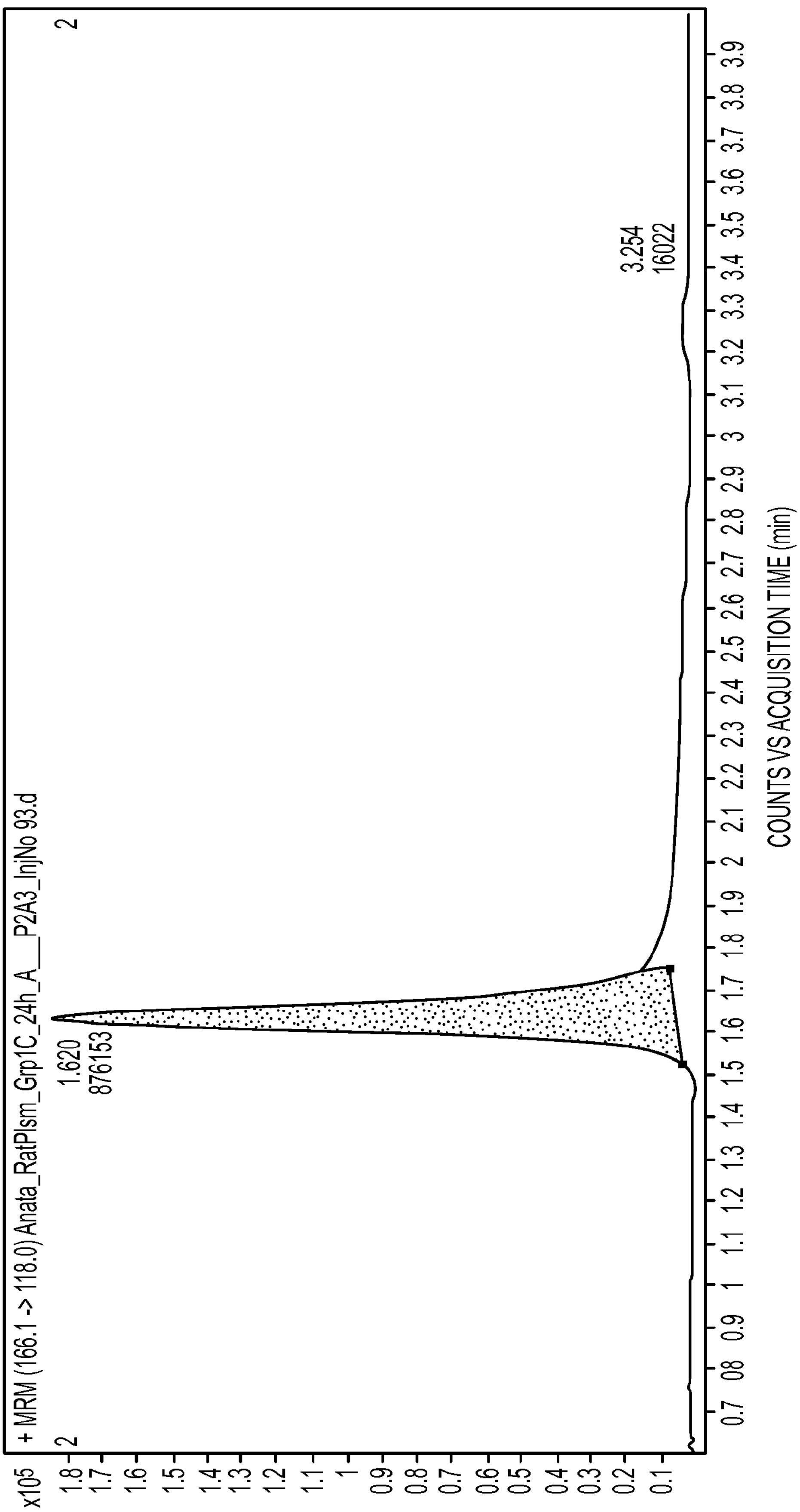
+ MRM (166.1 -> 118.0) Anata_RatPlsm_Grp1C_24h_A__P2A3_InjNo 93.d
x10^5
1.620
876153
3.254
16022
2
2
COUNTS VS ACQUISITION TIME (min)

FIG. 17

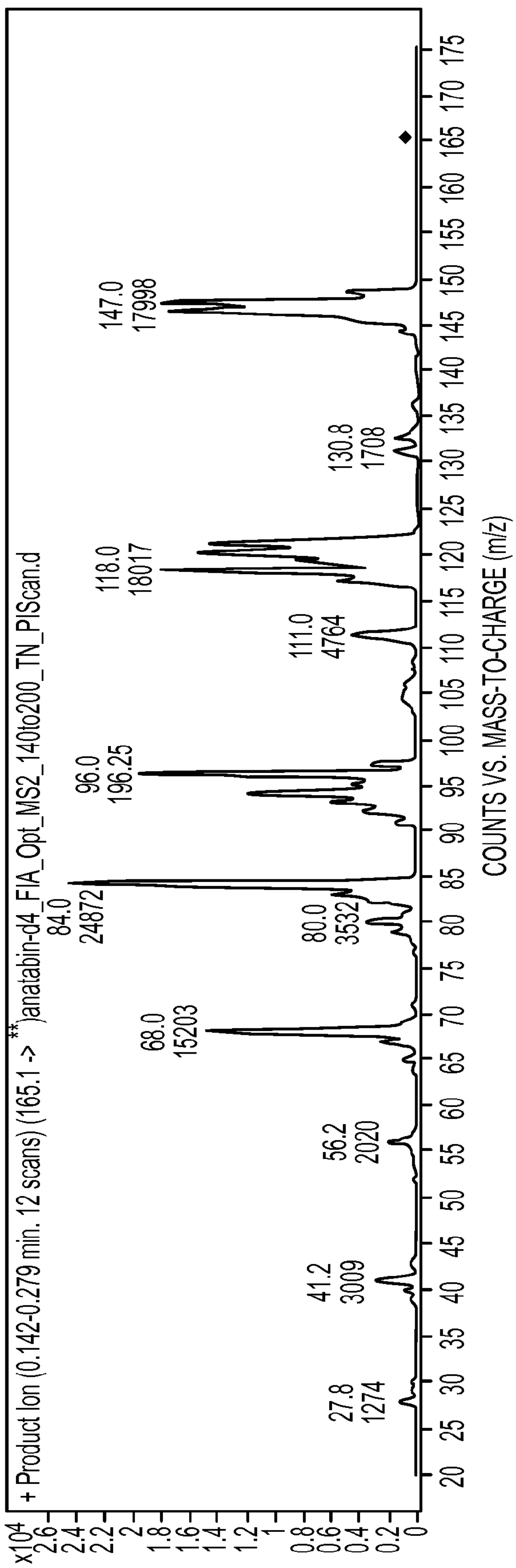
+ Product Ion (0.142-0.279 min. 12 scans) (165.1 -> **)anatabin-d4_FIA_Opt_MS2_140to200_TN_PIScan.d
x10^4
2.6
2.4
2.2
2
1.8
1.6
1.4
1.2
1
0.8
0.6
0.4
0.2
0
27.8
1274
41.2
3009
56.2
2020
68.0
15203
80.0
3532
84.0
24872
96.0
196.25
111.0
4764
118.0
18017
130.8
1708
147.0
17998
20 25 30 35 40 45 50 55 60 65 70 75 80 85 90 95 100 105 110 115 120 125 130 135 140 145 150 155 160 165 170 175
COUNTS VS. MASS-TO-CHARGE (m/z)

FIG. 18

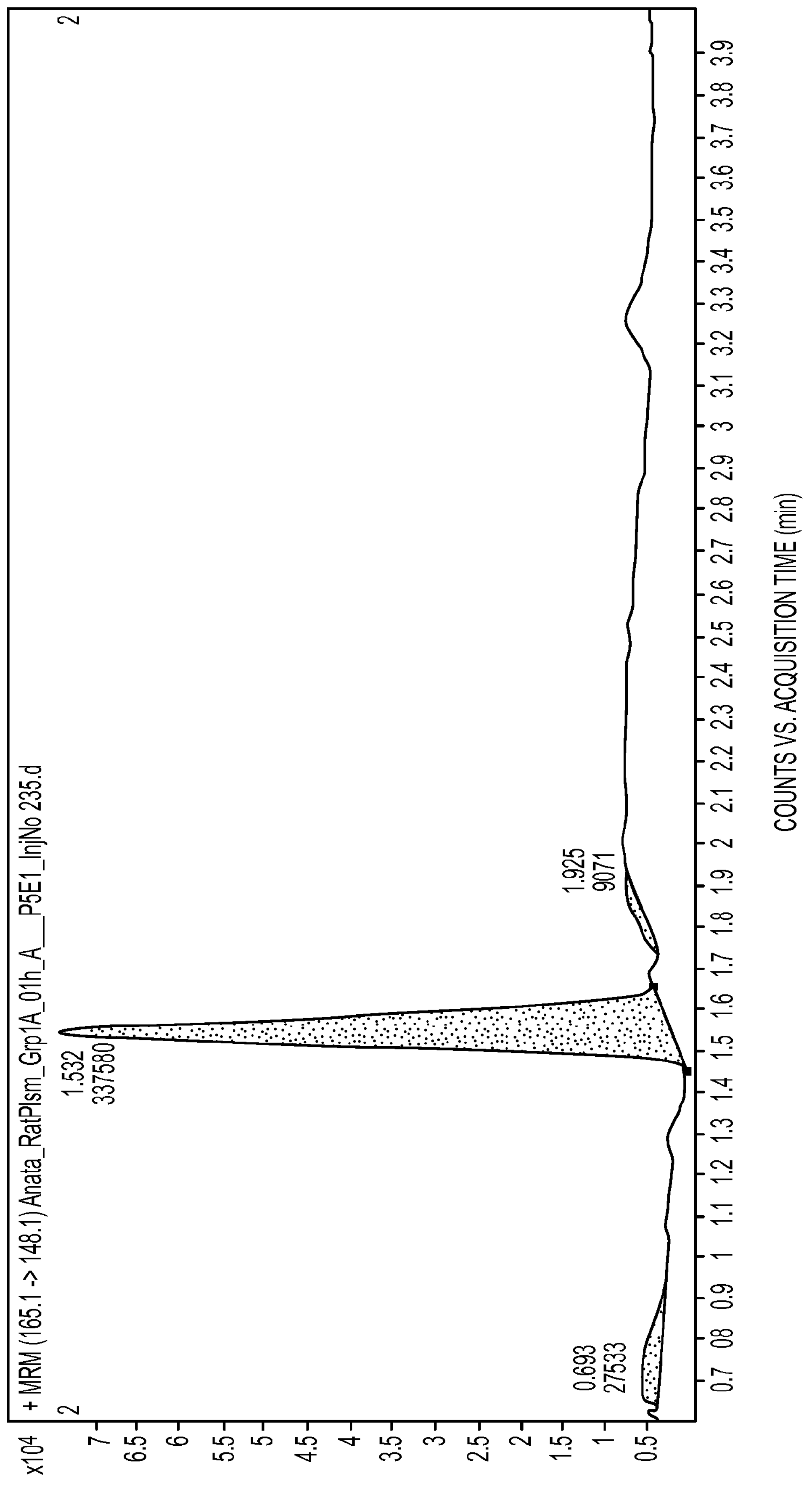
+ MRM (165.1 -> 148.1) Anata_RatPlsm_Grp1A_01h_A___P5E1_InjNo 235.d
x104
1.532
337580
0.693
27533
1.925
9071
COUNTS VS. ACQUISITION TIME (min)

FIG. 19

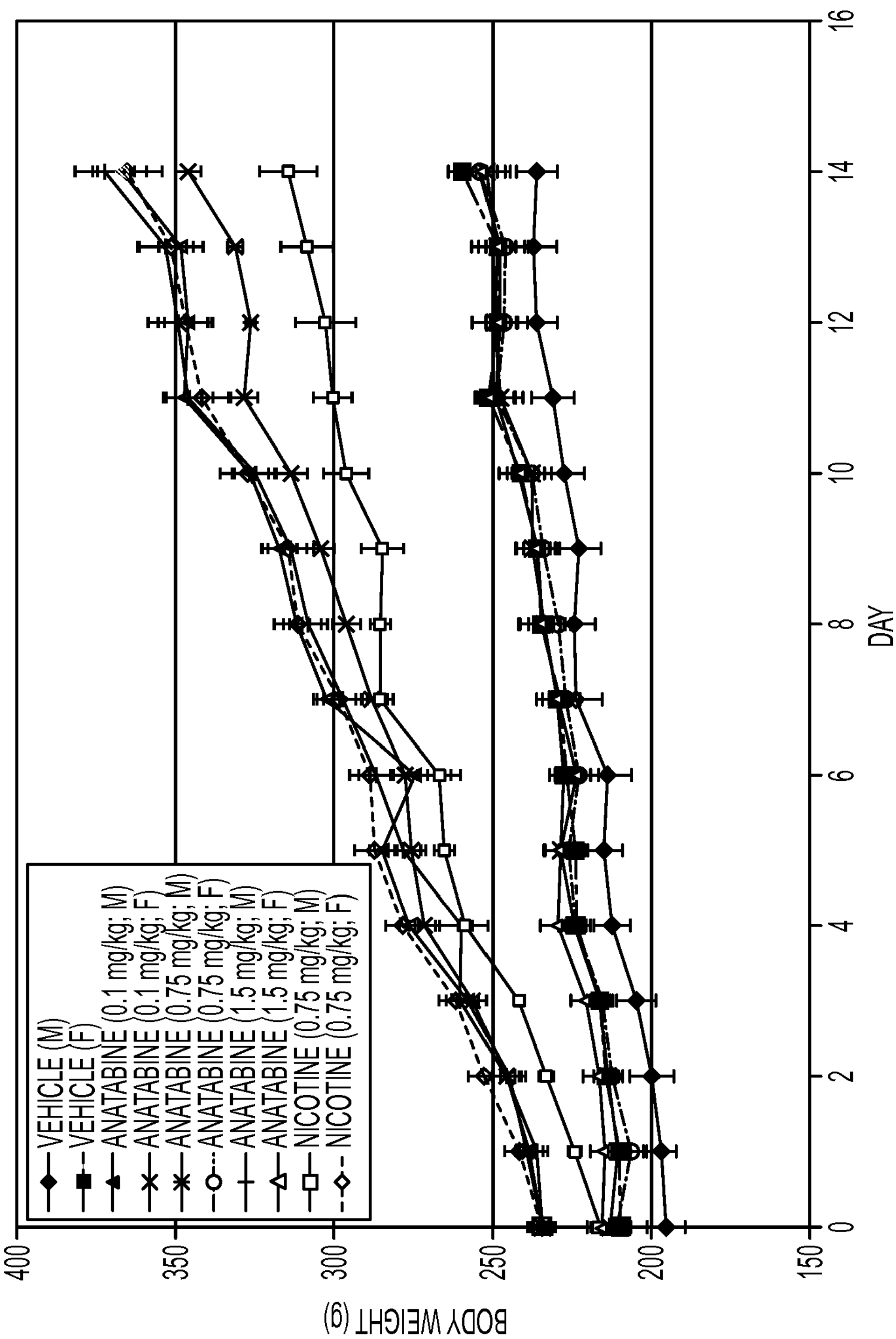
VEHICLE (M)
VEHICLE (F)
ANATABINE (0.1 mg/kg; M)
ANATABINE (0.1 mg/kg; F)
ANATABINE (0.75 mg/kg; M)
ANATABINE (0.75 mg/kg; F)
ANATABINE (1.5 mg/kg; M)
ANATABINE (1.5 mg/kg; F)
NICOTINE (0.75 mg/kg; M)
NICOTINE (0.75 mg/kg; F)
BODY WEIGHT (g)
400
350
300
250
200
150
DAY
0
2
4
6
8
10
12
14
16

FIG. 20

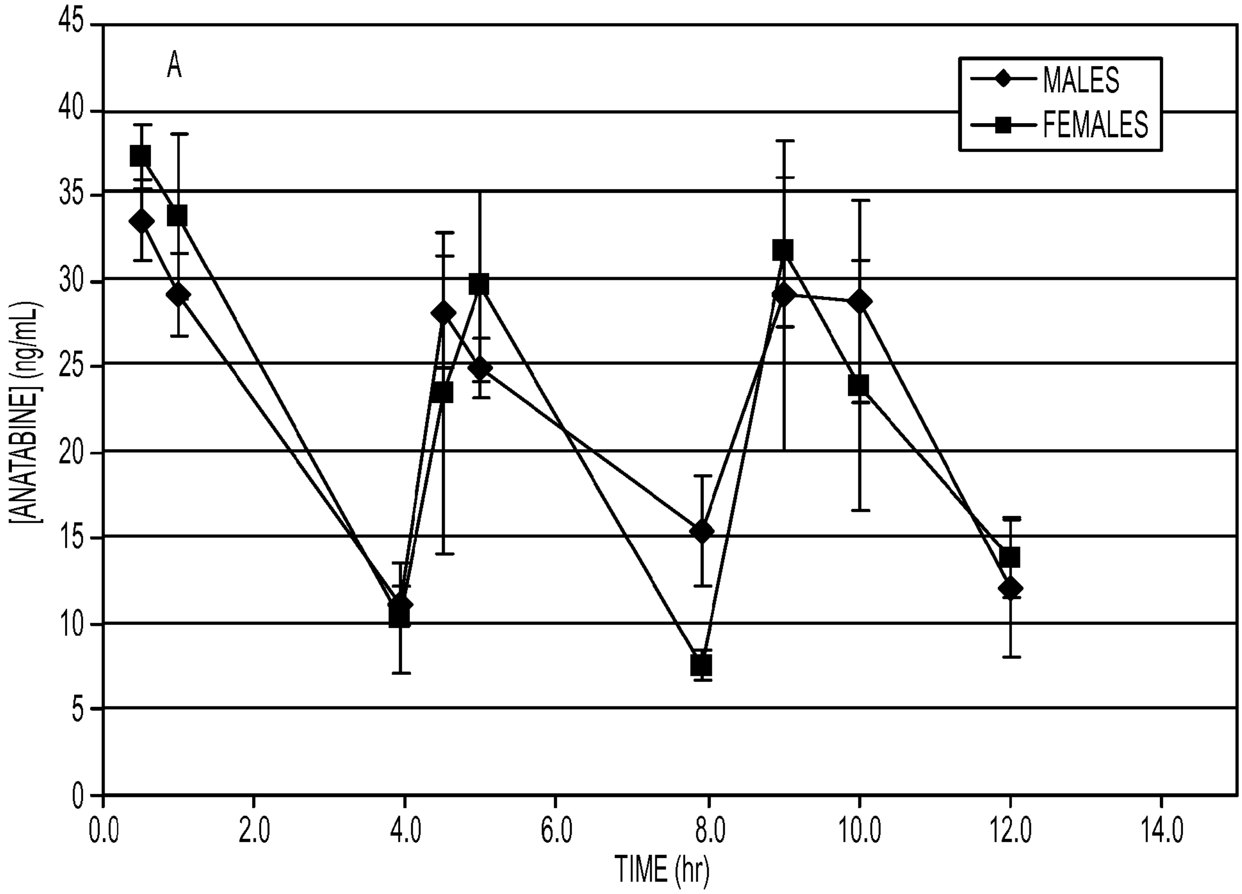
A
MALES
FEMALES
[ANATABINE] (ng/mL)
TIME (hr)
0
5
10
15
20
25
30
35
40
45
0.0
2.0
4.0
6.0
8.0
10.0
12.0
14.0

FIG. 21A

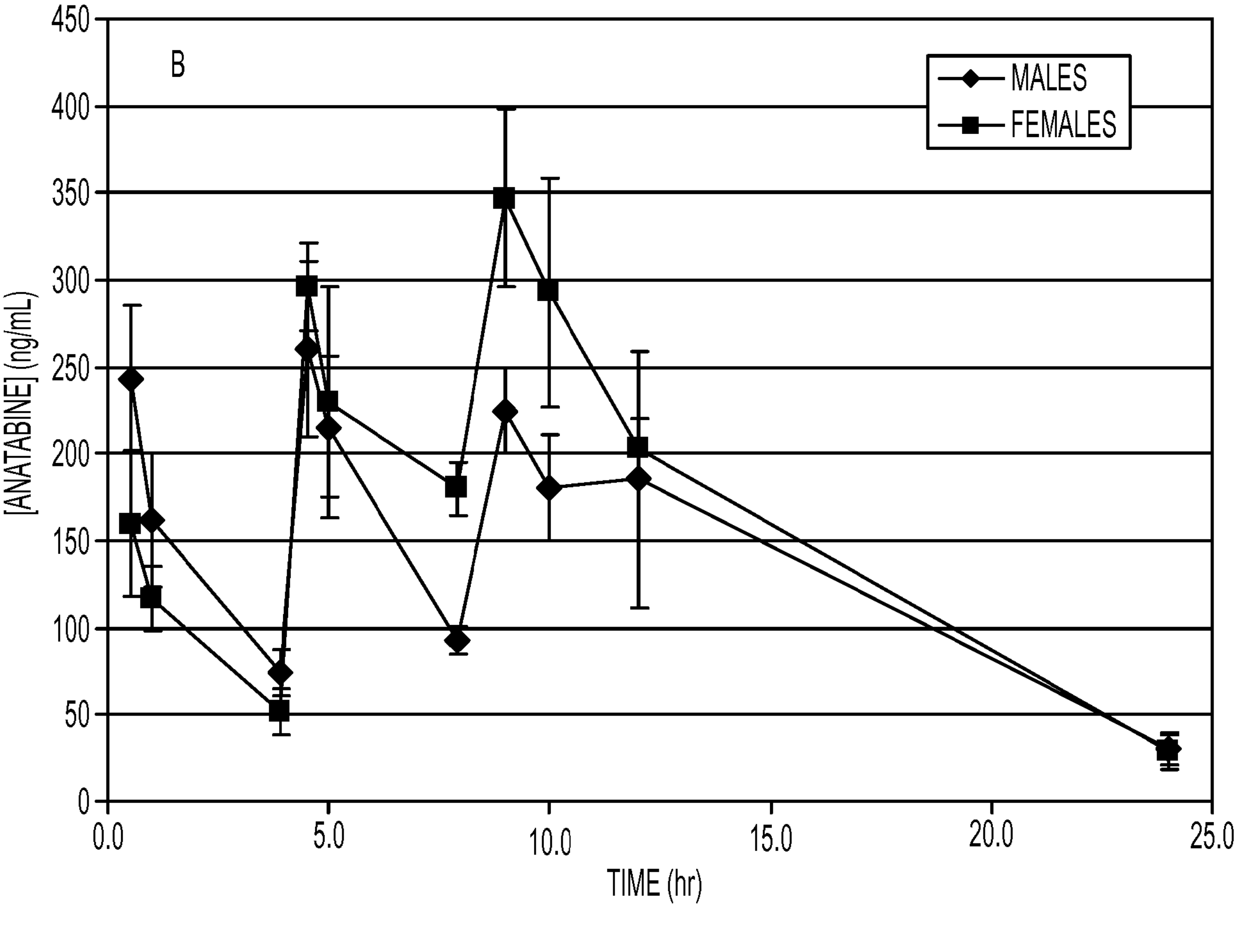
B
MALES
FEMALES
[ANATABINE] (ng/mL)
450
400
350
300
250
200
150
100
50
0
0.0
5.0
10.0
15.0
20.0
25.0
TIME (hr)

FIG. 21B

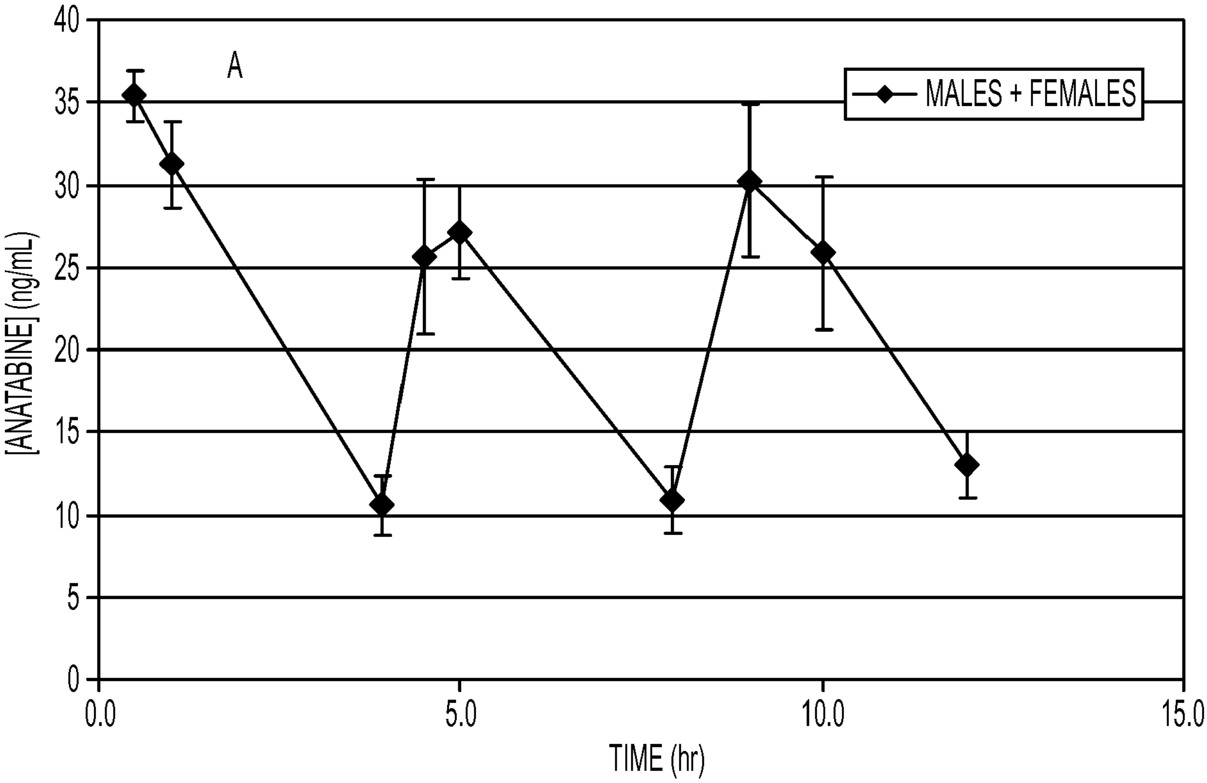
40
35
30
25
20
15
10
5
0
[ANATABINE] (ng/mL)
A
MALES + FEMALES
0.0
5.0
10.0
15.0
TIME (hr)

FIG. 22A

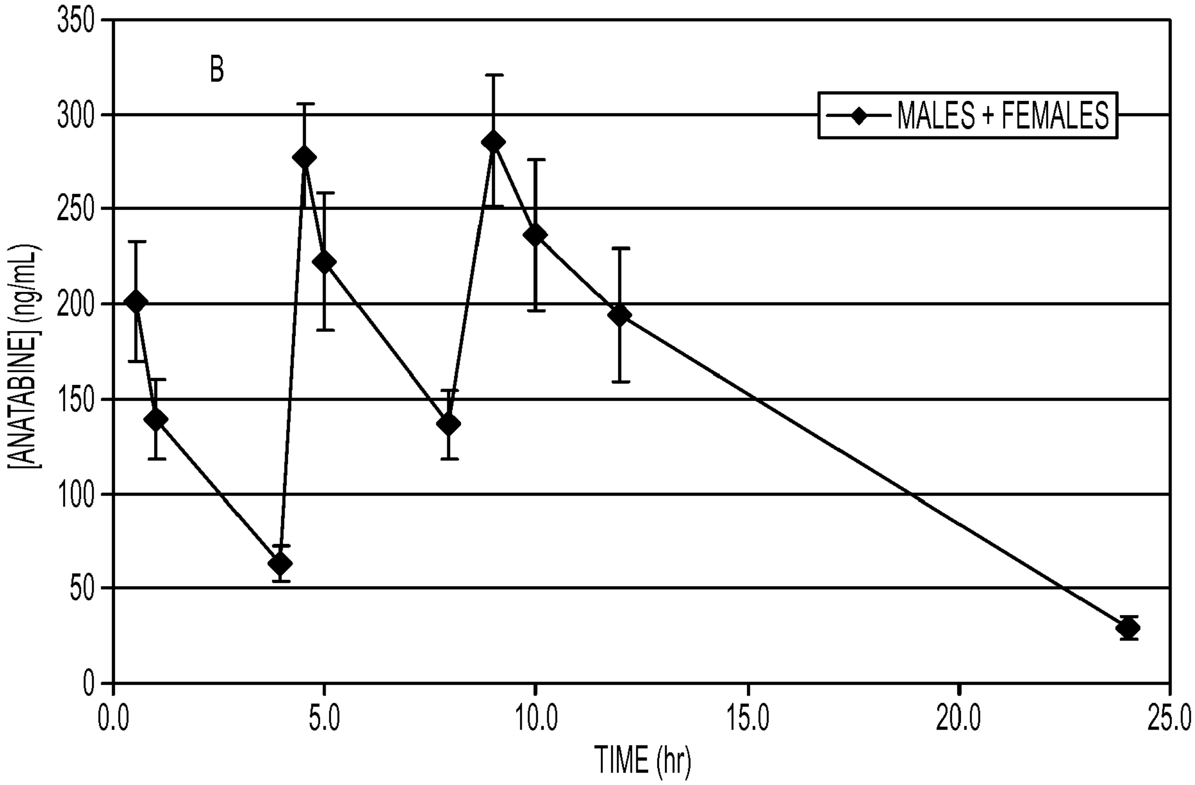
350
300
250
200
150
100
50
0
B
MALES + FEMALES
[ANATABINE] (ng/mL)
0.0
5.0
10.0
15.0
20.0
25.0
TIME (hr)

FIG. 22B

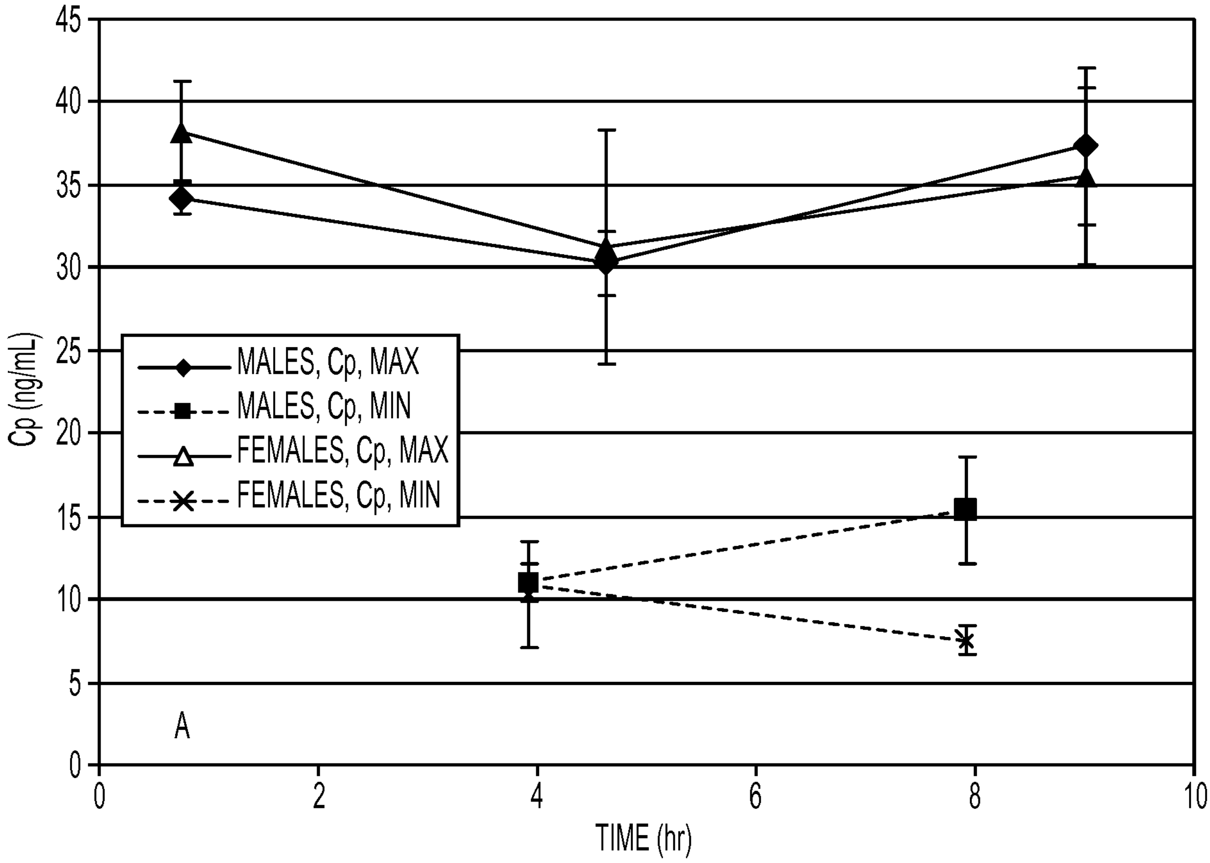
45
40
35
30
25
20
15
10
5
0
Cp (ng/mL)
MALES, Cp, MAX
MALES, Cp, MIN
FEMALES, Cp, MAX
FEMALES, Cp, MIN
A
0
2
4
6
8
10
TIME (hr)

FIG. 23A

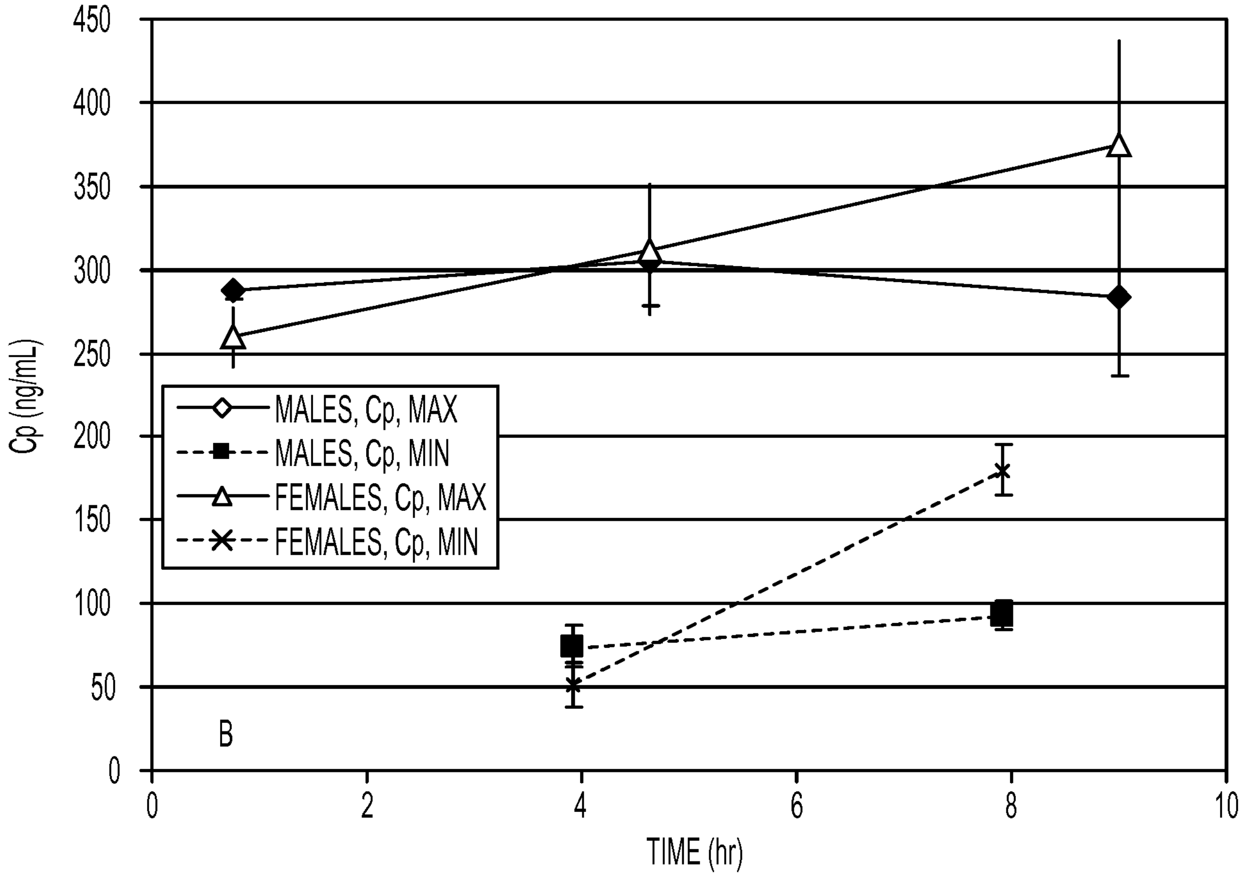

450
400
350
300
250
200
150
100
50
0
Cp (ng/mL)
MALES, Cp, MAX
MALES, Cp, MIN
FEMALES, Cp, MAX
FEMALES, Cp, MIN
B
0
2
4
6
8
10
TIME (hr)


FIG. 23B

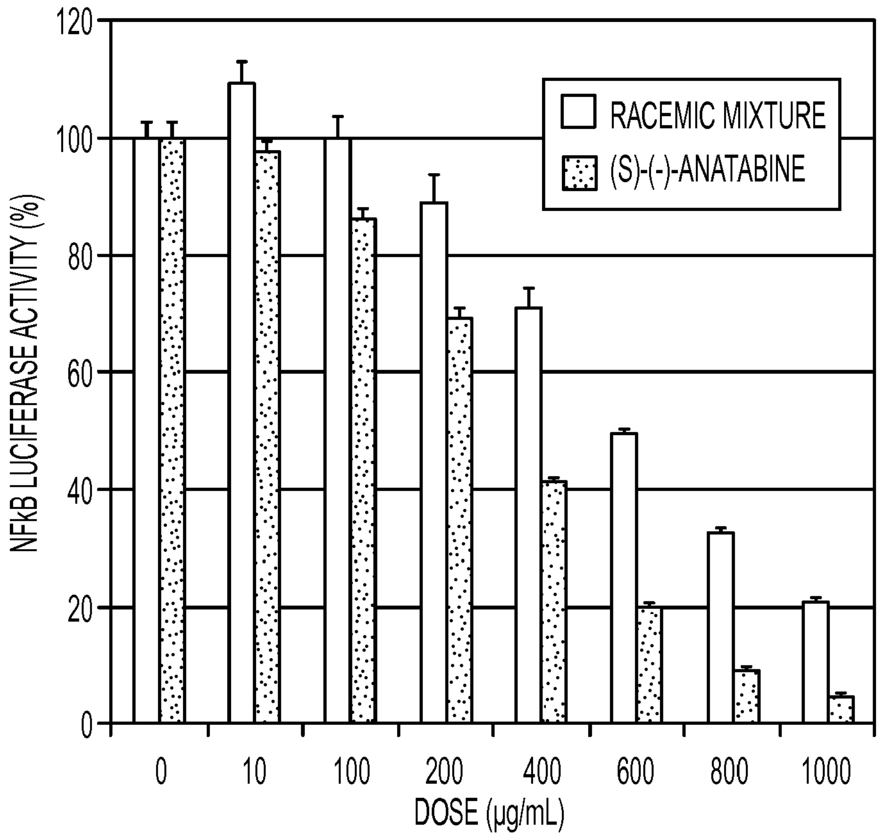

120
100
80
60
40
20
0
NFkB LUCIFERASE ACTIVITY (%)
RACEMIC MIXTURE
(S)-(-)-ANATABINE
0
10
100
200
400
600
800
1000
DOSE (μg/mL)


FIG. 24

Europäisches Patentamt
European Patent Office
Office européen des brevets

# EUROPEAN SEARCH REPORT

Application Number
EP 19 16 2452

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | WO 99/62531 A1 (REGENT COURT TECHNOLOGIES [US]) 9 December 1999 (1999-12-09) * See page 5, lines 20-25; page 8, lines 18-21; example 2; claims 1, 7, 8 and see figures 3 and 4: anatabine as a MAO inhibitor; uses for the treatment of neurological conditions, in particular Alzheimer and Parkinson * ----- | 1-15 | INV. A61K31/4458 A61P29/00 C07D213/38 C07D213/53 |
| Y | EP 0 567 251 A1 (REYNOLDS TOBACCO CO R [US]) 27 October 1993 (1993-10-27) * See page 2, lines 36-45, page 4, line 19-30, table I, claims 1, 2, 8-10, 13: anatabine as a nicotine receptor modulator and its use for the treatment of neurodegenerative disorders, Alzheimer and Parkinson in particular * ----- | 1-15 | |
| Y | WO 2010/030887 A1 (CATHOLIC HEALTHCARE WEST [US]; SHI FU-DONG [US]) 18 March 2010 (2010-03-18) * See claims 1, 12, 13, 14, 21, 23, 24, 25, 26, page 8, lines 7-27; page 12, lines 6-10, 32-34: nicotine receptor modulators for use in the treatment of inflammatory conditions of the central nervous system, including Alzheimer and Parkinson * ----- | 1-15 | TECHNICAL FIELDS SEARCHED (IPC) |
| | | | A61K A61P C07D |
| Y | WO 02/076434 A2 (UNIV LAVAL [CA]; CORMIER YVON [CA]; ISRAEL-ASSAYAG EVELYNE [CA]; BLANC) 3 October 2002 (2002-10-03) * See claims and examples: substances modulating the nicotine receptor for the treatment of inflammation * ----- | 1-15 | |
| | -/-- | | |

The present search report has been drawn up for all claims

1

| Place of search | Date of completion of the search | Examiner |
|---|---|---|
| Munich | 19 June 2019 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 16 2452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHI FU-DONG ET AL: "Nicotinic attenuation of central nervous system inflammation and autoimmunity.", 1 February 2009 (2009-02-01), JOURNAL OF IMMUNOLOGY (BALTIMORE, MD. : 1950) 1 FEB 2009 LNKD- PUBMED:19155522, VOL. 182, NR. 3, PAGE(S) 1730 - 1739, XP002647118, ISSN: 1550-6606 * See abstract and results: nicotin recceptor activation mediate the inflammatory process * ----- | 1-15 | |
| A | DENTON TRAVIS T ET AL: "Nicotine-related alkaloids and metabolites as inhibitors of human cytochrome P-450 2A6.", 15 February 2004 (2004-02-15), BIOCHEMICAL PHARMACOLOGY 15 FEB 2004 LNKD- PUBMED:14757175, VOL. 67, NR. 4, PAGE(S) 751 - 756, XP002647119, ISSN: 0006-2952 * See abstract, results and discussion: anatabine is a potent inhibitor of cytochrome P-450 * ----- | 1-15 | TECHNICAL FIELDS SEARCHED (IPC) |
| A | BRASIER ALLAN R: "The NF-kappaB regulatory network.", 2006, CARDIOVASCULAR TOXICOLOGY 2006 LNKD- PUBMED:17303919, VOL. 6, NR. 2, PAGE(S) 111 - 130, XP002647120, ISSN: 1530-7905 * See ubiquitary expression of the NF-KB regulatory network * ----- -/-- | 1-15 | |

The present search report has been drawn up for all claims

1

| Place of search | Date of completion of the search | Examiner |
|---|---|---|
| Munich | 19 June 2019 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 19 16 2452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FELPIN F -X ET AL: "Total synthesis of (S)-anabasine and (S)-anatabine", 2000, SYNLETT 2000 DE, NR. 11, PAGE(S) 1646 - 1648, XP002647121, ISSN: 0936-5214<br>* See total synthesis of anatabine *<br>----- | 1-15 | |
| A | FELPIN F X ET AL: "Efficient enantiomeric synthesis of pyrrolidine and piperidine alkaloids from tobacco.", 21 September 2001 (2001-09-21), THE JOURNAL OF ORGANIC CHEMISTRY 21 SEP 2001 LNKD- PUBMED:11559179, VOL. 66, NR. 19, PAGE(S) 6305 - 6312, XP002647122, ISSN: 0022-3263<br>* See synthesis of anatabine and other alkaloids from tobacco *<br>----- | 1-15 | |
| A | AYERS JOSHUA T ET AL: "A general procedure for the enantioselective synthesis of the minor tobacco alkaloids nornicotine, anabasine, and anatabine.", 2005, THE AAPS JOURNAL 2005 LNKD- PUBMED:16353951, VOL. 7, NR. 3, PAGE(S) E752 - E758, XP002647123, ISSN: 1550-7416<br>* See procedure for the synthesis of alkaloids from tobacco *<br>-----<br>-/-- | 1-15 | TECHNICAL FIELDS SEARCHED (IPC) |

The present search report has been drawn up for all claims

1

| Place of search | Date of completion of the search | Examiner |
|---|---|---|
| Munich | 19 June 2019 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 16 2452

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | LEE S ET AL: "Handbook of Pharmaceutical Salts: Properties, Selection , and Use, Chapter 8 (Large-Scale Aspects of Salt Formation: Processing of Intermediates and Final Products, Chapter 12 (Monographs on Acids and Bases)", 1 January 2002 (2002-01-01), HANDBOOK OF PHARMACEUTICAL SALTS : PROPERTIES, SELECTION, AND USE, ZÜRICH : VERL. HELVETICA CHIMICA ACTA ; WEINHEIM [U.A.] : WILEY-VCH, DE, PAGE(S) 191 - 192,211, XP002548973, ISBN: 978-3-906390-26-0 * the whole document * ----- | 1-15 | |
| | | | TECHNICAL FIELDS SEARCHED (IPC) |

1

The present search report has been drawn up for all claims

| Place of search | Date of completion of the search | Examiner |
|---|---|---|
| Munich | 19 June 2019 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 16 2452

This annex lists the patent family members relating to the patent documents cited in the above-mentioned European search report. The members are as contained in the European Patent Office EDP file on
The European Patent Office is in no way liable for these particulars which are merely given for the purpose of information.

19-06-2019

| Patent document cited in search report | | Publication date | Patent family member(s) | | | Publication date |
|---|---|---|---|---|---|---|
| WO 9962531 | A1 | 09-12-1999 | AU | 770309 | B2 | 19-02-2004 |
| | | | CA | 2334186 | A1 | 09-12-1999 |
| | | | EP | 1083913 | A1 | 21-03-2001 |
| | | | JP | 2002516869 | A | 11-06-2002 |
| | | | US | 6350479 | B1 | 26-02-2002 |
| | | | US | 2002054926 | A1 | 09-05-2002 |
| | | | US | 2003185908 | A1 | 02-10-2003 |
| | | | US | 2005176777 | A1 | 11-08-2005 |
| | | | WO | 9962531 | A1 | 09-12-1999 |
| EP 0567251 | A1 | 27-10-1993 | AT | 144898 | T | 15-11-1996 |
| | | | DE | 69305776 | D1 | 12-12-1996 |
| | | | DE | 69305776 | T2 | 15-05-1997 |
| | | | EP | 0567251 | A1 | 27-10-1993 |
| | | | JP | H069397 | A | 18-01-1994 |
| | | | US | 5276043 | A | 04-01-1994 |
| WO 2010030887 | A1 | 18-03-2010 | CA | 2736871 | A1 | 18-03-2010 |
| | | | US | 2012322832 | A1 | 20-12-2012 |
| | | | US | 2014364463 | A1 | 11-12-2014 |
| | | | US | 2017119752 | A1 | 04-05-2017 |
| | | | WO | 2010030887 | A1 | 18-03-2010 |
| WO 02076434 | A2 | 03-10-2002 | AT | 378051 | T | 15-11-2007 |
| | | | AU | 2002249023 | B2 | 02-02-2006 |
| | | | BR | 0208305 | A | 09-03-2004 |
| | | | CA | 2341952 | A1 | 23-09-2002 |
| | | | CN | 1498108 | A | 19-05-2004 |
| | | | DE | 60223508 | T2 | 25-09-2008 |
| | | | DK | 1370264 | T3 | 17-03-2008 |
| | | | EP | 1370264 | A2 | 17-12-2003 |
| | | | ES | 2295331 | T3 | 16-04-2008 |
| | | | JP | 4377586 | B2 | 02-12-2009 |
| | | | JP | 2004523588 | A | 05-08-2004 |
| | | | MX | PA03008423 | A | 19-03-2004 |
| | | | NO | 331674 | B1 | 20-02-2012 |
| | | | NZ | 528042 | A | 28-10-2005 |
| | | | PL | 365224 | A1 | 27-12-2004 |
| | | | RU | 2299731 | C2 | 27-05-2007 |
| | | | US | 2004132737 | A1 | 08-07-2004 |
| | | | US | 2010227871 | A1 | 09-09-2010 |
| | | | US | 2011245270 | A1 | 06-10-2011 |
| | | | WO | 02076434 | A2 | 03-10-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61439483 A
- US 61439473 A
- US 61384447 A
- US 61383811 A
- US 72934610 A
- US 3901248 A
- US 5119835 A
- US 5065775 A
- US 20100154810 A1
- US 5942244 A
- US 20060115903 A
- US 20040175754 A

### Non-patent literature cited in the description


- **DEO et al.** Regioselective alkylation of N-(diphenylmethylidine)-3-(aminomethyl) pyridine: A simple route to minor tobacco alkaloids and related compounds. *Tetrahedron Letters,* 19 February 1996, vol. 37 (8), 1137-1140
- **P. H. STAHL et al.** Handbook of Pharmaceutical Salts: Properties, Selection and Use. Wiley-VCH/VHCA, 2002
- A General Procedure for the Enantioselective Synthesis of the Minor Tobacco Alkaloids Nornicotine, Anabasine, and Anatabine. *The AAPS Journal,* 2005, vol. 7 (3
- **BARON et al.** *N. Engl. J. Med.,* 2003, vol. 348, 891-99
- **VISSER et al.** *JAMA,* 1999, vol. 282 (22), 2131-5
- **ROYTBLAT et al.** *Obes Res.,* 2000, vol. 8 (9), 673-5
- **STRACZKOWSKI et al.** *J Clin Endocrinol Metab.,* 2002, vol. 87 (10), 4602-6
- **HOTAMISLIGIL et al.** *Science,* 1996, vol. 271 (5249), 665-8
- **SARTIPY P ; LOSKUTOFF D.** *J. Proc Natl Acad Sci USA.,* 2003, vol. 100 (12), 7265-70
- **HOTAMISLIGIL et al.** *J Clin Invest.,* 1995, vol. 95 (5), 2409-15
- **REUTER et al.** *Headache,* 2006, vol. 43, 426-427
- **TEGEDER et al.** *Journal of Neuroscience,* 2004, vol. 24, 1637-1645
- **NIEDERBERGER et al.** *Neurosci.,* 2004, vol. 24, 1637-45
- **NIEDERBERGER et al.** *FASEB Journal,* 2008, vol. 22, 3432-3442
- **HETTNE et al.** *Journal of Biomedical Discovery and Collaboration,* 2007, vol. 2, 2
- **PURCELL ; MOLKENTIN.** *Circulation,* 2003, vol. 108, 638-640
- **FREUND et al.** *Circulation,* 2005, vol. 111, 2319-2325
- **SEN ; ROY.** *American Journal of Physiology: Heart and Circulatory Physiology,* 2005, vol. 289, H17-H19
- **BAGHDIGUIAN et al.** *Nature Medicine,* 1999, vol. 5, 503-511
- *Nature Medicine,* July 1999, vol. 5 (7), 849
- **HASSELGREN.** *Journal of Cellular Physiology,* 2007, vol. 293, R1545-R1551
- **HOLMES-MCNARY.** *Current Opinion in Clinical Nutrition and Metabolic Care,* 2002, vol. 5, 255-263
- **HO ; BRAY.** *Proceedings of the Society of Experimental Biology and Medicine,* 1999, vol. 222, 205-213
- **ELDOR et al.** *Proceedings of the National Academy of Sciences USA,* 2006, vol. 103, 5072-5077
- **YUAN et al.** *Science,* 2001, vol. 293, 1673-1677
- **LEHRKE et al.** *PLoS Medicine,* 2004, vol. 1, e45
- **CHEN F.** *Biochemical and Biophysical Research Communications,* 2005, vol. 332, 1-3
- **GIL et al.** *British Journal of Nutition,* 2007, vol. 98, S121-S126
- **MAMMON et al.** *Review of Diabetic Studies,* 2005, vol. 2, 27-34
- **WILSON et al.** *Atherosclerosis,* 2000, vol. 148, 23-30
- **BRAND et al.** *J Clin Invest,* 1996, vol. 97, 1715-1722
- **ROSS et al.** *American Journal of Clinical Pathology,* 2001, vol. 116, S97-S107
- **LI ; GAO.** *Medical Hypotheses,* 2005, vol. 64, 694-698
- **VALEN et al.** *Journal of the American College of Cardiology,* 2001, vol. 38, 307-314

- **FRANTZ et al.** *Cardiovascular Research,* 2003, vol. 57, 749-756
- **GONG et al.** *International Journal of Clinical Practice,* 2007, vol. 61, 611-621
- **TOLEDO-PEREYRA et al.** *Experimental and Clincal Transplantation,* 2004, vol. 2, 174-177
- **NICHOLS.** *Drug News and Perspectives,* 2004, vol. 17, 99-104
- **RIDDER ; SCHWANINGER.** *Neuroscience,* 2008, vol. 158 (3), 995-1006
- **HERRMANN et al.** *Nature Medicine,* 2005, vol. 11, 13322-1329
- **AOKI et al.** *Circulation,* 2007, vol. 116, 2830-2840
- **AOKI et al.** *Arteriosclerosis, Thrombosis, and Vascular Biology,* 2009, vol. 29, 1080-1086
- **RITCHIE.** *Circulation,* 1998, vol. 98, 1707-1713
- **CHRISTMAN et al.** *Chest,* 2000, vol. 117, 1482-1487
- **BODAS ; VIJ.** *Discovery Medicine,* April 2010, vol. 9 (47), 346-356
- **POLLARD et al.** *Proteomics,* 2005, vol. 5, 2210-2216
- **CARRABINO et al.** *Journal of Cystic Fibrosis,* 2006, vol. 5
- **ROTTNER et al.** *FASEB Journal,* 2007, vol. 21, 2939-2948
- **MADJDPOUR et al.** *Anesthesiology,* 2003, vol. 99, 1323-1332
- **SAWADA et al.** *Chest,* 2007, vol. 132, 1265-1274
- **BARNES.** *Nature Reviews Drug Discovery,* 2002, vol. 1, 437-446
- **RAHMAN ; KILTY.** *Current Drug Targets,* 2006, vol. 7, 707-720
- **SCHWARTZ et al.** *Crit Care Med,* 1996, vol. 24, 1285-1292
- **CHEAH et al.** *Pediatric Research,* 2005, vol. 57, 616-623
- **GUIJARRO ; EGIDO.** *Kidney International,* 2001, vol. 59, 415-424
- **CAMICI.** *Medical Hypotheses,* 2007, vol. 68, 900-905
- **GUZIK ; HARRISON.** *Circulation Research,* 2007, vol. 101
- **ZHENG et al.** *Virchows Archiv,* 2005, vol. 448, 172-183
- **ZIMA ; KALOUSOVA.** *Alcoholism, Clinical and Experimental Research,* 2005, vol. 29, 110S-115S
- **YANG et al.** *Nephrology Dialysis Transplantation,* 2001, vol. 16 (5), 73-77
- **NEURATH et al.** *Gut,* 1998, vol. 43, 856-860
- **GONZALEZ-RAMOS et al.** *Molecular Human Reproduction,* 2007, vol. 13 (7), 503-509
- **BELL et al.** *Cell Signaling,* 2003, vol. 15, 1-7
- **XU et al.** *Neurochemistry Research,* 2006, vol. 31, 1263-1269
- **PUEL et al.** *Journal of Endotoxin Research,* 2005, vol. 11, 220-224
- **TODARO et al.** *Arthritis and Rheumatism,* 2005, vol. 52, 2179-2191
- **COURTOIS ; ISRAEL.** *Sci STKE,* November 2000, vol. 14 (58), PE1
- **ZEA et al.** *Journal of Infectious Diseases,* 2006, vol. 194, 1385-1393
- **BARNES.** *Am J Respir Crit Care Med,* 1996, vol. 154, S21-S27
- **STACEY et al.** *Biochem Biophys Res Commun,* 1997, vol. 236, 522-526
- **PAHL ; SZELENYI.** *Inflammation Research,* 2002, vol. 51, 273-282
- **ROSHAK et al.** *Current Opinion in Pharmacology,* 2002, vol. 2, 316-321
- **ROMAN-BLAS ; JIMENEZ.** *Osteoarthritis and Cartilage,* 2006, vol. 14, 839-848
- **AUD ; PENG.** *Nature Clinical Practice: Rheumatology,* 2006, vol. 2, 434-442
- **OKAMOTO.** *Endocrine, Metabolic & Immune Disorders: Drug Targets,* 2006, vol. 6, 359-372
- **HANDEL et al.** *Arthritis Rheum,* 1995, vol. 38, 1762-1770
- **SAKURADA et al.** *Int Immunol,* 1996, vol. 8, 1483-1493
- **ROSHAK et al.** *J Biol Chem,* 1996, vol. 271, 31496-31501
- **PENA ; PENATE.** *Revista Espanola d Enfermedades Digestivas,* 2002, vol. 94, 351-360
- **CHEN et al.** *World Journal of Gastroenterology,* 2005, vol. 11, 1508-1514
- **BARNES ; KARIN.** *NEJM,* 1997, vol. 336, 1066-71
- **BIELORY et al.** *Current Opinion in Allergy and Clinical Immunology,* 2002, vol. 2, 435-445
- **ZHOU et al.** *Journal of Biological Chemistry,* 2005, vol. 280, 31240-31248
- **PENNINGTON et al.** *American Surgery,* 2000, vol. 66, 914-918
- **LIN et al.** *Journal of Dermatological Science,* 2007, vol. 45, 187-192
- **WEBER ; ADLER.** *Pancreatology,* 2001, vol. 1, 356-362
- **GRAY et al.** *Pancreas,* 2006, vol. 33, 260-267
- **NICHOLS et al.** *Annals of Periodontology,* 2001, vol. 6, 20-29
- **AMBILI et al.** *Journal of Periodontology,* 2005, vol. 76, 1148-1153
- **GUO.** *Gynecology and Obstetrics Investigations,* 2006, vol. 63, 71-97
- **CELIK et al.** *Human Reproduction,* 2008, vol. 23, 2458-2465
- **NEURATH et al.** *Nature Med,* 1996, vol. 2, 998-1004

- **DIJKSTRA et al.** *Scandinavian Journal of Gastroenterology Supplement,* 2002, vol. 236, 37-41
- **ATREYA et al.** *Journal of Internal Medicine,* 2008, vol. 263, 591-596
- **PARK ; CHRISTMAN.** *Current Drug Targets,* 2006, vol. 7, 661-668
- **WRATTEN et al.** *Contributions to Nephrology,* 2001, vol. 132, 400-414
- **MUKAIDA et al.** *J Leukoc Res,* 1996, vol. 59, 145-151
- **BOHRER et al.** *J Clin Invest,* 1997, vol. 100, 972-985
- **CHEN ; SHI.** *Molecular and Cellular Biochemistry,* 2002, vol. 234-235, 169-176
- **LAVIE.** *Sleep Medicine Review,* 2003, vol. 7, 35-51
- **HISCOTT et al.** *Journal of Clinical Investigation,* 2001, vol. 107, 143-151
- **LEE ; BURCKART.** *J. Clin. Pharmacol.,* 1998, vol. 38, 981-93
- **BACH et al.** *Nature Med,* 1997, vol. 13, 944-48
- **BACH et al.** *Transplant Proc,* 1997, vol. 29, 56-58
- **HAYASHI ; FAUSTMAN.** *Diabetes Technology & Therapeutics,* 2002, vol. 2, 415-428
- **LOPEZ-PEDRERA et al.** *Arthritis and Rheumatism,* 2005, vol. 54, 301-311
- **KAMMER ; TSOKOS.** *Current Directions in Autoimmunity,* 2002, vol. 5, 131-150
- **ZHENG et al.** *Human Pathology,* 2006, vol. 37, 637-647
- **MAES et al.** *Neuro Endocinol Letters,* 2007, vol. 28, 456-462
- **ONEN.** *Rheumatology International,* 2006, vol. 26, 489-496
- **JERU et al.** *Proceedings of the National Academy of Sciences USA,* 2008, vol. 105, 1614-1619
- **BIERHAUS et al.** *Pediatic Nephrology,* 2004, vol. 19, 1189-1191
- **CECHETTO.** *Progress in Brain Research,* 2001, vol. 132, 391-404
- **MAZZEO et al.** *Archives of Neurology,* 2004, vol. 61, 1097-1102
- **HANG et al.** *World Journal of Gastroenterology,* 2005, vol. 11, 1149-1154
- **BRAMBILLA et al.** *Journal of Experimental Medicine,* 2005, vol. 202, 145-156
- **SOOS et al.** *Neuroreport,* 2004, vol. 15, 1715-1718
- **SATOH et al.** *Neuroscience Letters,* 2007, vol. 422, 30-33
- **OKAMOTO T.** *Endocrine, Metabolic & Immune Disorders: Drug Targets,* 2006, vol. 6, 359-372
- **MATTSON ; CAMANDOLA.** *Journal of Clinical Investigation,* 2001, vol. 107, 247-254
- **KALTSCHMIDT et al.** *Proc. Natl. Acad. Sci. USA,* 1997, vol. 94, 2642-2647
- **LUKIW.** *J. Biol. Chem.,* 14 November 2008, vol. 283 (46), 31315-22
- **KHOSHNAN et al.** *Journal of Neuroscience,* 2004, vol. 24, 7999-8008
- **KITAOKA et al.** *Brain Research Molecular Brain Research,* 2004, vol. 131, 8-16
- **YANG et al.** *Chinese Journal of Traumatology,* 2006, vol. 9, 86-90
- **MERCHANT et al.** *Otology and Neurotology,* 2005, vol. 26, 86-90
- **GILMORE et al.** *Cancer Letters,* 2002, vol. 181, 1-9
- **PACIFICO ; LEONARDI.** *Biochemical Pharmacology,* 2006, vol. 72, 1142-1152
- **AKSHATRI et al.** *Molecular and Cellular Biology,* 1997, vol. 17, 3629-3639
- **NAIR et al.** *Oncogene,* 2003, vol. 22, 50-58
- **LIN et al.** *Clin. Cancer Res.,* 2007, vol. 13, 3423-30
- **SAMANTA et al.** *J. Biol. Chem.,* 2004, vol. 279, 7576-83
- **DEJARDIN et al.** *Oncogene,* 1999, vol. 18, 2567-2577
- **SEPPANEN ; VIHKO.** *Immunology Letters,* 2000, vol. 74, 103-109
- **HUANG et al.** *Oncogene,* 2001, vol. 20, 4188-4197
- **OYA et al.** *Oncogene,* 2001, vol. 20, 3888-3896
- **OYA et al.** *Carcinogenesis,* 2003, vol. 24, 377-384
- **HORIGUCHI et al.** *Expert Reviews in Anticancer Therapy,* 2003, vol. 3, 793-798
- **TICHELAAR et al.** *Chest,* 2004, vol. 125, 153S
- **BERTINO et al.** *International Journal of Cancer,* 2007, vol. 121, 2766-2774
- **ZHANG et al.** *Annals of Thoracic Surgery,* 2006, vol. 82, 243-248
- **TAI et al.** *Cancer,* 2000, vol. 89, 2274-2281
- **WANG et al.** *Clinical Cancer Research,* 1999, vol. 5, 119-127
- **ZHANG ; RIGAS.** *International Journal of Oncology,* 2006, vol. 29, 185-192
- **SUTTER et al.** *Onkologie,* 2004, vol. 27, 17-21
- **JACKSON ; EVERS.** *Cancer Treatment and Research,* 2006, vol. 130, 39-65
- **ZHU et al.** *Lin Chuang Er Bi Yan Hou Ke Za Zhi,* 2004, vol. 18, 745-6, 766
- **SASAKI et al.** *Clinical Cancer Research,* 2001, vol. 7, 4136-4142
- **LIND et al.** *Surgery,* 2001, vol. 130, 363-369
- **VISCONTI et al.** *Oncogene,* 1997, vol. 15, 1987-1994
- **CORBETTA et al.** *Endocrine Related Cancer,* 2005, vol. 12, 929-937
- **YANG ; RICHMOND.** *Cancer Research,* 2001, vol. 61, 4901-4909

- **AMIRI KI ; RICHMOND A.** *Cancer Metastasis Reviews,* 2005, vol. 24, 301-313
- **LOERCHER et al.** *Cancer Research,* 2004, vol. 64, 6511-6523
- **ONDREY et al.** *Molecular Carcinogenesis,* 1999, vol. 26, 119-129
- **JACKSON-BERNITSAS et al.** *Oncogene,* 2007, vol. 26, 1385-1397
- **PALLARES et al.** *Journal of Pathology,* 2004, vol. 204, 595-577
- **KOVALENKO et al.** *Nature,* 2003, vol. 424, 801-805
- **ALMEIDA et al.** *Dermatol.,* 2008, vol. 127, 587-93
- **CHEN et al.** *World Journal of Gastroenterology,* 2005, vol. 11, 726-728
- **NAKAYAMA et al.** *Cancer,* 2001, vol. 92, 3037-3044
- **RUAN et al.** *Phytotherapy Research,* 2007, vol. 22, 407-15
- **HAYASHI et al.** *Neurologia Medico-Chirufica,* 2001, vol. 41, 187-195
- **SMITH et al.** *Molecular and Cellular Biochemistry,* 2007, vol. 307, 1-2, 141-147
- **BIAN et al.** *Journal of Biological Chemistry,* 2002, vol. 277, 42144-42150
- **RAYCHAUDHURI et al.** *Journal of Neurooncology,* 2007, vol. 85, 39-47
- **BARGOU et al.** *Blood,* 1996, vol. 87, 4340-4347
- **BARGOU et al.** *Journal of Clinical Investigation,* 1997, vol. 100, 2961-2969
- **KORDES et al.** *Leukemia,* 2000, vol. 14, 399-402
- **GUZMAN et al.** *Blood,* 2001, vol. 98, 2301-2307
- **ARIMA ; TEI.** *Leukemia and Lymphoma,* 2001, vol. 40, 267-278
- **LEI ; ZHAO.** *Zhongguo Shi Yan Xue Za Zhi,* 2007, vol. 15, 253-257
- **FURMAN et al.** *Journal of Immunology,* 2000, vol. 164, 2200-2206
- **KNECHT et al.** *Oncology,* 2001, vol. 60, 289-302
- **MARTINEZ.** *Cancer Research,* 2003, vol. 63, 8226-8232
- **FABRE et al.** *Oncogene,* 2007, vol. 26, 4071-4083
- **GILMORE.** *Cancer Cell,* 2007, vol. 12, 95-97
- **BERENSON et al.** *Seminars in Oncology,* 2001, vol. 28, 626-633
- **DAVIS et al.** *Journal of Experimental Medicine,* 2001, vol. 194, 1861-1874
- **SAGAERT et al.** *Leukemia,* 2007, vol. 21, 389-396
- **LELEU et al.** *Blood,* 2008, vol. 111, 5068-5077
- **RAY ; COHN.** *J. Clin. Invest.,* 1999, vol. 104, 985-993